(19)

**Europäisches Patentamt**
**European Patent Office**
**Office européen des brevets**

(11)  **EP 2 925 395 B1**

(12)  **EUROPEAN PATENT SPECIFICATION**

(45) Date of publication and mention
of the grant of the patent:
**06.03.2019  Bulletin 2019/10**

(51) Int Cl.:
*A61M 11/04* (2006.01)      *A61M 15/06* (2006.01)
*A24F 47/00* (2006.01)

(21) Application number: **13858541.9**

(22) Date of filing: **27.11.2013**

(86) International application number:
**PCT/US2013/072426**

(87) International publication number:
**WO 2014/085719 (05.06.2014 Gazette 2014/23)**

(54) **DEVICE FOR GENERATING A CONDENSATION AEROSOL FROM A LIQUID FORMULATION**

VORRICHTUNG ZUR ERZEUGUNG EINES KONDENSATIONSAEROSOLS AUS EINER FLÜSSIGEN FORMULIERUNG

DISPOSITIF DE GÉNÉRATION D'UN AÉROSOL À CONDENSATION À PARTIR D'UNE FORMULATION LIQUIDE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **28.11.2012  US 201261730738 P**
**15.03.2013  US 201361794601 P**
**06.06.2013  US 201361831992 P**
**04.10.2013  US 201361887045 P**

(43) Date of publication of application:
**07.10.2015  Bulletin 2015/41**

(73) Proprietor: **Fontem Holdings 1 B.V.**
**1083 HN  Amsterdam (NL)**

(72) Inventors:
• **WENSLEY, Martin**
**Los Gatos, CA 95032 (US)**
• **HUFFORD, Michael**
**Chapel Hill, NC 27516 (US)**
• **WILLIAMS, Jeffrey**
**Draper, UT 84020 (US)**
• **LLOYD, Peter**
**Walnut Creek, CA 94597 (US)**

(74) Representative: **Lorenz, Sönke**
**Gulde & Partner**
**Patent- und Rechtsanwaltskanzlei mbB**
**Wallstraße 58/59**
**10179 Berlin (DE)**

(56) References cited:
**EP-A1- 0 845 220        EP-A1- 1 618 803**
**EP-A1- 2 218 760        EP-A1- 2 319 334**
**EP-A1- 2 489 391        WO-A1-95/01137**
**WO-A1-2012/039720        US-A- 5 666 977**
**US-A1- 2003 051 728        US-A1- 2005 235 991**
**US-B2- 7 117 867        US-B2- 8 156 944**

**Description**

**BACKGROUND**

There is a need for new methods and devices for administering compounds, such as pharmaceutical agents, to a subject. In particular, there is a need for methods and devices for delivery of compounds to a subject where the compounds are aerosolized to fall within a specified particle size range. In some cases, particles within a specified size range can be efficiently delivered to the deep lung. For example, there is an urgent need for improved methods and devices to deliver nicotine to a subject in specified doses and in a specified particle range size without the carcinogens and other chemicals associated with combustible tobacco products.

In 2011, an estimated 19% of U.S. adults were current smokers (43.8 million people), and an estimated 950 children become addicted to smoking daily. Smokers spend approximately $83 billion to support their habit, and half of smokers will die from their habit. Studies indicate that about 85% of smokers want to quit; however, only about 5% succeed.

Current nicotine replacement therapies (NRTs) are not effective for approximately 85% of users. In some cases, existing NRTs and electronic cigarettes (eCigs) fail to provide sufficient doses of nicotine. Many smokers using NRTs under-dose, resulting in break-through cravings, which can lead to smoking lapses and eventual relapse. Smokers also vary widely in terms of their daily nicotine intake, ranging from "social smokers" who may only consume 1 or 2 cigarettes in the presence of friends and/or with alcohol, to heavy smokers who consume 60 or more cigarettes per day. Thus, a need exists to provide effective, customized doses of nicotine to individuals attempting to use recreational nicotine products or to leverage these devices to help quit smoking or nicotine intake all together.

Furthermore, to facilitate nicotine delivery and smoking cessation using an electronic nicotine delivery device, a need exists to control nicotine particle size generated from an electronic nicotine delivery device to match the rapid nicotine pharmacokinetics (PK) from smoking, which can result in deep lung absorption of nicotine. Deep lung absorption of nicotine can facilitate rapid delivery of nicotine to the brain, which can result in a subsequent cessation of nicotine cravings. When smoking combustible tobacco products, nicotine laden smoke particles are carried proximally on tar droplets (0.1-1.0 $\mu$M in diameter), are inhaled and travel to the small airways and alveoli in the deep lung. Nicotine off-gasses from particles and defuses to, and deposits on, the alveoli wall where it can be rapidly absorbed into the blood stream. A typical electronic cigarette does not produce an aerosol of nicotine with a particle size for deep lung delivery. Aerosol particles with an aerodynamic diameter larger than 5 $\mu$m can be too large to reach the deep lung because the particles can impact in the mouth and upper airway, resulting in a slow PK. Conversely, aerosol particles with a median aerodynamic diameter of less than 1 $\mu$m can be small enough to reach the deep lung but can be too light to gravitationally settle and can be exhaled, which can result in low dose delivery. Additionally, aerosols with small aerosol particle size can contain a larger percentage of the mass in the gas phase, which rapidly diffuses to the mouth and upper airway. Aerosol particles with an aerodynamic diameter of about 1 $\mu$m to about 5 $\mu$m can be small enough to reach the deep lung but large enough to gravitationally settle in alveoli, which can result in a rapid PK. A need exists for electronic nicotine delivery devices that produce such particles. In addition, a need exists for producing nicotine aerosols that produce such particles using the liquid drug. Moreover, a need exists for methods of using such devices to help users achieve a particular health goal or goals.

Also, a need exists for considering behavioral factors in smoking relapse and for integrating smoking cessation programs with social media to facilitate and maintain behavior change.

There is also a need for a drug delivery platform that is capable of dispensing a variety of drugs to a subject in a specified dose or in a specified particle size range.

WO 95/01137 discloses a dispenser which comprises a reservoir of a physiologically active substance and a droplet ejection device, for example a bubble jet or piezoelectric device, which is controlled to issue a predetermined number of discrete droplets of the substance from ejection orifices upon actuation.

In EP 2 319 334 A1, it is disclosed a smoking system comprising a capillary wick for holding liquid, at least one air inlet, at least one air outlet and a chamber between the air inlet and air outlet. The air inlet, the air outlet and the chamber are arranged so as to define an air flow route from the air inlet to the air outlet via the capillary wick so as to convey aerosol formed from the liquid to the air outlet.

EP 1 618 803 A1 relates to a non-smokable electronic spray cigarette which only comprises nicotine without harmful tar. The cigarette includes a smoke mouth integer comprised with a shell, a cell, a high frequency ionzer, nicotine solution storage and its container, control circuit, a display screen, a human contact sensor, a piezoelectric supersound atomizer, a high temperature vaporization nozzle and attachments.

US 2005/0235991 A1 describes an aerosol generator that includes a flow passage having an inlet end, an outlet end, and a constriction in the flow passage at the outlet end. A heater is operable to heat liquid in the flow passage to produce a vapor, which is expelled from the outlet end of the flow passage.

**EP 2 925 395 B1**

## SUMMARY

The present invention provides a device for generating a condensation aerosol from a liquid formulation as defined in claim 1. Further preferred embodiments of the invention are defined in the dependent claims.

The following disclosure refers to different aspects or embodiments that do not necessarily fall under the scope of the claims, but which may be useful for understanding the present invention, the scope of which being only defined by the appended claims.

In one aspect, a condensation aerosol generating device for generating a condensation aerosol from a liquid formulation is provided, the device comprising: a) a reservoir comprising a liquid formulation; and b) a passageway comprising a heater element, an inlet, and an outlet, wherein the heater element is disposed in the passageway between the inlet and the outlet, wherein the reservoir is in fluid communication with the heater element, and wherein the passageway is configured to produce a condensation aerosol in the device, wherein the condensation aerosol has a mass median aerodynamic diameter (MMAD) of from about 1 $\mu$m to about 5 $\mu$m. In some cases, the heater element comprises a wick element. In some cases, the heater element comprises a coil. In some cases, the heater element comprises a wick element and a coil. In some cases, the wick element comprises an electrically resistive material. In some cases, the wick element and coil are formed from the same rod. In some cases, the rod comprises a pliable material. In some cases, the coil is wrapped around the wick element. In some cases, the wick element is capable of being heated. In some cases, the coil spans a length of from about 0.25 cm to about 0.39 cm (from about 0.1 inch to about 0.15 inches) along the length of the wick element. In some cases, the heater element comprises a wire coil.

The device further comprises a positive displacement pump in fluid communication with the reservoir. In some cases, the displacement pump is configured to pump the liquid formulation comprising the liquid formulation to the heater element. The positive displacement pump comprises a peristaltic pump.

In some cases, the passageway has an internal diameter of from 0.20 cm to about 1.3 cm (about 0.08 inches to about 0.5 inches). In some cases, an internal diameter of the passageway where the heater element is located is from about 0.44 cm to about 0.64 cm (about 0.175 to about 0.25 inches). In some cases, an internal diameter of the passageway before and after the heater element is from about 0.07 cm to about 1.3 cm (about 0.03 to about 0.5 inches). In some cases, the length of the passageway is from about 0.5 cm to about 15.3 cm (from about 0.2 inches to about 6 inches). The device further comprises a baffle disposed in the passageway between the outlet and the heater element. The baffle inhibits flow of particles having an MMAD of greater than 5 $\mu$m.

In some cases, the device further comprises a second passageway connected to the passageway. In some cases, the second passageway connects with the passageway between the outlet and the heater element. In some cases, the second passageway connects with the passageway between the air inlet and the heater element. In some cases, the device further comprises a power source in electric communication with the heater element. In some cases, the power source is a battery.

In some cases, the device further comprises a tube disposed within the passageway and connected to the reservoir, wherein the tube is configured to deliver the liquid formulation to the heater element. In some cases, the tube is a capillary. In some cases, the tube comprises a valve. In some cases, the tube comprises a second heater element. In some cases, the tube comprises a heatable region. In some cases, the device further comprises a valve located in the tube between the heatable region and the reservoir. In some cases, the tube comprises an electrically resistive material.

In some cases, the device further comprises a flow restrictor disposed in the passageway. In some cases, a flow rate of a gas through the device is capable of delivering the condensation aerosol of the liquid formulation to a deep lung of a user of the device. In some cases, a flow rate of gas through the device that is capable of delivering the condensation aerosol of the liquid formulation to a deep lung of a user of the device is from about 20 liters per minute (LPM) to about 80 LPM. In some cases, the device permits a flow resistance of from about 0.05 to about 0.15 sqrt (cm-H$_2$O)/LPM. In some cases, the device permits a flow resistance of from about 0.08 to about 0.12 sqrt (cm-H$_2$O)/LPM. In some cases, the condensation aerosol is formed at a flow rate of about 1 LPM to about 10 LPM past the heater element under a vacuum of between about 249 Pa to about 3738 Pa (about 1 inch of water to about 15 inches of water).

In some cases, the device further comprises a mouthpiece attached to the outlet. In some cases, the liquid formulation comprises a flavoring. In some cases, the liquid formulation comprises a pharmaceutically active agent. In some cases, the liquid formulation comprises nicotine and a carrier. In some cases, the carrier comprises propylene glycol or vegetable glycerin. In some cases, the device further comprises a programmable controller. In some cases, the programmable controller comprises a non-transitory computer readable medium, wherein the non-transitory computer readable medium comprises one or more algorithms for regulating dosage of the condensation aerosol of the liquid formulation, frequency of administration of the condensation aerosol of the liquid formulation, or a delivery schedule of the condensation aerosol of the liquid formulation. In some cases, the programmable controller is connected to one or more electronic devices. In some cases, the one or more electronic devices comprise a computer, a smartphone, or a mobile phone.

In some cases, the reservoir is within the passageway. In some cases, the device further comprises a sensor to detect inhalation. In some cases, the senor comprises an optical path. In some cases, the device further comprises a second heater element. In some cases, the heater element is a aerosol generating heater element. In some cases, the heater element is capable of vaporizing the liquid formulation when the liquid formulation is delivered to the heater element, wherein the vaporized liquid formulation is condensed into aerosol particles in the passageway. In some cases, the device further comprises an air flow regulator. In some cases, the device further comprises a second passageway, wherein the air flow regulator is capable of directing air through the second passageway in response to a pressure drop across the air flow regulator. In some cases, the air flow regulator comprises one or more gas control valves.

In some cases, the device further comprises a controller, wherein the controller comprises a non-transitory computer readable medium comprising one or more algorithms, wherein the one or more algorithms regulate dosing or a delivery schedule of the condensation aerosol of the liquid formulation. In some cases, the device further comprises an interface for communicating with the controller. In some cases, the controller is removably attached to the passageway and the reservoir. In some cases, the controller is capable of communicating with one or more electronic devices.

In another aspect, a condensation aerosol generating device for generating a condensation aerosol comprising a pharmaceutically active agent is provided, the device comprising: a) a housing comprising a heater element for vaporizing a liquid formulation comprising the pharmaceutically active agent, wherein the heater element is located between an outlet and an air inlet in the housing; b) a reservoir comprising the liquid formulation, wherein the reservoir is in fluid communication with the heater element; c) a passageway for condensing a vaporized liquid formulation comprising the pharmaceutically active agent, wherein the passageway is in fluid communication with the heater element; and d) a programmable controller. In some cases, the programmable controller comprises a non-transitory computer readable medium. In some cases, the non-transitory computer readable medium comprises one or more algorithms for regulating dosage of the pharmaceutically active agent, frequency of administration of the pharmaceutically active agent, or a delivery schedule of the pharmaceutically active agent. In some cases, the one or more algorithms regulate dosage of the pharmaceutically active agent, frequency of administration of the pharmaceutically active agent, or delivery schedule of the pharmaceutically active agent based on data provided to the device. In some cases, the device further comprises an interface for communicating with the programmable controller. In some cases, the programmable controller comprises a non-transitory computer readable medium, wherein the non-transitory computer readable medium comprises sequences of instructions, which, when executed by the device, cause the device to a) record data on use of the device; b) generate date on use of the device; c) retrieve data on use of the device; d) communicate data on use of the device; or e) input data regarding use of the device into one or more algorithms. In some cases, the programmable controller is connected to one or more electronic devices. In some cases, the connection is local or remote. In some cases, the connection comprises a wired connection. In some cases, the connection comprises a wireless connection. In some cases, the one or more electronic devices comprise a computer, smartphone, or a mobile phone. In some cases, the device further comprises the ability to access the Internet.

In some cases, the heater element comprises a wick element and a coil. In some cases, the wick element and the coil are formed from the same rod. In some cases, the coil is wrapped around the wick element. In some cases, the device further comprises a positive displacement pump in fluid communication with the reservoir. In some cases, the device further comprises a baffle disposed in the passageway between the outlet and the heater element. In some cases, the device further comprises a second passageway, wherein the second passageway connects with the passageway between the outlet and the heater element. In some cases, the device further comprises a second passageway, wherein the second passageway connects with the passageway between the inlet and the heater element.

In some cases, the device further comprises a tube disposed within the passageway and connected to the reservoir, wherein the tube is configured to deliver the liquid formulation comprising the pharmaceutically active agent to the heater element. In some cases, the device further comprises a mouthpiece attached to the outlet. In some cases, the liquid formulation comprises a flavoring. In some cases, the pharmaceutically active agent is nicotine. In some cases, the liquid formulation comprises nicotine and a carrier. In some cases, the device produces condensation aerosol particles having a mass median aerodynamic diameter (MMAD) of from about 1 $\mu$m to about 5 $\mu$m. In some cases, a flow rate of gas in the device is capable of delivering the condensation aerosol of the liquid formulation comprising the pharmaceutically active agent to a deep lung of a user, wherein the flow rate is from about 20 LPM to about 80 LPM at a vacuum of about 249 Pa to about 3738 Pa (about 1 inch of water to about 15 inches of water). In some cases, the device permits a flow resistance of from about 0.05 to about 0.15 sqrt (cm-H$_2$O)/LPM.

In another aspect, a method for generating a condensation aerosol of a liquid formulation is provided, the method comprising: a) generating a condensation aerosol, wherein the generating comprises vaporizing a liquid formulation using a heater element, wherein the heater element is disposed in a passageway of a condensation aerosol generating device, wherein the condensation aerosol has an MMAD of from about 1 $\mu$m to about 5 $\mu$m; and b) flowing a gas through the condensation aerosol generating device at a flow rate effective to allow delivery of the condensation aerosol. In some cases, the delivery is to the deep lung of a subject using the device. In some cases, the subject is a human. In some cases, the flowing comprises flowing a gas at a rate of about 20 LPM to about 80 LPM. In some cases, the condensation aerosol generating device permits a flow resistance of from about 0.05 to about 0.15 sqrt (cm-H$_2$O)/LPM. In some cases, the condensation aerosol is formed at a flow rate of about 1 LPM to about 10 LPM past the heater element under a vacuum of between about 1 to about 249 Pa to about 3738 Pa (about 1 to about 15 inches of water). In some cases, the subject is a human.

In some cases, the liquid formulation comprises a pharmaceutically active agent. In some cases, the pharmaceutically active agent is delivered orally to the subject. In some cases, the heater element comprises a wick element and a coil. In some cases, the wick element comprises an electrically resistive material. In some cases, the wick element and coil are formed from the same rod. In some cases, the coil is wrapped around the wick element.

In some cases, the condensation aerosol generating device comprises a programmable controller. In some cases, the programmable controller comprises a non-transitory computer readable medium. In some cases, the non-transitory computer readable medium comprises one or more algorithms for regulating dosage of a pharmaceutically active agent, frequency of administration of a pharmaceutically active agent, or a delivery schedule of a pharmaceutically active agent. In some cases, the method further comprises programming the condensation aerosol generating device to deliver a dose of the pharmaceutically active agent. In some cases, the method further comprises programming a delivery schedule for the condensation aerosol generating device. In some cases, method further comprises programming a dosing schedule for the condensation aerosol generating device. In some cases, the passageway comprises an inlet and an outlet. In some cases, the passageway is disposed between the inlet and the outlet.

In some cases, the deep lung comprises alveoli. In some cases, the liquid formulation comprises a pain medication. In some cases, the liquid formulation comprises a flavoring. In some cases, the liquid formulation comprises nicotine. In some cases, the liquid formulation comprises nicotine and a carrier. In some cases, the administering produces a nicotine blood concentration in the subject that is substantially similar to the nicotine blood concentration of a subject after smoking a cigarette. In some cases, the nicotine blood concentration is an arterial or venous nicotine blood concentration. In some cases, the administering produces a nicotine blood concentration in the subject that is at least 50% of the nicotine blood concentration of a subject after smoking a cigarette.

In some cases, the condensation aerosol generating device further comprises a baffle located in the passageway, wherein the baffle inhibits flow of particles having an MMAD of greater than 5 $\mu$m. In some cases, the condensation aerosol generating device further comprises a reservoir comprising the liquid formulation. In some cases, the reservoir is disposed an inlet and outlet. In some cases, the condensation aerosol generating device further comprises a tube located within the passageway and connected to the reservoir. In some cases, the tube is configured to deliver the liquid formulation to the heater element. In some cases, the tube is a capillary. In some cases, the tube is in fluid communication with the heater element. In some cases, the tube comprises a heatable region. In some cases, the condensation aerosol generating device further comprises a valve located in the tube between the heatable region and the reservoir. In some cases, the condensation aerosol generating device further comprises a controller, wherein the controller comprises a non-transitory computer readable medium comprising one or more algorithms, wherein the one or more algorithms regulate dosing or a delivery schedule of the condensation aerosol of the liquid formulation. In some cases, the condensation aerosol generating device further comprises an interface for communicating with the controller.

In some cases, the controller is removably attached to the passageway and the reservoir. In some cases, the controller is capable of communicating with one or more electronic devices. In some cases, the condensation aerosol generating device further comprises an air flow regulator located in the passageway. In some cases, the condensation aerosol generating device further comprises a second passageway, wherein an air flow regulator is capable of directing air through the second passageway in response to a pressure drop across the air flow regulator. In some cases, the air flow regulator comprises one or more gas control valves. In some cases, the heater element comprises a wire coil.

In another aspect, a method for treating a condition is provided, the method comprising: a) producing a condensation aerosol, wherein the producing comprises vaporizing a liquid formulation using a heater element, wherein the heater element is disposed in a passageway of a condensation aerosol generating device, wherein the condensation aerosol has an MMAD of from about 1 $\mu$m to about 5 $\mu$m; and b) flowing a gas through the condensation aerosol generating device at a flow rate effective to allow delivery of the condensation aerosol comprising the pharmaceutically active agent to the deep lung of a user of the device, thereby treating the condition. In some cases, the flowing comprises flowing a gas at a rate of about 20 LPM to about 80 LPM at a vacuum of about 249 Pa to about 3738 Pa (about 1 inch of water to about 15 inches of water). In some cases, the device permits a flow resistance of from about 0.05 to about 0.15 sqrt (cm-H$_2$O)/LPM. In some cases, the condensation aerosol is formed at a flow rate of about 1 LPM to about 10 LPM past the heater element under a vacuum of between about 249 Pa to about 3738 Pa (about 1 inch of water to about 15 inches of water).

In some cases, the condensation aerosol generating device comprises a programmable controller. In some cases, the method further comprises programming the condensation aerosol generating device to deliver a dose of the pharmaceutically active agent. In some cases, method further comprises programming a delivery schedule for the condensation aerosol generating device. In some cases, the method further comprises programming a dosing schedule for the condensation aerosol generating device.

In some cases, the subject is a human. In some cases, the deep lung comprises alveoli.

In some cases, the condition is pain. In some cases, the liquid formulation comprises a pain medication. In some cases, the pharmaceutically active agent is delivered orally to the subject. In some cases, the condition is addiction to smoking. In some cases, the condition is nicotine addiction. In some cases, the treating the condition comprises smoking cessation. In some cases, the treating the condition comprises transitioning a subject who smokes from use of cigarettes to use of the condensation aerosol generating device. In some cases, the liquid formulation comprises a flavoring. In some cases the liquid formulation comprises nicotine. In some cases, the liquid formulation comprises nicotine and a carrier. In some cases, the condition is addiction to smoking or nicotine addiction, and the delivery produces a nicotine blood concentration in the subject that is substantially similar to a nicotine blood concentration of a subject after smoking a cigarette. In some cases, the nicotine blood concentration is an arterial or venous nicotine blood concentration. In some cases, the delivery produces a nicotine blood concentration in the subject that is at least 50% of the nicotine blood concentration of a subject after smoking a cigarette.

In another aspect, a heater element is provided comprising: a) a coil comprising an electrically resistive material; and b) a wick element capable of being heated, wherein the coil is wrapped around the wick element. In some cases, the wick element comprises an electrically resistive material. In some cases, the wick element and the coil are independent. In some cases, the wick element and the coil are formed from the same rod. In some cases, the wick element and the coil are wire. In some cases, the coil comprises 1 to 9 turns. In some cases, a distance between each turn in a coil is from about 0.025 cm to about 0.51 cm (about 0.01 to about 0.02 inches). In some cases, the coil has a length to width aspect ratio of at least 2. In some cases, the coil comprises an inner diameter of from about 0.068 cm to about 0.11 cm (about 0.027 inches to about 0.040 inches). In some cases, the coil comprises an outside diameter of from about 0.11 cm to about 0.21 cm (about 0.047 inches to about 0.080 inches).

In another aspect, a method is provided comprising producing a nicotine blood concentration in a subject that is substantially similar to a nicotine blood concentration of a subject after smoking a cigarette, the method comprising delivering a condensation aerosol comprising nicotine to a subject, wherein the condensation aerosol has a MMAD of from about 1 $\mu$m to about 5 $\mu$m. In some cases, the nicotine blood concentration is an arterial or venous nicotine blood concentration. In some cases, the condensation aerosol is produced from a condensation aerosol generating device. In some cases, the condensation aerosol generating device comprises a) a passageway comprising a heater element, an inlet, and an outlet, wherein the heater element is disposed in the passageway between the inlet and the outlet; and b) a reservoir comprising a liquid formulation comprising nicotine, wherein the reservoir is in fluid communication with the heater element, wherein the heater element is capable of vaporizing the liquid formulation comprising the pharmaceutically active agent when the liquid formulation comprising nicotine is delivered to the heater element, wherein the vaporized liquid formulation comprising nicotine is condensed into aerosol particles.

In another aspect, a condensation aerosol generating device for generating a condensation aerosol of a liquid formulation is provided, the device comprising: a) a passageway comprising a heater element; and b) a reservoir comprising the liquid formulation, wherein the reservoir is in fluid communication with the heater element, wherein the heater element is capable of vaporizing the liquid formulation when the liquid formulation is delivered to the heater element, and wherein a flow of air through the device delivers the condensation aerosol of the liquid formulation to the deep lung of a subject using the device. In some cases, the device permits a flow resistance of from about 0.08 to about 0.12 sqrt (cm-H$_2$O)/LPM. In some cases, the passageway comprises an upstream opening and a downstream opening, wherein the upstream opening comprises an inlet for the air and the downstream opening serves as an outlet for the air, and wherein the heater element is located in the passageway between the upstream and downstream openings. In some cases, the reservoir is located within the passageway between the upstream and downstream openings.

In some cases, the device further comprises an air flow regulator located in the passageway. In some cases, the device further comprises a second passageway, wherein an air flow regulator is capable of directing the air through the second passageway in response to a pressure drop across the air flow regulator. In some cases, the air flow regulator comprises one or more gas control valves. In some cases, the heater element comprises a wire coil.

In some cases, the device further comprises a tube in fluid communication with the reservoir. In some cases, tube is a capillary. In some cases, the tube is in fluid communication with the heater element. In some cases, the tube comprises a heatable region.

In some cases, the device further comprises a controller, wherein the controller comprises a non-transitory computer readable medium comprising one or more algorithms wherein the one or more algorithms regulate dosing or a delivery schedule of the condensation aerosol of the liquid formulation. In some cases, the device further comprises an interface for communicating with the controller. In some cases, the controller is removably attached to the passageway and the reservoir. In some cases the controller is capable of communicating with one or more electronic devices. In some cases the liquid formulation comprises a flavoring.

In another aspect, a condensation aerosol generating device for producing a condensation aerosol is provided, the device comprising: a) a passageway comprising an aerosol generating heater element, an inlet, and an outlet, wherein the aerosol generating heater element is between the inlet and the outlet in the passageway, wherein the device is configured to produce a condensation aerosol from a liquid formulation in the device, wherein the condensation aerosol has a mass median aerodynamic diameter (MMAD) of from 1 $\mu$M to 5 $\mu$M; and b) one or more additional airflow regulating inlets for air located between the outlet and the heater element in the passageway, wherein the total airflow rate out of the outlet is between 20 LPM and 80 LPM at a vacuum of about 249 Pa to about 3738 Pa (about 1 inch of water to about 15 inches of water). In some cases, the device further comprises a reservoir comprising the liquid formulation, wherein the reservoir is in fluid communication with the aerosol generating heater element. In some cases, the device further comprises a tube located within the passageway and connected to the reservoir, wherein the tube is configured to deliver the liquid formulation to the aerosol generating heater element. In some cases, the reservoir is located within the passageway.

In some cases, the passageway further comprises a positive displacement pump configured to pump the liquid formulation comprising through the tube to the heater element. In some cases, the positive displacement pump comprises a peristaltic pump. In some cases, the passageway has an internal diameter of from about 0.20 cm to about 1.3 cm (about 0.08 inches to about 0.5 inches). In some cases, an internal diameter of the passageway where the heater element is located is from about 0.44 cm to about 0.64 cm (about 0.175 to about 0.25 inches). In some cases, an internal diameter of the passageway before and after the heater element is from about 0.07 cm to about 1.3 cm (about 0.03 to about 0.5 inches). In some cases, the length of the passageway is from about 0.5 cm to about 15.3 cm (from about 0.2 inches to about 6 inches).

In some cases, the passageway comprises a baffle located between the outlet and the heater element, wherein the baffle is configured to remove aerosol particles with an MMAD of greater than 5 $\mu$m from the aerosol. In some cases, the aerosol generating heater element comprises a wick element. In some cases, the aerosol generating heater element comprises a coil. In some cases, the aerosol generating heater element comprises a coil wrapped around a wick element, wherein the coil and the wick element are formed from the same rod. In some cases, the coil spans a length of from about 0.25 cm to about 0.39 cm (about 0.1 to about 0.15 inches) along the length of the wick element. In some cases, the wick element comprises an electrically resistive material. In some cases, the rod comprises a pliable material.

In some cases, the device further comprises a sensor to detect inhalation. In some cases, the sensor comprises an optical path. In some cases, the device further comprises a power source to power the heater element. In some cases, the power source is a battery. In some cases, the tube is a capillary tube. In some cases, the tube comprises a valve. In some cases, the tube comprises an electrically resistive material.

In some cases, the device further comprises a programmable controller. In some cases, the device is connected to one or more electronic devices. In some cases, the one or more electronic devices comprise a computer, smartphone, or mobile phone. In some cases, the device further comprises a second heater element. In some cases, the passageway is configured to produce a flow rate of from about 1 to about 10 LPM at the heater element under a vacuum of between about 249 Pa to about 3738 Pa (about 1 inch of water to about 15 inches of water).

In some cases, the liquid formulation comprises nicotine. In some cases, the liquid formulation comprises a carrier. In some cases, the carrier comprises propylene glycol or vegetable glycerin.

In another aspect, a condensation aerosol generating device for producing a condensation aerosol is provided, the device comprising: a passageway comprising an aerosol generating heater element, an inlet, and an outlet, wherein the aerosol generating heater element is between the inlet and the outlet in the passageway, wherein the passageway is configured to produce a condensation aerosol from a liquid formulation, wherein the condensation aerosol has a mass median aerodynamic diameter (MMAD) of from about 1 $\mu$m to about 5 $\mu$m, wherein the device is configured to produce an internal air resistance of from 0.05 to 0.15 sqrt (cm-$H_2O$)/LPM. In some cases, the device further comprises a reservoir comprising a liquid formulation, wherein the reservoir is in fluid communication with the heater element. In some cases, the device further comprises a tube located within the passageway and connected to the reservoir, wherein the tube is configured to deliver the liquid formulation comprising to the heater element. In some cases, the reservoir is located within the passageway. In some cases, the passageway further comprises a positive displacement pump configured to pump the liquid formulation comprising through the tube to the heater element. In some cases, the positive displacement pump comprises a peristaltic pump.

In some cases, the passageway has an internal diameter of from about 0.20 cm to about 1.3 cm (about 0.08 inches to about 0.5 inches). In some cases, an internal diameter of the passageway where the heater element is located is from about 0.44 cm to about 0.64 cm (about 0.175 to about 0.25 inches). In some cases, an internal diameter of the passageway before and after the heater element is from about 0.07 cm to about 1.3 cm (about 0.03 to about 0.5 inches). In some cases, the length of the passageway is from about 0.5 cm to about 15.3 cm (from about 0.2 inches to about 6 inches).

In some cases, the passageway comprises a baffle located between the outlet and the heater element, wherein the baffle is configured to remove aerosol particles with an MMAD of greater than 5 $\mu$m from the aerosol. In some cases, the heater element comprises a wick element. In some cases, the heater element comprises a coil. In some cases, the heater element comprises a coil wrapped around a wick element, wherein the coil and the wick element are formed from the same rod. In some cases, the coil spans a length of from about 0.25 cm to about 0.39 cm (about 0.1 to about 0.15 inches) along the length of the wick element. In some cases, the wick element comprises an electrically resistive material. In some cases, the rod comprises a pliable material.

In some cases, the device further comprises a sensor to detect inhalation. In some cases, the sensor comprises an optical path. In some cases, the device further comprises a power source to power the heater element. In some cases, the power source is a battery. In some cases, the tube is a capillary tube. In some cases, the tube comprises a valve. In some cases, the tube comprises an electrically resistive material. In some cases, the device further comprises a second passageway connected to the passageway. In some cases, the second passageway connects between the outlet and the heater element in the passageway. In some cases, the second passageway connects to the passageway between the inlet and the heater element.

In some cases, the device further comprises a programmable controller. In some cases, the device is connected to one or more electronic devices. In some cases, the one or more electronic devices comprise a computer, smartphone, or mobile phone. In some cases, the device further comprises a second heater element. In some cases, the passageway is configured to produce a flow rate of from 1 to 10 LPM past the heater element under a vacuum of between about 249 Pa to about 3738 Pa (about 1 inch of water to about 15 inches of water). In some cases, the liquid formulation comprises nicotine. In some cases, the liquid formulation comprises a carrier. In some cases, the carrier comprises propylene glycol or vegetable glycerin.

The above elements can be combined in any combination.

## BRIEF DESCRIPTION OF THE DRAWINGS

Novel features are set forth with particularity in the appended claims. A better understanding of the features and advantages will be obtained by reference to the following detailed description that sets forth illustrative embodiments, in which the principles are utilized, and the accompanying drawings of which:

FIG. 1 illustrates an embodiment of an electronic nicotine delivery device.

FIGs. 2A and 2B illustrate an embodiment of electronic agent (e.g., nicotine) delivery device.

FIGs. 3A and 3B illustrate embodiments of a heater element.

FIG. 4 illustrates an embodiment of an agent (e.g., nicotine) reservoir.

FIG. 5 illustrates another embodiment of an agent (e.g., nicotine) reservoir.

FIG. 6 illustrates another embodiment of an agent (e.g., nicotine) reservoir.

FIG. 7 illustrates an embodiment of a heater element.

FIG. 8 illustrates an embodiment of an electronic agent (e.g., nicotine) delivery device.

FIG. 9 illustrates another embodiment of a heater element.

FIGs. 10A and 10B illustrate additional embodiments of a heater element.

FIG 11 illustrates inertial impaction.

FIG. 12 illustrates an embodiment of a method of removal of an agent (e.g., nicotine) mixture from a reservoir and dispensing the nicotine into desired doses.

FIG. 13 illustrates another embodiment of a method for measuring an agent (e.g., nicotine) dose.

FIG. 14 illustrates another embodiment for measuring an agent (e.g., nicotine) dose.

FIG. 15 illustrates another embodiment for measuring an agent (e.g., nicotine) dose.

FIGs. 16A and 16B illustrate embodiments for applying an agent (e.g., nicotine) to a heater element.

FIGs. 17A and 17B illustrate embodiments of mechanisms for generating an aerosol.

FIG. 18 illustrates an embodiment of a mechanism for dispensing an agent (e.g., nicotine) mixture.

FIG. 19 illustrates feedback to a nicotine user regarding nicotine intake and mean craving over time.

FIG. 20 illustrates customized feedback to a user of an electronic nicotine delivery device.

FIG. 21 illustrates an embodiment of a method for flow control.

FIG. 22 illustrates an embodiment of a heater element.

FIG. 23 illustrates another embodiment for measuring an agent (e.g., nicotine) dose.

FIG. 24 illustrates another embodiment for measuring an agent (e.g., nicotine) dose.

FIGs. 25A and 25B illustrate another embodiment of a method of removal of an agent (e.g., nicotine) mixture from a reservoir.

FIG. 26 illustrates a schematic of a test apparatus used for testing the effects of altering system parameters of an aerosol delivery device on particle size distribution.

FIGs. 27A, 27B, 27C, and 27D illustrate a schematic of a test bed used for generating an aerosol in the test apparatus of FIG. 26.

FIG. 28 shows a comparison of particle sizes of an aerosol created by an e-cigarette (e-cig) vs. an aerosol created by a device as provided herein.

FIGs. 29A illustrate a schematic of a test apparatus used for testing flow control. FIG. 29B illustrates a close-up of the valve **(2904a)** that is part of the test apparatus in **FIG. 29A.**

**FIGs. 30A** illustrates an alternative valve flap for use in the valve **(2904a)** in **FIG. 29A. FIG. 30B** illustrates a slot for use in the bypass **(2908a)** in **FIG. 29A.**

**FIGs. 31A, 31B, 31C, 31D,** and **31E,** illustrate embodiments of airflow configurations and heater element.

**FIGs. 32A, 32B, 32C, 32D,** and **32E** illustrate embodiments of flow-through passageways.

**FIG. 33** illustrates an additional embodiment of a flow-through passageway.

**FIG. 34** illustrates an embodiment of a flow control valve.

**FIG. 35** illustrates an embodiment of a device comprising a primary and secondary airway.

**FIG. 36** illustrates another embodiment of a heater element.

**FIGs. 37A** and **37B** illustrate embodiments of a heater element similar to that shown in **FIG. 36. FIG. 37A** depicts a wire coil spanning a large percentage of the length of one end of the wire. **FIG. 37B** depicts a wire coil spanning a smaller percentage of the length of one end of the wire than shown in **FIG. 37A.**

**FIG. 38** is an enlarged representation of the wire coil from the heater element of **FIG. 36.**

**FIG. 39** illustrates components of eHealth-enabled electronic agent (e.g., nicotine) delivery system, in accordance with an embodiment.

**FIG. 40** illustrates example components of an electronic agent (e.g., nicotine) delivery system, in accordance with an embodiment.

**FIG. 41** illustrates example components of an electronic agent (e.g., nicotine) delivery device for implementing aspects described herein, in accordance with an embodiment.

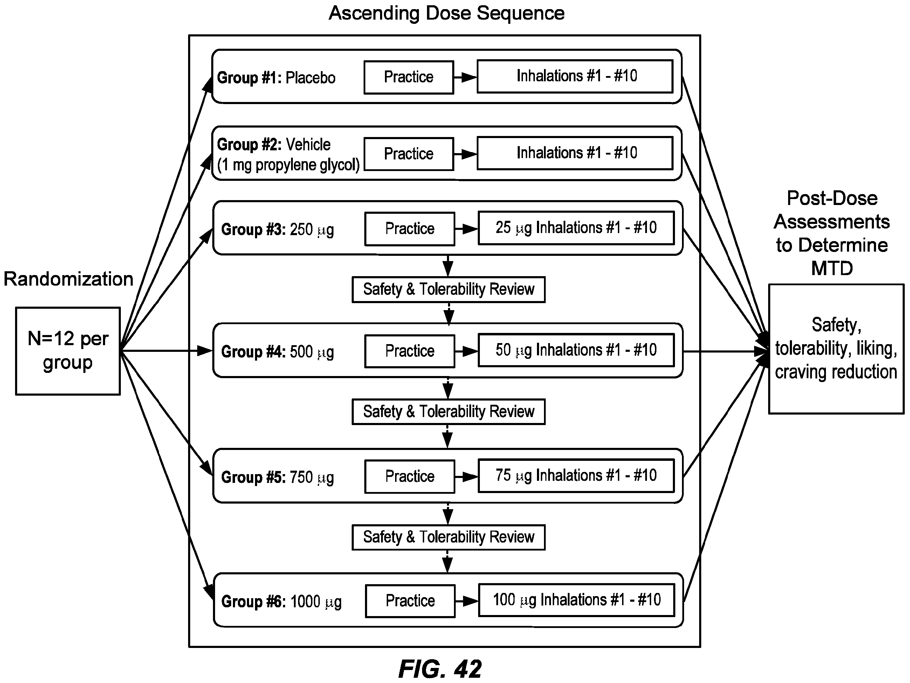

**FIG. 42** illustrates an escalating dose protocol utilized during part 1 of a two part study for assessing the safety, tolerability, pharmacokinetics, and pharmacodynamics of a condensation aerosol comprising nicotine and propylene glycol produced from an electronic agent (e.g., nicotine) delivery device as provided herein.

**FIG. 43** illustrates a trial design for part 2 of a two part study for assessing the safety, tolerability, pharmacokinetics, and pharmacodynamics of a condensation aerosol comprising nicotine and propylene glycol produced from an electronic agent (e.g., nicotine) delivery device as provided herein.

**FIGs. 44A, 44B, and 44C** illustrate embodiments of a passageway comprising a baffle for removing particles of a non-optimal size. **FIGs. 44A** and **44B** illustrate exterior views of a passageway comprising a baffle.

**FIG. 44C** illustrates an interior view of a passageway comprising a baffle.

## DETAILED DESCRIPTION

### I. Overview

Provided herein are devices, systems, kits, compositions, computer readable medium, and methods for electronic delivery of an agent to a subject. For example the devices, systems, computer readable medium, and methods can be used for electronic nicotine delivery, which can facilitate recreational nicotine delivery, full or partial smoking cessation, or facilitate full or partial cessation of nicotine intake. The subject can be a human. The human subject can be a smoker or an individual who uses tobacco or nicotine containing products. Devices described herein can generate an aerosol comprising an agent (e.g., nicotine), and the agent (e.g., nicotine) aerosol can have a known and consistent amount of agent (e g., nicotine). Also, devices and methods for dose titration are provided.

The devices, systems, kits, compositions, and computer readable medium provided herein can be part of an electronic agent (e.g., nicotine) delivery platform. The electronic platform for delivering an agent (e.g., nicotine) can be used to deliver the agent (e.g., nicotine) to a subject in a particular dose, with a particular mean particle size, pH, and airflow characteristics, which can affect back of the throat impaction and upper airway deposition. In one embodiment, the electronic delivery platform regulates a schedule of delivery of an agent (e.g., nicotine) to a user over time. Furthermore, provided herein are methods of tracking usage of an agent (e.g., nicotine) to suggest a dosing strategy based on the goal or goals of the user. In some cases, a user is a human. In some cases, a user is a human who smokes or otherwise uses tobacco or a nicotine containing product.

Provided herein are devices for generating a condensation aerosol comprising particles of a size suitable for delivery to the lungs of a subject. In some cases, a subject is a human. In some cases, a subject is a human who smokes or otherwise uses tobacco or nicotine containing products. The particles can be of a size suitable to delivery to the deep lung (i.e., alveoli) of the subject. The particles can be any of the sizes provided herein. In some cases, the particles can comprise a mass median aerodynamic diameter (MMAD) of from about 1 to about 5 $\mu$m. The particles can have a geometric standard deviation (GSD) of less than 2. The condensation aerosol can be generated from a formulation comprising a pharmaceutically active agent. The formulation can be in a liquid or solid phase prior to vaporization. The agent can be any agent as provided herein; in some cases, the agent is nicotine, and in some cases the nicotine is stabilized using one or more carriers (e.g., vegetable glycerin and/or propylene glycol). The device can comprise a heater element as provided herein and a configuration of flow-through passages or chambers suitable for generating condensation aerosols comprising particles of a size suitable for delivery to the deep lungs of a subject. For example, a device can comprise a primary flow-through chamber in fluid communication with a secondary flow-through chamber. The primary flow-through chamber can comprise an upstream and downstream opening, and the upstream opening can be an inlet for a carrier gas. The device can comprise an aerosol generation chamber, wherein the aerosol generation chamber is located (disposed) between the upstream and downstream openings within the primary flow through chamber. The aerosol generation chamber can comprise a heater element as provided herein and a source of a formulation comprising a pharmaceutically active agent (e.g. nicotine) as provided herein. The aerosol generation chamber can further comprise a configuration whereby the flow rate of the carrier gas entering the aerosol generation chamber is effective to condense a vapor generated from a formulation comprising a pharmaceutically active agent (e.g. nicotine) as provided herein within the aerosol generation chamber.

Devices and methods for aliquoting an agent (e.g., nicotine) to ensure dose-to-dose uniformity are provided herein. Furthermore, devices and methods are provided herein for sensing an inhalation by a user and triggering a device. Devices and methods are also provided herein for inhalation flow control.

Devices and methods of use of a closed loop design to control heating are provided herein. For example, a device provided herein can incorporate electronics that control for variability in battery condition and ensure consistent heating by direct measurement of resistance through the heater element to control for changes in battery voltage/charge.

Devices and methods are provided herein for transitioning a smoker away from cigarettes. For example, devices and methods are provided for enabling a subject to achieve full smoking or nicotine cessation. Devices and methods are provided for enabling a subject to achieve full smoking or nicotine cessation without relapse. Also, devices and methods are provided for enabling a subject to achieve full smoking or nicotine cessation with reduced, minimal, or no withdrawal symptoms. In some cases, a subject is a human. In some cases, a subject is a human who smokes or otherwise uses tobacco or a nicotine containing product.

eHealth tools provided herein can yield customized doses of an agent (e.g., nicotine) to a subject. In some cases, customized dosing regimens are provided, which can include instructions to dose at specific intervals, driven by reminders on the device. Devices and methods for providing customized feedback and behavioral support to a subject are also provided. In some cases, the customized feedback and/or behavioral support comprise simple instructions. The customized feedback and/or behavioral support can comprise use of social media to leverage social networks to help induce and/or maintain behavior change.

Also provided herein are methods of identifying individual user goals and matching user goals to an agent (e.g., nicotine) dose algorithm. Furthermore, provided herein are devices and methods for giving customized feedback to achieve a nicotine administration goal. Also, provided herein are devices and methods for giving customized feedback to achieve an agent administration goal. In some cases, an individual is a human. In some cases, an individual is a human who smokes or otherwise uses tobacco or a nicotine containing product.

## II. Devices

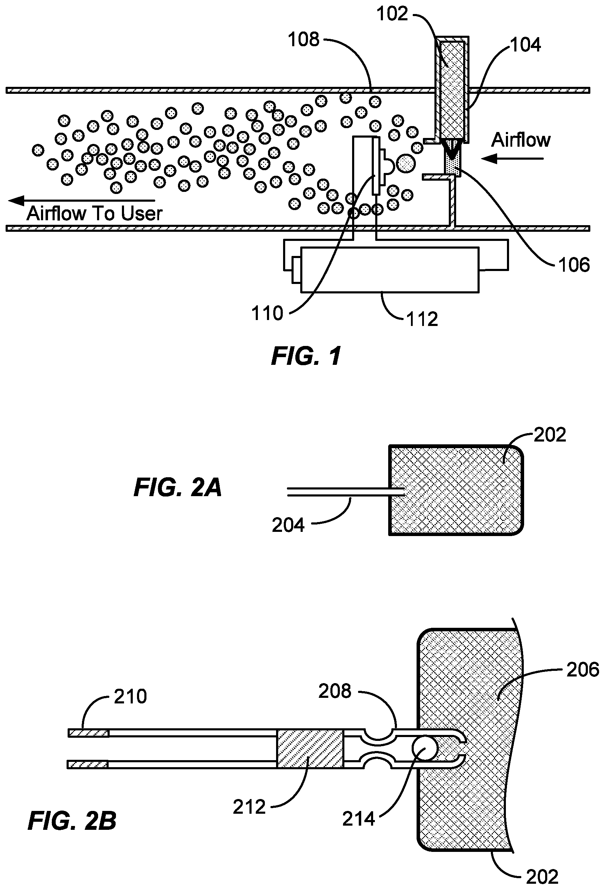

**FIG. 1** illustrates an embodiment of an electronic agent (e.g., nicotine) delivery device for controlling and reducing aerosol particle size for deep lung delivery and rapid pharmacokinetics. An agent, e.g., nicotine (**102**) is held in an agent (e.g., nicotine) reservoir (**104**), and can be wicked into a dosing mechanism (**106**). Upon inhalation, agent (e.g., nicotine) droplets are pulled out of the dosing mechanism. Small droplets are entrapped in airflow in the airway (**108**). A heater (**110**) can be in electrical communication with a battery (**112**). Larger droplets inertially impact with a heater (**110**), deposit, and are vaporized and reduced in size. Vapor condenses to form an optimum size aerosol by controlling airflow and vaporization rate.

## Agent doses

An electronic agent (e.g., nicotine) delivery device provided herein can provide doses of agent (e.g., nicotine) in a consistent and known amount. A dose of an agent (e.g., nicotine) can about, more than, less than, or at least 1, 2, 3, 4, 5, 6, 7, 8, 9, 10, 11, 12, 13, 14, 15, 16, 17, 18, 19, 20, 21, 22, 23, 24, 25, 26 27, 28, 29, 30, 31, 32, 33, 34, 35, 36, 37, 38, 39, 40, 41, 42, 43, 44, 45, 46, 47, 48, 94, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145, 146, 147, 148, 149, 150, 151, 152, 153, 154, 155, 156, 157, 158, 159, 160, 161, 162, 163, 164, 165, 166, 167, 168, 169, 170, 171, 172, 173, 174, 175, 176, 177, 178, 179, 180, 181, 182, 183, 184, 185, 186, 187, 188, 189, 190, 191, 192, 193, 194, 195, 196, 197, 198, 199, 200, 201, 202, 203, 204, 205, 206, 207, 208, 209, 210, 211, 212, 213, 214, 215, 216, 217, 218, 219, 220, 221, 222, 223, 224, 225, 226, 227, 228, 229, 230, 231, 232, 233, 234, 235, 236, 237, 238, 239, 240, 241, 242, 243, 244, 245, 246, 247, 248, 249, 250, 260, 270, 280, 290, 300, 310, 320, 330, 340, 350, 360, 370, 380, 390, 400, 410, 420, 430, 440, 450, 460, 470, 480, 490, 500, 510, 520, 530, 540, 550, 560, 570, 580, 590, 600, 610, 620, 630, 640, 650, 660, 670, 680, 690, 700, 710, 720, 730, 740, 750, 760, 770, 780, 790, 800, 810, 820, 830, 840, 850, 860, 870, 880, 890, 900, 910, 920, 930, 940, 950, 960, 970, 980, 990, 1000 $\mu$g of agent (e.g., nicotine). In some cases, a device can deliver a dose of an agent of about, more than, less than, or at least 1, 2, 3, 4, 5, 6, 7, 8, 9, 10, 11, 12, 13, 14, 15, 16, 17, 18, 19, 20, 21, 22, 23, 24, 25, 26 27, 28, 29, 30, 31, 32, 33, 34, 35, 36, 37, 38, 39, 40, 41, 42, 43, 44, 45, 46, 47, 48, 94, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, or 100 mg.

In one embodiment, a dose of an agent (e.g., nicotine) is about 1 $\mu$g to about 1000 $\mu$g, about 1 $\mu$g to about 500 $\mu$g, about 1 $\mu$g to about 1000 $\mu$g, about 10 $\mu$g to about 500 $\mu$g, about 20 $\mu$g to about 500 $\mu$g, about 25 $\mu$g to about 500 $\mu$g, about 30 $\mu$g to about 500 $\mu$g, about 40 $\mu$g to about 500 $\mu$g, about 50 $\mu$g to about 500 $\mu$g, about 10 $\mu$g to about 250 $\mu$g, about 20 $\mu$g to about 250 $\mu$g, about 30 $\mu$g to about 250 $\mu$g, about 40 $\mu$g to about 250 $\mu$g, about 50 $\mu$g to about 250 $\mu$g, about 1 $\mu$g to about 200 $\mu$g, about 10 $\mu$g to about 200 $\mu$g, about 20 $\mu$g to about 200 $\mu$g, about 30 $\mu$g to about 200 $\mu$g, about 40 $\mu$g to about 200 $\mu$g, about 50 $\mu$g to about 200 $\mu$g, about 25 $\mu$g to about 50 $\mu$g, about 25 $\mu$g to about 100 $\mu$g, about 25 $\mu$g to about 150 $\mu$g, about 25 $\mu$g to about 200 $\mu$g, about 25 $\mu$g to about 250 $\mu$g, about 25 $\mu$g to about 300 $\mu$g, about 25 $\mu$g to about 350 $\mu$g, about 25 $\mu$g to about 400 $\mu$g, about 25 $\mu$g to about 450 $\mu$g, about 25 $\mu$g to about 500 $\mu$g, about 50 $\mu$g to about 750 $\mu$g, or about 25 $\mu$g to about 1000 $\mu$g of agent (e.g., nicotine). In some cases, a dose of an agent is about 1 mg to about 100 mg, about 1 mg to about 50 mg, about 10 mg to about 50 mg, about 20 mg to about 50 mg, about 25 mg to about 50 mg, about 30 mg to about 50 mg, about 40 mg to about 50 mg, about 50 mg to about 100 mg, about 1 mg to about 25 mg, about 2 mg to about 25 mg, about 3 mg to about 25 mg, about 4 mg to about 25 mg, about 5 mg to about 25 mg, about 1 mg to about 20 mg, about 1 mg to about 20 mg, about 2 mg to about 20 mg, about 3 mg to about 20 mg, about 4 mg to about 20 mg, or about 5 mg to about 20 mg of agent.

An emitted dose of an agent (e.g., nicotine) can be about, more than, less than, or at least 1, 2, 3, 4, 5, 6, 7, 8, 9, 10, 11, 12, 13, 14, 15, 16, 17, 18, 19, 20, 21, 22, 23, 24, 25, 26 27, 28, 29, 30, 31, 32, 33, 34, 35, 36, 37, 38, 39, 40, 41, 42, 43, 44, 45, 46, 47, 48, 94, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145, 146, 147, 148, 149, 150, 151, 152, 153, 154, 155, 156, 157, 158, 159, 160, 161, 162, 163, 164, 165, 166, 167, 168, 169, 170, 171, 172, 173, 174, 175, 176, 177, 178, 179, 180, 181, 182, 183, 184, 185, 186, 187, 188, 189, 190, 191, 192, 193, 194, 195, 196, 197, 198, 199, 200, 201, 202, 203, 204, 205, 206, 207, 208, 209, 210, 211, 212, 213, 214, 215, 216, 217, 218, 219, 220, 221, 222, 223, 224, 225, 226, 227, 228, 229, 230, 231, 232, 233, 234, 235, 236, 237, 238, 239, 240, 241, 242, 243, 244, 245, 246, 247, 248, 249, 250, 260, 270, 280, 290, 300, 310, 320, 330, 340, 350, 360, 370, 380, 390, 400, 410, 420, 430, 440, 450, 460, 470, 480, 490, 500, 510, 520, 530, 540, 550, 560, 570, 580, 590, 600, 610, 620, 630, 640, 650, 660, 670, 680, 690, 700, 710, 720, 730, 740, 750, 760, 770, 780, 790, 800, 810, 820, 830, 840, 850, 860, 870, 880, 890, 900, 910, 920, 930, 940, 950, 960, 970, 980, 990, or 1000 $\mu$g of agent (e.g., nicotine). In some cases, an emitted dose of an agent is about, more than, less than, or at least 1, 2, 3, 4, 5, 6, 7, 8, 9, 10, 11, 12, 13, 14, 15, 16, 17, 18, 19, 20, 21, 22, 23, 24, 25, 26 27, 28, 29, 30, 31, 32, 33, 34, 35, 36, 37, 38, 39, 40, 41, 42, 43, 44, 45, 46, 47, 48, 94, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, or 100 mg. In one embodiment, an emitted dose of an agent (e.g., nicotine) is about 1 $\mu$g to about 1000 $\mu$g, about 1 $\mu$g to about 500 $\mu$g, about 1 $\mu$g to about 1000 $\mu$g, about 10 $\mu$g to about 500 $\mu$g, about 20 $\mu$g to about 500 $\mu$g, about 25 $\mu$g to about 500 $\mu$g, about 30 $\mu$g to about 500 $\mu$g, about 40 $\mu$g to about 500 $\mu$g, about 50 $\mu$g to about 500 $\mu$g, about 10 $\mu$g to about 250 $\mu$g, about 20 $\mu$g to about 250 $\mu$g, about 30 $\mu$g to about 250 $\mu$g, about 40 $\mu$g to about 250 $\mu$g, about 50 $\mu$g to about 250 $\mu$g, about 1 $\mu$g to about 200 $\mu$g, about 10 $\mu$g to about 200 $\mu$g, about 20 $\mu$g to about 200 $\mu$g, about 30 $\mu$g to about 200 $\mu$g, about 40 $\mu$g to about 200 $\mu$g, about 50 $\mu$g to about 200 $\mu$g, about 25 $\mu$g to about 50 $\mu$g, about 25 $\mu$g to about 100 $\mu$g, about 25 $\mu$g to about 150 $\mu$g, about 25 $\mu$g to about 200 $\mu$g, about 25 $\mu$g to about 250 $\mu$g, about 25 $\mu$g to about 300 $\mu$g, about 25 $\mu$g to about 350 $\mu$g, about 25 $\mu$g to about 400 $\mu$g, about 25 $\mu$g to about 450 $\mu$g, about 25 $\mu$g to about 500 $\mu$g, about 50 $\mu$g to about 750 $\mu$g, or about 25 $\mu$g to about 1000 $\mu$g (e.g., nicotine). In some cases, an emitted dose of an agent is about 1 mg to about 100 mg, about 1 mg to about 50 mg, about 10 mg to about 50 mg, about 20 mg to about 50 mg, about 25 mg to about 50 mg, about 30 mg to about 50 mg, about 40 mg to about 50 mg, about 50 mg to about 100 mg, about 1 mg to about 25 mg, about 2 mg to about 25 mg, about 3 mg to about 25 mg, about 4 mg to about 25 mg, about 5 mg to about 25 mg, about 1 mg to about 20 mg, about 1 mg to about 20 mg, about 2 mg to about 20 mg, about 3 mg to about 20 mg, about 4 mg to about 20 mg, or about 5 mg to about 20 mg of agent. In another embodiment, a device according to any of the embodiments described herein delivers only a single emitted dose of an agent (e.g., nicotine).

In some cases, the emitted dose can be about, more than, less than, or at least 1, 2, 3, 4, 5, 6, 7, 8, 9, 10, 11, 12, 13, 14, 15, 16, 17, 18, 19, 20, 21, 22, 23, 24, 25, 26 27, 28, 29, 30, 31, 32, 33, 34, 35, 36, 37, 38, 39, 40, 41, 42, 43, 44, 45, 46, 47, 48, 94, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, or 100% of the dose (or loaded dose). In some cases, the emitted dose can be between 1%-10%, 10%-20%, 20%-30%, 30%-40%, 40%-50%, 50%-60%, 60%-70%, 70%-80%, 80%-90%, or 90%-100% of the dose (or loaded dose). In some cases, the emitted dose is more than 20% of the dose (or loaded dose). In some cases, the emitted dose is less than 20% of the dose (or loaded dose). The dose (or loaded dose) is the amount of nicotine solution delivered onto the heater element prior to the creation of the aerosol and can be about 2% of the target dose (the label claimed dose or goal dose). The emitted dose can be 92% to 97% of the dose. For example, the amount actually delivered to the lung if the label claim dose is 100 $\mu$g can be between 90% and 99%.

**Dosing**

Provided herein are methods for administering agent (e.g., nicotine) challenge doses to a subject. In some cases, a subject is a human. In some cases, a subject is a human who smokes or otherwise uses tobacco or nicotine containing products. Methods are provided herein for generating condensation aerosols comprising particles comprising a mass median aerodynamic diameter (MMAD) effective for delivery to the deep lung of a subject. The methods can comprise supplying or delivering a liquid formulation comprising a pharmaceutically active agent (e.g. nicotine) to a passageway; vaporizing the liquid formulation using a heater element in the passageway to produce a vaporized liquid formulation; and flowing a carrier gas through the passageway at a flow rate effective to allow condensation of the vaporized liquid formulation into particles comprising a size effective for delivery to the deep lung. The size of the particles following condensation can be an MMAD of from about 1 to about 5 $\mu$m. The flow rate can be about 1 to about 10 liters per minute (LPM) (a range from about 1.667 x $10^{-5}$ $m^3$/s to about 1.667 x $10^{-4}$ $m^3$/s), e.g., at a vacuum of about 1 to about 15 inches of water (a range from about 249 Pa to about 3738 Pa). The flow resistance of the device can be about 0.05 to about 0.15 (cm of $H_2O$)$^{\frac{1}{2}}$/LPM. The liquid formulation can be supplied or delivered from a reservoir. The reservoir can comprise a tube, e.g., a capillary tube. The reservoir can be in fluid communication with the heater element. In some cases, the liquid formulation comprising a pharmaceutically active agent (e.g., nicotine) is delivered to the heater element through the use of a positive displacement pump. The positive displacement pump can be a reciprocating, metering, rotary-type, hydraulic, peristaltic, gear, screw, flexible impeller, diaphragm, piston, or progressive cavity pump, or any other pump utilizing positive displacement as known in the art. The positive displacement pump can be in fluid communication with the heater element. The positive displacement pump can be in fluid communication or fluidically coupled to a reservoir comprising a pharmaceutically active agent (e.g., nicotine). The positive displacement pump can be in fluid communication with the heater element and a reservoir comprising a pharmaceutically active agent (e.g., nicotine). The pharmaceutically active agent (e.g., nicotine) can be a liquid formulation. The positive displacement pump can be within the passageway or external to the passageway. The heater element can be any heater element as provided herein. The carrier gas can be air.

Methods for aliquoting an agent (e.g., nicotine) to ensure dose-to-dose uniformity are provided herein. For example, an element comprising porous materials can wick out fluid comprising agent (e.g., nicotine) at a particular rate in order to measure out a dose to provide dose-to-dose uniformity. A tube, e.g., a capillary tube can be used to measure out a dose. In one embodiment, heat is used as a means of ejecting a dose. A material or geometry of a device can be used to measure out a dose. In one embodiment, providing dose consistency controls for variability in environment and device. In another embodiment, inhalation flow control ensures that variability in inhalations by a user are controlled and corrected for, which can result in dose-to-dose consistency and predictable and desirable aerosol particle sizes.

In some cases, an agent (e.g., nicotine) is metered out into a pre-vaporization area in a device (dosing mechanism) through capillary action. The metering can occur between inhalations of a user of a device. Upon inhalation by a subject, an agent (e.g., nicotine) can be drawn into a vaporization chamber or onto a heater element. The agent can be a pharmaceutically active agent. The agent can be in a formulation that is liquid. The liquid formulation comprising a pharmaceutically active agent (e.g., nicotine) can be drawn or metered out into a vaporization chamber or onto a heater element upon inhalation by a subject. The subject can be a human. The human subject can be a smoker or user of tobacco or nicotine containing substances. The agent (e.g., nicotine) in the vaporization chamber or heater element can be vaporized and subsequently condense to form an aerosol. The aerosol can comprise agent (e.g., nicotine) particles of an optimum size to achieve certain biological effects (e.g., deep lung delivery producing rapid pharmacokinetics). Devices described herein can comprise a mechanism for separating out and reducing large aerosol particles to a size that can navigate to the deep lung of a subject. In the deep lung, the particles can settle and be rapidly absorbed. Also provided herein are methods for controlling aerosol particle size, pH, and other inhalation characteristics, which can ensure deep lung delivery and rapid pharmacokinetics. For example, the aerosol size control can result in rapid, cigarette-like nicotine absorption, which can help to satisfy nicotine cravings. In some cases, aerosol particles comprising nicotine produced by a heater element or device as provided herein can achieve peak plasma concentrations similar to peak plasma concentrations achieved by smoking a cigarette. In some cases, aerosol particles comprising nicotine produced by a heater element or device as provided herein can achieve peak plasma concentrations in a time frame similar to the time frame required to achieve peak plasma concentrations achieved by smoking a cigarette. The condensation aerosol comprising nicotine produced by any of the devices provided herein can result in rapid, cigarette-like nicotine absorption resulting in nicotine plasma concentrations similar or substantially similar to the nicotine plasma concentration achieved from smoking a cigarette. In some cases, the plasma concentration can be an arterial plasma concentration. In some cases, the plasma concentration can be a venous plasma concentration. Smoking a single cigarette can produce peak increments of plasma nicotine concentration of 5-30 ng/ml.

In some cases, use of a device described herein can produce an arterial plasma nicotine concentration in the user of the device of about 1 ng/mL to about 200 ng/mL, about 1 ng/mL to about 150 ng/mL, about 1 ng/mL to about 100 ng/mL, about 1 ng/mL to about 75 ng/mL, about 1 ng/mL to about 50 ng/mL, about 1 ng/mL to about 40 ng/mL, about 1 ng/mL to about 30 ng/mL, about 1 ng/mL to about 20 ng/mL, about 1 ng/mL to about 10 ng/mL, about 10 ng/mL to about 200 ng/mL, about 10 ng/mL to about 150 ng/mL, about about 10 ng/mL to about 100 ng/mL, about 10 ng/mL to about 75 ng/mL, about 10 ng/mL to about 50 ng/mL, about 10 ng/mL to about 40 ng/mL, about 10 ng/mL to about 30 ng/mL, about 10 ng/mL to about 20 ng/mL, about 10 ng/mL to about 15 ng/mL, about 20 ng/mL to about 200 ng/mL, about 20 ng/mL to about 150 ng/mL, about 20 ng/mL to about 100 ng/mL, about 20 ng/mL to about 75 ng/mL, about 20 ng/mL to about 50 ng/mL, about 20 ng/mL to about 40 ng/mL, about 20 ng/mL to about 30 ng/mL, about 20 ng/mL to about 24 ng/mL, about 30 ng/mL to about 200 ng/mL, about 30 ng/mL to about 150 ng/mL, about 30 ng/mL to about 100 ng/mL, about 30 ng/mL to about 75 ng/mL, about 30 ng/mL to about 50 ng/mL, about 30 ng/mL to about 40 ng/mL, or about 30 ng/mL to about 35 ng/mL. In some cases, use of a device described herein can produce an arterial plasma nicotine concentration in a user of the device of about, more than, less than, or at least 1, 2, 3, 4, 5, 6, 7, 8, 9, 10, 11, 12, 13, 14, 15, 16, 17, 18, 19, 20, 21, 22, 23, 24, 25, 26 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100 ng/mL. The arterial plasma nicotine concentration can be produced after receiving at least 1, 2, 3, 4, 5, 6, 7, 8, 9, or 10 doses from the condensation aerosol generating device.

In some cases, use of a device described herein can produce a peak plasma nicotine concentration in a user of the device within about 30 seconds to 30 minutes, 30 seconds to 20 minutes, 30 seconds to 10 minutes, 30 seconds to 5 minutes, 30 seconds to 2 minutes, 1 to about 30 minutes, about 1 minute to about 25 minutes, about 1 minute to about 20 minutes, about 1 minute to about 15 minutes, about 1 minute to about 10 minutes, about 5 minutes to about 30 minutes, about 5 minutes to about 25 minutes, about 5 minutes to about 20 minutes, about 5 minutes to about 15 minutes, about 5 minutes to about 10 minutes, about 10 minutes to about 30 minutes, about 10 minutes to about 25 minutes, about 10 minutes to about 20 minutes, or about 10 minutes to about 15 minutes of use of the device. A use of the device can be an inhalation of a dose delivered by the device.

The peak increments of plasma nicotine concentration from smoking a cigarette can be achieved within 10 mintues. The nicotine arterial plasma concentration can be about, more than, less than, or at least 1%, 2%, 3%,4%,5%, 6%, 7%, 8%, 9%, 10%, 11%, 12%, 13%, 14%, 15%, 16%, 17%, 18%, 19%, 20%, 30%, 31%, 32%, 33%, 34%, 35%, 36%, 37%, 38%, 39%, 40%, 41%, 42%, 43%, 44%, 45%, 46%, 47%, 48%, 49%, 50%, 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99%, or 100% of the nicotine plasma concentration achieved by smoking a cigarette. The nicotine arterial plasma concentration can be between 1%-10%, 10%-20%, 20%-30%, 30%-40%, 40%-50%, 50%-60%, 60%-70%, 70%-80%, 80%-90%, or 90%-100% of the nicotine plasma concentration achieved by smoking a cigarette. The nicotine arterial plasma concentration can be about 1% to about 10%, about 10% to about 20%, about 20% to about 30%, about 30% to about 40%, about 40% to about 50%, about 50% to about 60%, about 60% to about 70%, about 70% to about 80%, about 80% to about 90%, or about 90% to about 100% of the nicotine plasma concentration achieved by smoking a cigarette.

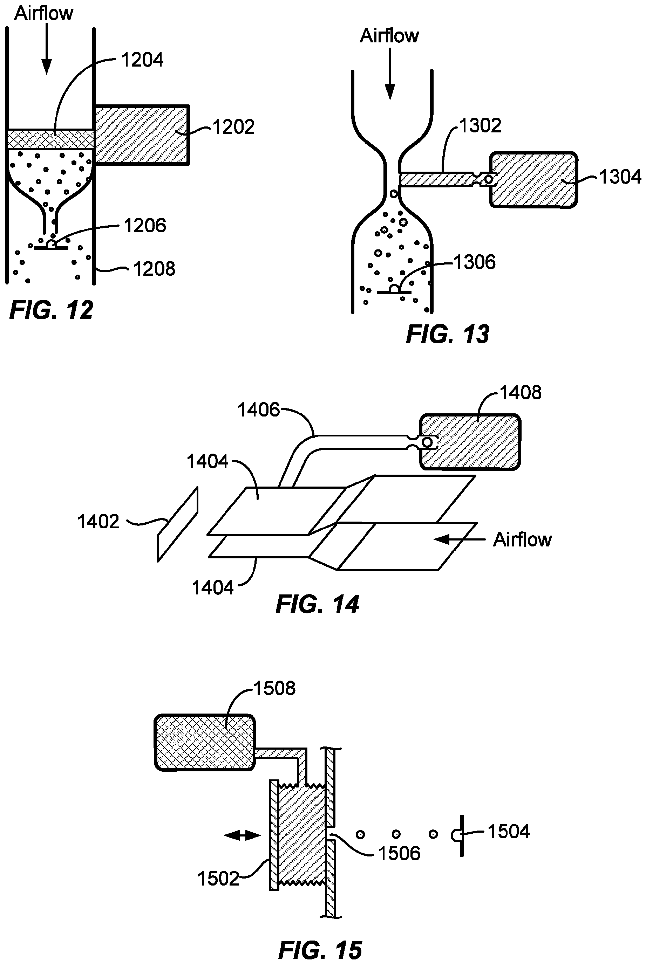

**FIG. 12** illustrates an embodiment of a method of removal of an agent (e.g., nicotine) mixture from a reservoir and dispensing the agent (e.g., nicotine) into desired doses. **FIG. 12** shows an agent (e.g., nicotine) reservoir (**1202**) next to a frit (**1204**) or porous material, such as a metal (stainless steel) or a ceramic, and allowing the agent (e.g., nicotine) to wick into it. Then, upon inhalation, the air can draw the agent (e.g., nicotine) into the airway (**1208**) and onto the heater element (**1206**). In some cases, the mixture is a liquid formulation comprising an agent (e.g., nicotine).

**FIG. 13** illustrates another embodiment of a method for measuring a dose. Another method of dosing out the mixture is to draw the material out using a venturi. The device can comprise a tube, e.g., a capillary tube (**1302**), an agent (e.g., nicotine) reservoir (**1304**), and a heater element (**1306**). In some cases, the mixture is a liquid formulation comprising an agent (e.g., nicotine).

**FIG. 14** illustrates another embodiment of a method for measuring a dose. In this embodiment, an agent (e.g., nicotine) mixture can be wicked into a space between two parallel plates. The device can comprise a heater element (**1402**), plates (**1404**), tube, e.g., capillary tube (**1406**), and an agent (e.g., nicotine) reservoir (**1408**). In some cases, the mixture is a liquid formulation comprising an agent (e.g., nicotine).

**FIG. 15** illustrates another embodiment for measuring a dose. An agent (e.g., nicotine) mixture can be ejected using a piezoelectric device (**1502**) and an attached chamber with an opening or orifice (**1506**). When the piezo is activated, either as a single pulse or as a series of pulses (vibrated) the mixture can be driven from the opening. By controlling the amplitude of the pulse or the number of pulses, the amount of material dosed can be controlled. The device can comprise an agent (e.g., nicotine) reservoir (**1508**) and a heater element (**1504**). In one embodiment, a piezo electric device is mounted on an end or a side of the reservoir and receipt of an electrical pulse causes the piezo to deflect and push a small amount of the agent (e.g., nicotine) formulation out of a tube, e.g., capillary tube mounted on another end of the reservoir onto a heater element. In some cases, the agent formulation is liquid.

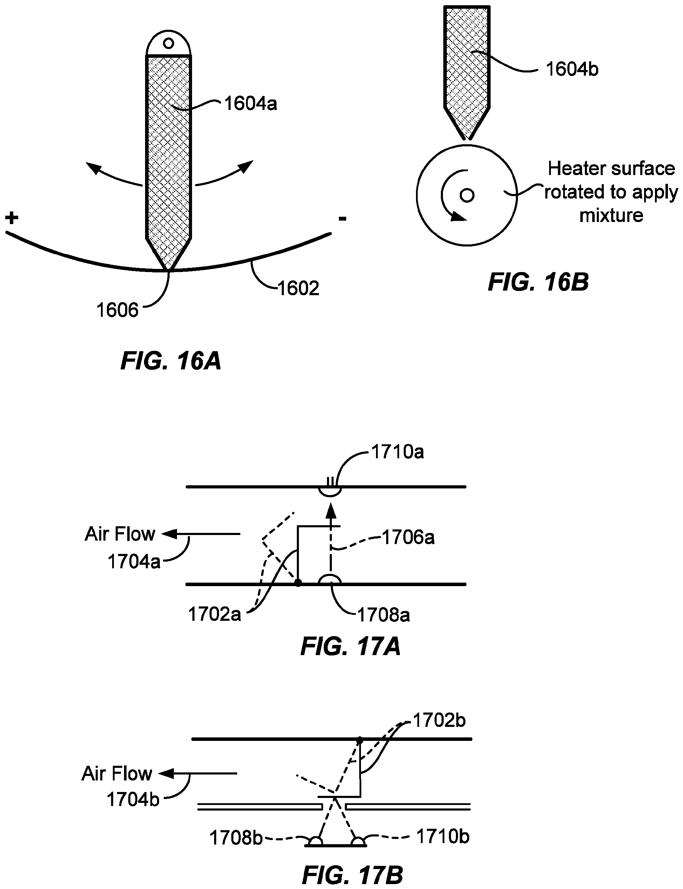

All of the forgoing mechanisms to power the dispensing of a mixture (heat, piezo) can be powered by a user performing a maneuver such as pushing a button or lever. Mechanical energy from the user can also allow for alternative methods of applying agent (e.g., nicotine) to a heater surface. An agent (e.g., nicotine) can be applied to the heater element (**1602**), where the reservoir is moved over the heater surface in a sweeping (see **FIG. 16A**) or rolling motion (see **FIG. 16B**). The heater surface can be etched or pitted to accept the mixture.

To have the device generate an agent (e.g., nicotine) aerosol upon inhalation by a user, a movable member (e.g., vane (**1702a** or **1702b**)) can be used that moves upon air flow (**1704a** or **1704b**) caused by inhalation (see e.g., **FIG. 17A** or **17B**). This member can break an optical path (**1706a**) (e.g., when no inhalation is occurring), move out of an optical path (**1706a**) when inhalation occurs (see e.g., **FIG**. **17A**), or can complete an optical path when inhalation occurs (by, e.g., reflection; see e.g., **FIG. 17B**). An LED (**1708a** or **1708b**) can be used to generate the light. To ensure that a sensor or detector (**1710a** or **1710b**) does not get confused by stray light, the LED (**1708a** or **1708b**) can be strobed in a particular pattern and only when that pattern is detected is an inhalation present. In some cases, optical light pipes can be used to route the light to the valve and to route the light back to the detector.

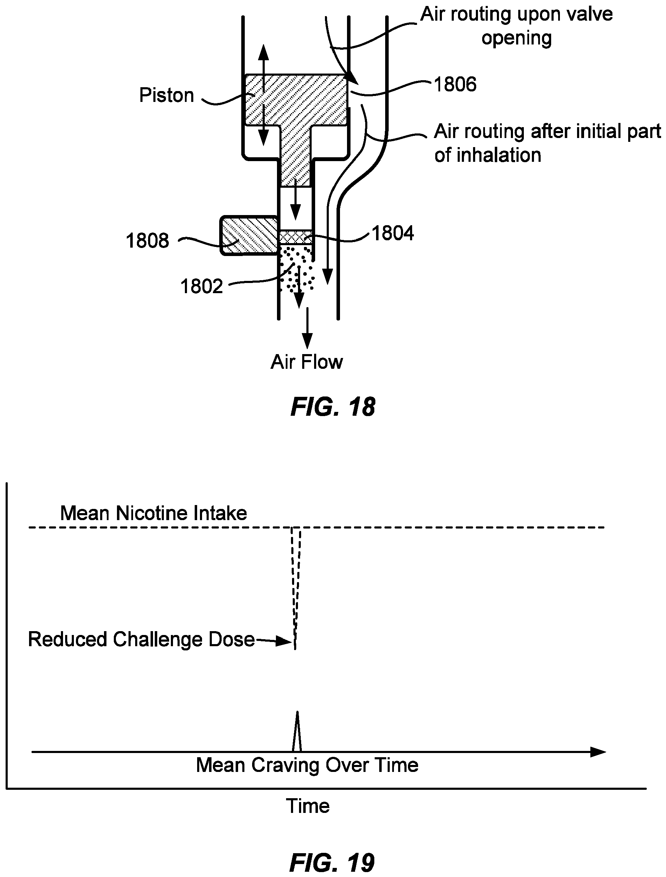

To dispense the agent (e.g., nicotine) mixture (**1802**) out of some of the frits (**1804**) or capillaries using the pressure from the inhalation a valve can be designed to create increased pressure in the initial part of the inhalation and decrease the resistance for the duration of the inhalation (see e.g., **FIG. 18**).

In one embodiment, an electronic agent (e.g., nicotine) delivery device is provided that provides a dose of from 25 to 200 μg of freebase agent (e.g., nicotine). The agent (e.g., nicotine) can be in a mixture of propylene glycol at a ratio of agent (e.g., nicotine) to propylene glycol of from about 1:1 to about 1:20, or about 1:5 to about 1:10. In some cases, a mixture comprises propylene glycol and about 1.25% to about 20% nicotine. In some cases, the mixture is liquid formulation comprising an agent (e.g., nicotine). In some cases, the mixture is liquid formulation comprising an agent (e.g., nicotine) during use of the device. An aerosol can have an MMAD of about 1 to about 5 microns with a geometric standard deviation (GSD) of less than 2.0. Dose to dose consistency over the lifetime of the product can be no greater than ± 30%. The device can have a dose to dose consistency over the lifetime of the product that can be about, more than, less than, at least, or at most ± 1%, 2%, 3%,4%,5%, 6%, 7%, 8%, 9%, 10%, 11%, 12%, 13%, 14%, 15%, 16%, 17%, 18%, 19%, 20%, 30%, 31%, 32%, 33%, 34%, 35%, 36%, 37%, 38%, 39%, 40%, 41%, 42%, 43%, 44%, 45%, 46%, 47%, 48%, 49%, or 50%. The device can be activated by an inhalation. The device can have an interior air resistance (to inhalation) no greater than that of a cigarette. The device can have an interior air resistance (to inhalation) no greater than 0.08 (cm $H_2O$)$^{1/2}$/LPM. The device can have an interior air resistance (to inhalation) about, more than, less than, at least, or at most 0.01, 0.02, 0.03, 0.04, 0.05, 0.06, 0.07, 0.08, 0.09, 0.10, 0.11, 0.12, 0.13, 0.14, 0.15, 0.16, 0.17, 0.18, 0.19, or 0.20(cm $H_2O$)$^{1/2}$/LPM.

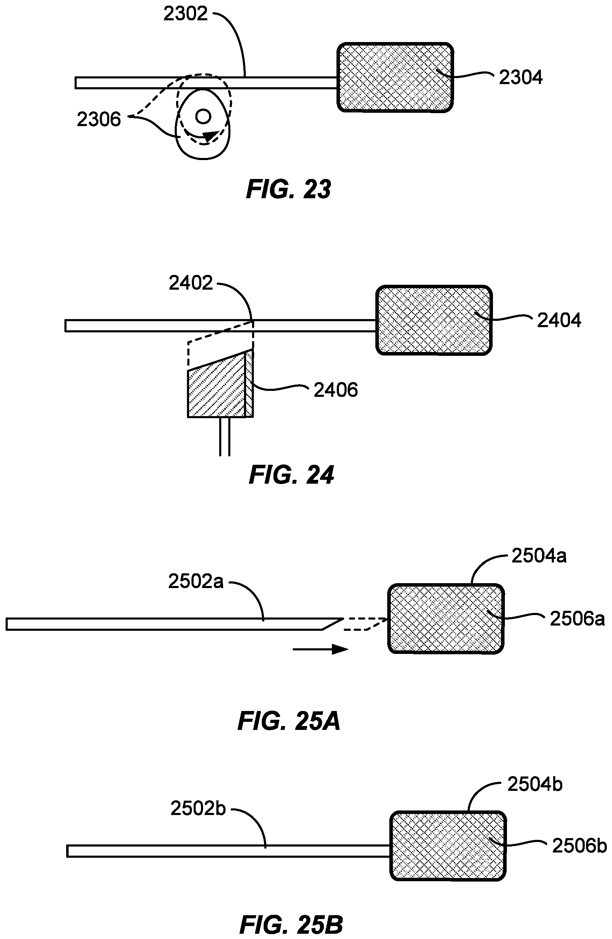

**FIG. 23** illustrates another embodiment of a method for measuring a dose. Another method of dosing out the mixture is to draw the material out using a peristaltic pump comprising a rotatable cam. The device can comprise a tube, e.g., capillary tube (**2302**), agent (e.g., nicotine) reservoir (**2304**), and a rotatable cam (**2306**) to pull or draw an agent (e.g., nicotine) mixture from the nicotine reservoir. In one embodiment, an agent (e.g., nicotine) delivery device comprises a disposable component that comprises the tube, e.g., capillary tube, and agent (e.g, nicotine) reservoir and a reusable component comprising the rotatable cam, wherein the tube, e.g., capillary tube and agent (e.g., nicotine) reservoir are mechanically connected to the rotatable cam by mating the disposable component to the reusable component. In some cases, the mixture is a liquid formulation comprising an agent (e.g., nicotine).

**FIG. 24** illustrates another embodiment of a method for measuring a dose. The device can comprise a tube, e.g, capillary tube (**2402**), agent (e.g., nicotine) reservoir (**2404**), and a cam made of variable durometer material (**2406**). The cam can comprise an area of high durometer material surrounded by low durometer material, wherein the tube, e.g., capillary tube can be sealed within the high durometer material. In one embodiment, an agent (e.g., nicotine) mixture can be pushed out of the tube, e.g., capillary tube by compression, wherein pressure is exerted on the low durometer material of the cam to cause compression of the tube, e.g., capillary tube, within the high durometer material. In one embodiment, an agent (e.g., nicotine) delivery device comprises a disposable component that comprises the tube, e.g., capillary tube and the agent (e.g., nicotine) reservoir and a reusable component comprising the cam made of variable durometer material, wherein the tube, e.g., capillary tube and agent (e.g., nicotine) reservoir are mechanically connected to the cam made of variable durometer material by mating the disposable component to the reusable component. In some cases, the mixture is a liquid formulation comprising an agent (e.g., nicotine).

**FIG. 25** illustrates an embodiment of a method of removal of an agent (e.g., nicotine) mixture from a reservoir.

**FIG. 25A** shows a tube, e.g., capillary tube **(2502a)** adjacent to, but separate from, an agent (e.g., nicotine) reservoir **(2504a)** comprising an agent (e.g., nicotine) mixture **(2506a)**. **FIG. 25B** shows that the tube, e.g., capillary tube **(2502b)** can pierce the agent (e.g., nicotine) reservoir **(2504b)** such that the agent (e.g., nicotine) mixture **(2506b)** within the agent (e.g., nicotine) reservoir can move into the tube, e.g., capillary tube and subsequently onto a heater element as provided herein. In one embodiment, the agent (e.g., nicotine) reservoir comprises a septum or seal, wherein the tube, e.g., capillary tube pierces the septum or seal. In one embodiment, the agent (e.g., nicotine) reservoir is a collapsible bag or container. In one embodiment, the collapsible bag or container is made of plastic, foil, or any other collapsible material known in the art. In a further embodiment, the tube, e.g., capillary tube can directly pierce an agent (e.g., nicotine) reservoir that is made of a collapsible material. In one embodiment, the tube, e.g., capillary tube is not inserted into the agent (e.g., nicotine) reservoir prior to a first use of the device, wherein upon first use, the tube, e.g., capillary tube, is inserted into the agent (e.g., nicotine) reservoir such that an agent (e.g., nicotine) mixture can move from the agent (e.g., nicotine) reservoir into the tube, e.g., capillary tube and subsequently onto a heater element as provided herein. In some cases, the mixture is a liquid formulation comprising an agent (e.g., nicotine).

## Carriers/excipients

In some cases, an agent (e.g., nicotine) is mixed with one or more other substances. When mixed with an agent (e.g., nicotine) as provided herein, the mixture can be liquid at room temperature. When mixed with an agent (e.g., nicotine) as provided herein, the mixture can be liquid during use of the device such that the liquid mixture is delivered to the heater element during use of the device. The one or more other substances can be pharmaceutically acceptable excipients or carriers. The suitable pharmaceutically acceptable excipients or carriers can be volatile or nonvolatile. The volatile excipients, when heated, can be volatilized, aerosolized and inhaled with the agent (e.g. nicotine). Classes of such excipients are known in the art and include, without limitation, gaseous, supercritical fluid, liquid and solid solvents. The excipient/carriers can be water; terpenes, such as menthol; alcohols, such as ethanol, propylene glycol, glycerol and other similar alcohols; dimethylformamide; dimethylacetamide; wax; supercritical carbon dioxide; dry ice; and mixtures or combinations thereof.

The one or more other substances can be, e.g., propylene glycol (1,2-dihydroxypropane, 1,2-propanediol, methyl glycol, or trimethyl glycol). The ratio of agent (e.g., nicotine) to propylene glycol can be about, more than, less than, or at least 100:1, 95:1, 90:1, 85:1, 80:1, 75:1, 70:1, 65:1, 60:1, 55:1, 50:1, 45:1, 40:1, 35:1, 30:1, 25:1, 20:1, 15:1, 10:1, 5:1, 2:1, 1:1:, 1:2, 1:5, 1:10, 1:15, 1:20, 1:25, 1:30, 1:35, 1:40, 1:45, 1:50, 1:55, 1:60, 1:65, 1:70, 1:75, 1:80, 1:85, 1:90, 1:95, or 1:100. The ratio of agent (e.g., nicotine) to propylene glycol can be from about 100:1 to about 1:100, about 75:1 to about 1:100, about 50:1 to about 1:100, about 25:1 to about 1:100, about 25:1 to about 1:50, about 10:1 to about 1:100, about 10:1 to about 1:50, about 10:1 to about 1:20, about 5:1 to about 1:20, or about 1:1 to about 1:20. In one example, a 100 $\mu$g dose of agent (e.g., nicotine) and 1:10 ratio yields a volume of 1 mm$^3$ (1 mg). A mixture of agent (e.g., nicotine) and another substance, e.g., propylene glycol, can be held in an agent (e.g., nicotine) reservoir (e.g., as a liquid).

In one embodiment, the one or more other substances is vegetable glycerin. The ratio of an agent (e.g., nicotine) to vegetable glycerin can be about, more than, less than, or at least 100:1, 95:1, 90:1, 85:1, 80:1, 75:1, 70:1, 65:1, 60:1, 55:1, 50:1, 45:1, 40:1, 35:1, 30:1, 25:1, 20:1, 15:1, 10:1, 5:1, 2:1, 1:1:, 1:2, 1:5, 1:10, 1:15, 1:20, 1:25, 1:30, 1:35, 1:40, 1:45, 1:50, 1:55, 1:60, 1:65, 1:70, 1:75, 1:80, 1:85, 1:90, 1:95, or 1:100. The ratio of an agent (e .g., nicotine) to vegetable glycerin can be from about 100:1 to about 1:100, about 75:1 to about 1:100, about 50:1 to about 1:100, about 25:1 to about 1:100, about 25:1 to about 1:50, about 10:1 to about 1:100, about 10:1 to about 1:50, about 10:1 to about 1:20, about 5:1 to about 1:20, or about 1:1 to about 1:20. In one example, a 100 $\mu$g dose of agent (e.g., nicotine) and 1:10 ratio yields a volume of 1 mm$^3$ (1 mg). A mixture of agent (e.g., nicotine) and vegetable glycerin can be held in an agent (e.g., nicotine) reservoir (e.g., as a liquid).

In another embodiment, the one or more other substances comprise vegetable glycerin and propylene glycol. The ratio of vegetable glycerin to propylene glycol can be about, more than, less than, or at least 100:1, 95:1, 90:1, 85:1, 80:1, 75:1, 70:1, 65:1, 60:1, 55:1, 50:1, 45:1, 40:1, 35:1, 30:1, 25:1, 20:1, 15:1, 10:1, 5:1, 2:1, 1:1:, 1:2, 1:5, 1:10, 1:15, 1:20, 1:25, 1:30, 1:35, 1:40, 1:45, 1:50, 1:55, 1:60, 1:65, 1:70, 1:75, 1:80, 1:85, 1:90, 1:95, or 1:100. The ratio of agent (e.g., nicotine) to vegetable glycerin can be from about 100:1 to about 1:100, about 75:1 to about 1:100, about 50:1 to about 1:100, about 25:1 to about 1:100, about 25:1 to about 1:50, about 10:1 to about 1:100, about 10:1 to about 1:50, about 10:1 to about 1:20, about 5:1 to about 1:20, or about 1:1 to about 1:20.

The ratio of agent (e.g., nicotine) to mixture of vegetable glycerin and propylene glycol can be about, more than, less than, or at least 100:1, 95:1, 90:1, 85:1, 80:1, 75:1, 70:1, 65:1, 60:1, 55:1, 50:1, 45:1, 40:1, 35:1, 30:1, 25:1, 20:1, 15:1, 10:1, 5:1, 2:1, 1:1:, 1:2, 1:5, 1:10, 1:15, 1:20, 1:25, 1:30, 1:35, 1:40, 1:45, 1:50, 1:55, 1:60, 1:65, 1:70, 1:75, 1:80, 1:85, 1:90, 1:95, or 1:100. The ratio of agent (e.g., nicotine) to vegetable glycerin and glycerin can be from about 100:1 to about 1:100, about 75:1 to about 1:100, about 50:1 to about 1:100, about 25:1 to about 1:100, about 25:1 to about 1:50, about 10:1 to about 1:100, about 10:1 to about 1:50, about 10:1 to about 1:20, about 5:1 to about 1:20, or about 1:1 to about 1:20.

In another embodiment, the one or more other substances can be polyethylene glycol (PEG). The PEG can be PEG200, PEG300, PEG400, PEG600, PEG1000, PEG2000, PEG4000, or PEG6000.

In one embodiment, the one or more other substances is glycerol.

In another embodiment, an electronic agent (e.g., nicotine) delivery device comprises a mixture of agent (e.g., nicotine) and polyethylene glycol. A mixture can comprise an agent (e.g., nicotine), polyethylene glycol, and vegetable glycerin. A mixture can comprise an agent (e.g., nicotine), polyethylene glycol, vegetable glycerin, and propylene glycol. In another embodiment, a mixture comprises an agent (e.g., nicotine), polyethylene glycol, and propylene glycol. A mixture can comprise an agent (e.g., nicotine), propylene glycol, and vegetable glycerin.

In one embodiment, the percentage of an agent (e.g., nicotine) in a formulation (e.g., solution) comprising an agent (e.g., nicotine) can be about, more than, less than, or at least 0.25, 0.5, 0.75, 1, 1.25, 1.5, 1.75, 2, 2.25, 2.5, 2.75, 3, 3.25, 3.5, 3.75, 4, 4.25, 4.5, 4.75, 5, 5.25, 5.5, 5.75, 6, 6.25, 6.5, 6.75, 7, 7.25, 7.5, 7.75, 8, 8.25, 8.5, 8.75, 9, 9.25, 9.5, 9.75, 10, 10.25, 10.5, 10.75, 11, 11.25, 11.5, 11.75, 12, 12.25, 12.5, 12.75, 13, 13.25, 13.5, 13.75, 14, 14.25, 14.5, 14.75, 15, 15.25, 15.5, 15.75, 16, 16.25, 16.5, 16.75, 17, 17.25, 17.5, 17.75, 18, 18.25, 18.5, 18.75, 19, 19.25, 19.5, 19.75, 20, 20.5, 21, 21.5, 22, 22.5, 23, 23.5, 24, 24.5, or 25% by volume. The percentage of an agent (e.g., nicotine) in a formulation (e.g., solution) comprising an agent (e.g., nicotine) can be from about 0.25 to about 1.25, about 1.25 to about 2.5, about 2.5 to about 5, about 5 to about 7.5, about 7.5 to about 10, about 10 to about 12.5, about 12.5 to about 15, about 15 to about 17.5, about 17.5 to about 20, or about 20 to about 25 % by volume. The formulation (e.g., solution) can further comprise one or more substances. The one or more substances can be propylene glycol and/or vegetable glycerin. The formulation can be liquid at room temperature or at temperatures at which the device is generally used by a subject.

The source of nicotine for use in the devices and methods as provided herein can be a tobacco or tobacco material. Here, a tobacco or tobacco material can be defined as any combination of natural and synthetic material that can be vaporized for pleasure or medicinal use. The formulation comprising nicotine can comprise flue-cured tobacco, glycerin, and flavorings. The formulation comprising nicotine can comprise flue-cured tobacco, propylene glycol, and flavorings. A liquid formulation comprising nicotine can be produced by chopping tobacco into fine pieces (less than 3 mm diameter, less than 2 mm), adding the other ingredients (e.g., propylene glycol, vegetable glycerin, water, and/or flavorings), and mixing until even consistency is achieved.

## pH

An electronic agent (e.g., nicotine) delivery device described herein can control a pH of an agent (e.g., nicotine) mixture or aerosol. The pH of the agent (e.g., nicotine) mixture or aerosol can be about, more than, less than, or at least 1, 1.5, 2, 2.5, 3, 3.5, 4, 4.5, 5, 5.5, 5.6, 5.7, 5.8, 5.9, 6, 6.1, 6.2, 6.3, 6.4, 6.5, 6.6, 6.7, 6.8, 6.9, 7, 7.1, 7.2, 7.3, 7.4, 7.5, 7.6, 7.7, 7.8, 7.9, 8, 8.1, 8.2, 8.3, 8.4, 8.5, 8.6, 8.7, 8.8, 8.9 9, 9.5, 10, 10.5, 11, 11.5, 12, 12.5, 13, 13.5, or 14. In some cases, the pH of an agent (e.g., nicotine) mixture or aerosol can be about 1 to about 14, about 2 to about 13, about 3 to about 12, about 4 to about 11, about 5 to about 10, about 6 to about 9, about 6 to about 8, about 6 to about 7, about 4 to about 6, about 4 to about 8, about 5 to about 8, about 5 to about 7, about 7 to about 9, about 5.5 to about 8.5, about 6.5 to about 8.5, about 6.5 to about 7.5, about 7.5 to about 9, or about 7 to about 8.5. One or more buffers can be added to a mixture to adjust the pH of the mixture.

The one or more buffers can be any buffers known in the art. The one or more buffers can be acidic buffers or alkaline buffers. The one or more buffers can be a phosphate, bicarbonate or protein buffer system. Examples of buffers can include, but are not limited to, TAPS (3-{[tris(hydroxymethyl)methyl]amino}propanesulfonic acid), Bicine (N,N-bis(2-hydroxyethyl)glycine), Tris (tris(hydroxymethyl)methylamine), Tricine (N-tris(hydroxymethyl)methylglycine), TAPSO (3-[N-Tris(hydroxymethyl)methylamino]-2-hydroxypropanesulfonic Acid), HEPES (4-2-hydroxyethyl-1-piperazineethanesulfonic acid), TES (2-{[tris(hydroxymethyl)methyl]amino}ethanesulfonic acid), MOPS (3-(N-morpholino)propanesulfonic acid), PIPES (piperazine-N,N'-bis(2-ethanesulfonic acid)), cacodylate (dimethylarsinic acid), SSC (saline sodium citrate), MES (2-(N-morpholino)ethanesulfonic acid), succinic acid (2(R)-2-(methylamino)succinic acid), sodium acetate/acetic acid, sodium citrate/citric acid, CHES, sodium borate/boric acid, diethyl barbituric acid, potassium dihydrogen phosphate, Carmody buffer, Britton-Robinson buffer, or mixtures thereof.

## Flavorings

In one embodiment, a mixture comprises one or more flavorings. The one or more flavorings can be a flavor offered by, e.g., Flavourart (Italy), Flavor Apprentice, or LorAnn. A flavor can be, e.g., almond, almond amaretto, apple, Bavarian cream, black cherry, black sesame seed, blueberry, brown sugar, bubblegum, butterscotch, cappuccino, caramel, caramel cappuccino, cheesecake (graham crust), cinnamon redhots, cotton candy, circus cotton candy, clove, coconut, coffee, clear coffee, double chocolate, energy cow, graham cracker, grape juice, green apple, Hawaiian punch, honey, Jamaican rum, Kentucky bourbon, kiwi, koolada, lemon, lemon lime, tobacco, maple syrup, maraschino cherry, marshmellow, menthol, milk chocolate, mocha, Mountain Dew, peanut butter, pecan, peppermint, raspberry, banana, ripe banana, root beer, RY4, spearmint, strawberry, sweet cream, sweet tarts, sweetner, toasted almond, tobacco, tobacco blend, vanilla bean ice cream, vanilla cupcake, vanilla swirl, vanillin, waffle, Belgian waffle, watermelon, whipped cream, white chocolate, wintergreen, amaretto, banana cream, black walnut, blackberry, butter, butter rum, cherry, chocolate hazelnut, cinnamon roll, cola, creme de menthe, eggnog, English toffee, guava, lemonade, licorice, maple, mint chocolate chip, orange cream, peach, pina colada, pineapple, plum, pomegranate, pralines and cream, red licorice, salt water taffy, strawberry banana, strawberry kiwi, tropical punch, tutti frutti, or vanilla. The number of flavors in a mixture can be about, more than, less than, or at least 1, 2, 3, 4, 5, 6, 7, 8, 9, 10.

A flavoring can be used to pair nicotine administration with certain gustatory and/or olfactory sensations. Subsequent administration of agent (e.g., nicotine) doses can be reduced while retaining the flavoring to help the user reduce their agent (e.g., nicotine) dependency and enable cravings to be fully or partially sated using the flavoring as a conditioned stimulus.

## Particle size

A device provided herein can generate an aerosol that can be particles of an optimum size for delivery to the deep lung. The aerosol can be a condensation aerosol. The particle size can be about, more than, less than, or at least 0.01, 0.015, 0.02, 0.025, 0.03, 0.035, 0.04, 0.045, 0.05, 0.055, 0.06, 0.065, 0.07, 0.075, 0.08, 0.085, 0.09, 0.095, 0.1, 0.11, 0.12, 0.13, 0.14, 0.15, 0.16, 0.17, 0.18, 0.19, 0.2, 0.21, 0.22, 0.23, 0.24, 0.25, 0.26, 0.27, 0.28, 0.29, 0.3, 0.31, 0.32, 0.33, 0.34, 0.35, 0.36, 0.37, 0.38, 0.39, 0.4, 0.41, 0.42, 0.43, 0.44, 0.45, 0.46, 0.47, 0.48, 0.49, 0.5, 0.51, 0.52, 0.53, 0.54, 0.55, 0.56, 0.57, 0.58, 0.59, 0.6, 0.61, 0.62, 0.63, 0.64, 0.65, 0.66, 0.67, 0.68, 0.69, 0.7, 0.71, 0.72, 0.73, 0.74, 0.75, 0.76, 0.77, 0.78, 0.79, 0.8, 0.81, 0.82, 0.83, 0.84, 0.85, 0.86, 0.87, 0.88, 0.89, 0.9, 0.91, 0.92, 0.93, 0.94, 0.95, 0.96, 0.97, 0.98, 0.99, 1, 1.5, 2, 2.5, 3, 3.5, 4, 4.5, 5, 5.5, 6, 6.5, 7, 7.5, 8, 8.5, 9, 9.5, 10, 10.5, 11, 11.5, 12, 12.5, 13, 13.5, 14, 14.5, 15, 15.5, 16, 16.5, 17, 17.5, 18, 18.5, 19, or 20 microns. The particle size can be from about 1 to about 10 microns, about 1 to about 9 microns, about 1 to about 7 microns, about 1 to 6 microns, about 1 to about 5 microns, about 1 to about 4 microns, about 1 to about 3 microns, or about 1 to about 2 microns. The particle size can be from about 0.5 to about 10 microns, about 0.5 to about 9.5 microns, about 0.5 to about 9 microns, about 0.5 to about 8.5 microns, about 0.5 to about 8 microns, about 0.5 to about 7.5 microns, about 0.5 to about 7microns, about 0.5 to about 6.5 microns, about 0.5 to about 6 microns, about 0.5 to about 5.5 microns, about 0.5 to about 5 microns, about 0.5 to about 4.5 microns, about 0.5 to about 4.0 microns, about 0.5 to about 3.5 microns, about 0.5 to about 3 microns, about 0.5 to about 2.5 microns, about 0.5 to about 2 microns, about 0.5 to about 1.5 microns, or about 0.5 to about 1 microns. The particle size can be less than 1 micron. The particle size can be greater than 5 microns. The particle size can be less than 5 microns. The particle size can be greater than 1 micron. In one embodiment, the particle size is from about 1 to about 5 microns. In one embodiment, the particle size is from about 1 to about 3 microns. In one embodiment, the particle size is a mass median aerodynamic diameter (MMAD).

A device provided herein can generate an aerosol that can create particles of an optimum size for delivery to the deep lung. The geometric standard deviation (GSD) of the particles can be about, more than, less than, or at least 1, 1.1, 1.2, 1.3, 1.4, 1.5, 1.6, 1.7, 1.8, 1.9, 2, 2.1, 2.2, 2.3, 2.4, 2.5, 2.6, 2.7, 2.8, 2.9, or 3. The GSD can be from about 1 to about 3 or about 1 to about 2. The GSD can be between about 1 to about 1.5, about 1.5 to about 2, or about 2 to about 3. In one embodiment, the GSD is less than 2.

## Agent (e.g., nicotine) reservoir

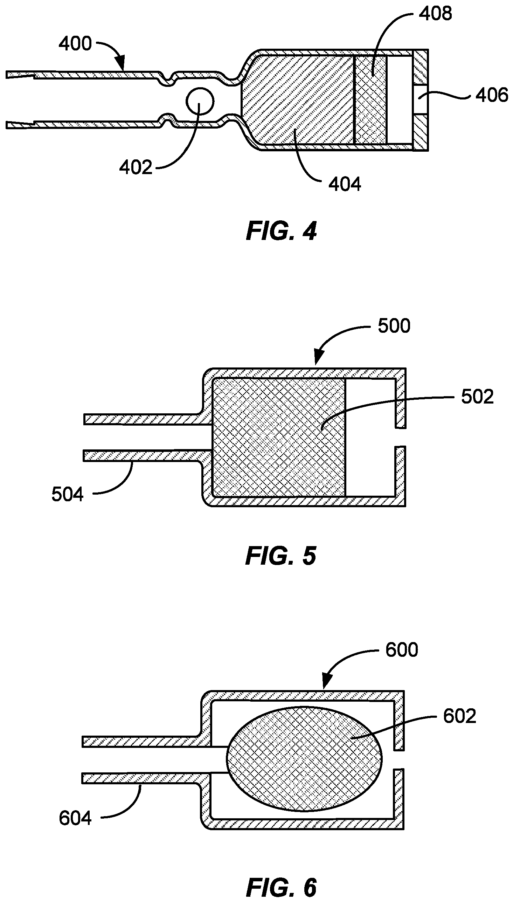

FIG. 4 illustrates an embodiment of an agent (e.g., nicotine) reservoir (404) that can be used in an electronic agent (e.g., nicotine) delivery device provided herein. A tube, e.g., capillary tube (400) with a valve (402) does not need to be inserted into a separate reservoir, but can be the reservoir (404) itself by extending away from the ejection end. The diameter of the tube, e.g., capillary tube, can be increased to store more mixture. To allow for the mixture to be pulled from the reservoir without creating a low pressure, which could resist the mixture leaving, the back end can have a vent (406). To stop an agent (e.g., nicotine) from vaporizing or evaporating from the back end a section of the reservoir could be filled with a soft material such as a wax or grease plug. This plug (408) can be drawn along the reservoir as the mixture is used. In one embodiment, the agent (e.g., nicotine) reservoir is cylindrical. In one embodiment, the agent (e.g., nicotine) reservoir holds a formulation comprising 200 mg of agent (e.g., nicotine) mixed with 1000 mg of propylene glycol. In one embodiment, the agent (e.g., nicotine) reservoir holds a formulation comprising 200 ug of agent (e.g., nicotine) mixed with 1000 ug of propylene glycol. In some cases, the agent (e.g., nicotine) formulation is a liquid formulation.

FIG. 5 illustrates another embodiment of a reservoir. An agent (e.g., nicotine) reservoir (500) can be a porous, open cell foam (502) within a cartridge; a tube, e.g., capillary tube (504) can extend from the reservoir.

**FIG. 6** illustrates another embodiment of an agent (e.g., nicotine) reservoir. The mixture can be held in a collapsible bag (**602**) which can be held within a secondary container (**600**). A tube, e.g., capillary tube (**604**) can extend from the reservoir.

In one embodiment, doses of a liquid agent (e.g., liquid nicotine) are held in a safe dose cartridge container until needed. A container for an agent (e.g., nicotine) can comprise a sealing mechanism that can keep the agent (e.g., nicotine) in the container even if the container is crushed. In one embodiment, the sealing mechanism comprises septum sealing. Methods are provided herein for safely puncturing and reclosing access to a drug (e.g., nicotine) cartridge. In one embodiment, a septum and a puncturing needle is used to extract an agent (e.g., nicotine) from a cartridge. A semi-porous material can be used to ensure that the rate of agent (e.g., nicotine) transfer is safe. For example, materials can include a frit or other material (e.g., ceramic, foam, or metal) that has a convoluted or open structure.

In one embodiment, a device comprises a dose cartridge. In one embodiment, the dose cartridge is a disposable dose cartridge. In another embodiment, the dose cartridge houses an agent (e.g., nicotine) formulation and an aerosol creation mechanism as described herein. In another embodiment, the agent (e.g., nicotine) formulation is housed in a reservoir. In one embodiment, the dose cartridge comprises a reservoir comprising an agent (e.g., nicotine) formulation. In one embodiment, the dose cartridge comprises a reservoir comprising an agent (e.g., nicotine) formulation and dispensing tube, e.g., capillary tube, for dispensing the agent (e.g., nicotine) formulation. In one embodiment, the dose cartridge comprises a mouthpiece. In another embodiment, the mouthpiece comprises a cap. The cap can help prevent contamination. The cap can provide a tamper resistance feature. The cap can provide a child resistance feature. In one embodiment, the cap covers both the mouthpiece and any air inlets. In another embodiment, the cap is reusable. In one embodiment, the dose cartridge comprises a mouthpiece at one end and a mating mechanism whereby the dose cartridge can connect to a controller at another end. In one embodiment, the dose cartridge comprises a mechanism for breath detection. In one embodiment, the dose cartridge comprises a flow control valve. In one embodiment, the dose cartridge comprises a flow control valve that can regulate inhalation. The mechanism for breath detection or inhalation sensing can comprise breath sensory components. The breath sensory components can comprise an optical chase whereby light can be routed to and from a flow sensor.

In one embodiment, the dose cartridge comprises a heater element. In one embodiment, the heater element comprises a metal foil. The metal foil can be made of stainless steel or any other electrically resistive material. In one embodiment, the metal foil is made of stainless steel. In one embodiment, the heater element comprises a steel or metal foil that can be about 0.013 mm thick in order to ensure rapid vaporization. In one embodiment, the heater element comprises a coil of wire or wire coil. The coil of wire or wire coil can be from about 0.12 to about 0.5 mm in diameter. In another embodiment, the dose cartridge comprises more than one heater element. In one embodiment, the dose cartridge comprises two heater elements. In some cases, the heater element can be rapidly heated. In one embodiment, a heating element can comprise a heating rate of about 1600° C (1873.15°K) per second for a duration of 250 msec, which can cause a 400° C (673.15°K) rise in the temperature of the heater element. In some cases, a heater element is activated for a duration of about 10 msec to about 2000 msec, about 10 msec to about 1000 msec, about 10 msec to about 500 msec, about 10 msec to about 250 msec, about 10 msec to about 100 msec, about 50 msec to about 1000 msec, about 50 msec to about 500 msec, about 50 msec to about 250 msec, about 100 msec to about 1000 msec, about 100 msec to about 500 msec, about 100 msec to about 400 msec, or about 100 msec to about 300 msec. In some cases, a heater element is activated for about 10, 50, 100, 150, 200, 250, 300, 350, 400, 450, 500, 550, 600, 650, 700, 750, 800, 850, 900, 950, or 1000 msec. In some cases, a heater element is activated for at least 10, 50, 100, 150, 200, 250, 300, 350, 400, 450, 500, 550, 600, 650, 700, 750, 800, 850, 900, 950, or 1000 msec. In some cases, the maximum temperature of the heater element is about 100, 150, 200, 250, 300, 350, 400, 450, 500, 550, 600°C (a range from about 373.15°K to about 873.15°K). In some cases, the maximum temperature of the heater element is at least 100, 150, 200, 250, 300, 350, 400, 450, 500, 550, 600°C (a range from about 373.15°K to about 873.15°K).

In one embodiment, a device provided herein is made up of multiple components. In one embodiment, the device provided herein is comprised of two components wherein one component comprises a controller and the other component comprises a dose cartridge. In a further embodiment, the controller is reusable and the dose cartridge is replaceable. In yet another embodiment, the dose cartridge is mated to the controller. Mating of the dose cartridge to the controller can be accomplished by inserting the dose cartridge into an interlocking channel in the controller and engaging a locking mechanism. The locking mechanism can comprise a tab or button on the controller which can be depressed. In one embodiment, the dose cartridge is detachable from the controller. In one embodiment, detachment of the dose cartridge is accomplished by releasing the locking mechanism. In one embodiment, releasing the locking mechanism entails depressing the tab or button on the controller. Electrical connection between the dose cartridge and the controller can be accomplished through a set of mating electrical contacts. In one embodiment, attachment or mating of the dose cartridge to the controller establishes a breath detection mechanism. The breath detection mechanism can comprise breath sensory components. In one embodiment, the breath detection mechanism comprises detecting an alteration in an optical signal, wherein attachment or mating of the dose cartridge to the controller establishes an optical path through which the optical signal can be sent and received. In one embodiment, a source and detector of an optical signal is present in the controller, while the dose cartridge comprises an optical path. The optical path can comprise reflectors for reflecting an optical signal. The optical path can comprise a vane, wherein an inhalation can move the vane in such a way as to cause an alteration in an optical signal. In one embodiment, the dose cartridge comprises a vane, wherein an inhalation can move the vane in such a manner as to cause an alteration in an optical signal. The optical signal can be light of any wavelength.

## Tube, e.g., Capillary Tube

**FIGs. 2A** and **2B** illustrate embodiments of components of an electronic nicotine delivery device. **FIG. 2A** illustrates an agent (e.g., nicotine) reservoir (**202**) and a tube, e.g., capillary tube (**204**). **FIG. 2B** illustrates an expanded view of the device. The agent (e.g., nicotine) reservoir can comprise an agent (e.g., nicotine)/propylene glycol (PG) mixture (**206**). The tube, e.g., capillary tube can comprise a region on the interior which has been coated with an agent (e.g., nicotine)/PG philic material (**208**) to promote wicking out of a reservoir. A region on the interior which has been coated with an agent (e.g., nicotine)/PG phobic material (**210**) (such as polytetrafluoroethylene (PTFE)) can lie at the open end. This coating can cause the agent (e.g., nicotine)/PG to stop wicking short of the open end, thereby reducing the surface area of the mixture exposed to air, and air devoid of agent (e.g., nicotine) vapor. The tube, e.g., capillary tube can comprise a heated section (**212**) of the tube, e.g., capillary tube which, upon heating, can cause the mixture in the tube to vaporize and expand, pushing the mixture from the open end. A ball valve (**214**) can be trapped between two indentations in the tube, e.g., capillary tube, the end indentation being such that the ball, if pushed by fluid, will form a seal. This configuration can allow the liquid to be ejected from the end upon heating rather than back into the reservoir. All four of these elements can form a pump which can eject a known dose of the mixture from the end of the tube, e.g., capillary tube.

To eject a dose of an agent (e.g., nicotine)/PG mix with a 1:10 ratio, 1 mm$^3$ of material can be in the tube, e.g., capillary tube. For a tube, e.g., capillary tube with an interior diameter of 0.5 mm, the length can be ~5 mm.

## Valve

A valve can be a check valve, and the check valve can be a ball which can be made of a metal, such as stainless steel or can be made of a plastic, such as nylon, delrin, or a homopolymer acetal. The ball can have a diameter less than the interior diameter of the tube, e.g., capillary tube sufficient to allow an agent (e.g., nicotine)/PG mix to wick by it.

## Heater element

A heater element can be any heater element as provided herein. The heater element can be used to generate a condensation aerosol from a liquid formulation comprising a pharmaceutically active agent as provided herein. The condensation aerosol can comprise particles of a size suitable for delivery to the lungs of a subject as provided herein. In some cases, the heater element comprises a coil. The coil can be a wire coil. The coil can further comprise a wick element capable of being heated. The heater element can vaporize the liquid formulation when the liquid formulation is delivered to the heater element. In some cases, the liquid formulation comprising a pharmaceutically active agent (e.g., nicotine) is delivered to the heater element through the use of a positive displacement pump. The positive displacement pump can be a reciprocating, metering, rotary-type, hydraulic, peristaltic, gear, screw, flexible impeller, diaphragm, piston, or progressive cavity pump, or any other pump utilizing positive displacement as known in the art. The positive displacement pump can be in fluid communication with the heater element. The positive displacement pump can be in fluid communication or fluidically coupled to a reservoir comprising a pharmaceutically active agent (e.g., nicotine). The positive displacement pump can be in fluid communication with the heater element and a reservoir comprising a pharmaceutically active agent (e.g., nicotine). The pharmaceutically active agent (e.g., nicotine) can be a liquid formulation. The positive displacement pump can be within the passageway or external to the passageway. The heater element can comprise a rod comprising electrically resistive material. The rod can be a wire. The rod can be a pliable rod. The rod can comprise a coil, wherein a segment of the rod can pass through the interior of the coil. The coil can be a wire coil. The heater element can comprise a wick element capable of being heated. The wick element capable of being heated can pass through and exit the center of the coil. In some cases the heater element comprises a heatable wick element. The rod can comprise the heatable wick element. In some cases, the rod comprises a coil and a heatable wick element, wherein the heatable wick element is a segment of the rod that passes through the interior or center of the coil.

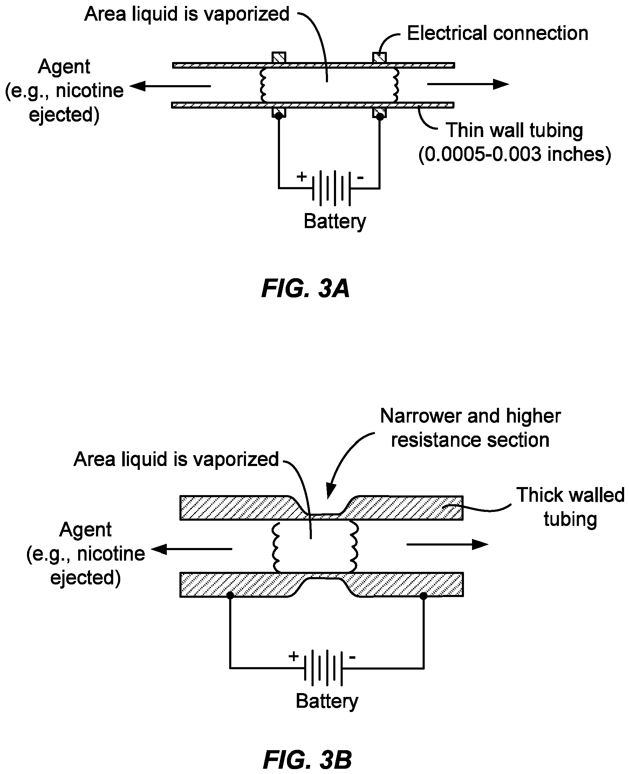

**FIGs. 3A** and **3B** illustrate configurations of a heater element. The tube, e.g., capillary tube can be made of stainless steel, or a similar matter, which has an electrical resistance substantially greater than other metals (aluminum, brass, iron). The tube, e.g., capillary tube can be made of a thin wall material (**FIG. 3A**), or a section of the wall can be narrowed (**FIG. 3B**) to result in that section having an electrical resistance such that when an electrical current is passed across the section heating happens. Alternately the tube, e.g., capillary tube can be wrapped with a heater wire. This configuration can allow for the tube, e.g., capillary tube to be made of a non-electrically conductive material such as Kapton (polyimide), which can withstand heat. Electrical heating can be powered directly from a battery or can be powered from a charged capacitor.

A heater element can be used to vaporize an agent (e.g., nicotine)/PG mixture to form an aerosol with a particle size (MMAD= Mass Median Aerodynamic Diameter) of about 1 to about 5 $\mu$m. Aerosols with this particle size can deposit in the deep lung and result in rapid PK.

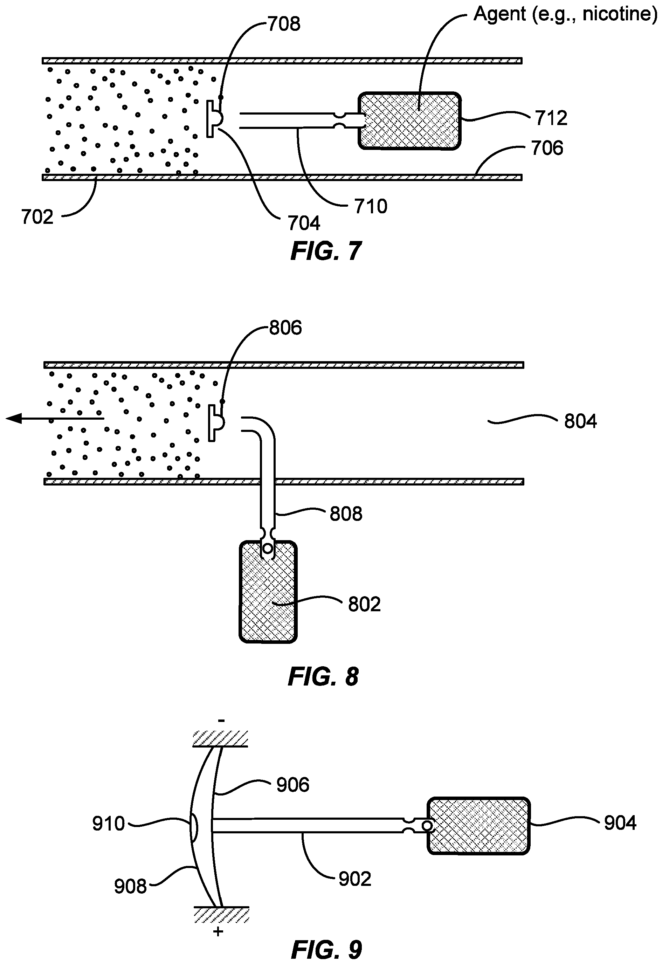

FIG. 7 illustrates a configuration of a heater element (**704**) in an airway (**706**). The heater element can be made of a thin stainless steel foil. The foil can be of a thickness of about 0.0005 to about 0.005 inches (a range from about 0.01 mm to about 0.13 mm) thick, or from about 0.0005 to about 0.001 inches (a range from about 0.01 mm to about 0.025 mm) so that less electrical current is needed to vaporize the mixture. The foil can be of a thickness of about, less than, more than, at least or at most 0.0005, 0.0006, 0.0007, 0.0008, 0.0009, 0.001, 0.002, 0.003, 0.003, 0.004, or 0.005 inches (a range from about 0.01 mm to about 0.13 mm). The heater element (**704**) can be positioned at the exit of the tube, e.g., capillary tube (**710**) so that the mixture can deposit (**708**) on the heater element (**704**). The heater element (**704**) can be positioned in an airway (**706**) so that a user upon inhalation can cause the aerosol to pass through the mouthpiece (**702**) and be drawn into the lungs. The agent (e.g., nicotine) reservoir (**712**) can be in the airway. **FIG. 8** illustrates that in some cases, an agent (e.g., nicotine) reservoir (**802**) can be placed outside of an airway (**804**), while the heater element (**806**) can be in the airway (**804**). A tube, e.g., capillary tube (**808**) can enter the airway (**804**).

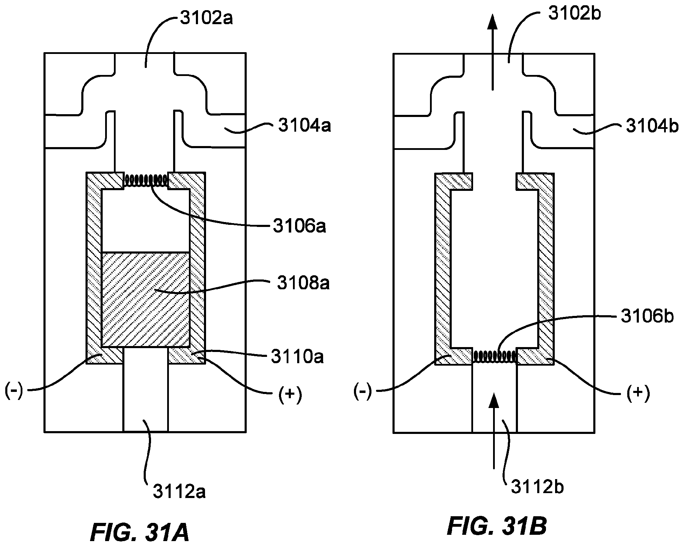

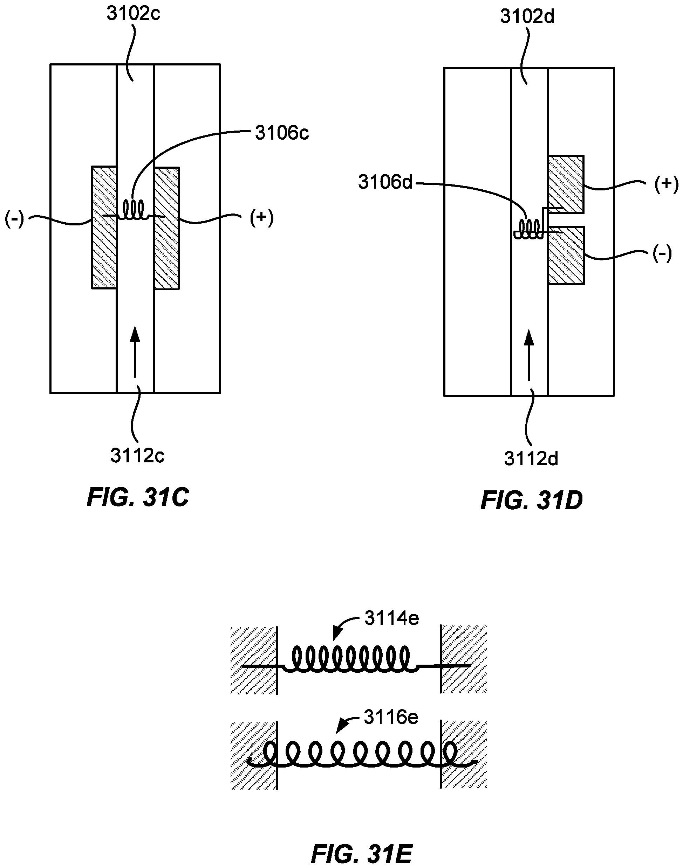

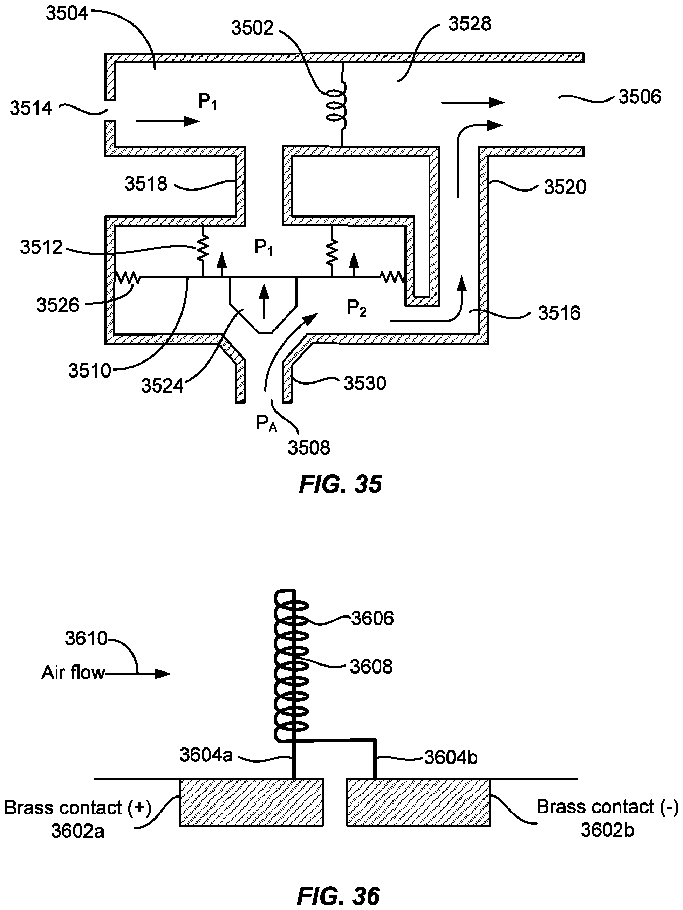

FIGs. 31A-D illustrates another configuration of a heater element (3106a-d) in an airway (**3112a-d**). FIG. 31A depicts a device (ENT-100-A), comprising a primary carrier gas inlet (**3112a**), positive and negative brass contacts (**3110a**), a heater element (**3106a**) comprising a coil located distally from the inlet to the primary airway (**3112a**) and two bypass inlets (**3104a**) located (disposed) downstream of the heater element but prior to the outlet (**3102a**). **FIG. 31B** depicts a device designated ENT-100-B, which is the same as ENT-100-A except that the heater element has been moved to be proximal to the inlet of the primary airway (**3112b**). **FIG. 31C** depicts a device designated ENT-100-C, which is similar to the ENT-100-A device except that the wire coil heater element has been moved to an intermediate position relative to the location of the coil in ENT-100-A and ENT-100-B. Any of the devices depicted in **FIG. 31A-C** can comprise the wire coil heater element designated "A Coil" (**3114e**) or "B Coil" (**3116e**) as illustrated in **FIG. 31E**. The coil in both types of heater elements comprise inner diameter of 0.26 inches (about 6.6 mm). The "A Coil" comprises a stretch of coil followed by a straight lead on either end of the coil which connects to the brass contacts. The "B Coil" comprises a stretch of coil, wherein the coil itself connects to the brass contacts. **FIG. 31D** depicts a device designated ENT-100-D with a primary passageway (**3112d**) for air to flow through, brass contacts (+/-) embedded within the wall of the primary passageway, and a heater element (**3106d**) comprising a wire wherein one end of the wire wraps around another segment of the wire, wherein a wire coil is formed with an end of the wire passes through the center of the wire coil. An example of this type of heater element is shown in **FIGs. 36-38.** In some cases, a liquid formulation comprising a pharmaceutically active agent (e.g., nicotine) is delivered to the heater element of **FIGs. 31A-D** from a reservoir comprising the liquid formulation comprising a pharmaceutically active agent (e.g., nicotine) through the use of a tube, e.g., capillary tube as provided herein, wherein the tube, e.g., capillary tube is coupled or capable of being coupled to the reservoir. In some cases, a liquid formulation comprising a pharmaceutically active agent (e.g., nicotine) is delivered to the heater element of **FIGs. 31A-D** from a reservoir comprising the liquid formulation comprising a pharmaceutically active agent (e.g., nicotine) through the use of a positive displacement pump as provided herein, wherein the positive displacement pump is fluidically coupled to the reservoir.

FIG. 9 illustrates another embodiment for a heater element. To aid in reducing an agent (e.g., nicotine) from evaporating from the end of a tube, e.g., capillary tube (**902**) (attached to an agent (e.g., nicotine) reservoir (**904**)), the heater element (**906**) can be positioned to cover the end of the tube, e.g., capillary tube when cold. Upon heating the heater would move away from the end (**908**) due to thermal expansion, opening up the end and allowing the mixture to leave. The position of deposited material (**910**) is shown.

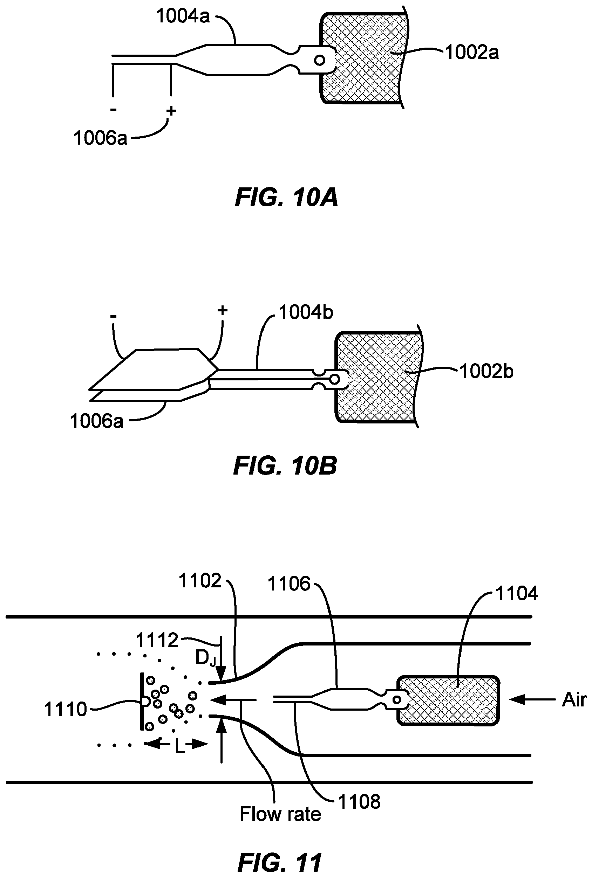

FIGs. 10A and 10B illustrate additional configurations of a heater element. FIG. 10A illustrates that a heater element (**1006a**) can be positioned at the end of the tube, e.g., capillary tube (**1004a**), where the tube, e.g., capillary tube can be attached to an agent (e.g., nicotine) reservoir (**1002a**). **FIG. 10B** illustrates an agent (e.g., nicotine) reservoir (**1002b**) and a tube, e.g., capillary tube (**1004b**), where the geometry of the tube, e.g., capillary tube is modified at the end (**1006b**) by narrowing or flattening to aid in vaporization.

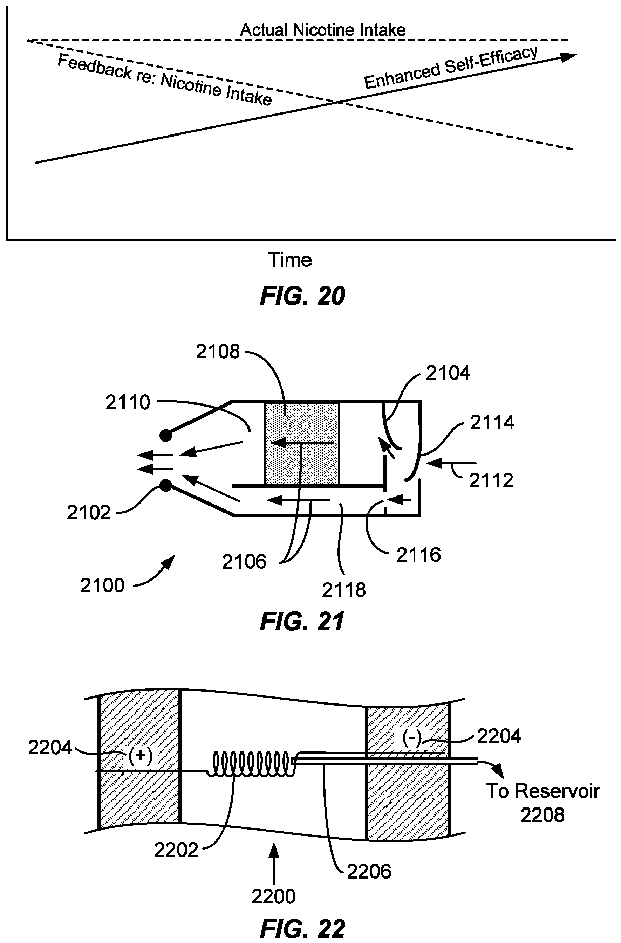

FIG. 22 illustrates another embodiment of a heater element. The heater element (**2200**) can be a rod comprising a coil (**2202**) that can be made of stainless steel, or a similar matter, which has an electrical resistance substantially greater than other metals (aluminum, brass, iron). In some cases, the rod is a wire, wherein the coil is a wire coil. The rod can comprise an electrically resistive material. The electrically resistive material can have an electrical resistance such that when an electrical current is passed across the rod heating happens. The rod is connected to brass contacts (**2204**) through segments of the rod that do not form the coil. In some cases, the segments of the rod that connect to the brass contacts comprise leads. The brass contacts can serve to pass electrical current across the rod, including the coil. The electrical current can serve to heat the coil and vaporize material (i.e. an agent (e.g., nicotine) mixture) that contacts or is delivered to the coil. The coil can be an open coil that can allow for air to flow between the coils and carry away the vaporized material. In **FIG. 22,** the brass contacts (**2204**) are located (disposed) on either side of an airflow channel and the rod, including the coil, span the channel. In some cases, the coil can be oriented parallel to the flow of a carrier gas (e.g, air). In some cases, the coil can be oriented perpendicular to the flow of a carrier gas (e.g., air). In **FIG. 22,** a tube, e.g., capillary tube (**2206**) attached to a reservoir (**2208**) comprising an agent (e.g., nicotine) mixture is located at one end of the coil and an agent (e.g., nicotine) mixture is dispensed from the end of the tube, e.g., capillary tube onto the coil. The agent (e.g., nicotine) mixture, once dispensed, can wick along the coil to cover the entire or part of the coil. The coil can be heated which can vaporize the agent (e.g., nicotine) mixture.

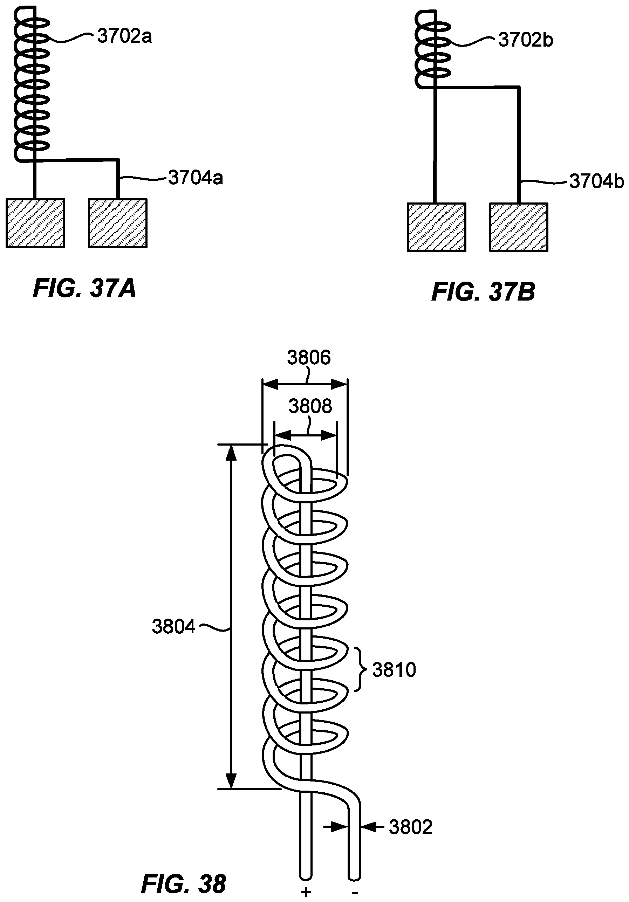

**FIGs. 36-38** illustrate yet another embodiment of a heater element. In this embodiment, a first (**3602a**; +) and a second (**3602b**; -) brass contact or terminal are located adjacent to each other. The brass contacts can be embedded within or placed proximal to a wall of a housing or channel of a device for generating an aerosol as provided herein. The heater element can be a rod comprising electrically resistive material, wherein a first end or lead (**3604a**) is connected to one brass contact (**3602a**; +), while a second end or lead (**3604b**) is connected to another, separate brass contact (**3602b**; -). As illustrated in **FIG. 36,** a portion or segment of the rod between the leads is configured into a coil (**3606**). In addition, a separate portion or segment (**3608**) of the rod passes through the interior of the coil (**3606**). Supplying current to the rod through the brass contacts (**3602a,b**) can serve to heat both the coil (**3606**) as well as the segment (**3608**) of the rod that passes through the interior of the coil (**3606**). In some cases, the segment of the rod that runs through the center of the coil is capable of holding a liquid formulation comprising an agent (i.e. nicotine) as provided herein. The liquid formulation can wick or be delivered by any of dosing mechanisms provided herein onto the segment of the rod that runs through the center of the coil from a source of the liquid formulation (e.g., a reservoir). In some cases, supplying current to the rod through the brass contacts (**3602a,b**) serves to heat both the coil (**3606**) as well as the segment (**3608**) of the rod that passes through the interior of the coil (**3606**), wherein a liquid formulations that wicks or is delivered by any of dosing mechanisms provided herein onto the segment of the rod running through the coil is vaporized. In **FIG. 36,** the coil is oriented perpendicular to the the flow of a carrier gas (e.g. air flow) (**3610**). In some cases, the coil is oriented parallel to the flow of a carrier gas (e.g. air flow) in a device for generating a condensation aerosol as described herein. **FIGs. 37A** and **37B** depict alternate embodiments to the heater element illustrated in **FIG. 36,** wherein the number of coils shown in the heater element of **FIG. 37A** is reduced in the heater element of **FIG. 37B.** As shown in **FIG. 37,** alternating the number of coils (**3702b, 3702b**) in the coil serves to increase the length of the non-coil segments (**3704a, 3704b**) of the rod and decrease the length of the rod covered by the coil. **FIG. 38** illustrates components of the rod and coil in the heater element illustrated in **FIG. 36,** including the diameter of the rod (**3802**), total length of the coil (**3804**) (e.g., 0.1 to 0.15 inches (a range from about 2.54 mm to about 3.81mm)), inner diameter of the coil (**3808**) (e.g., 0.027-0.040 inches (about 0.6 mm to about 1.02 mm)), outer diameter of the coil (**3806**) (e.g., 0.047-0.06 inches (a range from about 1.19 mm to about 1.53 mm)), and pitch of the coil (**3810**).

An electronic agent (e.g., nicotine) delivery device provided herein can comprise a heater element comprising a coil, wherein the coil comprises electrically resistive material. The coil can be a wire coil. The electrically resistive material can include but is not limited to: semiconductors such as doped ceramics, electrically conductive ceramics such as molybdenum disilicide, carbon, graphite, metals, metal alloys and composite materials made of a ceramic material and a metallic material. Such composite materials may comprise doped or undoped ceramics. Examples of suitable doped ceramics include doped silicon carbides. Examples of suitable metals include titanium, zirconium, tantalum and metals from the platinum group. Examples of suitable metal alloys include stainless steel-, Constantan-, nickel-, cobalt-, chromium-, aluminium- titanium- zirconium-, hafnium-, niobium-, molybdenum-, tantalum-, tungsten-, tin-, gallium-, manganese- and iron-containing alloys, and super-alloys based on nickel, iron, cobalt, stainless steel, Timetal® and iron-manganese-aluminium based alloys. Timetal® is a registered trade mark of Titanium Metals Corporation, 1999 Broadway Suite 4300, Denver Colorado. In composite materials, the electrically resistive material can optionally be embedded in, encapsulated or coated with an insulating material or vice-versa, depending on the kinetics of energy transfer and the external physicochemical properties required. In one embodiment, the coil comprises stainless steel.

A heater element comprising a rod as provided herein can comprise a coil and a wick element around which the coil can be wrapped. The wick element can be capable of being heated. The wick element can be connected to the rod. The wick element can be independent of the rod. In some cases, the wick element is capable of being heated, and wherein the wick element is connected to the rod. The rod can be a wire. The coil can be a wire coil. The rod can comprise a coil along the entire length of the wick element. The wick element can be capable of wicking or holding a liquid formulation comprising an agent as provided herein. The wick element can be a capillary (a self wicking tube). The liquid formulation comprising an agent as provided herein can be in fluid communication with a source of the liquid formulation. The source of the liquid formulation can be any source as provided herein, including but not limited to, a reservoir. The liquid formulation comprising an agent as provided herein can be delivered to the wick element by any means known in the art. The delivery can be through capillary action or through the use of a pump. In some cases, the rod comprises a capillary wherein the capillary is in fluid communication with a reservoir, wherein the reservoir comprises a liquid formulation comprising a pharmaceutically active agent (e.g. nicotine), and wherein the capillary is capable of holding the liquid formulation comprising a pharmaceutically active agent (e.g. nicotine). The wick element can be made of any material known in the art capable of wicking or holding a liquid formulation comprising an agent as provided herein. In some cases, the coil connects to a source of electricity. The coil can connect to the source of electricity through one or more leads protruding from both ends of the coil. The source of electricity can be a battery or a charged capacitor. The battery can be rechargeable.

In some cases, the coil can wrap around or span exactly, about, more than, less than, at least, or at most 1%, 2%, 3%,4%,5%, 6%, 7%, 8%, 9%, 10%, 11%, 12%, 13%, 14%, 15%, 16%, 17%, 18%, 19%, 20%, 21%, 22%, 23%, 24%, 25%, 26%, 27%, 28%, 29%, 30%, 31%, 32%, 33%, 34%, 35%, 36%, 37%, 38%, 39%, 40%, 41%, 42%, 43%, 44%, 45%, 46%, 47%, 48%, 49%, 50%, 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99%, 100% of the length of the wick element. In some cases, the coil can wrap around or span between 1-10%, 10-20%, 20-30%, 30-40%, 40-50%, 50-60%, 60-70%, 70-80%, 80-90%, or 90-100%, 10-20% of the length of the wick element. In some cases, the coil can wrap around or span of about 1 to about 10%, about 10 to about 20%, about 20 to about 30%, about 30 to about 40%, about 40 to about 50%, about 50 to about 60%, about 60 to about 70%, about 70 to about 80%, about 80 to about 90%, or about 90 to about 100% of the length of the wick element.

The total length of the coil can be exactly, about, more than, less than, at least, or at most 0.01, 0.0125, 0.015, 0.0175, 0.02, 0.0225, 0.025, 0.0275, 03, 0.0325, 0.035, 0.0375, 0.04, 0.0425, 0.045, 0.0475, 0.05, 0.0525, 0.055, 0.0575, 0.06, 0.0625, 0.065, 0.0675, 0.07, 0.0725, 0.075, 0.0775, 0.08, 0.0825, 0.085, 0.0875, 0.09, 0.0925, 0.095, 0.0975, 0.1, 0.11, 0.115, 0.12, 0.125, 0.13, 0.135, 0.14, 0.145, 0.15, 0.155, 0.16, 0.165, 0.17, 0.175, 0.18, 0.185, 0.19, 0.195 or 0.2 inches (a range from about 0.25 mm to about 5.08 mm). The total length of the coil can be between 0.01- 0.015, 0.015-0.02, 0.02-0.025, 0.025-030, 0.03-0.035, 0.035-0.04, 0.04-0.045, 0.045-0.05, 0.05-0.055, 0.055-0.06, 0.06-0.065, 0.065-0.07, 0.07-0.075, 0.075-0.08, 0.08-0.085, 0.085-0.09, 0.09-0.095, 0.095-0.1, 0.1-0.12, 0.12-0.13, 0.13-0.14, 0.14-0.15, 0.15-0.16, 0.16-0.17, 0.17-0.18, 0.18-0.19, or 0.19-0.2 inches (a range from about 0.25 mm to about 5.08 mm). The total length of the coil can be about 0.01 to about 0.015, about 0.015 to about 0.02, about 0.02 to about 0.025, about 0.025 to about 03, about 0.03 to about 0.035, about 0.035 to about 0.04, about 0.04 to about 0.045, about 0.045 to about 0.05, about 0.05 to about 0.055, about 0.055 to about 0.06, about 0.06 to about 0.065, about 0.065 to about 0.07, about 0.07 to about 0.075, about 0.075 to about 0.08, about 0.08 to about 0.085, about 0.085 to about 0.09, about 0.09 to about 0.095, about 0.095 to about 0.1, about 0.1 to about 0.12, about 0.12 to about 0.13, about 0.13 to about 0.14, about 0.14 to about 0.15, about 0.15 to about 0.16, about 0.16 to about 0.17, about 0.17 to about 0.18, about 0.18 to about 0.19, or about 0.19 to about 0.2 inches (a range from about 0.25 mm to about 5.08 mm).

A heater element comprising a rod as provided herein can comprise one or more segments comprising a coil and one or more segments not comprising a coil (i.e. non-coil segment). The rod can be a wire. The coil can be a wire coil. One or more non-coil segments of the rod can be capable of wicking or holding a liquid formulation comprising an agent as provided herein. The non-coil segment can act as a capillary or wick. In some cases, one or more non-coil segments of the rod comprise a wick element. One or more wick elements can be capable of being heated, thereby forming one or more heated wick elements. The liquid formulation comprising an agent as provided herein can be in fluid communication with a source of the liuid formulation. The source of the liquid formulation can be any source as provided herein, including, but not limited to, a reservoir. The liquid formulation comprising an agent as provided herein can be delivered to a non-coil segment of the rod by any means known in the art. The delivery can be through capillary action or through the use of a pump. In some cases, the non-coil segment is in fluid communication with a reservoir, wherein the reservoir comprises a liquid formulation comprising a pharmaceutically active agent (e.g. nicotine), and wherein the non-coil segment is capable of holding the liquid formulation comprising a pharmaceutically active agent (e.g., nicotine).

The non-coil segments can serve as electrical leads for connecting the rod to a source of electricity. The rod can comprise a coil along the entire length of the rod. In some cases, the coil connects to the source of electricity. The source of electricity can be a battery or a charged capacitor. The battery can be rechargeable.

In some cases, a distance between the first and second leads of the rod when the first lead is connected to either the first or second terminal of the power source while the second lead is connected to the other of the first or second terminal of the power source is about, more than, less than, or at least 0.01, 0.015, 0.02, 0.025, 0.03, 0.035, 0.04, 0.045, 0.05, 0.055, 0.06, 0.065, 0.07, 0.075, 0.08, 0.085, 0.09, 0.095, 0.1, 0.11, 0.12, 0.13, 0.14, 0.15, 0.16, 0.17, 0.18, 0.19, or 0.2 inches (a range from about 0.254 mm to about 5.08 mm). A distance between the first and second leads of the rod when the first lead is connected to either the first or second terminal of the power source while the second lead is connected to the other of the first or second terminal of the power source is from about 0.01 to about 0.1 inches, about 0.02 to about 0.09 inches, or about 0.025 to about 0.8 inches (a range from about 0.254 mm to about 20.32 mm).

In some cases, the coil can wrap around a non-coil segment of the rod, wherein the non-coil segment passes through the coil. In these cases, the coil can wrap around or span exactly, about, more than, less than, at least, or at most 1%, 2%, 3%,4%,5%, 6%, 7%, 8%, 9%, 10%, 11%, 12%, 13%, 14%, 15%, 16%, 17%, 18%, 19%, 20%, 21%, 22%, 23%, 24%, 25%, 26%, 27%, 28%, 29%, 30%, 31%, 32%, 33%, 34%, 35%, 36%, 37%, 38%, 39%, 40%, 41%, 42%, 43%, 44%, 45%, 46%, 47%, 48%, 49%, 50%, 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99%, 100% of the length a non-coil segment of the rod. In these cases, the coil can wrap around or span between 1-10%, 10-20%, 20-30%, 30-40%, 40-50%, 50-60%, 60-70%, 70-80%, 80-90%, or 90-100%, 10-20% of the length a non-coil segment of the rod. In these cases, the coil can wrap around or span of about 1 to about 10%, about 10 to about 20%, about 20 to about 30%, about 30 to about 40%, about 40 to about 50%, about 50 to about 60%, about 60 to about 70%, about 70 to about 80%, about 80 to about 90%, or about 90 to about 100% of the length of a non-coil segment of the rod.

The total length of the coil can be exactly, about, more than, less than, at least, or at most 0.01, 0.0125, 0.015, 0.0175, 0.02, 0.0225, 0.025, 0.0275, 03, 0.0325, 0.035, 0.0375, 0.04, 0.0425, 0.045, 0.0475, 0.05, 0.0525, 0.055, 0.0575, 0.06, 0.0625, 0.065, 0.0675, 0.07, 0.0725, 0.075, 0.0775, 0.08, 0.0825, 0.085, 0.0875, 0.09, 0.0925, 0.095, 0.0975, 0.1, 0.11, 0.115, 0.12, 0.125, 0.13, 0.135, 0.14, 0.145, 0.15, 0.155, 0.16, 0.165, 0.17, 0.175, 0.18, 0.185, 0.19, 0.195 or 0.2 inches (a range from about 0.254 mm to about 5.08 mm). The total length of the coil can be between 0.01-0.015, 0.015-0.02, 0.02-0.025, 0.025-030, 0.03-0.035, 0.035-0.04, 0.04-0.045, 0.045-0.05, 0.05-0.055, 0.055-0.06, 0.06-0.065, 0.065-0.07, 0.07-0.075, 0.075-0.08, 0.08-0.085, 0.085-0.09, 0.09-0.095, 0.095-0.1, 0.1-0.12, 0.12-0.13, 0.13-0.14, 0.14-0.15, 0.15-0.16, 0.16-0.17, 0.17-0.18, 0.18-0.19, or 0.19-0.2 inches (a range from about 0.254 mm to about 5.08 mm). The total length of the coil can be about 0.01 to about 0.015, about 0.015 to about 0.02, about 0.02 to about 0.025, about 0.025 to about 03, about 0.03 to about 0.035, about 0.035 to about 0.04, about 0.04 to about 0.045, about 0.045 to about 0.05, about 0.05 to about 0.055, about 0.055 to about 0.06, about 0.06 to about 0.065, about 0.065 to about 0.07, about 0.07 to about 0.075, about 0.075 to about 0.08, about 0.08 to about 0.085, about 0.085 to about 0.09, about 0.09 to about 0.095, about 0.095 to about 0.1, about 0.1 to about 0.12, about 0.12 to about 0.13, about 0.13 to about 0.14, about 0.14 to about 0.15, about 0.15 to about 0.16, about 0.16 to about 0.17, about 0.17 to about 0.18, about 0.18 to about 0.19, or about 0.19 to about 0.2 inches (a range from about 0.254 mm to about 5.08 mm).

A heater element comprising a rod as provided herein can comprise one or more coils. The rod can be a wire. The coil can be a wire coil. The coil can have exactly, about, more than, less than, at least or at most 1, 1.5, 2, 2.5, 3, 3.5, 4, 4.5, 5, 5.5, 6, 6.5, 7, 7.5, 8, 8.5, 9, 9.5 10, 10.5, 11, 11.5, 12, 12.5, 13, 13.5, 14, 14.5, 15, 15.5, 16, 16.5, 17, 17.5, 18, 18.5, 19, 19.5, 20, 20.5, 21, 21.5, 22, 22.5, 23, 23.5, 24, 24.5, 25, 25.5, 26, 26.5, 27, 27.5, 28, 28.5, 29, 29.5, 30, 30.5, 31, 31.5, 32, 32.5, 33, 33.5, 34, 34.5, 35, 35.5, 36, 36.5, 37, 37.5, 38, 38.5, 39, 39.5, 40, 41, 42, 43, 44, 45, 46, 47, 4, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 105, 110, 115, 120, 125, 130, 135, 140, 145, 150, 155, 160, 165, 170, 175, 180, 185, 190, 195, or 200 coils. The coil can comprise 1-2, 2-4, 4-6, 6-8, 8-10, 10-12, 12-14, 14-16, 16-18, or 18-20 coils. The coil can comprise 2-20, 4-20, 6-20, 8-20, 10-20, 12-20, 14-20, or 16-20 coils. The coil can comprise between 1-5, 5-10, 10-15, or 15-20 coils. The coil can comprise between 1-10, 1-20, 1-30, 1-40, 1-60, 1-70, 1-80, 1-90, 1-100 coils. The coil can comprise from about 1 to about 5, about 5 to about 10, about 10 to about 15, or about 15 to about 20 coils. The coil can comprise from about 1 to about 10, about 1 to about 20, about 1 to about 30, about 1 to about 40, about 1 to about 60, about 1 to about 70, about 1 to about 80, about 1 to about 90, or about 1 to about 100 coils. In one embodiment, the coil comprises from about 5 to about 10 coils. In one embodiment, the coil comprises from about 1 to about 10 coils. The distance between successive coils or the pitch of the coils can be exactly, about, more than, less than, at least or at most 0.01, 0.0125, 0.015, 1.17, 0.0175, 0.02, 0.0225, 0.025, 0.0275, 03, 0.0325, 0.035, 0.0375, 0.04, 0.0425, 0.045, 0.0475, 0.05, 0.0525, 0.055, 0.0575, 0.06, 0.0625, 0.065, 0.0675, 0.07, 0.0725, 0.075, 0.0775, 0.08, 0.0825, 0.085, 0.0875, 0.09, 0.0925, 0.095, 0.0975, 0.1, 0.11, 0.115, 0.12, 0.125, 0.13, 0.135, 0.14, 0.145, 0.15, 0.155, 0.16, 0.165, 0.17, 0.175, 0.18, 0.185, 0.19, 0.195 or 0.2 inches (a range from about 0.254 mm to about 5.08 mm). The distance between successive coils or the pitch of the coils can be between 0.01-0.015, 0.015-0.3, 0.01-0.02, 0.015-0.02, 0.020-0.025, 0.025-03, 0.03-0.035, 0.035-0.04, 0.04-0.045, 0.045-0.05, 0.05-0.055, 0.055-0.06, 0.06-0.065, 0.065-0.07, 0.07-0.075, 0.075-0.08, 0.08-0.085, 0.085-0.09, 0.09-0.095, 0.095-0.1, 0.1-0.12, 0.12-0.13, 0.13-0.14, 0.14-0.15, 0.15-0.16, 0.16-0.17, 0.17-0.18, 0.18-0.19, or 0.19-0.2 inches (a range from about 0.254 mm to about 5.08 mm). The distance between successive coils or the pitch of the coils can be about 0.01 to about 0.015, about 0.01 to about 0.02, about 0.015 to about 0.3, about 0.015 to about 0.02, about 0.02 to about 0.025, about 0.025 to about 03, about 0.03 to about 0.035, about 0.035 to about 0.04, about 0.04 to about 0.045, about 0.045 to about 0.05, about 0.05 to about 0.055, about 0.055 to about 0.06, about 0.06 to about 0.065, about 0.065 to about 0.07, about 0.07 to about 0.075, about 0.075 to about 0.08, about 0.08 to about 0.085, about 0.085 to about 0.09, about 0.09 to about 0.095, about 0.095 to about 0.1, about 0.1 to about 0.12, about 0.12 to about 0.13, about 0.13 to about 0.14, about 0.14 to about 0.15, about 0.15 to about 0.16, about 0.16 to about 0.17, about 0.17 to about 0.18, about 0.18 to about 0.19, or about 0.19 to about 0.2 inches (a range from about 0.254 mm to about 5.08 mm).

A rod in a heater element comprising a rod as provided herein can have a diameter of exactly, about, more than, less than, at least or at most 0.005, 0.006, 0.007, 0.008, 0.009, 0.01, 0.011, 0.012, 0.013, 0.014, 0.015, 0.016, 0.017, 0.018, 0.019, or 0.02 inches (a range from about 0.127 mm to about 0.51 mm). The rod can have a diameter between 0.005 and 0.01, 0.01 and 0.015, or 0.015 and 0.02 inches (a range from about 0.127 mm to about 0.51 mm). In one embodiment, the rod has a diameter between 0.005 and 0.02 inches (a range from about 0.127 mm to about 0.51 mm). In one embodiment, the rod has a diameter between 0.008 and 0.0012 inches (a range from about 0.2032 mm to about 0.03 mm).. The rod can have a diameter of about 0.005 to about 0.01, about 0.01 to about 0.015, or about 0.015 to about 0.02 inches (a range from about 0.127 mm to about 0.508 mm). In one embodiment, the rod has a diameter of about 0.005 to about 0.02 inches (a range from about 0.127 mm to about 0.508 mm). In one embodiment, the rod has a diameter of about 0.008 to about 0.0012 inches (a range from about 0.2031 mm to about 0.03 mm). The rod can be a wire.

A heater element comprising a coil as provided herein can have a coil with an inner or internal diameter of exactly, about, more than, less than, at least or at most 0.01, 0.012, 0.0125, 0.015, 0.0175, 0.02, 0.022, 0.0225, 0.025, 0.0275, 03, 0.032, 0.0325, 0.035, 0.0375, 0.04,0.042 0.0425, 0.045, 0.0475, 0.05, 0.0520.0525, 0.055, 0.0575, 0.06, 0.062, 0.0625, 0.065, 0.0675, 0.07, 0.072, 0.0725, 0.075, 0.0775, 0.08, 0.0825, 0.085, 0.0875, 0.09, 0.0925, 0.095, 0.0975, 0.1, 0.11, 0.115, 0.12, 0.125, 0.13, 0.135, 0.14, 0.145, 0.15, 0.155, 0.16, 0.165, 0.17, 0.175, 0.18, 0.185, 0.19, 0.195, 0.2, 0.21, 0.22, 0.23, 0.24, 0.25, 0.26, 0.27, 0.28, 0.29, 0.3, 0.35, 0.4, 0.45, or 0.5 inches (a range from about 0.254 mm to about 12.7 mm). The inner or internal diameter of the coil can be between 0.01-0.015, 0.015-0.02, 0.02-0.025, 0.0250-03, 0.03-0.035, 0.035-0.04, 0.04-0.045, 0.045-0.05, 0.05-0.055, 0.055-0.06, 0.06-0.065, 0.065-0.07, 0.07-0.075, 0.075-0.08, 0.08-0.085, 0.085-0.09, 0.09-0.095, 0.095-0.1, 0.1-0.12, 0.12-0.13, 0.13-0.14, 0.14-0.15, 0.15-0.16, 0.16-0.17, 0.17-0.18, 0.18-0.19, 0.19-0.2, 0.2-0.21, 0.21-0.22, 0.22-0.23, 0.23-0.24, 0.24-0.25, 0.25-0.26, 0.26-0.27, 0.27-0.28, 0.28-0.29, 0.29-0.3, 0.3-0.35, 0.35-0.4, 0.4-0.45, or 0.45-0.5 inches (a range from about 0.254 mm to about 12.7 mm). The inner or internal diameter of the coil can be about 0.01 to about 0.015, about 0.015 to about 0.02, about 0.02 to about 0.025, about 0.025 to about 0.3, about 0.03 to about 0.035, about 0.035 to about 0.04, about 0.04 to about 0.045, about 0.045 to about 0.05, about 0.05 to about 0.055, about 0.055 to about 0.06, about 0.06 to about 0.065, about 0.065 to about 0.07, about 0.07 to about 0.075, about 0.075 to about 0.08, about 0.08 to about 0.085, about 0.085 to about 0.09, about 0.09 to about 0.095, about 0.095 to about 0.1, about 0.1 to about 0.15, about 0.15 to about 0.2, about 0.2 to about 0.25, about 0.25 to about 0.3, about 0.3 to about 0.35, about 0.35 to about 0.4, about 0.4 to about 0.45, or about 0.45 to about 0.5 inches (a range from about 0.254 mm to about 12.7 mm). The inner or internal diameter of the coil can be between 0.02 and 0.04, 0.04 and 0.06, or 0.02 and 0.06 inches (a range from about 0.508 mm to about 1.524 mm). In one embodiment, the inner or internal diameter of the coil is between 0.03 and 0.04 inches (a range from about 0.3 mm to about 1.02 mm). The inner or internal diameter of the coil can be about 0.02 to about 0.04, about 0.04 to about 0.06, or about 0.02 to about 0.06 inches (a range from about 0.508 mm to about 1.524 mm). In one embodiment, the inner or internal diameter of the coil is about 0.03 to about 0.04 inches (a range from about 0.3 mm to about 1.02 mm). In one embodiment, the inner or internal diameter of the coil is about 0.02 to about 0.04 inches (a range from about 0.508 mm to about 1.02 mm). In one embodiment, the inner or internal diameter of the coil is between 0.02 to about 0.04 inches (a range from about 0.508 mm to about 1.02 mm). The coil can be a wire coil.

A heater element comprising a coil as provided herein can have a coil with an outer or external diameter of exactly, about, more than, less than, at least or at most 0.01, 0.0125, 0.015, 0.0175, 0.02, 0.0225, 0.025, 0.0275, 0.03, 0.0325, 0.035, 0.0375, 0.04, 0.0425, 0.045, 0.0475, 0.05, 0.0525, 0.055, 0.0575, 0.06, 0.0625, 0.065, 0.0675, 0.07, 0.0725, 0.075, 0.0775, 0.08, 0.0825, 0.085, 0.0875, 0.09, 0.0925, 0.095, 0.0975, 0.1, 0.11, 0.115, 0.12, 0.125, 0.13, 0.135, 0.14, 0.145, 0.15, 0.155, 0.16, 0.165, 0.17, 0.175, 0.18, 0.185, 0.19, 0.195, 0.2, 0.21, 0.22, 0.23, 0.24, 0.25, 0.26, 0.27, 0.28, 0.29, 0.3, 0.35, 0.4, 0.45, or 0.5 inches (a range from about 0.254 mm to about 12.7 mm). The outer or external diameter of the coil can be between 0.01-0.015, 0.015-0.02, 0.02-0.025, 0.025-0.03, 0.03-0.035, 0.035-0.04, 0.04-0.045, 0.045-0.05, 0.05-0.055, 0.055-0.06, 0.06-0.065, 0.065-0.07, 0.07-0.075, 0.075-0.08, 0.08-0.085, 0.085-0.09, 0.09-0.095, 0.095-0.1, 0.1-0.12, 0.12-0.13, 0.13-0.14, 0.14-0.15, 0.15-0.16, 0.16-0.17, 0.17-0.18, 0.18-0.19, 0.19-0.2, 0.2-0.21, 0.21-0.22, 0.22-0.23, 0.23-0.24, 0.24-0.25, 0.25-0.26, 0.26-0.27, 0.27-0.28, 0.28-0.29, 0.29-0.3, 0.3-0.35, 0.35-0.4, 0.4-0.45, or 0.45-0.5 inches (a range from about 0.254 mm to about 12.7 mm). The outer or external diameter of the coil can be about 0.01 to about 0.015, about 0.015 to about 0.02, about 0.02 to about 0.025, about 0.025 to about 0.3, about 0.03 to about 0.035, about 0.035 to about 0.04, about 0.04 to about 0.045, about 0.045 to about 0.05, about 0.05 to about 0.055, about 0.055 to about 0.06, about 0.06 to about 0.065, about 0.065 to about 0.07, about 0.07 to about 0.075, about 0.075 to about 0.08, about 0.08 to about 0.085, about 0.085 to about 0.09, about 0.09 to about 0.095, about 0.095 to about 0.1, about 0.1 to about 0.15, about 0.15 to about 0.2, about 0.2 to about 0.25, about 0.25 to about 0.3, about 0.3 to about 0.35, about 0.35 to about 0.4, about 0.4 to about 0.45, or about 0.45 to about 0.5 inches (a range from about 0.254 mm to about 12.7 mm). The outer or external diameter of the coil can be between 0.02 and 0.04, 0.04 and 0.06, or 0.02 and 0.06 inches (a range from about .02 mm and 1.02 mm to about 0.02 mm and 1.524 mm). In one embodiment, the outer or external diameter of the coil is between 0.03 and 0.04 inches (a range from about 0.0.762 mm to about 1.02 mm). The outer or external diameter of the coil can be about 0.02 to about 0.04, about 0.04 to about 0.06, about 0.02 to about 0.06 inches, about 0.02 to about 0.08 inches, about 0.02 to about 0.1 inches (a range from about 0.508 mm to about 2.54 mm). In one embodiment, the outer or external diameter of the coil is about 0.03 to about 0.04 inches (a range from about 0.762 mm to about 1.02 mm). In one embodiment, the outer or external diameter of the coil is about 0.02 to about 0.04 inches (a range from about 0.508 mm to about 1.02 mm). In one embodiment, the outer or external diameter of the coil is between 0.02 to about 0.04 inches (a range from about 0.508 mm to about 1.02 mm). The coil can be a wire coil.

A heater element comprising a coil as provided herein can have a coil with a length to width aspect ratio exactly, about, more than, less than, at least or at most 0.1, 0.2, 0.3, 0.4, 0.5, 0.6, 0.7, 0.8, 0.9, 1, 1.1, 1.2, 1.3, 1.4, 1.5, 1.6, 1.7, 1.8, 1.9, 2.0, 2.1, 2.2, 2.3, 2.4, 2.5, 2.6, 2.7, 2.8, 2.9, 3, 3.1, 3.2, 3.3, 3.4, 3.5, 3.6, 3.7, 3.8, 3.9, 4, 4.1, 4.2, 4.3, 4.4, 4.5, 4.6, 4.7, 4.8, 4.9, 5, 5.1, 5.2, 5.3, 5.4, 5.5, 5.6, 5.7, 5.8, 5.9, 6, 6.1, 6.2, 6.3, 6.4, 6.5, 6.6, 6.7, 6.8, 6.9, 7, 7.1, 7.2, 7.3, 7.4, 7.5, 7.6, 7.7, 7.8, 7.9, 8, 8.1, 8.2, 8.3, 8.4, 8.5, 8.6, 8.7, 8.8, 8.9, 9, 9.1, 9.2, 9.3, 9.4, 9.5, 9.6, 9.7, 9.8, 9.9, 10, 10.5, 11, 11.5, 12, 12.5, 13, 13.5, 14, 14.5, or 15. The length to width aspect ratio of the coil can be between 0.1-0.15, 0.15-0.2, 0.2-0.25, 0.25-0.3, 0.3-0.35, 0.35-0.4, 0.4-0.45, 0.45-0.5, 0.5-0.55, 0.55-0.6, 0.6-0.65, 0.65-0.7, 0.7-0.75, 0.75-0.8, 0.8-0.85, 0.85-0.9, 0.9-0.95, 0.95-1, 1-1.5, 1.5-2, 2-2.5, 2.5-3, 3-3.5, 3.5-4, 4-4.5, 4.5-5, 5-5.5, 5.5-6, 6-6.5, 6.5-7, 7-7.5, 7.5-8, 8-8.5, 8.5-9, 9-9.5, 9.5-10, 10-10.5, 10.5-11, 11-11.5, 11.5-12, 12.5-13, 13-13.5, 13.5-14, 14-14.5, or 14.5-15. The length to width aspect ratio of the coil can be about 0.1 to about 0.15, about 0.15 to about 0.2, about 0.2 to about 0.25, about 0.25 to about 0.3, about 0.3 to about 0.35, about 0.35 to about 0.4, about 0.4 to about 0.45, about 0.45 to about 0.5, about 0.5 to about 0.55, about 0.55 to about 0.6, about 0.6 to about 0.65, about 0.65 to about 0.7, about 0.7 to about 0.75, about 0.75 to about 0.8, about 0.8 to about 0.85, about 0.85 to about 0.9, about 0.9 to about 0.95, about 0.95 to about 1, about 1 to about 1.5, about 1.5 to about 2, about 2 to about 2.5, about 2.5 to about 3, about 3 to about 3.5, about 3.5 to about 4, about 4 to about 4.5, about 4.5 to about 5, about 5 to about 5.5, about 5.5 to about 6, about 6 to about 6.5, about 6.5 to about 7, about 7 to about 7.5, about 7.5 to about 8, about 8 to about 8.5, about 8.5 to about 9, about 9 to about 9.5, about 9.5 to about 10, about 10 to about 10.5, about 10.5 to about 11, about 11 to about 11.5, about 11.5 to about 12, about 12.5 to about 13, about 13 to about 13.5, about 13.5 to about 14, 14 to about 14.5, or about 14.5 to about 15. The width of the coil in the length to width aspect ratio can be the inner or internal diameter, or the outer or external diameter. The coil can be a wire coil.

A heater element comprising a rod as provided herein, wherein the rod can have a coil wherein a ratio of the diameter of the rod to the diameter of the coil can be exactly, about, more than, less than, at least or at most 0.1, 0.2, 0.3, 0.4, 0.5, 0.6, 0.7, 0.8, 0.9, 1, 1.1, 1.2, 1.3, 1.4, 1.5, 1.6, 1.7, 1.8, 1.9, 2, 2.1, 2.2, 2.3, 2.4, 2.5, 2.6, 2.7, 2.8, 2.9, 3, 3.1, 3.2, 3.3, 3.4, 3.5, 3.6, 3.7, 3.8, 3.9, 4, 4.1, 4.2, 4.3, 4.4, 4.5, 4.6, 4.7, 4.8, 4.9, 5, 5.1, 5.2, 5.3, 5.4, 5.5, 5.6, 5.7, 5.8, 5.9, 6, 6.1, 6.2, 6.3, 6.4, 6.5, 6.6, 6.7, 6.8, 6.9, 7, 7.1, 7.2, 7.3, 7.4, 7.5, 7.6, 7.7, 7.8, 7.9, 8, 8.1, 8.2, 8.3, 8.4, 8.5, 8.6, 8.7, 8.8, 8.9, 9, 9.1, 9.2, 9.3, 9.4, 9.5, 9.6, 9.7, 9.8, 9.9, 10, 10.5, 11, 11.5, 12, 12.5, 13, 13.5, 14, 14.5, or 15. The ratio of the diameter of the rod to the diameter of the coil can be between 0.1-0.15, 0.15-0.2, 0.2-0.25, 0.25-0.3, 0.3-0.35, 0.35-0.4, 0.4-0.45, 0.45-0.5, 0.5-0.55, 0.55-0.6, 0.6-0.65, 0.65-0.7, 0.7-0.75, 0.75-0.8, 0.8-0.85, 0.85-0.9, 0.9-0.95, 0.95-1, 1-1.5, 1.5-2, 2-2.5, 2.5-3, 3-3.5, 3.5-4, 4-4.5, 4.5-5, 5-5.5, 5.5-6, 6-6.5, 6.5-7, 7-7.5, 7.5-8, 8-8.5, 8.5-9, 9-9.5, 9.5-10, 10-10.5, 10.5-11, 11-11.5, 11.5-12, 12.5-13, 13-13.5, 13.5-14, 14-14.5, or 14.5-15. The ratio of the diameter of the rod to the diameter of the coil can be about 0.1 to about 0.15, about 0.15 to about 0.2, about 0.2 to about 0.25, about 0.2 to about 0.4, about 0.25 to about 0.3, about 0.3 to about 0.35, about 0.35 to about 0.4, about 0.4 to about 0.45, about 0.45 to about 0.5, about 0.5 to about 0.55, about 0.55 to about 0.6, about 0.6 to about 0.65, about 0.65 to about 0.7, about 0.7 to about 0.75, about 0.75 to about 0.8, about 0.8 to about 0.85, about 0.85 to about 0.9, about 0.9 to about 0.95, about 0.95 to about 1, about 1 to about 1.5, about 1.5 to about 2, about 2 to about 2.5, about 2.5 to about 3, about 3 to about 3.5, about 3.5 to about 4, about 4 to about 4.5, about 4.5 to about 5, about 5 to about 5.5, about 5.5 to about 6, about 6 to about 6.5, about 6.5 to about 7, about 7 to about 7.5, about 7.5 to about 8, about 8 to about 8.5, about 8.5 to about 9, about 9 to about 9.5, about 9.5 to about 10, about 10 to about 10.5, about 10.5 to about 11, about 11 to about 11.5, about 11.5 to about 12, about 12.5 to about 13, about 13 to about 13.5, about 13.5 to about 14, 14 to about 14.5, or about 14.5 to about 15. The width of the coil in the ratio of the diameter of the rod to a diameter of the coil can be the inner or internal diameter, or the outer or external diameter. The rod can be a wire. The coil can be a wire coil.

A heater element comprising a rod is provided herein, wherein the rod can have a coil wherein the volume of the rod can be less than the volume of the coil. The volume of the rod can be exactly, about, more than, less than, at least or at most 1%, 2%, 3%, 4%, 5%, 6%, 7%, 8%, 9%, 10%, 11%, 12%, 13%, 14%, 15%, 16%, 17%, 18%, 19%, 20%, 21%, 22%, 23%, 24%, 25%, 26%, 27%, 28%, 29%, 30%, 31%, 32%, 33%, 34%, 35%, 36%, 37%, 38%, 39%, 40%, 41%, 42%, 43%, 44%, 45%, 46%, 47%, 48%, 49%, 50%, 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99%, 100% of the volume of the coil. The volume of the rod can be between 1-10%, 10-20%, 20-30%, 30-40%, 40-50%, 50-60%, 60-70%, 70-80%, 80-90%, or 90-100% of the volume of the coil. The volume of the rod can be about 1 to about 10%, about 10 to about 20%, about 20 to about 30%, about 30 to about 40%, about 40 to about 50%, about 50 to about 60%, about 60 to about 70%, about 70 to about 80%, about 80 to about 90%, or about 90 to about 100% of the volume of the coil. The rod can be a wire. The coil can be a wire coil. In some cases, the volume of the coil be about, more than, less than, at least, or no greater than 1.1, 1.2, 1.3, 1.4, 1.5, 1.6, 1.7, 1.9, 2, 2.1, 2.2, 2.3, 2.4, 2.5, 2.6, 2.7, 2.8, 2.9, 3, 3.1, 3.2, 3.3, 3.4, 3.5, 3.6, 3.7, 3.8, 3.9, 4, 4.1, 4.5, 5, 5.5, 6, 6.5, 7, 7.5, 8, 8.5, 9, 9.5, or 10 times the volume of the rod.

A heater element comprising a rod is provided herein, wherein the rod can have a coil, wherein the surface area of the rod can be less than, greater than or equal to the surface area of the outer or external surface of the coil. The surface area of the rod can be exactly, about, more than, less than, at least or at most 1%, 2%, 3%,4%,5%, 6%, 7%, 8%, 9%, 10%, 11%, 12%, 13%, 14%, 15%, 16%, 17%, 18%, 19%, 20%, 21%, 22%, 23%, 24%, 25%, 26%, 27%, 28%, 29%, 30%, 31%, 32%, 33%, 34%, 35%, 36%, 37%, 38%, 39%, 40%, 41%, 42%, 43%, 44%, 45%, 46%, 47%, 48%, 49%, 50%, 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99%, 100% greater than or less than the outer surface area of the coil. The surface area of the rod can be between 1-10%, 10-20%, 20-30%, 30-40%, 40-50%, 50-60%, 60-70%, 70-80%, 80-90%, or 90-100% greater than or less than the outer surface area of the coil. The surface area of the rod can be about 1 to about 10%, about 10 to about 20%, about 20 to about 30%, about 30 to about 40%, about 40 to about 50%, about 50 to about 60%, about 60 to about 70%, about 70 to about 80%, about 80 to about 90%, or about 90 to about 100% greater than or less than the outer surface area of the coil. The rod can be a wire. The coil can be a wire coil. In some cases, a surface area of a rod can be 1.1, 1.2, 1.3, 1.4, 1.5, 1.6, 1.7, 1.8, 1.9, 2, 2.1, 2.2, 2.3, 2.4, 2.5, 2.6, 2.7, 2.8, 2.9, 3, 3.1, 3.2, 3.3, 3.4, 3.5, 3.6, 3.7, 3.8, 3.9, 4, 4.1, 4.2, 4.3, 4.4, 4.5, 4.6, 4.7, 4.8, 4.9, 5, 5.1, 5.2, 5.3, 5.4, 5.5, 5.6, 5.7, 5.8, 5.9, 6, 6.1, 6.2, 6.3, 6.4, 6.5, 6.6, 6.7, 6.8, 6.9, 7, 7.1, 7.2, 7.3, 7.4, 7.5, 7.6, 7.7, 7.8, 7.9, 8, 8.1, 8.2, 8.3, 8.4, 8.5, 8.6, 8.7, 8.8, 8.9, 9, 9.1, 9.2, 9.3, 9.4, 9.5, 9.6, 9.7, 9.8, 9.9, or 10 times greater than a surface area of a coil.

A heater element as provided herein can comprise an electrical resistance that can be exactly, about, more than, less than, at least or at most 0.1, 0.2, 0.3, 0.4, 0.5, 0.6, 0.7, 0.8, 0.9, 1, 1.1, 1.2, 1.3, 1.4, 1.5, 1.6, 1.7, 1.8, 1.9, 2, 2.1, 2.2, 2.3, 2.4, 2.5, 2.6, 2.7, 2.8, 2.9, 3, 3.1, 3.2, 3.3, 3.4, 3.5, 3.6, 3.7, 3.8, 3.9, 4, 4.1, 4.2, 4.3, 4.4, 4.5, 4.6, 4.7, 4.8, 4.9, 5, 5.1, 5.2, 5.3, 5.4, 5.5, 5.6, 5.7, 5.8, 5.9, 6, 6.1, 6.2, 6.3, 6.4, 6.5, 6.6, 6.7, 6.8, 6.9, 7, 7.1, 7.2, 7.3, 7.4, 7.5, 7.6, 7.7, 7.8, 7.9, 8, 8.1, 8.2, 8.3, 8.4, 8.5, 8.6, 8.7, 8.8, 8.9, 9, 9.1, 9.2, 9.3, 9.4, 9.5, 9.6, 9.7, 9.8, 9.9, or 10 Ohms. The electrical resistance can be between 0.1-0.15, 0.15-0.2, 0.2-0.25, 0.25-0.3, 0.3-0.35, 0.35-0.4, 0.4-0.45, 0.45-0.5, 0.5-0.55, 0.55-0.6, 0.6-0.65, 0.65-0.7, 0.7-0.75, 0.75-0.8, 0.8-0.85, 0.85-0.9, 0.9-0.95, 0.95-1, 1-1.5, 1.5-2, 2-2.5, 2.5-3, 3-3.5, 3.5-4, 4-4.5, 4.5-5, 5-5.5, 5.5-6, 6-6.5, 6.5-7, 7-7.5, 7.5-8, 8-8.5, 8.5-9, 9-9.5, or 9.5-10 Ohms. The electrical resistance can be about 0.1 to about 0.15, about 0.15 to about 0.2, about 0.2 to about 0.25, about 0.25 to about 0.3, about 0.3 to about 0.35, about 0.35 to about 0.4, about 0.4 to about 0.45, about 0.45 to about 0.50, about 0.5 to about 0.55, about 0.55 to about 0.6, about 0.6 to about 0.65, about 0.65 to about 0.7, about 0.7 to about 0.75, about 0.75 to about 0.8, about 0.8 to about 0.85, about 0.85 to about 0.9, about 0.9 to about 0.95, about 0.95 to about 1, about 1 to about 1.5, about 1.5 to about 2, about 2 to about 2.5, about 2.5 to about 3, about 3 to about 3.5, about 3.5 to about 4, about 4 to about 4.5, about 4.5 to about 5, about 5 to about 5.5, about 5.5 to about 6, about 6 to about 6.5, about 6.5 to about 7, about 7 to about 7.5, about 7.5 to about 8, about 8 to about 8.5, about 8.5 to about 9, about 9 to about 9.5, or about 9.5 to about 10 Ohms. The electrical resistance can be the electrical resistance at room temperature.

A heater element as provided herein can vaporize a liquid formulation comprising a pharmaceutically active agent (e.g., nicotine) as provided herein, wherein substantially all of the liquid formulation in contact with or delivered to the heater element is vaporized. The vaporization of the liquid formulation that contacts or is delivered to a heater element as provided herein can be exactly, about, more than, less than, at most, or at least 1%, 2%, 3%,4%,5%, 6%, 7%, 8%, 9%, 10%, 11%, 12%, 13%, 14%, 15%, 16%, 17%, 18%, 19%, 20%, 30%, 31%, 32%, 33%, 34%, 35%, 36%, 37%, 38%, 39%, 40%, 41%, 42%, 43%, 44%, 45%, 46%, 47%, 48%, 49%, 50%, 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99%, or 100%. The vaporization of the liquid formulation that contacts or is delivered to a heater element as provided herein can be between 1%-10%, 10%-20%, 20%-30%, 30%-40%, 40%-50%, 50%-60%, 60%-70%, 70%-80%, 80%-90%, or 90%-100%. The vaporization of the liquid formulation that contacts or is delivered to a heater element as provided herein can be about 1% to about 10%, about 10% to about 20%, about 20% to about 30%, about 30% to about 40%, about 40% to about 50%, about 50% to about 60%, about 60% to about 70%, about 70% to about 80%, about 80% to about 90%, or about 90% to about 100%. The vaporization of the liquid formulation that contacts or is delivered to a heater element as provided herein can be greater than 95%, 99%, or 99.5%.

The amount of residue or build-up of non-vaporized liquid formulation comprising a pharmaceutically active agent (e.g., nicotine) that contacts or is delivered to a heater element as provided herein can be reduced by exactly, about, more than, less than, at most, or at least 1%, 2%, 3%,4%,5%, 6%, 7%, 8%, 9%, 10%, 11%, 12%, 13%, 14%, 15%, 16%, 17%, 18%, 19%, 20%, 30%, 31%, 32%, 33%, 34%, 35%, 36%, 37%, 38%, 39%, 40%, 41%, 42%, 43%, 44%, 45%, 46%, 47%, 48%, 49%, 50%, 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99%, or 100%. The amount of residue or build-up of non-vaporized liquid formulation comprising a pharmaceutically active agent that contacts or is delivered to a heater element as provided herein can be between 1%-10%, 10%-20%, 20%-30%, 30%-40%, 40%-50%,

50%-60%, 60%-70%, 70%-80%, 80%-90%, or 90%-100%. The amount of residue or build-up of non-vaporized liquid formulation comprising a pharmaceutically active agent that contacts or is delivered to a heater element as provided herein can be reduced by about 1% to about 10%, about 10% to about 20%, about 20% to about 30%, about 30% to about 40%, about 40% to about 50%, about 50% to about 60%, about 60% to about 70%, about 70% to about 80%, about 80% to about 90%, or about 90% to about 100%. The amount of residue or build-up of non-vaporized liquid formulation comprising a pharmaceutically active agent that contacts or is delivered to a heater element as provided herein can be reduced by greater than 95%, 99%, or 99.5%.

Methods of renewal of a heater element are provided herein. Heating elements can be renewed with changes in an agent (e.g., nicotine) dose cartridge to ensure dose consistency by removal of any build up of combusted material on the heater element.

In some cases, the heater element comprises a coil and a wick element, wherein the coil wraps around the wick element, and wherein the liquid formulation wicks onto the heated wick element, wherein the liquid formulation is vaporized through heating of the coil and wick element.

The heater element can be in fluid communication with a source of liquid formulation comprising an agent (e.g., nicotine) as provided herein. In some cases, the heater element further comprises a source of a liquid formulation comprising an agent (e.g., nicotine), wherein the source is in fluid communication with the wick element capable of being heated, wherein the liquid formulation comprising an agent (e.g., nicotine) wicks onto the wick element capable of being heated, whereby the liquid formulation is aerosolized by heating of the coil and wick element capable of being heated upon activation of a power source, wherein the power source is electrically coupled to the heater element. In some cases, the heater element further comprises a source of a liquid formulation comprising an agent, wherein the source is in fluid communication with the heatable wick element, wherein the liquid formulation comprising an agent wicks onto the heatable wick element, wherein the heatable wick element is heated after the formulation has wicked onto the heatable wick element, whereby the liquid formulation is aerosolized by heating of the coil and heatable wick element upon activation of the power source.

The heater element comprising a coil with a center exit wick element capable of being heated as described herein can vaporize substantially all of the liquid formulation comprising the pharmaceutically active agent (e.g., nicotine) that wicks onto the center wick element. The heater element comprising a coil with a center exit wick element capable of being heated can have a reduced or substantially no splatter. In some cases, the heater element comprises a coil with a center exit wick element capable of being heated, wherein a liquid formulation comprising a pharmaceutically active agent (e.g., nicotine) is held or wicks onto the center exit wick element capable of being heated, and wherein both the wick element capable of being heated and coil are heated, thereby vaporizing the liquid formulation, wherein substantially all of the liquid formulation is vaporized. The heater element comprising a coil with a center exit wick element capable of being heated can vaporize greater than 95 % of the liquid formulation wicked onto the wick element. The amount of residue or build-up of non-vaporized liquid formulation comprising a pharmaceutically active agent (e.g., nicotine) can be substantially reduced. Following vaporization of a liquid formulation as provided herein by a heater element comprising a coil and a center exit wick element capable of being heated less than 5% residue of non-vaporized liquid formulation can remain on the heater element.

In some cases, a heater element is connected to a timing device.

## Removal of Particles

In some cases, an issue with vaporization within the capillary can arise. First, liquid droplets can be ejected by vapor pushing the material out. Second, because the high vapor concentration can be high within the capillary end, rapid condensation and aggregation leading to larger than optimum particle size can result. To reduce the particle size of the aerosol the large particles can be removed and revaporized. Removal can be accomplished thru inertial impaction (**FIG. 11**). **FIG. 11** shows an agent (e.g., nicotine) reservoir (**1104**), tube, e.g., capillary tube (**1106**), heater element 1 (**1108**), and a heater element 2 (**1110**). One consideration is whether a restriction in a nozzle (**1102**) can cause an unacceptable increase in the air flow resistance. The following formula can be used to calculate the diameter of an orifice ($D_J$) (**1112**).

$$d_{50}\sqrt{C_c} = \left[\frac{9\pi N D_j^3 (Stk_{50})}{4 P_p Q}\right]^{1/2}$$

Where $d_{50}$ = is the average aerosol practice size.

Where:

N = viscosity (of air) = $1.81 \times 10^{-5}$ $P_a$ sec
$D_J$ = The nozzle diameter in meters

$Stk_{50}$ =Stokes number for a round nozzle = .24 (dimensionless)

$P_p$ = Density of particle, for liquids assumed to be $1000 kg/meter^3$

Q = Flow rate in liters/mixture (assume 15L/min (about $2.5 \times 10^{-4} m^3/s$))

Additionally to correct for slip factor the following equation can be used:

$$d_{50} = d_{50}\sqrt{C_c} - 0.078 \ \ in \ microns$$

Using the above, a table of nozzle sizes vs. particle sizes that will impact can be generated as shown in Table 1:

**Table 1.**

| Nozzle Size (mm) | Particle Size ($\mu$m) |
|---|---|
| 7 | 6.41 |
| 6 | 5.07 |
| 5 | 3.84 |
| 4 | 2.72 |

If a particle size of approximately 5 $\mu$m is desired, a nozzle with a diameter of about 6 mm can be used, which can be acceptable for a pressure drop at 15L/min (about $2.5 \times 10^{-4} m^3/s$) flow rate of inhalation.

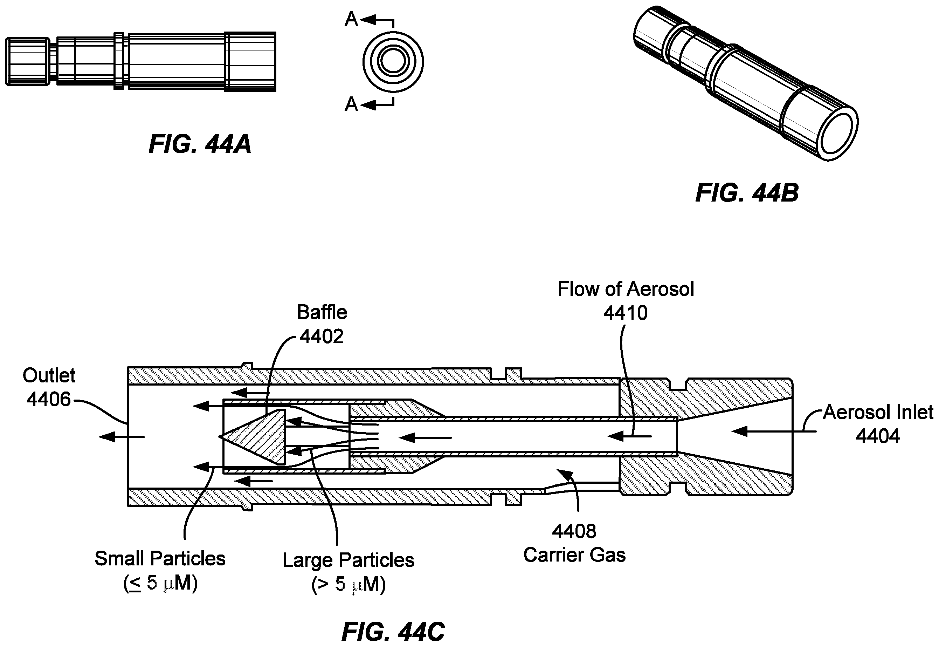

In some cases, a device for generating a condensation aerosol from a liquid formulation comprising a pharmaceutically active agent (e.g., nicotine) as provided herein comprises a means for removing aerosol particles of a size not optimal for deep lung delivery and subsequent rapid PK. The non-optimal particles can have an MMAD of greater than 5 $\mu$m. The means for removing the non-optimal particles can be a solid structure within a passageway in which a condensation aerosol generated as provided herein flows. In some cases, the structure is a planar surface attached to one or more walls of the passageway, wherein the planar structure comprises one or more holes wherein particles of specific sizes (e.g. less than 5 $\mu$m) pass through. In some cases, the structure comprises a planar surface attached to the passageway such that the planar surface has a diameter or width that occupies a portion of the diameter or width of the passageway such that only particles of an optimal size flow or are diverted around the planar surface while non-optimally sized particles impact the surface and are incapable of flowing around the surface. In some cases, the optimally sized particles have an MMAD of less than or equal to 5 $\mu$m. In some cases, the optimally sized particles have an MMAD of about 1 to about 5 $\mu$m. In some cases, the optimally sized particles have an MMAD of greater than 5 $\mu$m. The structure can be a baffle or baffle plate. **FIGs. 44 A-C** illustrate an embodiment of a passageway comprising a baffle for removing condensation aerosol particles whose size is not optimal for deep lung delivery and subsequent rapid PK. **FIGs. 44A** and **B** illustrate exterior views of the passageway comprising the baffle, while **FIG. 44C** provides an interior view of a cone shaped baffle (**4402**) and its orientation within a passageway through which a condensation aerosol flows (**4410**). In **FIG. 44C,** a condensation aerosol comprising a pharmaceutically active agent (e.g., nicotine) generated by any means as provided herein enters a portion of a passageway comprising the baffle (**4402**) through an aerosol inlet (**4404**). The aerosol inlet can be a portion of a passageway downstream of a heater element that narrows following the area of the passageway that comprises the heater element. The aerosol inlet (**4404**) serves to funnel the aerosol through a narrowed passageway prior to the aerosol encountering the planar surface of the cone-shaped baffle (**4402**). Prior to the baffle (**4402**), the passageway widens, wherein the diameter of the planar surface of the baffle occupies a substantial portion of the diameter of the widened passageway. Upon entry into the widened passageway, the aerosol flows toward the baffle (**4402**), wherein large particles (> 5 $\mu$m) flow into the planar surface of the baffle, while small particles ($\leq$ 5 $\mu$m), flow around the edges of the baffle (**4402**). As the small particles flow around the baffle (**4402**), they flow into a wider passageway towards the outlet (**4406**) of the passageway. The widened passageway downstream of the baffle (**4402**) entrains the small particles into additional carrier gas (**4408**) that enters through secondary carrier gas (**4408**) inlets. In some cases, a flow of carrier gas through the passageway is about 1 to about 10 LPM (a range from about 1.667 x $10^{-5} m^3/s$ to about $1.667 \times 10^{-4} m^3/s$) (e.g., at a vacuum of about 1 to about 15 inches of water (a range from about 249 Pa to about 3738 Pa)), while the carrier gas (**4408**) entering through the secondary carrier gas (**4408**) inlets entrains the small particles in an air flow of about 20 to about 80 LPM (a range from about $3 \times 10^{-4} m^3/s$ to about $1.3 \times 10^{-3} m^3/s$). In some cases, the passageway depicted in **FIG. 44C** is connected to and downstream of the passageways depicted in any one of **FIGs. 31A-D,** wherein the passageway depicted in **FIG. 44C** is connected at the aerosol inlet (**4404**). In some cases, the aerosol inlet of the passageway depicted in **FIG. 44C** is a downstream extension of the passageways depicted in any one of **FIGs. 31A-D.**

The inner diameter of the passageway at the aerosol inlet of **FIG. 44C** and downstream of the narrow channel can be can be exactly, about, more than, less than, at least or at most 0.2, 0.21, 0.22, 0.23, 0.24, 0.25, 0.26, 0.27, 0.28, 0.29, 0.3, 0.35, 0.4, 0.45, or 0.5 inches (a range from about 0.508 mm to about 12.7 mm). The inner diameter of the passageway at the aerosol inlet of **FIG. 44C** and downstream of the narrow channel can be between 0.2-0.21, 0.21-0.22, 0.22-0.23, 0.23-0.24, 0.24-0.25, 0.25-0.26, 0.26-0.27, 0.27-0.28, 0.28-0.29, 0.29-0.3, 0.3-0.35, 0.35-0.4, 0.4-0.45, or 0.45-0.5 inches (a range from about 0.508 mm to about 12.7 mm). The inner diameter of the passageway at the aerosol inlet of **FIG. 44C** and downstream of the narrow channel can be about 0.2 to about 0.25, about 0.25 to about 0.3, about 0.3 to about 0.35, about 0.35 to about 0.4, about 0.4 to about 0.45, or about 0.45 to about 0.5 inches (a range from about 0.508 mm to about 12.7 mm). The inner diameter of the outlet (**4406**) can be exactly, about, more than, less than, at least or at most 0.2, 0.21, 0.22, 0.23, 0.24, 0.25, 0.26, 0.27, 0.28, 0.29, 0.3, 0.35, 0.4, 0.45, or 0.5 inches (a range from about 0.508 mm to about 12.7 mm). The inner diameter of the outlet (**4406**) can be between 0.2-0.21, 0.21-0.22, 0.22-0.23, 0.23-0.24, 0.24-0.25, 0.25-0.26, 0.26-0.27, 0.27-0.28, 0.28-0.29, 0.29-0.3, 0.3-0.35, 0.35-0.4, 0.4-0.45, or 0.45-0.5 inches (a range from about 0.508 mm to about 12.7 mm). The inner diameter of the outlet (**4406**) can be about 0.2 to about 0.25, about 0.25 to about 0.3, about 0.3 to about 0.35, about 0.35 to about 0.4, about 0.4 to about 0.45, or about 0.45 to about 0.5 inches (a range from about 0.508 mm to about 12.7 mm). The inner diameter of the narrow channel can be exactly, about, more than, less than, at least or at most 0.01, 0.0125, 0.015, 0.0175, 0.02, 0.0225, 0.025, 0.0275, 0.03, 0.0325, 0.035, 0.0375, 0.04, 0.0425, 0.045, 0.0475, 0.05, 0.0525, 0.055, 0.0575, 0.06, 0.0625, 0.065, 0.0675, 0.07, 0.0725, 0.075, 0.0775, 0.08, 0.0825, 0.085, 0.0875, 0.09, 0.0925, 0.095, 0.0975, 0.1, 0.11, 0.115, 0.12, 0.125, 0.13, 0.135, 0.14, 0.145, or 0.15 inches (a range from about 0.254 mm to about 3.81 mm). The inner diameter of the narrow channel can be between 0.01-0.015, 0.015-0.02, 0.02-0.025, 0.025-0.03, 0.03-0.035, 0.035-0.04, 0.04-0.045, 0.045-0.05, 0.05-0.055, 0.055-0.06, 0.06-0.065, 0.065-0.07, 0.07-0.075, 0.075-0.08, 0.08-0.085, 0.085-0.09, 0.09-0.095, 0.095-0.1, 0.1-0.12, 0.12-0.13, 0.13-0.14, or 0.14-0.15 inches (a range from about 0.254 mm to about 3.81 mm). The inner diameter of the narrow channel can be about 0.01 to about 0.015, about 0.015 to about 0.02, about 0.02 to about 0.025, about 0.025 to about 03, about 0.03 to about 0.035, about 0.035 to about 0.04, about 0.04 to about 0.045, about 0.045 to about 0.05, about 0.05 to about 0.055, about 0.055 to about 0.06, about 0.06 to about 0.065, about 0.065 to about 0.07, about 0.07 to about 0.075, about 0.075 to about 0.08, about 0.08 to about 0.085, about 0.085 to about 0.09, about 0.09 to about 0.095, about 0.095 to about 0.1, or about 0.1 to about 0.15 inches (a range from about 0.254 mm to about 3.81 mm).

## Flow regulation

A device provided herein can be configured to limit a flow of a carrier gas through the passageway or aerosol generation area/chamber to permit condensation of the vaporized liquid formulation. The carrier gas can be air. The flow of a carrier gas through the aerosol generation chamber or passageway comprising or in fluid communication with the heater element can be limited to about 1 to about 10 liters per minute (LPM) (a range from about 1.667 x $10^{-5}$ m$^3$/s to about 1.667 x $10^{-4}$ m$^3$/s). The device can be configured to comprise a flow resistance (to inhalation) of about 0.05 to about 0.15 sqrt (cm-H$_2$O)/LPM. The device can be configured to comprise an inhalation resistance comprising a vacuum pressure of about 1 to about 10 inches of H20 (a range from about 249 Pa to about 2488 Pa).

Methods are provided herein for sensing an inhalation by a user and triggering a device. For example, an optical sensor that uses a deformable member (e.g., a vane) that moves during inhalation can be used to either open or close an optical path. In some embodiments, a Hall effect sensor is used to measure inhalation. In one embodiment, inhalation sensing is accomplished using an optical signal wherein a unique pattern of light pulses is sent along an optical path or light pipe and resent back along the optical path to a light detector. In one embodiment, the optical signal is sent from a controller into a dose cartridge whereby it is resent back into the controller to a light detector. In one embodiment, a vane is positioned in the path of an airway such that when an inhalation occurs, the vane is deflected out of the way and interrupts the optical signal. In this case, the device notes the absence of the optical signal and triggers the creation of an aerosol.

Methods are provided herein for inhalation flow control. In some cases, a valve system to allow for a user to experience an initial high pressure and low flow rates, followed by low pressure is used. An initial high-pressure drop through the device to facilitate the ejection of an agent (e.g., nicotine) from a dosing mechanism can be used. The following high flow rate can facilitate deep lung delivery. In one embodiment, a slide valve with an attached piston mechanism is used to eject an agent (e.g., nicotine) from a dosing reservoir. In one embodiment, air flow over a vaporizing agent (e.g., nicotine) formulation is regulated and controlled to an optimum level using a valve system, resulting in optimum particle sizing and dosing effectiveness. In a one embodiment, a valve system is used to create an internal air or inhalation resistance that is low (e.g., 0.08 to 0.12 (cm H$_2$O)$^{1/2}$/LPM).

In some cases, a device for generating a condensation aeorosol as provided herein can comprise a heater element. In some cases, a device provided herein can comprise a passageway, wherein the passageway comprises a heater element and a reservoir. In some cases, the device comprises a passageway, a reservoir, and a housing which comprises a heater element, wherein the passageway is in fluid communication with the heater element. The passageway comprising the heater element or in fluid communication with the heater element can comprise an aerosol generation area or chamber. In some cases, the aerosol generation area or chamber comprises the heater element. In some cases, the aerosol generation area or chamber comprises the heater element and a source of a formulation comprising an agent as provided herein. The source can be a tube, e.g., capillary tube, or a reservoir. The tube, e.g., capillary tube can be coupled to the reservoir. The reservoir can comprise the liquid formulation. The reservoir can be in fluid communication with the heater element. The reservoir can serve to deliver the liquid formulation to the heater element, wherein the liquid formulation can wick onto the heater element. The reservoir can comprise a tube, e.g., capillary tube, wherein the tube, e.g., capillary tube can deliver the liquid formulation onto the heater element.

In some cases, a device for generating a condensation aeorosol as provided herein comprises an aerosol generation chamber. The aerosol generation chamber can comprise a heater element. The aerosol generation chamber can comprise a source of a liquid formulation comprising a pharmaceutically active agent (e.g. nicotine). In some cases, the aerosol generation chamber comprises a heater element and a source of a liquid formulation comprising a pharmaceutically active agent (e.g. nicotine). The aerosol generation chamber can be within a primary flow-through passageway. In some cases, a device for producing a condensation aerosol as provided herein comprises a flow-through passageway, wherein the flow-through passageway comprises an upstream opening and a downstream opening, wherein the flow-through passageway comprises an aeorosol generation chamber between the upstream and downstream openings of the flow-through passageway. The passageway can be a primary flow-through passageway. The primary flow-through passageway can be in fluid communication with a secondary flow-through passageway as provided herein. In some cases, the aerosol generation chamber further comprises a nozzle as provided herein. In some cases, a device for generating a condensation aeorosol as provided herein comprises an aerosol generation chamber, wherein the aerosol generation chamber is within a passageway configured to limit the flow of a carrier gas through the aerosol generation chamber to a flow rate effective for producing a condensation aerosol comprising particles of a size suitable for delivery to the deep lung of a subject. The flow rate can be limited to about 1 to about 10 liters per minute (LPM) (a range from about $1.667 \times 10^{-5}$ m$^3$/s to about $1.667 \times 10^{-4}$ m$^3$/s) at, e.g., a vacuum of about 1 to about 15 inches of water (a range from about 249 Pa to about 3738 Pa).

In some cases, a device for producing a condensation aerosol as provided herein comprises a primary flow-through passageway, wherein the primary flow-through passageway comprises an upstream opening and a downstream opening, wherein the upstream opening comprises an inlet for a carrier gas (e.g., air) and the downstream opening comprises an outlet for the carrier gas (e.g., air). The passageway can be a primary flow-through passageway. The primary flow-through passageway can be in fluid communication with a secondary flow-through passageway as provided herein. The inlet can comprise a flow restrictor configured to limit the flow of the carrier gas through primary flow-through passageway to a flow rate effective for producing a condensation aerosol comprising particles of a size suitable for delivery to the deep lung of a subject. The flow restrictor can limit the flow rate to about 1 to about 10 liters per minute (LPM) (a range from about $1.667 \times 10^{-5}$ m$^3$/s to about $1.667 \times 10^{-4}$ m$^3$/s), e.g., at a vacuum of about 1 to about 15 inches of water (a range from about 249 Pa to about 3738 Pa). The flow restrictor can be a valve or an orifice comprising dimensions that limit the flow of a carrier gas (e.g., air) to a rate suitable for producing a condensation aerosol comprising particles of a size suitable for delivery to the deep lung of a subject.

An orifice for air that passes over the heater element can have a diameter of about, more than, less than, or at least 0.01, 0.012, 0.015, 0.02, 0.022, 0.025, 0.03, 0.032, 0.035, 0.04, 0.042, 0.045, 0.05, 0.052, 0.055, 0.06, 0.062, 0.065, 0.07, 0.075, 0.08, 0.085, 0.09, 0.1, 0.105, 0.11, 0.12, 0.13, 0.14, 0.15, 0.16, 0.17, 0.18, 0.19, 0.2, 0.21, 0.22, 0.23, 0.24, 0.25, 0.26, 0.27, 0.28, 0.29, 0.3, 0.31, 0.32, 0.33, 0.34, 0.35, 0.36, 0.37, 0.38, 0.39, 0.4, 0.41, 0.42, 0.43, 0.44, 0.45, 0.46, 0.47, 0.48, 0.49, 0.5, 0.51, 0.52, 0.53, 0.54, 0.55, 0.56, 0.57, 0.58, 0.59, 0.6, 0.61, 0.62, 0.63, 0.64, 0.65, 0.66, 0.67, 0.68, 0.69, 0.7, 0.71, 0.72, 0.73, 0.74, 0.75, 0.76, 0.77, 0.78, 0.79, or 0.8 inches (a range from about 0.254 mm to about 20.32 mm). In some cases, an orifice for air that passes over a heater element has a diameter of about 0.01 to about 0.12 inches, about 0.02 to about 0.1 inches, about 0.03 to about 0.09 inches, about 0.04 to about 0.08 inches, or about 0.05 to about 0.07 inches, or about 0.15 to about 3 inches (a range from about 0.254 mm to about 76.2 mm). An orifice for bypass air (air that is routed around a heater element) can have a diameter of about, more than, less than, or at least 0.02, 0.04, 0.06, 0.08, 0.1, 0.12, 0.14, 0.16, 0.18, 0.2, 0.22, 0.24, 0.26, 0.28, 0.3, 0.32, 0.34, 0.36, 0.38, 0.4, 0.42, 0.44, 0.46, 0.48, 0.5, 0.52, 0.54, 0.56, 0.58, 0.6, 0.62, 0.64, 0.66, 0.68, 0.7, 0.8, 0.9, 1, or 1.2 inches (a range from about 0.508 mm to about 30.48 mm). In some cases, an orifice for bypass air (air that is routed around a heater element) has a diameter of about 0.05 to about 0.4 inches, about 0.1 to about 0.3 inches, or about 0.1 to about 0.4 inches (a range from about 1.27 mm to about 10.16 mm).

In some cases, a device for producing a condensation aerosol as provided herein comprises a flow-through passageway, wherein the flow-through passageway comprises an upstream opening and a downstream opening, wherein the flow-through passageway is configured to facilitate formation of a condensation aerosol comprising particles of a size effective for delivery to the deep lung of a subject. The particles can comprise an MMAD of about 1 to about 5 $\mu$m. The subject can be a human. The subject can be a human who smokes and/or uses tobacco or nicotine containing products. The condensation aerosol can comprise a pharmaceutically active agent (e.g. nicotine). The passageway can be a primary flow-through passageway. The primary flow-through passageway can be in fluid communication with a secondary flow-through passageway as provided herein. The upstream opening can be an inlet. The inlet can comprise a flow restrictor as provided herein. The downstream opening can comprise an outlet. The outlet can be a mouthpiece.

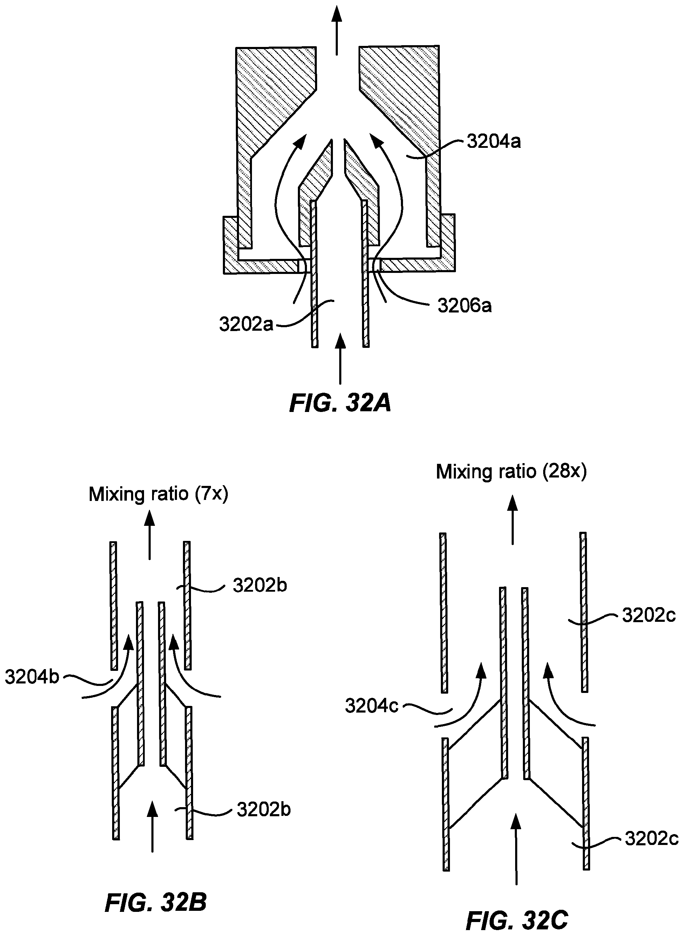

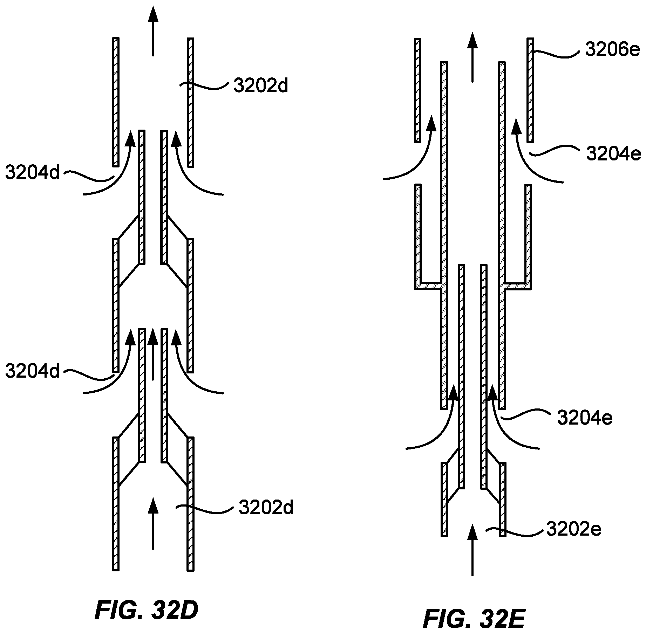

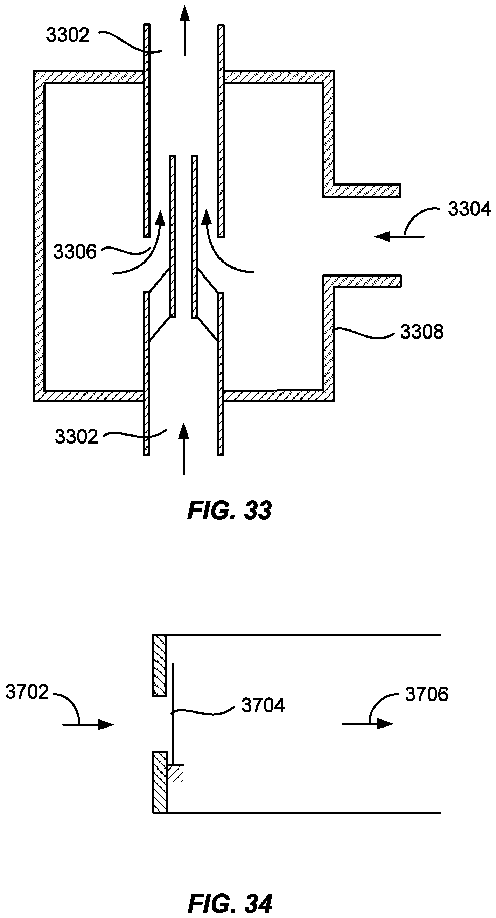

The flow-through passageway can be configured to form a narrow channel between the upstream and downstream openings. The passageway can be further configured to widen downstream of the narrow channel prior to the downstream opening of the passageway. The narrow channel can comprise an inner diameter and an outer diameter (see, e.g., **FIG. 32** and **33**). The inner diameter of the narrow channel can be exactly, about, more than, less than, at least or at most 0.01, 0.0125, 0.015, 0.0175, 0.02, 0.0225, 0.025, 0.0275, 0.03, 0.0325, 0.035, 0.0375, 0.04, 0.0425, 0.045, 0.0475, 0.05, 0.0525, 0.055, 0.0575, 0.06, 0.0625, 0.065, 0.0675, 0.07, 0.0725, 0.075, 0.0775, 0.08, 0.0825, 0.085, 0.0875, 0.09, 0.0925, 0.095, 0.0975, 0.1, 0.11, 0.115, 0.12, 0.125, 0.13, 0.135, 0.14, 0.145, or 0.15 inches (a range from about 0.254 mm to about 3.81 mm). The inner diameter of the narrow channel can be between 0.01-0.015, 0.015-0.02, 0.02-0.025, 0.025-0.03, 0.03-0.035, 0.035-0.04, 0.04-0.045, 0.045-0.05, 0.05-0.055, 0.055-0.06, 0.06-0.065, 0.065-0.07, 0.07-0.075, 0.075-0.08, 0.08-0.085, 0.085-0.09, 0.09-0.095, 0.095-0.1, 0.1-0.12, 0.12-0.13, 0.13-0.14, or 0.14-0.15 inches (a range from about 0.254 mm to about 3.81 mm). The inner diameter of the narrow channel can be about 0.01 to about 0.015, about 0.015 to about 0.02, about 0.02 to about 0.025, about 0.025 to about 03, about 0.03 to about 0.035, about 0.035 to about 0.04, about 0.04 to about 0.045, about 0.045 to about 0.05, about 0.05 to about 0.055, about 0.055 to about 0.06, about 0.06 to about 0.065, about 0.065 to about 0.07, about 0.07 to about 0.075, about 0.075 to about 0.08, about 0.08 to about 0.085, about 0.085 to about 0.09, about 0.09 to about 0.095, about 0.095 to about 0.1, or about 0.1 to about 0.15 inches (a range from about 0.254 mm to about 3.81 mm). The outer diameter of the narrow channel can be exactly, about, more than, less than, at least or at most 0.08, 0.0825, 0.085, 0.0875, 0.09, 0.0925, 0.095, 0.0975, 0.1, 0.11, 0.115, 0.12, 0.125, 0.13, 0.135, 0.14, 0.145, or 0.15 inches (a range from about 2.0 mm to about 3.81 mm). The outer diameter of the narrow channel can be between 0.08-0.085, 0.085-0.09, 0.09-0.095, 0.095-0.1, 0.1-0.12, 0.12-0.13, 0.13-0.14, or 0.14-0.15 inches (a range from about 2.0 mm to about 3.81 mm). The outer diameter of the narrow channel can be about 0.08 to about 0.085, about 0.085 to about 0.09, about 0.09 to about 0.095, about 0.095 to about 0.1, or about 0.1 to about 0.15 inches (a range from about 2.0 mm to about 3.81 mm). The inner diameter of the flow-through passageway prior to and/or downstream of the narrow channel can be exactly, about, more than, less than, at least or at most 0.2, 0.21, 0.22, 0.23, 0.24, 0.25, 0.26, 0.27, 0.28, 0.29, 0.3, 0.35, 0.4, 0.45, or 0.5 inches (a range from about 5.08 mm to about 12.7 mm). The inner diameter of the flow-through passageway prior to and/or downstream of the narrow channel can be between 0.2-0.21, 0.21-0.22, 0.22-0.23, 0.23-0.24, 0.24-0.25, 0.25-0.26, 0.26-0.27, 0.27-0.28, 0.28-0.29, 0.29-0.3, 0.3-0.35, 0.35-0.4, 0.4-0.45, or 0.45-0.5 inches (a range from about 5.08 mm to about 12.7 mm). The inner diameter of the flow-through passageway prior to and/or downstream of the narrow channel can be about 0.2 to about 0.25, about 0.25 to about 0.3, about 0.3 to about 0.35, about 0.35 to about 0.4, about 0.4 to about 0.45, or about 0.45 to about 0.5 inches (a range from about 5.08 mm to about 12.7 mm).

In some cases, a device for generating a condensation aeorosol comprising a primary flow-through passageway as provided herein further comprises a secondary flow-through passageway. The secondary flow-through passageway can be in fluid communication with the primary flow through passageway. The secondary flow-through passageway can comprise one or more channels. In some cases, the secondary flow -through channel comprises a first, a second, and a third channel. The first channel can be in fluid communication with a primary flow-through chamber upstream of an aerosol generation chamber as provided herein. The second channel can be in fluid communication with a primary flow through passageway between an aerosol generation chamber as provided herein and a downstream opening of the primary flow through passageway. The third channel can comprise a second inlet for a carrier gas (e.g. air) and can be in fluid communication with the second channel. The secondary flow-through passageway can also comprise an articuable element. The articuable element can be a diaphragm. The articuable element can be further connected to springs. The springs can control the movement of the articuable element. The articuable element can be articulated by changes in pressure within the device. The pressure that articulates the articuable element can be inhalation resistance or vacuum pressure. The inhalation resistance can be a vacuum of about 1 to about 10 inches of $H_2O$ (a range from about 249 Pa to about 2488 Pa). An increase in pressure can compress the springs. Inhalation through a device for generating a condensation aerosol as provided herein can increase the pressure in the device. The articuable element can comprise a protruding member. In some cases, one or more springs are located on a first side of an articuable element, while the protruding member is located on a second side opposite the first side. The protruding member can be configured to enter and block the third channel. A pressure differential between primary and secondary flow-through passageways within the device can cause articulation or movement of the articuable element. The pressure differential can be affected by inhalation through the downstream opening of the primary flow chamber. The pressure differential can be across the first channel of the secondary flow chamber. Under conditions of low pressure or inhalation resistance, the articuable element can block the third channel, thereby preventing entry of the carrier gas (e.g. air). Under conditions of increased pressure or inhalation resistance, the articuable element can be articulated or removed from blocking the third channel, thereby allowing the carrier gas to enter the device. In some cases, inhalation through the downstream opening of the primary flow -through passageway serves to articulate the articuable element, whereby the articulation serves to open the third channel, wherein the opening permits the carrier gas (e.g. air) to flow through the third channel of the secondary flow-through passageway and enter the primary flow through passageway through the second channel in the secondary flow-through passageway, thereby entraining the condensation aerosol in the carrier gas from the secondary flow-through passageway. Additional carrier gas entering the primary flow-through passageway through the secondary flow-through passageway as described herein can entrain the condensation aerosol in the carier gas (e.g. air) to produce a total flow rate of about 20 to about 80 LPM (a range from about $3 \times 10^{-4}$ m$^3$/s to about $1.3 \times 10^{-3}$ m$^3$/s). The device can have an interior air resistance (to inhalation) no greater than that of a cigarette. The device can have an interior air resistance (to inhalation) of about 0.05 to about 0.15 (cm H$_2$O)$^{1/2}$/LPM.

A device for generating condensation aerosols comprising a primary flow-through passageway as provided herein can further comprise one or more additional sources of carrier gas, wherein the additional sources permit the flow of carrier gas to enter the device in addition to the carrier gas flowing through the primary flow-through passageway. The one or more additional sources can be inlets or channels. The one or more additional sources can be bypass inlets or bypass channels, wherein carrier gas entering a device through the bypass inlets or channels is bypass carrier gas. The bypass carrier gas can be air. The one or more sources can be within one or more walls of the primary flow-through passageway. The one or more sources can be components of a secondary flow-through passageway as provided herein, wherein the secondary flow-through passageway can be in fluid communication with the primary flow-through passageway. The one or more sources can be within one or more walls of the secondary flow-through passageway. The one or more sources can be within one or more walls of a housing, wherein the housing surrounds or encompasses the primary flow-through passageway. The one or more sources can be flow regulators. The carrier gas entering the device through the one or more sources can be the same type or a different type of carrier gas as that flowing through a primary flow-through passageway. In some cases, the carrier gas entering through the one or more sources can be air. In some cases, the one or more sources permit flow of carrier gas to enter the device downstream of a heater element or aerosol generation chamber or area as provided herein. The flow of carrier gas entering the device through the one or more sources can mix with the carrier gas flowing through a primary flow through passageway. The mixing can be downstream of a heater element or aerosol generation chamber as provided herein but before a downstream opening or outlet of a primary passageway comprising the heater element or aersol generation chamber. The mixing of the carrier gases can produce a total flow rate exiting the device that can be similar to normal breathing of a subject. The total flow rate can be about 20 to about 80 LPM (a range from about $3 \times 10^{-4}$ m$^3$/s to about $1.3 \times 10^{-3}$ m$^3$/s). The subject can be a human. The subject can be a human who smokes and/or uses tobacco or nicotine containing products.

**FIG. 21** illustrates an embodiment of an electronic agent (e.g., nicotine) delivery device comprising a valve system **(2100)** for controlling air flow for deep lung delivery and rapid PK. Upon inhalation, negative pressure in a mouthpiece **(2102)** increases causing a pressure drop across a gas control valve **(2104)**. An increase in the pressure drop can cause the valve **(2104)** to close and prevent airflow **(2106)** into an aerosol generating area **(2108)** within a flow through chamber **(2110)**. The aerosol generating area **(2108)** can comprise an agent (e.g., nicotine) reservoir comprising an agent (e.g., nicotine) formulation, any of the dosing mechanisms described herein, and a heater for vaporizing an agent (e.g., nicotine) droplets that can be released from the dosing mechanism. Closing of the valve **(2104)** can subsequently cause an increase in airflow **(2106)** from an air inlet **(2112)** across a backflow valve **(2114)** through a diversion air orifice **(2116)** and into a diversion air channel **(2118)**. In this manner, the airflow over a vaporizing agent (e.g., nicotine) formulation can be regulated and controlled to an optimal level in order to achieve optimum particle sizing and dosing effectiveness. In one embodiment, the valve system produces an inhalation resistance no greater than that of a cigarette. In one embodiment, the valve system produces an inhalation resistance no greater than 0.08 (cm H$_2$O)$^{1/2}$/LPM.

**FIG. 32 A-E** illustrates multiple embodiments of a device for regulating the flow of a carrier gas (e.g. air). In each embodiment, the device comprises a primary flow-through passageway **(3202A-E)** and one or more sources of bypass or additional carrier gas **(3204A-E).** In each embodiment, the one or more sources of bypass or additional carrier gas **(3204A-E)** permit an additional or bypass flow of carrier gas (e.g. air) to mix with the carrier gas flowing through the primary flow-through passageway **(3202A-E)**. In some cases, the mixing occurs downstream of an aerosol generation chamber, thereby mixing a condensation aerosol produced in the aerosol generation chamber with a larger volume of carrier gas (e.g. air). The mixing can produce a total flow rate downstream of the mixing of about 20 to about 80 liters per minute (LPM) (a range from about $3 \times 10^{-4}$ m$^3$/s to about $1.3 \times 10^{-3}$ m$^3$/s). **FIG. 32A** shows a device comprising a primary flow-through passageway **(3202a)** comprising an upstream and downstream section comprising an inner diameter of 0.25 inches (about 6.35 mm), and two secondary flow-through chambers **(3204a),** wherein bypass or additional carrier gas enters the device through two inlets **(3206a)** adjacent to the primary flow-through chamber **(3202a)**. The inner diameter of the primary flow through chamber **(3202a)** narrows just prior to entry of the bypass carrier gas. In some cases, the narrowing of the primary flow-through passageway permits formation of condensation aerosol particles comprising particles with an MMAD of about 1 to about 5 uM. The device in **FIG. 32A** can permit the mixing of the bypass carrier gas with the carrier gas flow through the primary chamber at a ratio of 10:1.

**FIG. 32B** shows a device comprising a primary flow-through passageway **(3202b)** comprising an upstream and downstream section comprising an inner diameter of 0.25 inches (about 6.35 mm), and two inlets **(3204b)** within the wall of the primary flow-through chamber **(3202b),** wherein bypass or additional carrier gas enters the device. The primary flow through chamber **(3202b)** narrows just prior to entry of the bypass carrier gas to comprise an inner diameter of 0.084 inches (about 2.13 mm) and an outer diameter of 0.108 inches (about 2.74 mm). In some cases, the narrowing of the primary flow-through passageway **(3202b)** permits formation of condensation aerosol particles comprising particles with an MMAD of about 1 to about 5 um. The device in **FIG. 32B** can permit the mixing of the bypass carrier gas with the carrier gas flow through the primary chamber at a ratio of 7:1.

**FIG. 32C** shows a device comprising a primary flow-through passageway **(3202c)** comprising an upstream and downstream section comprising an inner diameter of 0.5 inches (about 12.7 mm), and two inlets **(3204c)** within the wall of the primary flow-through chamber **(3202c),** wherein bypass or additional carrier gas enters the device. The primary flow through chamber **(3202c)** narrows just prior to entry of the bypass carrier gas to comprise an inner diameter of 0.084 inches (about 2.13 mm) and an outer diameter of 0.108 inches (about 2.74 mm). In some cases, the narrowing of the primary flow-through passageway **(3202c)** permits formation of condensation aerosol particles comprising particles with an MMAD of about 1 to about 5 $\mu$m. The device in **FIG. 32C** can permit the mixing of the bypass carrier gas with the carrier gas flow through the primary chamber at a ratio of 28:1.

**FIG. 32D** shows a device comprising a primary flow-through passageway **(3202d)** comprising an upstream and downstream section comprising an inner diameter of 0.25 inches (about 6.35 mm), and two sets of two inlets **(3204d)** adjacent to the primary flow-through chamber **(3202d),** wherein bypass or additional carrier gas enters the device. The flow through chamber narrows just prior to entry of the bypass carrier gas from each set of two inlets to comprise an inner diameter of 0.096 inches (about 2.44 mm) and an outer diameter of 0.125 inches (about 3.175 mm). Following the first set of two inlets, the primary flow through passageway widens to an inner diameter of 0.250 inches (about 6.35 mm), before narrowing again. In some cases, the narrowing of the primary flow-through passageway permits formation of condensation aerosol particles comprising particles with an MMAD of about 1 to about 5 $\mu$m. The device in **FIG. 32D** can permit the mixing of the bypass carrier gas with the carrier gas flow through the primary chamber at a ratio of 35:1.

The device in **FIG. 32E** is similar to the device in **FIG. 32D,** wherein **FIG. 32E** shows a device comprising a primary flow-through passageway **(3202e)** comprising an upstream and downstream section comprising an inner diameter of 0.250 inches (about 6.35 mm), and two sets of two inlets **(3204e)** adjacent to the primary flow-through chamber **(3202e),** wherein bypass or additional carrier gas enters the device. The primary flow through chamber **(3202e)** narrows just prior to entry of the bypass carrier gas from the first set of two inlets to comprise an inner diameter of 0.096 inches (about 2.44 mm) and an outer diameter of 0.125 inches (about 3.175 mm). Following the first set of two inlets, the primary flow through passageway **(3202e)** widens to an inner diameter of 0.250 inches (about 6.35 mm) and an out diameter of 0.280 inches (about 7.112 mm). Subsequently, the primary flow-through passageway **(3202e)** opens into a secondary housing **(3206e)**, which has an inner diameter of 0.466 inches (about 11.8 mm). In **FIG. 32E,** the second pair of inlets **(3204e)** are located in the wall of a secondary housing **(3206e)**, which is coupled to and encompasses the primary flow-through passageway.

**FIG. 33** illustrates another embodiment of a device for regulating the flow of a carrier gas (e.g. air). **FIG. 33** shows a device comprising a primary flow-through passageway **(3302)** comprising an upstream and downstream section comprising an inner diameter of 0.25 inches (about 6.35 mm), and two inlets **(3306)** within the wall of the primary flow-through chamber **(3302)**, wherein bypass or additional carrier gas enters the device. The primary flow-through chamber narrows **(3302)** just prior to entry of the bypass carrier gas to comprise an inner diameter of 0.086 inches (about 2.18 mm) and an outer diameter of 0.106 inches (about 2.69 mm). As depicted in **FIG. 33,** the section of the primary flow-through chamber **(3302)** is coupled to and encased by a secondary housing **(3308)**. The secondary housing comprises a bypass inlet **(3304)**, which permits entry of bypass or additional carrier gas (e.g. air) to enter the primary flow-through passageway through the inlets **(3306)**. In some cases, the narrowing of the primary flow-through passageway permits formation of condensation aerosol particles comprising particles with an MMAD of about 1 to about 5 $\mu$m.

**FIG. 35** illustrates another embodiment a device for regulating the flow of a carrier gas (e.g. air). The device comprises a primary airway **(3504)** that comprises an aerosol generation chamber **(3528)** comprising a heater element **(3502)**, a restrictive orifice **(3514)** and a mouthpiece **(3506)**. The heater element **(3502)** comprises a coil. The heater element can be any heater element comprising a coil as provided herein. The primary airway **(3504)** is fluidically connected to a secondary airway **(3516)**, through a first channel **(3518)** located (disposed) between the restrictive orifice **(3514)** and heater element **(3502)**, and a second channel **(3520)** located (disposed) between the heater element **(3502)** and the mouthpiece **(3506)**. The secondary airway **(3516)** further comprises a third channel **(3530)** that is a secondary inlet **(3508)** for a carrier gas (e.g. air) and a diaphragm **(3510)**. The diaphragm **(3510)** comprises a base member that is connected to a pair of springs **(3512)** on a first side and a protruding member **(3524)** on a second side. The springs **(3512)** are additionally connected to a wall opposite the first side of the base member that is part of the housing of the secondary airway **(3516)**. The base member of the diaphragm **(3510)** is also connected to a pair of lateral springs **(3526)** on its lateral edges, which are further connected to the walls of the housing of the secondary airway **(3516)** opposite the lateral edges of the base member. The restrictive orifice **(3514)** is configured to limit the flow rate of the carrier gas (e.g.

air) through the aerosol generation chamber (**3528**) in order to allow for the condensation of a liquid formulation comprising a pharmaceutically active agent as provided herein vaporized by the heater element (**3502**) to particles comprising about 1 to about 5 um MMAD. The restrictive orifice (**3514**) limits the flow rate of the carrier gas (i.e. air) about 1 to about 10 liters per minute (LPM) (a range from about $1.667 \times 10^{-5}$ $m^3/s$ to about $1.667 \times 10^{-4}$ $m^3/s$) at, e.g., a vacuum of about 1 to about 15 inches of water (a range from about 249 Pa to about 3738 Pa). Inhalation through the mouthpiece (**3506**) can produce a flow of carrier gas (e.g. air) through the restrictive orifice (**3514**) that can produce an inhalation resistance. The inhalation resistance produces a pressure differential across the opening of the first channel (**3518**) connecting the primary airway (**3504**) with the secondary airway (**3516**). The inhalation resistance causes the springs (**3512**) coupled to the first side of the diaphragm (**3510**) to compress and the lateral springs (**3526**) coupled to the lateral edges of the diaphragm (**3510**) to extend, whereby the protruding member of coupled to the second side of the diaphragm (**3510**) is removed from the third channel (**3530**) of the secondary airway (**3516**). Removal of the protruding member (**3524**) causes an additional flow of carrier gas (e.g. air) to enter the device. The additional flow of carrier gas (e.g. air) then enters the primary airway (**3504**) downstream of the heater element (**3502**) and aerosol generation area (**3528**) through the second channel (**3520**). The additional flow of carrier gas (e.g. air) can serve to mix or entrain the condensation aerosol comprising particles of about 1 to about 5 $\mu$m to produce a total flow rate suitable for delivery of the particles to the deep lung of a user of the device.

A device for producing a condensation aerosol as provided herein can have an interior air resistance (to inhalation) no greater than 0.08 $(cm \ H_2O)^{1/2}/LPM$. The device can have an interior air resistance (to inhalation) exactly, about, more than, less than, at least, or at most 0.01, 0.02, 0.03, 0.04, 0.05, 0.06, 0.07, 0.08, 0.09, 0.10, 0.11, 0.12, 0.13, 0.14, 0.15, 0.16, 0.17, 0.18, 0.19, 0.20, or 0.25 $(cm \ H_2O)^{1/2}/LPM$. The device can have an interior air resistance (to inhalation) between 0.01-0.02, 0.02- 0.03, 0.03-0.04, 0.04-0.05, 0.05-0.06, 0.06- 0.07, 0.07-0.08, 0.08-0.09, 0.09-0.10, 0.1-0.11, 0.11-0.12, 0.12- 0.13, 0.13-0.14, 0.14-0.15, 0.15-0.16, 0.16-0.17, 0.17-0.18, 0.18-0.19, 0.19-0.20, or 0.20-0.25 $(cm \ H_2O)^{1/2}/LPM$. The device can have an interior air resistance (to inhalation) of about 0.01 to about 0.03, about 0.03 to about 0.05, about 0.05 to about 0.07, about 0.07 to about 0.09, about 0.09 to about 0.11, about 0.11 to about 0.13, about 0.13 to about 0.15, about 0.15 to about 0.17, about 0.17 to about 0.19, or about 0.19 to about 0.25 $(cm \ H_2O)^{1/2}/LPM$.

A device for producing a condensation aerosol as provided herein can produce a total flow rate of a carrier gas (e.g. air) of exactly, about, more than, less than, at least, or at most 1, 2, 3,4,5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, or 100 liters per min (LPM) (a range from about $1.667 \times 10^{-5}$ $m^3/s$ to about $1.667 \times 10^{-3}$ $m^3/s$). The total flow rate can be between 1-10, 10-20, 20-30, 30-40, 40-50, 50-60, 60-70, 70-80, 80-90, or 90-100 LPM (a range from about $1.667 \times 10^{-5}$ $m^3/s$ to about $1.667 \times 10^{-3}$ $m^3/s$). The total flow rate can be about 1 to about 10, about 10 to about 20, about 20 to about 30, about 30 to about 40, about 40 to about 50, about 50 to about 60, about 60 to about 70, about 70 to about 80, about 80 to about 90, or about 90 to about 100 LPM (a range from about $1.667 \times 10^{-5}$ $m^3/s$ to about $1.667 \times 10^{-3}$ $m^3/s$). The device can comprise a primary flow-through passageway for a carrier gas and one or more sources of additional or bypass carrier gas as provided herein. These flow rates can be at a vacuum of about 1 to about 15 inches of water (a range from about 249 Pa to about 3738 Pa).

The one or more sources of additional or bypass carrier gas (e.g. air) can be configured to limit the flow rate of additional or bypass carrier gas to produce a total flow rate as provided herein. The flow rate can be limited by using a restrictive orifice on the one or more sources of additional or bypass carrier gas (e.g. air). The restrictive orifice can comprise any valve or flap as known in the art. The valve or flap can be moderated at specific flow rates. The flow rates that moderate the valve or flap can be the limited to flow rates provided herein. The valve or flap can be opened at specific inhalation resistance levels. The restrictive orifice can be opened at inhalation resistances comprising a vacuum of about 1 to about 10 inches of water (a range from about 249 Pa to about 2488 Pa).

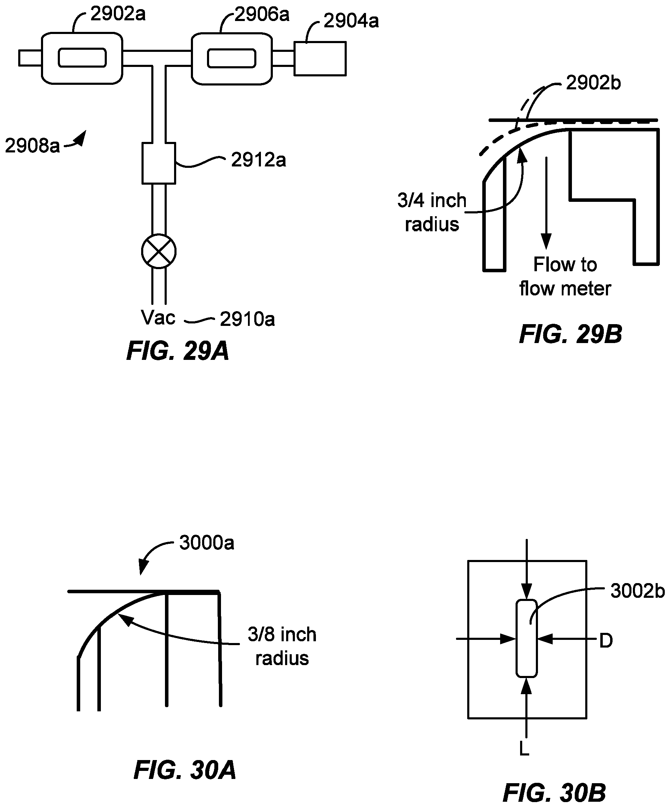

A device for producing a condensation aerosol as provided herein can be configured to limit the flow rate of a carrier gas across or through a aerosol generation area or heater element as provided herein to a flow rate of exactly, about, more than, less than, at least, or at most 1, 1.5, 2, 2.5, 3, 3.5, 4, 4.5, 5, 5.5, 6, 6.5, 7, 7.5, 8, 8.5, 9, 9.5, 10, 10.5, 11, 11.5, 12, 12.5, 13, 13.5, 14, 14.5, 15, 15.5, or 16 liters per minute (LPM) (a range from about $1.667 \times 10^{-5}$ $m^3/s$ to about $2.667 \times 10^{-4}$ $m^3/s$). A device for producing a condensation aerosol as provided herein can be configured to limit the flow rate of a carrier gas across or through a aerosol generation area or heater element to between 1-2, 2-4, 4-6, 6-8, 8-10, 10-12, 12-14, or 14-16 LPM a range from (about $1.667 \times 10^{-5}$ $m^3/s$ to about $2.667 \times 10^{-4}$ $m^3/s$). A device for producing a condensation aerosol as provided herein can be configured to limit the flow rate of a carrier gas across or through a aerosol generation area or heater element to about 1 to about 2, about 2 to about 4, about 4 to about 6, about 6 to about 8, about 8 to about 10, about 10 to about 12, about 12 to about 14, or about 14 to about 16 LPM (a range from about $1.667 \times 10^{-5}$ $m^3/s$ to about $2.667 \times 10^{-4}$ $m^3/s$). The flow rate can be limited by using a restrictive orifice on the inlet for a carrier gas (e.g. air). The restrictive orifice can comprise any valve or flap as known in the art. The valve or flap can be moderated at specific flow rates. The flow rates that moderate the valve or flap can be the limited flow rates provided herein. The valve or flap can be opened at specific inhalation resistance levels. The restrictive orifice can be opened at inhalation resistances comprising a vacuum of about 1 to about 10 inches of water (a range from about 249 Pa to about 2488 Pa). The restrictive orifice can be configured to limit the flow rates to flow rates as provided herein. The restrictive orifice can be configured into a slot as depicted in **FIG. 30B.** An aerosol generation area or heater element as provided herein can be within a flow-through passageway. The flow-through passageway can be a primary flow through passageway.

A device for producing a condensation aerosol comprising a primary flow-through passageway and one or more sources of additional or bypass carrier gas (e.g. air) as provided herein can produce a mixing ratio of bypass or additional carrier gas to carrier gas flowing through the primary flow through chamber of 1:1, 2:1, 3:1, 4:1, 5:1, 6:1, 7:1, 8:1, 9:1, 10:1, 11:1, 12:1, 13:1, 14:1, 15:1, 16:1, 17:1, 18:1, 19:1, 20:1, 21:1, 22:1, 23:1, 24:1, 25:1, 26:1, 27:1, 28:1, 29:1, 30:1, 31:1, 32:1, 33:1, 34:1, 35:1, 36:1, 37:1, 38:1, 39:1, 40:1, 41:1, 42:1, 43:1, 44:1, 45:1, 46:1, 47:1, 48:1, 49:1, or 50:1. The mixing ratio can be between 1:1 and 5:1, 5:1 and 10:1, 10:1 and 15:1, 15:1 and 20:1; 20:1 and 25:1, 25:1, and 30:1, 30:1, and 35:1, 35:1 and 40:1, 40:1 and 45:1, or 45:1 and 50:1. The mixing ratio can be about 1:1 to about 5:1, about 5:1 to about 10:1, about 10:1 to about 15:1, about 15:1 to about 20:1; about 20:1 to about 25:1, about 25:1 to about 30:1, about 30:1 to about 35:1, about 35:1 to about 40:1, about 40:1 to about 45:1, or about 45:1 to about 50:1.

## Device Dimensions

In some cases, an electronic agent (e.g., nicotine) delivery device comprises the dimensions of an electronic cigarette. The electronic agent (e.g., nicotine) delivery device can have an overall cyclindrical shape. The electronic agent (e.g., nicotine) delivery device can resemble a combustible cigarette. An_electronic agent (e.g., nicotine) delivery device as provided herein can have an outer diameter of about, more than, less than, or at least 0.001, 0.0015, 0.002, 0.0025, 0.003, 0.0035, 0.004, 0.0045, 0.005, 0.0055, 0.006, 0.0065, 0.007, 0.0075, 0.008, 0.0085, 0.009, 0.0095, 0.01, 0.015, 0.02, 0.025, 0.03, 0.035, 0.04, 0.045, 0.05, 0.055, 0.06, 0.065, 0.07, 0.075, 0.08, 0.085, 0.09, 0.095, 0.1, 0.11, 0.12, 0.13, 0.14, 0.15, 0.16, 0.17, 0.18, 0.19, 0.2, 0.21, 0.22, 0.23, 0.24, 0.25, 0.26, 0.27, 0.28, 0.29, 0.3, 0.31, 0.32, 0.33, 0.34, 0.35, 0.36, 0.37, 0.38, 0.39, 0.4, 0.41, 0.42, 0.43, 0.44, 0.45, 0.46, 0.47, 0.48, 0.49, 0.5, 0.51, 0.52, 0.53, 0.54, 0.55, 0.56, 0.57, 0.58, 0.59, 0.6, 0.61, 0.62, 0.63, 0.64, 0.65, 0.66, 0.67, 0.68, 0.69, 0.7, 0.71, 0.72, 0.73, 0.74, 0.75, 0.76, 0.77, 0.78, 0.79, 0.8, 0.81, 0.82, 0.83, 0.84, 0.85, 0.86, 0.87, 0.88, 0.89, 0.9, 0.91, 0.92, 0.93, 0.94, 0.95, 0.96, 0.97, 0.98, 0.99, 1, 2, 3, 4, 5, 6, 7, 8, 9, 10, 11, 12, 13, 14, 15, 16, 17, 18, 19, 20, 21, 22, 23, 24, 25, 26 27, 28, 29, or 30 cm. An electronic agent (e.g., nicotine) delivery device as provided herein can have an outer diameter of about 0.5 cm to about 1 cm, about 0.25 cm to about 0.75 cm, about 0.25 cm to about 1 cm, or about 0.25 cm to about 1.5 cm.

An electronic agent (e.g., nicotine) delivery device as provided herein can have a length of about, more than, less than, or at least 20, 21, 22, 23, 24, 25, 26 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145, 146, 147, 148, 149, or 150 mm. An_electronic agent (e.g., nicotine) delivery device as provided herein can have a length of about 25 mm to about 75 mm, about 75 mm to about 125 mm, about 125 mm to about 150 mm, or about 75 mm to about 150 mm

An electronic agent (e.g., nicotine) delivery device as provided herein can have a transverse dimension of about, more than, less than, or at least 1, 2, 3, 4, 5, 6, 7, 8, 9, 10, 11, 12, 13, 14, 15, 16, 17, 18, 19, 20, 21, 22, 23, 24, 25, 26 27, 28, 29, 30, 31, 32, 33, 34, 35, 36, 37, 38, 39, or 40 mm.

## Agents

Any suitable agent (e.g., drug) can be used in the methods and devices described herein. Agents (e.g., pharmaceutically active agents) that can be used include, for example, drugs of one of the following classes: anesthetics, antibiotic, anticonvulsants, antidepressants, antidiabetic agents, antidotes, antiemetics, antihistamines, anti-infective agents, antineoplastics, antiparkisonian drugs, antirheumatic agents, antipsychotics, anxiolytics, appetite stimulants and suppressants, blood modifiers, cardiovascular agents, central nervous system stimulants, drugs for Alzheimer's disease management, a cold medication, COPD (chronic obstructive pulmonary disease) drug, cough medication, drugs for cystic fibrosis management, diagnostics, dietary supplements, drugs for erectile dysfunction, gastrointestinal agents, hormones, drugs for the treatment of alcoholism, drugs for the treatment of addiction, immunosuppressives, mast cell stabilizers, migraine preparations, motion sickness products, drugs for multiple sclerosis management, muscle relaxants, drugs for treating myocardial infarction, nonsteroidal antiinflammatories, opioids, other analgesics and stimulants, opthalmic preparations, osteoporosis preparations, pain medication, panic medication, prostaglandins, respiratory agents, sedatives and hypnotics, skin and mucous membrane agents, smoking cessation aids, Tourette's syndrome agents, urinary tract agents, insomnia medication, weight loss drug, and vertigo agents. In some cases, an agent is an herb, supplement, or vitamin.

An anesthetic can be ketamine, procaine, amethocaine, cocaine, prilocaine, bupivacaine, levobupivacaine, ropivacaine, mepivacaine, dibucaine, or lidocaine. An anesthetic can be desflurane, enflurane, halothane, isofurane, methoxyflurane, or sevoflurane, amobaribital, methohexital, thiamylal, thiopental, diazepam, lorazepam, midzolam, etomidate, or propofol. An anesthetic can be atracurium, ciastracurium besyalte, rapacuronium, rocuronium, succinylcholine, or suxamethonium chloride. An anesthetic can be articaine, benzocaine, benzonatate, butacaine, butanilicaine, chloroprocaine, cinchocaine, dimethocaine, eucaine, etidocaine, hexylcaine, levobupivacaine, mepivacaine, meprylcaine, metabutoxycaine, orthocaine, oxybuprocaine, phenacaine, piperocaine, pramocaine, prilocaine, procaine, proparacaine, propoxycaine, quinisocaine, ropivacaine, trimecaine, or tetracaine.

An antibiotic can be an aminoglycoside (e.g., amikacin, gentamicin, kanamycin, neomycin, netilmicin, tobramycin, paromomycin, spectinomycin); an ansamycin (e.g., geldanamycin, herbimycin, rifaximin, streptomycin); a carbacephem (e.g., loracarbef); a carbapenem (e.g., ertapenem, doripenem, imipenem/cilastatin, meropenem); a cephalosporin (first generation) (e.g., cefadroxil, cefazolin, cefalotin or cefalothin, cefalexin); a cephalosporin (second generation) (e.g., cefaclor, cefamandole, cefoxitin, cefprozil, cefuroxime); a cephalosporin (third generation) (e.g., cefixime, cefdinir, cefditoren, cefoperazone, cefotaxime, cefpodoxime, ceftazidime, ceftibuten, ceftizoxime, ceftriaxone); a cephalosporin (fourth generation) (e.g., cefepime); a cephalosporin (fifth generation) (e.g., ceftaroline fosamil, ceftobiprole); a glycopeptide (e.g., teicoplanin, vancomycin, telavancin); a lincosamide (e.g., clindamycin, lincomycin); a lipopeptide (e.g., daptomycin); a macrolide (e.g., azithromycin, clarithromycin, dirithromycin, erythromycin, roxithromycin, troleandomycin, telithromycin, spiramycin); a monobactam (e.g., aztreonam); a nitrofuran (e.g., furazolidone, nitrofurantoin); an oxazolidonone (e.g., linezolid, posizolid, radezolid, torezolid); a penicillin (e.g., amoxicillin, ampicillin, azlocillin, carbenicillin, cloxacillin, dicloxacillin, flucloxacillin, mezlocillin, methicillin, nafcillin, oxacillin, penicillin g, penicillin v, piperacillin, penicillin g, temocillin, ticarcillin); a penicillin combination (e.g., amoxicillin/clavulanate, ampicillin/sulbactam, piperacillin/tazobactam, ticarcillin/clavulanate); a polypeptide (e.g., bacitracin, colistin, polymyxin b); a quinolone (e.g., ciprofloxacin, enoxacin, gatifloxacin, levofloxacin, lomefloxacin, moxifloxacin, nalidixic acid, norfloxacin, ofloxacin, trovafloxacin, grepafloxacin, sparfloxacin, temafloxacin); a sulfonamide (e.g., mafenide, sulfacetamide, sulfadiazine, silver sulfadiazine, sulfadimethoxine, sulfamethizole, sulfamethoxazole, sulfanilimide (archaic), sulfasalazine, sulfisoxazole, trimethoprim-sulfamethoxazole (co-trimoxazole) (tmp-smx), sulfonamidochrysoidine (archaic)); a tetracycline (e.g., demeclocycline, doxycycline, minocycline, oxytetracycline, tetracycline); a drug against mycobacteria (e.g., clofazimine, dapsone, capreomycin, cycloserine, ethambutol, ethionamide, isoniazid, pyrazinamide, rifampicin (rifampin in US), rifabutin, rifapentine, streptomycin); or another antibiotic (e.g., arsphenamine, chloramphenicol, fosfomycin, fusidic acid, metronidazole, mupirocin, platensimycin, quinupristin/dalfopristin, thiamphenicol, tigecycline, tinidazole, trimethoprim).

An anticonvulsant can be an aldehyde (e.g., paraldehyde), an aromatic allylic alcohol (e.g., stiripentol), a GABA analog (e.g., gabapentin, pregabalin); a barbiturate (e.g., pentobarbital, methylpenobarital, or barbexaclone); a benzodiazepine (e.g., clonazepam, clobazam, clorazepate, diazepam, midazolam, nitrazepam, temezepam, nimetazepam, or lorazepam); a bromide (e.g., potassium bromide), a carbamate (e.g., felbamate), a caroxamide (e.g., carbamazepine, oxcarbazepine, eslicarbazepine acetate), a fatty acid (e.g., vaproate (e.g., valproic acid, sodium valproate, divalproex sodium), vigabatrin, progabide, or tiagabine), a fructose derivative (e.g., topiramate), a hydantoin (e.g., phenyloin, ethotoin, mephenytoin, or fospheytoin); an oxazolidinedione (e.g., paramethadoine, trimethadione, or ethadione), a propionate (e.g., beclamide), a pyrimidinedione (e.g., primidone), a pyrrolidine (e.g., brivaracetam, levetiracetam, seletracetam), a succinimide (e.g., ethosuximide, phensuximide, mesuximide), a sulfonamide (e.g., acetazoamide, sultiame, methazolamide, or zonisamide), a triazine (e.g., lamatrigine), a urea (e.g., pheneturide, phenacemide), a valproylamide (e.g., valpromide or valnoctamide), or a phenyltriazine (e.g., lamotrigine).

An antidepressant can be a selective serotonin reuptake inhibitor (SSRI, e.g., citalopram, escitalopram, paroxetine, fluoxetine, fluvoxamine, sertraline), a norepinephrine reuptake inhibitor (NRI, e.g., atomoxetine, reboxetine, viloxazine), a noradrenergic and specific serotonergic antidepressant (NaSSA e.g., mianserin, mirtazapine), a serotonin-norepinephrine reuptake inhibitor (SNRIs, e.g., desvenlafaxine, duloxetine, milnacipran, venlafaxine), a serotonin antagonist and reuptake inhibitor (SARIs, e.g., etoperidone, nefazodone, trazodone), a norepinephrine-dopamine reuptake inhibitor (e.g., bupropion), a selective serotonin reuptake enhancer (e.g., tianeptine, amineptine), a norepinephrine-dopamine disinhibitor (NDDIs e.g., agomelatine), a tricyclic antidepressant (e.g., tertiary amine tricyclic antidepressants (amitriptyline, clomipramine, doxepin, imipramine, trimipramine) or secondary amine tricyclic antidepressants (e.g., desipramine, nortriptyline, protriptyline)), a monoamine oxidase inhibitor (MAOIs e.g., isocarboxazid, mocolobemide, phenelzine, selegiline, tranylcypromine), nicotine, caffeine, or lithium. In some cases, the antidepressant is agomelatine, amitriptyline, amoxapine, atomoxetine, buspirone, benmoxine, butriptyline, citalopram, clomipramine, desipramine, dosulepin, doxepin, duloxetine, escitalopram, etoperidone, femoxetine, fluvoxamine, imipramine, kitanserin, lofepramine, medifoxamine, mianserin, maprotiline, mazindol, milnacipran, mirtazapine, nefzaodone, nisoxetine, nomifensine, nortriptyline, protriptyline, oxaprotiline, paroxetine, reboxetine, sertaline, trazodone, trimipramine, venlafaxine, viloxazine, zimelidine, citalopram, cotinine, duloxetine, fluoxetine, fluvoxamine, milnacipran, nisoxetine, paroxetine, reboxetine, sertraline, tianeptine, acetaphenazine, binedaline, brofaromine, cericlamine, clovoxamine, iproniazid, isocarboxazid, moclobemide, phenyhydrazine, phenelzine, selegiline, sibutramine, tranylcypromine, ademetionine, adrafinil, amesergide, amisulpride, amperozide, benactyzine, bupropion, caroxazone, gepirone, idazoxan, metralindole, milnacipran, minaprine, nefazodone, nomifensine, ritanserin, roxindole, S-adenosylmethionine, escitalopram, tofenacin, trazodone, tryptophan, or zalospirone.

An antidiabetic agent can be insulin, a sufonylurea (e.g., tolbutamide, acetohexamide, tolazmide, chlorpropamide, glyburide, glibenclamide, glimepiride, gliclazide, glycopyramide, gliquidone, or glipizide), a biguanide (e.g., metformin, phenformin, or buformin), an alpha-glucosidase inhibitor (e.g., acarbose, miglitol, or voglibose), a meglitinide (e.g., repaglinide, nateglinide), or a thiazolidinedione (e.g., pioglitazone rosiglitazone, or troglitazone). An antidiabetic agent can be an injectable glucagon-like peptide analog (e.g., exenatide, liraglutide), or a dipeptidyl peptidase-4 inhibitor (e.g., vildagliptin, sitagliptin, saxagliptin, linagliptin, allogliptin, septagliptin).

An antidote can be edrophonium chloride, flumazenil, deferoxamine, nalmefene, naloxone, or naltrexone. An antidote can be activated charcoal (e.g., with sortibal), adenosine, atropine, beta blocker, calcium chloride, calcium gluconate, a chelator (e.g, EDTA, dimercaptrol, penicillamine, EGTA, or 2,3-dimercaptosuccinic acid), a cyanide antidote (amyl nitrite, sodium nitrite, thiosulfate), cyproheptadine, deferoxamine mesylate, digoxin immune Fab antibody, diphenhydramine hydrochloride, benztorpine mesylate, ethanol, fomepizole, flumazenil, glucagon, insulin, insulin with glucagon, leucovorin, methylene blude, naloxone hydrochloride, N-acetylcysteine, octreotide, pralidoxime chloride (2-PAM), protamine sulfate, Prussian blue, physostigmine sulfate, pyridoxine, phytomenadione (vitamin K), or sodium bicarbonate.

An antiemetic can be a 5-HT3 receptor antagonist (e.g., dolasetron, granisetron, ondansetron, tropisetron, palonosetron, or mirtazapine), a dopamine antagonist (e.g., doperidone, olanzapine, droperidol, haloperidol, chlorpormaine, promethazine, prochloperazine, alizapride, prochlorperazine, metoclopramide), an NK1 receptor antagonist (e.g., aprepitant, casopitant), an antihistamine (HI histamine receptor antagonist; e.g., cyclizine, diphenhydramine, dimenhydrinate, doxylamine, meclozine, promethazine, hydroxyzine), a cannabinoid (e.g., cannabis, dronabinol, nabilone, one of a JWH cannabinoid series), a benzodiazepine (e.g., midazolam, lorazepam), an anticholinergic (e.g., hyoscine), a steroid (e.g., dexamethasone), trimethobenzamide, ginger, emetrol, propofol, peppermint, muscimol, or ajwain. In some cases, the antiemetic is alizapride, azasetron, benzquinamide, bromopride, buclizine, chlorpromazine, cinnarizine, clebopride, cyclizine, diphenhydramine, diphenidol, dolasetron, droperidol, granisetron, hyoscine, lorazepam, dronabinol, metoclopramide, metopimazine, ondansetron, perphenazine, promethazine, prochlorperazine, scopolamine, triethylperazine, trifluoperazine, triflupromazine, trimethobenzamide, tropisetron, domperidone, or palonosetron.

An antihistamine can be an HI-receptor antagonist (e.g., acrivastine, azelastine, brompheniramine, buclizine, bromodiphenhydramine, carbinoxamine, cetirizine, chlorpromazine, cyclizine, chlorpheniramine, chlorodiphenhydramine, celmastine, cyproheptadine, desloratadine, dexbrompheniramine, dexchlorpheniramine, dimenhydramine, doxylamine, ebastine, embramine, fexofenadine, levocetirizine, loratadine, meclozine, mirtazapine, olopatadine, orphenadrine, pheninadamine, pheniramine, phenyltooxamine, promethazine, pyrilamine, quetiapine, rupatadine, tripelennamine, triprolidine), an H2-receptor antagonist (e.g., cimetidine, famotidien, lafutidien, mizatidine, ranitidine, roxatidine), and H3-receptor antagonist (e.g., A-349,821, ABT-239, ciproxifam, clobenpropit, conessine, thioperamide), or and H4-receptor antagonist (e.g., thioperamide, JNJ 7777120, or VUF-6002). In some cases, an antihistamine can be astemizole, azatadine, brompheniramine, carbinoxamine, cetrizine, chlorpheniramine, cinnarizine, clemastine, cyproheptadine, dexmedetomidine, diphenhydramine, doxylamine, fexofenadine, hydroxyzine, loratidine, hyroxyizine, promethazine, pyrilamine or terfenidine.

A drug can be an allergy medication. In some cases, the allergy medication can be an antihistamine, montelukast, azelastine/fluticaseon propionate, beclomethasone dipropionate, budesonide, ciclesonide, cromlyn sodium, flunisollide, fluticaonse furoate, fluticasone propionate, ipratropium bromide, mometasone furoate monohydrate, olopatadine, oxymetazoline, triamcinolone acetonide, azelastine, cromolyn, emadastine, epinastine, ketorolac, ketotifen, lodoxamine, loteprednol, naphazoline, naphazoline/pheniramine, nedocromil, olopatadine, pemirolast, epinephrine, aclometasone, fluocinolone, fluocinonide, triamcinolone, desonide, fluocinolone, flurandrenolide, fluandrenolide, fluticaonse, hydrocortisone butyrate, hydrocortisone probuate, hydrocortisone valerate, mometasone, prednicarbate, triamcinolone, amcinonide, betamethazone valerate, desoximetasone, diflorasone, fluocinonide, halcononide, triamcinolone, betamethasone bipropionate, clobetasol priopionate, diflorasone, flurandrenolide, halobetasol propionate, doxepin, pimecrolimus, tacrolimus, C1 inhibitor, ecallantide, cortisone acetate, dexamethasone, hydrocortisone, methylprednisolone, prednisolone, or prednisone.

An anti-infective agent can be selected from one of the following classes: antivirals (e.g., abacavir, acyclovir, acyclovir, adefovir, amadtamine, amprenavir, ampligen, arbidol, atazanavir, atripla, balavir, boceprevirertet, cidofovir, combivir, darunavir, delavirdine, didanosine, docosanol, edoxudine, efavirenz, emtricitabine, enfuvirtide, entecavir, famciclovir, fomivirsen, fosamprenavir, foscarnet, fosfonet, ganciclovir, ibacitabine, imunovir, idoxuridine, imiquimod, indinavir, inosine, lamivudine, lipinavir, loviride, maraviroc, moroxydine, methisazone, nelfinavir, nevirapine, nexavir, oseltamivir, peginterferon alpha-2a, penciclovir, peramivir, pleconaril, podophyllotoxin, raltegravir, ribavirin, rimantadine, ritonavir, pyramidine, saquinavir, atavudine, telepevir, tenofovir, tenofovir disoproxil, tipranavir, trifluidine, trizivir, tromantadine, truvada, valaciclovir, valganciclovir, vicriviroc, vidarabine, viramidine, zalcitabine, zanamivir, or zidovudine); AIDS adjunct agents such as dapsone; aminoglycosides (e.g., streptomycin, neomycin, framycetin, paromomycin, ribostamycin, kanamycin, amikacin, arbekacin, bekanamycin, dibekacin, tobramycin, spectinomycin, hygromycin B, paromoycin sulfate, gentamicin, netilmicin, sisomicin, isepamicin, verdamicin, or astromicin); antifungals (e.g., imidazoles, e.g., bifonazole, butoconazole, clotrimazole, econazole, fenticonazole, isoconazole, ketoconazole, miconazole, omoconazole, oxiconazole, sertaconazole, sulconazole, or tioconazole; trizoles, e.g., albaconazole, fluconazole, isavuconazole, itraconazole, posaconazole, ravuconazole, terconazole, voriconazole; thiazoles e.g., abafungin; allyamines, e.g., amorolfin, butenafine, naftifine, terbinafine; echinocandins e.g., anidulafugin, caspofungin, micafungin; benzoic acid, ciclopirox, flucytosine, griseofulvin, haloprogin, polygodial, tolnaftate, undecylenic acid, or crystal violet); antimalarial agents (e.g., quinine, chloroquine, amodiaquine, pyrimethamine, proguanil, sulfadoxine, sulfamethoxypryidazine, mefloquine, atovaquone, atovaquone-proguanil, primaquine, artemisinin, artemether, artesuante, dihyroartemisinin, arteether, halofantrine, doxycycline, clindamycin); antituberculosis agents (e.g., ethambutol, isoniazid, pyrazinamide, rifampicin); .beta.-lactams (e.g., cefmetazole, cefazolin, cephalexin, cefoperazone, cefoxitin, cephacetrile, cephaloglycin, cephaloridine; cephalosporins, such as cephalosporin C, cephalothin; cephamycins such as cephamycin A, cephamycin B, and cephamycin C, cephapirin, cephradine); leprostatics (e.g., acedapsone, clofazimine, dapsone, desoxyfructo-serotonin, diucifon, ethionamide, rifampicin, rifapentine, sulfameter, thalidomide); penicillins (e.g., ampicillin, amoxicillin, hetacillin, carfecillin, carindacillin, carbenicillin, amylpenicillin, azidocillin, benzylpenicillin, clometocillin, cloxacillin, cyclacillin, methicillin, nafcillin, 2-pentenylpenicillin, penicillin N, penicillin O, penicillin S, penicillin V, dicloxacillin; diphenicillin; heptylpenicillin; and metampicillin); quinolones (e.g., cinoxacin, nalidixic acid, oxolinic acid, piromidic acid, pipemidic acid, rosoxacin, ciprofloxacin, enoxacin, fleroxacin, lomefloxacin, madifloxacin, norfloxacin, ofloxacin, pefloxacin, rufloxacin, balofoxacin, clinafloxacin, difloxacin, grepafloxacin, levofloxacin, pazufloxacin, sparfloxacin, tosufloxacin, norfloxacin, ofloxacine, temafloxacin, clinafloxacin, gatifloxacin, gemifloxacin, moxifloxacin, sitafloxacin, trovafloxacin, prulifloxacin); tetracyclines (e.g., tetracycline, chlortetracycline, demeclocycline, doxycycline, oxytetracycline, lymecycline, meclocycline, methacycline, minocycline, rolitetracycyline, tigecycline; miscellaneous anti-infectives such as linezolide, trimethoprim and sulfamethoxazole.

An anti-neoplastic agent can be, e.g., lomustine, carmustine, steptozocin, mechlorethamine, melphalan, uracil nitrogen mustard, chlorambucil, cyclophosphamide, iphosphamide, cisplatin, carboplatin, mitomycin, thiotepa, dacarbazin, procarbazine, hexamethyl melamine, triethylene melamine, busulfan, pipobroman, mitotane, methotrexate, trimetrexate, pentostatin, cytarabine, Ara-CMP, fludarabine phosphate, hydroxyurea, fluorouracil, floxuridine, chlorodeoxyadenosine, gemcitabine, thioguanine, 6-mercaptopurine, bleomycin, toptecan, irinotecan, camptothecin sodium salt, daunorubicin, doxorubicin, idarubicin, mitoxantrone, teniposide, etoposide, dactinomycin, mithramycin, vinblastine, vincristine, nvalebine, paclitaxel, docetaxel, droloxifene, tamoxifen, or toremifene.

An antiparkisonian drug can be amantadine, baclofen, biperiden, benztropine, orphenadrine, procyclidine, trihexyphenidyl, levodopa, carbidopa, andropinirole, apomorphine, benserazide, bromocriptine, budipine, cabergoline, eliprodil, eptastigmine, ergoline, galanthamine, lazabemide, lisuride, mazindol, memantine, mofegiline, pergolide, piribedil, pramipexole, propentofylline, rasagiline, remacemide, ropinerole, selegiline, spheramine, terguride, entacapone, or tolcapone.

An antirheumatic agent can be abatacept, adalimumab, azathioprine, chloroquine, diclofenac, hydroxychloroquine, methotrexate, ciclosporin, D-penicillamine, etanercept, golimumab, infliximab, leflunomide, miocyline, rituximab, or sulfasalzine.

An antipsychotic can be acetophenazine, alizapride, amisulpride, amoxapine, amperozide, aripiprazole, asenapine, benperidol, benzquinamide, bromperidol, buramate, butaclamol, butaperazine, carphenazine, carpipramine, chlorpromazine, chlorprothixene, clocapramine, clomacran, clopenthixol, clospirazine, clothiapine, clopenthixol, clozapine, cyamemazine, droperidol, flupenthixol, fluphenazine, fluspirilene, haloperidol, loxapine, melperone, mesoridazine, levomepromazine, pimozide, metofenazate, molindrone, olanzapine, paliperidone, lloperidone, lurasidone, penfluridol, periciazine, perphenazine, pimozide, pipamerone, piperacetazine, pipotiazine, prochlorperazine, promazine, quetiapine, remoxipride, risperidone, sertindole, spiperone, sulpiride, thioridazine, thiothixene, trifluperidol, triflupromazine, trifluoperazine, ziprasidone, zotepine, or zuclopenthixol.

An anxiolytic can be a benzodiazepine (e.g., alprazolam, chlordiazepoxide, clonazepam, diazepam, etizolam, lorazepam, oxazepam); tofisopam; a selective serotonin reuptake inhibitor (SSRI); afobazole; selank; bromantane; an azapirone (e.g., buspirone, tandospirone, gipeirone); a barbiturate; hydroxyzine; pregalalin; validol; an herbal treatment (e.g., Bacopa monnieri, Lactuca virosa, Rohodiola rosea, Hypericum perforatum, Matricaria recutita, Passiflora incarnate, Piper methysticum; Sceletium tortuosum, Scutellaria lateriflora; Valeriana officinalis; Salvia splendens; Coriandrum sativum; Myristica; Salvia elegans; Inositol; Cannabidiol); an over-the counter pharmaceutical drug (e.g., picamilon; chlorpheniramine; diphenhydramine; melatonin); BNC210; CL-218,872; L-838,417; SL-651,498; or aloradine. In some cases, an anxiolytic can be alprazolam, bromazepam, oxazepam, buspirone, hydroxyzine, mecloqualone, medetomidine, metomidate, adinazolam, chlordiazepoxide, clobenzepam, flurazepam, lorazepam, loprazolam, midazolam, alpidem, alseroxlon, amphenidone, azacyclonol, bromisovalum, captodiamine, capuride, carbcloral, carbromal, chloral betaine, enciprazine, flesinoxan, ipsapiraone, lesopitron, loxapine, methaqualone, methprylon, propanolol, tandospirone, trazadone, zopiclone, or zolpidem.

An appetite stimulant (orexigenic) can be ghrelin, orexin, neuropeptide Y; a 5-HT2c receptor antagonist (e.g., mirtazapine, alanzapine, quetiapin, amitriptyline, cyrpoheptadine); an HI receptor antagonist (e.g., mirtazapine, olanzapine, quetiapine, amitriptyline, cyproheptadine); a dopamine antagonist (e.g., haloperidol, chlorpromazine, olanzapine, risperidone, quetiapine); an adrenergic antagonist (e.g., carvedilol, propranolol; alpha2-adrenergi agonist (e.g., clonidine); a CB1 receptor agonist (e.g., THC/dronabinol, nabilone); a corticosteroid (e.g., dexamethasone; prednisone, hydrocortisone); a pregnene steroid (e.g., oxandrolone, nandrolone, testosterone); a sufonylurea (e.g., glibenclamide, chlopropamide).

An appetite suppressant can be diethylpropion, rimonabant, oxymetazoline, fenfluramine, phentermine, sibutramine, benfluorex, butenolide, cathine, diethylpropion, FG-7142, phenmetrazine, phenylpropanolamine, pryoglutamylhistidyl-glycine, amfepramon, amphetamine, benzphetamine, dexmethylphenidate, dextroamphetamine, glucagon, lisdexamfetamine, methamphetamine, methylphenidate, phendimetrazine, phenethylamine, or bupropion.

A blood modifier can be an anticoagulant (e.g., heparin); colony stimulating factor (e.g., fligrastim, pegfilgrastim; sargramostim); phytonadione (Vitamin K); iron; iron combination (e.g., iron + vitamin C) cilostazol, dipyridamol, abbokinase, abciximab, activase, advate, aggrastat, aggrenox, agrylin, albumin, alteplase, amicar, aminocaproic acid, anadrol, anagrelide, angiomax, anti-inhibitor coagulant complex; antihemophilic factor, antithrombin III, aprotinin, aquamephyton, aranesp, argtroban, arixtra, aspirin, aspirin + dipryidamole, benefix, bivalirudin, buminate 25%, buminate 5%, cathflo activas, clopidogrel, Coagulation Factor IX, Coagulation Factor IX Human, Coagulation Factor VIIA, Coumadin, Cyanocobalamin Nasal, cykokapron, Dalteparin, Ddavp, drotrecogin alpha, ecotrin, eltrombopag, enoxaparin, epoetin alpha, epogen, epoprostenol, eptifibatide, erythropoiesis stimulating protein, feiba VH, ferrlecit, fibrinogen human, flolan, fondaparinux subcutaneous, fragmin, gammaplex, hemofil M, human immunoglobulin G, infed, integrilin, iron dextran, jantoven, kinlytic, koate-DVI, kogenate, lepirudin, leukine, lovenox, mephyton, mononine, mozobil, nascobal, neulasta, neumega, novoseven, nplate, oprelvekin, pegfilgrastim, pentoxifylline, pentoxil, persantine, phytondione, plasbumin-25, pasbumin-5, plasma protein fraction, plasmanate, plavix, plerixafor, pletal, procrit, promacta, recombinate, refacto, refludan, reopro, riastap, romiplostim, sargramostim, sodium ferric gluconate, tenecteplase, thrombate III, thrombin, ticlid, ticlopidien, tirofiban, tnkase, tranexamic acid, trasylol, trental, urokinase, vitamin KI, warfarin, or xigris.

An asthma agent can be fluticaone, budeonside, mometasone, beclomethasone, zariflukast, zileuton, flunisolide, ciclesonide, triamcinolone, ipratropium, dyphylllin/guaifenesin, dexamethasone, prednisone, methylprednisolne, formoterol/mometeasone, triamcinolone, montelukast, isoetharine, dyphylline, salmeterol, budeonside/formoterol, mometasone/formoterol, theophylline, albuterol, levabulterol, ipratropium, omalizumab, or guaifenesin/theophylline. In some cases, the asthma medication can be an inhaled corticosteroid (e.g., beclomethasone propionate, budesonide, budesonide/formoterol, ciclesonide, blunisolide, fluticasone propionate, fluticaonse/salmeterol, mometasone, memetasone/formoterol, or triamcinolone acetonide). In some cases the asthma agent can be a long-acting beta-agonist (LABA; e.g., albuterol sulfate, formoterol fumarate, salmeterol xinafoate, or arformoterol tartrate). In some cases, the asthma agent can be cromolyn sodium or theophylline. In some cases an asthma agent can be a leukotriene modifier (e.g., montelukast, zafirlukast, zileuton). In some cases, an asthma agent can be an immunomodulator (e.g., omalizumab). In some cases, an asthma agent can be a short-acting beta-agonist (SABA; e.g., albuterol sulfate, ipratropium bromide/albuterol sulfate, ipratropium bromide HFA, levalbuterol HCl, pirbuterol, tiotropium bromide). In some cases, an asthma agent is duplilumab. In some cases, the asthma agent is bambuterol, bitolerol, doxofylline, ephedrine, epinephrine/chlorpheniramine, erythromycin, hydrocortisone, ipratropium bromide, isoetharine, isoprenaline, isoproterenol, ketotifen, metaproterenol, mometasone furoate and formoterol fumarate, nedocromil, oxtriphylline, salmeterol/fluticasone, terbutaline, tinocordin, triamcinolone, zafirlukast, or zileuton.

A cardiovascular agent can be fenoldopam, diazoxide, nitroprusside, ambrisentan, epoprostenol, treprostinil, sildenafil, bosentan, iloprost, treprostinil, epoprostenol; an aldosterone receptor antagonist (e.g., spironolactone, eplerenone); an angiotensin converting enzyme inhibitor (e.g., fosinopril, ramipril, captopril, trandolapril, moexipril, lisinopril, quinapril, enalapril, lisinopril, perinodpril, benazepril); an angiontensin II inhibitor (e.g., eprosartan, olemsartan, azilsartan medoxomil, telmisartan, losartan, valsartan, candesartan, irbesartan); an antiadrenergic agent, centrally acting (e.g., clonidine, fuanfacine, methyldopa, guanabenz); an antiadrenergic agent, peripherally acting (e.g., doxazosin, prazosin, terazosin, silodosin, alfuzosin, tamsulosin, dutasertide/tamsulosin, guanadrel, mecemylamine, guanethidine); an antianginal agent (e.g., nitroglycerin, ranolazine, isosorbide mononitrate, isosorbide dinitrate); an antiarrhythmic agent (e.g., group I (e.g., moricizine, guanidine, disopyramide, phenytoin, propafenone, flecainide, disopyramide, phenytoin, mexiletine, quinidine, tocainide, lidocaine, procainamide); group II (e.g., propranolol, esmolol, acebutolol); group III (e.g., amiodarone, sotalol, dofetilide, dronedarone, amiodarone, sotalol, ibutilid); group IV (e.g., ditiazem, verapamil); group V (e.g., adenosine, digoxin); and anticholinergic chronotropic agent (e.g., atropine); an antihypertensive combination (e.g., bendroflumethiazide/nadolol, eprosartan/hydrochlorothiazide, amlodipine/hydrochlorothiazide/valsartan, amplodipine/atorvastatin, hydrochlorothiazide/telmisartan, trandolapril/verapamil; hydrochlorthiazide/irbesartan, hydralazine/hydrochlorothiazide, hydrochlorothiazide/triamterene, diltiazem/enalapril, aliskiren/hdrochlorothiazise, amlodipine/telmisartan, amlodipine/olmesartan, atenolol/chlorthalidone, hydrochlorothiazide/moexipril, hydrochlorothiazide/olmesartan, hydrochlorothiazide/lisinopril, hydrochlorothiazide/valsartan, hydrochlorothiazide/losartan, hydrochlorthiaxide/quinapril, hyrodchlorothiazide/spironolactone, azilsartan medoxomil/chlorthalidone, amlodipine/benazepril, amiloride/hydrochlorothiazise, hydrochlrothiazide/lisinopril, amlodipine/hydrochorothiazide/olmesartan, amlodipine/valsartan, aliskirne/valsartan, hydrocholorthiazide/triamterene, bisoprolol/hydrochlorothiazide, candesartan/hydrochlorothiazide, chrlorthiazide/methyldopa, hydrochlorothiazide/triamterene, hydroclorothiazide/methyldopa, chlorothiazide/methyldopa, hydrochlrothiazide/methyldopa, amlodipine/benazepril, aliskiren/amlodipine/hydrochlorothiazide, hydrazine/hydrochlorothiazide, hydralazine/isosrbide dinitrate, captopril/hydrochlorothiazide, chlorthalidone/clonidine, bendroflumethiazide/nadolol, bendrofluemethiazide/nadolol, chlorthalidone/reserpine, hydralazine/hydrochlorothiazide/reserpine, hydrochlorothiazide/metoprolol, deserpidine/methyclothiazide, guanethidine/hydrochlorothiazide, hydrochlorothiazide/propranolol, enalapril/felodipine, polythiazide/prazosin, amiloride/hydrochlorothiazise, fosinopril/hydrochlorothiazide, hydrochlorothiazide/quinapril, chlorthalidone/reserpine, polythiazide/reerpine, aliskiren/amlodipine, atenolol/chlorthalidone, hydrochlorothiazide/timolol); a beta-adrenergic blocking agent (e.g., cardioselective beta blocker (e.g., betaxolol, bisoprolol, atenolol, metoprolol, nibivolol, esmolol, acebutolol); non-cardioselective beta blocker (e.g., propranolol, nadolol, sotalol, carvedilol, labetalol, timolol, carteolol, penbutolol, pindolol)); a calcium channel blocking agent (e.g., nifedipine, diltiazem, nimodipine, verapamil, felodipine, nicardipine, isradipine, nisoldipine, clevidipine, bepridil); a peripheral vasodilator (e.g., cyclandelate, papverine, isoxsuprine); a catecholamine (e.g., epinephrine, isoproterenol, norepinephrine); a diuretic (e.g., carbonic anhydrase inhibitor (e.g., acetazolamide, dichlophenamide, methazolamide), loop diuretic (e.g., torsemide, furosemide, bumetanide, ethacrynic acid); pamabrom, mannitol; a potassium-sparing diuretic (e.g., triamterene, spironolactone, amiloride); a thiazide diuretic (e.g., indapamide, hydrochlorothiazide, metolazone, methylclothizode, hydrochlorothiazide, chlorothiazide, methyclothizide, metolazone, bendroflumethiazide, polythiazide, hydrofluemethiazide, chlorthalidone)); a inotropic agent (e.g., digoxin, dobutamine, milrinone); icatibant, cilostazol, midodrine, metyrosine, phenoxybenzamine, EDTA, phentolamine; rennin inhibitor (e.g., aliskiren); a peripheral vasodilator (e.g., cyclandelate, papaverine, isoxsuprine); a sclerosing agent (e.g., laureth-9, ethanolamine oleate, morrhuate sodium, sodium tetrdecyl sulfate); a vasodilator (e.g., nitroglycerin, alprostadil, hydralazine, minoxidil, mesiritide, nitroprusside); a vasopression antagonist (e.g., conivaptan, tolvaptan); or a vasopressor (e.g., epinephrine, isoproterenol, phenylephrine, norepinephrine, dobutamine, isoproterenol). In some cases, the cardiovascular agent can be benazepril, captopril, enalapril, quinapril, ramipril, doxazosin, prazosin, clonidine, labetolol, candesartan, irbesartan, losartan, telmisartan, valsartan, disopyramide, flecanide, mexiletine, procainamide, propafenone, quinidine, tocamide, amiodarone, dofetilide, ibutilide, adenosine, gemfibrozil, lovastatin, acebutalol, atenolol, bisoprolol, esmolol, metoprolol, nadolol, pindolol, propranolol, sotalol, diltiazem, nifedipine, verapamil, spironolactone, bumetanide, ethacrynic acid, furosemide, torsemide, amiloride, triamterene, or metolazone.

A central nervous system stimulant can be phendimetrazine, methamphetamine, diethylpropion, amphetamine/dextroamphetamine, benzphetamine, phendimetrazine, lisdexamfetamine, diethylpropion, phendimetrazine, dexmethylphenidate, armodafinil, atomexetine, doxapram, amphetamine, brucine, caffeine, dexfenfluramine, dextroamphetamine, ephedrine, fenfluramine, mazindol, methyphenidate, pemoline, phentermine, sibutramine, or modafinil.

An agent for Alzheimer's disease management can be donepezil, galanthamine, rivastigmine, tacrine, or memantine.

An agent for cystic fibrosis management can be an antibiotic (e.g., ciprofloxacin, tobramycin); a bronchodilator (e.g., albuterol or salmeterol); an anticholinergic (e.g., atrovent); a DNase (e.g., pulmozyme); a mucolytic (e.g., acetylcysteine); a saltwater solution (e.g., hypertonic saline); a nonsteroidal anti-inflammatory (NSAID; e.g., ibuprofen); a corticosteroid (e.g., fluticasone or prednisone); an enzyme replacement therapy (e.g., creon or pancreaze); CPX, IBMX, XAC and analogues; 4-phenylbutyric acid; genistein and analogous isoflavones; azithromycin, aztreonam, pancrelipase, gentamicin, ivacaftor, azithromycin, vitamin E, pancreatin, or milrinone.

A diagnostic agent can be adenosine or aminohippuric acid.

A homeopathic cold medication can be *Aconitum napellus, Allium cepa, Antimonium tartaricum, Apsi mellifica, Arsenicum album, Arum triphyllum, Belladonna, Bryonia alba, Dulcamara, Eupatorium perforliatum, Euphrasia, Ferrum phosphoricum, Gelsemium, Hepar sulphuris, Kali bichromicum, Mercurius solubilis, Natrum muriaticum, Nux vomica, Oscillococinum* (*Anas barbariase*), phosphorus, *Rhus toxicodendron,* sulphur, or *Pulsatilla Sticta.*

A COPD drug can be montelukast, budesonide/formoterol, roflumilast, aclidinium, prednisone, isoetharine, dyphylline, guaifenesin/theophylline, or fluticasone/vilanterol. In some cases, a COPD drug is a bronchodilator (e.g., albuterol, levabuterol, ipratropium; or a long-acting bronchodilator (e.g., tiotropium, salmeterol, formoterol, arformoterol, indacaterol, aclidinium). In some cases, a COPD drug is a steroid (e.g., fluticasone, budesonide). In some cases, a COPD drug is a combination (e.g., salmeterol/fluticasone and formoterol/budesonide). In some cases a COPD drug is a phosphodiesterase-4 inhibitor (e.g., roflumilast). In some cases, a COPD drug is theophylline or an antibiotic.

A cough medication can be guaifenesin/hydrocodone, acetaminophen/codeine, diphenhydramine, guaifenesin/potassium guaiacolsulfonate, carbetapentane/guaifenesin, codeine/guaifenesin, dextromethorphan/guaifenesin, guaifenesin, carbinoxamine/dextromethorphan/pseudoephedrine, dextromethorphan, brompheniramine/codeine, carbetapentane/chlorpheniramine/phenylephrine, benzocaine/dextromethorphan, menthol, acetaminophen/dextromethorphan, chlophedianol/guaifenesin, acetaminophen/dextromethrophan/doxylamine, aceteaminophen/hydrocodone, glycerin, acetaminophen/dextromethorphan/phenylephrine, dexbrompheniramine/hydrocodone/phenylephrine, hydromorphone, acetaminophen/chlorpheniramine/dextromethorphan/phenylephrine, guaifenesin, carbetapentane/guaifenesin, carbinoxamine/dextromethorphan/pseudoephredrine, chlorpheniramine/dextromethorphan/methscopolamine, guaifenesin/potassium guaiacolsulfonate, homatropine/hydrocodone, dihdrocodeine/guaifenesin/pseudoephredrine, chlropheniramine/hydrocodone, codeine/guaifenesin, potassium iodide, dihydrocodeine/guaifenesin, dihydrocodeine/hydrocodone, acetaminophen/hydrocodone, chlorcyclizine/codeine/phenylephrine, codeine/pseudoephedrine/pyrilamine, hydromorphone, dihydrocodeine/guaifensesin/pseudoephedrine, chlophedianol/triprolidine, dextromethorphan/promethazine, codeine/promethazine, dextromethorphan/promethazine, carbetapentane/guaifenesin, carbetapentane/guaifenesine, dextromethorphan/guaifenesin, dextromethorphan/doxylamine, carbetapentanse, dyclonine/menthol, dextromethorphan/guaifenesin, benzonatate, acetaminophen/dextromethorphan/phenylephrine, guaifenesin/hydrocodone, carbinoxamine/hydrocodone/pseudeoephedrine, codeine/guaifenesin, guaifenesin/hydrocodoen, homatropine/hydrocodone, chlorpheniramine/hydrocodone, carbetapentane/guaifenesin, acetaminophen/dextromethorphan/doxylamine/phenylephrine, acetaminophen/dextromethorphan, acetaminophen/dextromethorphan/phenylephrine, acetaminophen/hydrocodone, dihydrocodein/guaifenesin/pseudoephedrine, or benzonatate. In some cases, the cough medication can be dextromethorphan, codeine, noscapine, bromhexine, acetylcysteine, ephedrine, guaifenesin, honey, cinnaomon, honey/cinnamon, lemon, elderberry syrup, tea, Slippery Elm, peppermint, Chinese Hot Mustard, cayenne pepper (capsaicin), apple cider vinegar, wasabi, horseradish, Echinacea, vitamin c, zinc, ginger (zingiber officinale), vinegar, water, red onion, garlic, hyssop, or mullein. In some cases, a cough medication can be an antihistamine, decongestant, inhaled asthma drug, antibiotic, acid blocker, or cough suppressant. In some cases, a cough medication is licorice, horehound, mullein, peppermint, elderflower, yarrow, *Belladonna, bryonia, Gelsemium, Coccus catcti, Drosera, Dulcamara, Eupatorium, Euphrasia,* hepar suphuratum, *Kali bic, Nux vomica,* phosphorus, *Pulsatilla, Antimonium tartaricu, Rhus tox, Spongia,* or *Vincetoxicum.*

A dietary supplement can be acai, aloe vera, an anabolic steroid, astragalus, vitamin A, bilberry, beta carotene, bitter orange, Black Cohosh, Butterbur, vitamin B12, vitamin B6, calcium, carnitine, cartilage, cat's claw, chamomile, chasteberry, chondroitin, chromium, cinnamon, coenzyme Q10, colloidal silver, cranberry, vitamin C, dandelion, vitamin C, Echinacea, ephedra, essiac/flor-essence, European elder, evening primrose oil, vitamin E, fenugreek, feverfew, fish oil, flaxseed, folate, folic acid, garlic, ginger, ginkgo, ginseng, glucosamine, glucosamine with chondroitin sulfate, goldenseal, grape seed extract, green tea, hawthorn, hoodia, horse chestnut, iodine, iron, kava, vitamin K, lavender, licorice root, L-lysine, magnesium, melatonin, milk thistle, mistletoe, noni, omega-3 fatty acids, PC-SPES, peppermint oil, red clover, sage, S-adenosyl-L-methionine, saw palmetto, selenium, soy, St. John's Wort, tea, thunder god vince, turmeric, valerian, vitamin A, vitamin B1, vitamin B12, vitamin B6, vitamin C, vitamin D, vitamin E, vitamin K, yohimbe, or zinc.

An agent for erectile dysfunction can be tadalafil, sildenafil, vardenafil, alprostadil, avanafil, apomorphine, apomorphine diacetate, phentolamine, and yohimbine.

A gastrointestinal agent can be a 5-aminosalicylate (e.g., mesalamine, balsalazide, sulfasalazine, olsalazine), an antacid (e.g., aluminum hydroxide/magnesium hydroxide/simethicone; sodium barcarbonate, magaldrate/simethicone, calcium carbonate, aluminum hydroxide/magnesium hydroxide/simethicone, magnesium hydroxide, aluminum hydroxide/magnsesium hydroxide, magnesium hydroxide, alginic acid/aluminum hydroxide/magnesium trisilicate, alginic acid/aluminum hydroxide/magnesium carbonate, aluminum hydroxide/magnesium hydroxide/simethicone, calcium carbonate/magnesium hydroxide, magaldrate, magaldrate/simethicon), an antidiarrheal (e.g., bismuth subsalicylate, atropine/difenoxin, attapulgite, lactobacillus acidophilus, loperamide, atropine/diphenoxylate, saccharomyces boulardii lyo, crofelemer systemic, kaolin/pectin systemic, kaolin systemic, lactobacillus acidophilus/lactobacillus bulgaricus, loperamide/simethicone systemic), a digestive enzyme (e.g., pancrelipase, amylase/cellulose/hyoscyamine/lipase/phenyltoloxamine/protease, pancreatin, lactase), a functional bowel disorder agent (e.g., an antichoinergic/antispasmodic, e.g., hyoscyamine, atropine/hyoscyamine/Phenobarbital/scopolamine, methscopolamine, scopolamine, chlordiazepoxide/clidinium, dicyclomine, glycopyrrolate, belladonna, atropine, atropine/hyoscyamin/Phenobarbital/scopolamine, belladonna/ergotamine/phenobarbital, mepenzolate, hyoscyamine/phenyltoloxamine), a chloride channel activator (e.g., lubiprostone), a guanylate cyclase-C agonist (e.g., linaclotide), a peripheral opioid receptor antagonist (e.g., methylnaltrexone, alivmopan); a serotoninergic neuroenteric modulator (e.g., tegaserod, alosetron), a gallstone solubilizing agent (e.g., ursodiol, chenodeoxycholic acid), a gastrointestinal stimulant (e.g., metoclopramide, cisapride, choline bitartrate/dexpanthenol), H. pylori eradication agent (e.g., amoxicillin/clarithromycin/lansoprazole, bismuth subcitrate potassium/metronidazole/tetracycline, bismuth subsalicylate/metronidazole/tetracycline, amoxicillin/clarithromycin/omeprazole), an H2 antagonist (e.g., nizatidine, cimetidine, ranitidine, famotidine, cimetidine, calcium carbonate/famotdine/magnesium hydroxide), a laxative (e.g., magnesium citrate, polyethylene glycol 3350, lactulose, senna, bisacodyl, psyllium, methylcellulose, docusate, polycarbophil, sodium biphophate/sodium phosphate, docusate/senna, sodium biphosphate/sodium phosphate, polyethylene glycol 3350 with electrolytes, bisacodyl/polyethylene glycol 3350/potassium chloride/sodium bicarbonate/sodium chloride, magnesium sulfate, polycarbophil, magnesium hydroxide, mineral oil), citric acid/simethicone/sodium bicarbonate, simethicone, misoprostol, charcoal/simethicone, sucralfate, teduglutide; or a proton pump inhibitor (e.g., pantoprazole, omeprazole/sodium bicarbonate, rebeprazole, esomeprazole, lansoprazole, dexlansoprazole). In some cases, the gastrointestinal agent can be loperamide, atropine, hyoscyamine, famotidine, lansoprazole, omeprazole, or rebeprazole.

A hormone can be estrogen, progesterone, hydrocortisone, fludocortisone, throxine, progestin, testosterone, estradiol, cortisone, 1-androstendediol, 1-androstenedione, bolandiol, bolasterone, boldenone, boldione, calusterone, clostebol, danazol, dehydrochlormethyltestosterone, desoxymethyltestosterone, drostanolone, ethylestrenol, fluoxymesterone, formeboone, furazabol, gestrinone, 4-hydroxytestosterone, mestanolone, mesterolone, meenolone, methandienone, methandriol, methasterone, methyldienolone, methyl-1-testosterone, methylnortestosterone, methyltestosterone, mitribolone, mibolerone, nandrolone, 19-noradrostenedione, norboletone, norclostebol, norethandrolone, oxabolone, oxandrolone, oxymesterone, oxymetholone, prostanozol, quinbolone, stnozolol, stenbolone, 1-testosterone, tetrhydrogestrinone, trenbolone, androstenediol, androstenedionne, dihydrotestosterone, or prasterone.

An agent for the treatment of alcoholism can be naloxone, naltrexone, acamprostate, or disulfuram.

An agent for the treatment of addiction can be disulfiram, naltrexone, acamprosate, methadone, levo-alph acetyl methadol (LAAM), or buprenorphine.

An immunosupressive can be a glucocorticoid, a cytostatic (e.g., alkyating agent, antimetabolite (e.g., methotrexate, azathiopurine, mercaptopurine, fluorouracil, a cytotoxic antibiotic (e.g., dactinomycin, an antracycline, mitomycin C, bleomycin, mithramycin), an antibody (e.g., a monoclonal antibody (e.g., IL-2 receptor directed antibody, CD3 directed antibody; a T-cell receptor directed antibody (e.g., muromonab-CD3)), a drug acting on immunophilin (e.g., ciclosporin, tacrolimus, sirolimus), other drugs (e.g., an interferon, an opioid, a TNF binding protein, a mycophenolate). In some cases, the immunosuppressive is mycophenolic acid, cyclosporin, azathioprine, tacrolimus, everolimus, or rapamycin. In some cases, the immunosuppressive agent is a calcineurin inhibitor (e.g., cyclosporine, tacrolimus), an interleukin inhibitor (e.g., rilonacept, tocilizumab, anakinra, ustekinumab, canakinumab, basiliximab, daclizumab), omalizumab, lenalidomide, azathioprine, methotrexate, pomalidomide, thalidomide, alefacept, efalizumab, mycophenolic acid, mycophenolate mofetil, fingolimod, natalizumab, belimumab, lefunomide, abatacept, lymphocyte immune globulin anti-thy (equine), teriflunomide, belatacept, muromonab-cd3, eculizumab, anti-thymocyte globulin (rabbit), or a TNF alpha inhibitor (e.g., infliximab, adalimumab, etanercept, certolizumab, golimumab).

A mast cell stabilizer can be cromolyn, pemirolast, or nedocromil.

An agent for migraine headache can be naproxen, ibuprofen, acetaminophen, almotriptan, alperopride, codeine, dihydroergotamine, ergotamine, eletriptan, frovatriptan, isometheptene, lidocaine, lisuride, metoclopramide, naratriptan, oxycodone, propoxyphene, rizatriptan, sumatriptan, tolfenamic acid, zolmitriptan, amitriptyline, atenolol, clonidine, cyproheptadine, diltiazem, doxepin, fluoxetine, lisinopril, methysergide, metoprolol, nadolol, zolmitriptan, nortriptyline, paroxetine, pizotifen, pizotyline, propanolol, protriptyline, sertraline, timolol, ergotamine/caffeine, isometheptine/dichlorphenazone/apap, or verapamil.

An agent that can be used to treat motion sickness can be diphenhydramine, dimehydrinate, cinnaizine, meclozine, promethazine, metoclopramide, prochlorperazine, ginger root, or scopolamine.

An agent for managing multiple sclerosis can be corticotropin, dalfampridine, teriflunomide, interferon beta-la, interferon beta-lb, glatiramer, cyclophosphamide, dexamethasone, prednisone, fingolimod, azathioprine, natalizumab, bencyclane, methylprednisolone, azathioprine, mitoxantrone, or prednisolone.

A muscle relaxant can be a neuromuscular blocking agent (e.g., succinylcholine, mivacurium, cisatracurium, vecuronium, doxacurium, pancuronium, atracurium); a skeletal muscle relaxant combination (e.g., aspirin/caffeine/orphenadrine, aspirin/carisoprodol, aspirin/carisoprodol/codeine, aspirin/methocarbamol, aspirin/meprobamate); or a skeletal muscle relaxant (e.g., dantrolene, botulinum toxin type b, carisprodol, onabotulinumtoxin A, cyclobenzaprine, chlorzoxazone, chlrophenesin, tizanidine, baclofen, cyclobenzaprine, metaxalone, methocarbamol, cyclobenzaprine, orphenadrine, carisoprodol, incobotulinumtoxinA). In some cases, the muscle relaxant is decamethonium, rapacuronium, atracurium, rocuronium, alcuronium, gallamine, metocurine, pipecuronium, bubocurarine, baclofen, chlorzoxazone, cyclobenzaprine, methocarbamol, orphenadrine, quinine, carisoprodol, gabapentin, metaxalone, diazepam, dantrolene, botulinum toxin type b, onabotulinumtoxinA, chloroxazone, chlorphenesin, baclofen, methocarbamol, or ortizanidine.

A drug for treating mycocardial infarction can be urokinase, perindopril, alteplase, ramipril, aspirin, aluminum hydroxide/aspirin/calcium carbonate/magnesium hydroxide, timolol, magnesium chloride, warfarin, dalteparin, heparin, propranolol, eptifibatide, metoprolol, enoxaparin, trandolapril, nitroglycerin, clopidogrel, lisinopril, reteplase, streptokinase, atenolol, tenecteplase, or moexipril. In some cases, the drug for treating myocardial infarction can be a vasodilator. A vasodilator can be an alpha-adrenoceptor antagonist (e.g., prazosin, terazosin, doxazosin, trimazosin, phentolamine, phenoxybenzamine); an angiotensin converting enzyme (ACE) inhibitor (e.g., benazepril, captopril, enalapril, fosinopril, lisinopril, moexipril, quinapril, ramipril); an angiotensin receptor blocker (ARB) (e.g., candesartan, eprosartan, irbesartan, losartan, olmesartan, telmisartan, valsartan); a beta2-adrenoceptor agonist (beta2-agonist) (e.g., epinephrine, norepinephrine, dopamine, dobutamin, isoproterenol); a calcium-channel blocker (CCB) (e.g., amlodipine, felodipine, isradipine, nicardipine, nifedipine, nimodipine, nitrendipine); a centrally acting sympatholytic (e.g., clonidine, guanabenz, guanfacine, alpha-methyldopa); a direct acting vasodilator (e.g., hydralazine); an endothelin receptor antagonist (e.g., bosetan); a ganglionic blocker (e.g., trimethaphan camsylate); a nitrodilator (e.g., isosorbide dinitrate, isosorbide mononitrate, nitroglycerin, erhthrityl tetranitrate, pentaerythritol tetranitrate, sodium nitroprusside); a phosphodiesterase inhibitor (e.g., a PDE3 inhibitor (e.g., milrinone, inamrinone, cliostazol; a PDE5 inhibitor (e.g., sildenafil, tadalafil)); a potassium-channel opener (e.g., minoxidil); or a rennin inhibitor (e.g., aliskiren). In some cases, a drug for treating myocardial infarction can be a cardiac depressant drug (e.g., a beta-adrenoceptor antagonist (beta-blocker), e.g., a non-selective betal/beta2 drug (e.g., carteolol, carvedilol, labetalol, nadolol, penbutolol, pindolol, propranolol, sotalol, timolol) or a beta1-selective drug (e.g., acebutolol, atenolol, betaxolol, bisoprolol, esmolol, metoprolol, nebivolol); a calcium-channel blocker (e.g., amlodipine, felodipine, isradipine, nicardipine, nifedipine, nimodipine, nitrendipine); or a centrally acting sympatholytic (e.g., clonidine, guanabenz, guanfacine, alpha-methyldopa). In some cases, a drug for treating myocardial infarction can be an antiarrhythmic drug (e.g., Class I -sodium-channel blocker (e.g., Class 1A (e.g., quinidine, procainamide, disopryamide); Class 1B (e.g., lidocaine, tocainide, mexiletine); Class 1C (e.g., flecainide, propafenone, moricizine); Class II-beta blocker (e.g., a non-selective betal/beta2 drug (e.g., carteolol, carvedilol, labetalol, nadolol, penbutolol, pindolol, propranolol, sotalol, timolol) or a betal-selective drug (e.g., acebutolol, atenolol, betaxolol, bisoprolol, esmolol, metoprolol, nebivolol); a Class III-potassium channel blocker (e.g., amiodarone, dronedarone, bretylium, sotalol, ibutilide, dofetilide); a Class IV calcium channel blocker (e.g., amlodipine, felodipine, isradipine, nicardipine, nifedipine, nimodipine, nitrendipine); adenosine, an electrolyte supplement (e.g., magnesium, potassium); a digitalis compound (e.g., digoxin, digitoxin, ouabain); or atropine. In some cases, the drug for treating myocardial infarction is a thrombolytic drug (e.g., a tissue plasmiogen activator (e.g., alteplase, retaplase, tenecteplase); streptokinase, anistreplase, or urokinase.

A nonsteroidal anti-inflammatory can be a salicylate (e.g., aspirin (acetylsalicylic acid), diflunisal, salsalate); a propionic acid derivative (e.g., ibuprofen, dexibuprofen, naproxen, fenoprofen, ketoprofen, dexketoprofen, flurbiprofen, oxaprozin, loxoprofen); an acetic acid derivative (e.g., indomethacin, tolmetin, sulindac, etodolac, ketorolac, diclofeanc, nabumetone) a enolic acid (oxicam) derivative (e.g., piroxicam meloxicam, tenoxicam, droxicam, lornoxicam, isoxicam); a fenamic acid derivative (fenamate; e.g., mefenamic acid, meclofenamic acid, flufenamic acid, tolfenamic acid); a selective COX-2 inhibitor (coxib; e.g., celecoxib, rofecoxib, vadexocib, parecoxib, lumiracoxib, etoricoxib, firoxib); a sulphonanilide (e.g., nimesulide); licofelone, lysine clonixinate, hyperforin, figwort, calcitriol (vitamin D). In some cases, a nonsteroidal anti-inflammatory can be aceclofenac, alminoprofen, amfenac, aminopropylon, amixetrine, aspirin, benoxaprofen, bromfenac, bufexamac, carprofen, celecoxib, choline salicylate, cinchophen, cinmetacin, clopriac, clometacin, diclofenac, diclofenac potassium, diclofenac sodium, diclofenac sodium with misoprostol, diflunisal, etodolac, fenoprofen, fenoprofen calcium, flurbiprofen, ibuprofen, indomethacin, indoprofen, ketoprofen, ketorolac, magnesium salicylate, mazipredone, meclofenamate, meclofenamate sodium, mefanamic acid, meloxicam, nabumetone, naproxen, naproxen sodium, oxaprozin, parecoxib, piroxicam, pirprofen, rofecoxib, salsalate, sodium salicylate, sulindac, tolfenamate, tolmetin, tolmetin sodium,or valdecoxib.

An opioid, opioid antagonist, or inverse agonist can be an opium alkaloid (e.g., codeine, morphine, oripavine, pseudomorphine, thebaine); an alkaloid salt mixture (e.g., pantopon, papaveretum); 14-hydroxymorphine, 2,4-dinitorphenylmorphe, 6-methyldihydromorphine, 6-methylenedihydrodesoxymorphine, acetyldihydromorphine, azidomorphine, chlornaltrexamine, chloroxymorphamine, desomorphine, dihydromorphine, ehtyldihydromorphine, hydromorphinol, methyldesorphine, N-henethylnormorphine, RAM-378, 6-nicotinoyldihydromorphine, acetlypropionylmorphin, diacetyldihydromorphine, dibutyrylmorphine, dibenzoylmorphine, diformylmorphine, dipropanoylmorphine, heroin, nicomorphine, 6-Monoacetylcodeine, Benzylmorphine, Codeine methylbromide, Desocodeine, Dimethylmorphine (6-O-Methylcodeine), Ethyldihydromorphine, Methyldihydromorphine (dihydroheterocodeine), Ethylmorphine (dionine), Heterocodeine, Isocodeine, Pholcodine (morpholinylethylmorphine), Myrophine, Nalodeine (N-allyl-norcodeine), Transisocodeine, 14-Cinnamoyloxycodeinone, 14-Ethoxymetopon, 14-Methoxymetopon, 14-Phenylpropoxymetopon, 7-Spiroindanyloxymorphone, 8,14-Dihydroxydihydromorphinone, Acetylcodone, Acetylmorphone, $\alpha$-hydrocodol (Dihydrocodeine), Bromoisopropropyldihydromorphinone, Codeinone, Codorphone, Codol (Codeine Phosphate), Codoxime, IBNtxA, Thebacon (acetyldihydrocodeinone, dihydrocodeinone enol acetate), Hydrocodone, Hydromorphone, Hydroxycodeine, Metopon (Methyldihydromorphinone), Morphenol Morphinone, Morphol, N-Phenethyl-14-ethoxymetopon, Oxycodone, Oxymorphol, Oxymorphone, Pentamorphone, Semorphone, $\alpha$-chlorocodide (Chlorocodide), $\beta$-chlorocodide, $\alpha$-chloromorphide (Chloromorphide), Bromocodide, Bromomorphide, Chlorodihydrocodide, Chloromorphide, Codide, 14-Hydroxydihydrocodeine, Acetyldihydrocodeine, Dihydrocodeine, Dihydrodesoxycodeine (desocodeine), Dihydroisocodeine, Nicocodeine, Nicodicodeine, 1-Nitrocodeine cas, Codeine-N-oxide, Morphine-N-oxide, Oxymorphazone, 1-Bromocodeine, 1-Chlorocodeine, 1-Iodomorphine, Codeine-N-oxide (genocodeine), Heroin-7,8-oxide, Morphine-6-glucuronide, 6-Monoacetylmorphine, Morphine-N-oxide (genomorphine), Naltrexol, Norcodeine, Normorphine, Levomethorphan, 4-chlorophenylpyridomorphinan, Cyclorphan, Dextrallorphan, Levargorphan, Levorphanol, Levophenacylmorphan, Levomethorphan, Norlevorphanol, N-Methylmorphinan, Oxilorphan, Phenomorphan, Methorphan (racemethorphan), Morphanol (racemorphanol), Ro4-1539, Stephodeline, Xorphanol, 1-Nitroaknadinine, 14-episinomenine, 5,6-Dihydronorsalutaridine, 6-Keto Nalbuphine, Aknadinine, Butorphanol, Cephakicine, Cephasamine, Cyprodime, Drotebanol, Fenfangjine G, Nalbuphine, Sinococuline, Sinomenine (cocculine), Tannagine, 5,9 alpha-diethyl-2-hydroxybenzomorphan (5,9-DEHB), 8-Carboxamidocyclazocine (8-CAC), Alazocine, Anazocine, Bremazocine, Butinazocine, Carbazocine, Cogazocine, Cyclazocine, Dezocine, Eptazocine, Etazocine, Ethylketocyclazocine, Fedotozine, Fluorophen, Gemazocine, Ibazocine, Ketazocine, Metazocine, Moxazocine, Pentazocine, Phenazocine, Quadazocine, Thiazocine, Tonazocine, Volazocine, Zenazocine, Pethidine, 4-Fluoromeperidine, Allylnorpethidine, Anileridine, Benzethidine, Carperidine, Difenoxin, Diphenoxylate, Etoxeridine (carbetidine), Furethidine, Hydroxypethidine (bemidone), Morpheridine, Meperidine-N-oxide, Oxpheneridine (carbamethidine), Pethidine (meperidine), Pethidine intermediate A, Pethidine intermediate B (norpethidine), Pethidine intermediate C (pethidinic acid), Pheneridine, Phenoperidine, Piminodine, Properidine (ipropethidine), Sameridine, Allylprodine, (α/β)-Meprodine, Desmethylprodine (MPPP), PEPAP, (α/β)-Prodine, Prosidol, Trimeperidine (promedol), Acetoxyketobemidone, Droxypropine, Ketobemidone, Methylketobemidone, Propylketobemidone, Alvimopan, Loperamide, Picenadol, Methadone, Dextromethadone, Dipipanone, Isomethadone, Levoisomethadone, Levomethadone, Methadone, Methadone intermediate, Normethadone, Norpipanone, Phenadoxone (heptazone), Pipidone (Dipipanone Hydrochloride) (6-piperidine-4,4-diphenyl-5-methyl-hexanone-3 hydrochloride), Alphaacetylmethadol, Dimepheptanol (racemethadol), Levacetylmethadol, Noracetylmethadol, Desmethylmoramide, Dextromoramide, Levomoramide, Moramide intermediate, Racemoramide, Diethylthiambutene, Dimethylthiambutene, Ethylmethylthiambutene, Piperidylthiambutene, Pyrrolidinylthiambutene, Thiambutene, Tipepidine, Dextropropoxyphene (propoxyphene), Dimenoxadol, Dioxaphetyl butyrate, Levopropoxyphene, Norpropoxyphene, Diampromide, Phenampromide, Propiram, IC-26, Isoaminile, Lefetamine, R-4066, Fentanyl, 3-Allylfentanyl, 3-Methylfentanyl, 3-Methylthiofentanyl, 4-Phenylfentanyl, Alfentanil, Alphamethylacetylfentanyl, Alphamethylfentanyl, Alphamethylthiofentanyl, Benzylfentanyl, Betahydroxyfentanyl, Betahydroxythiofentanyl, Betamethylfentanyl, Brifentanil, Carfentanil, Fentanyl, Lofentanil, Mirfentanil, Ocfentanil, Ohmefentanyl, Parafluorofentanyl, Phenaridine, Remifentanil, Sufentanil, Thenylfentanyl, Thiofentanyl, Trefentanil, Thienorphine, 7-PET, Acetorphine, Alletorphine (N-allyl-noretorphine), BU-48, Buprenorphine, Cyprenorphine, Dihydroetorphine, Etorphine, Homprenorphine, 18,19-Dehydrobuprenorphine (HS-599), N-cyclopropylmethylnoretorphine, Nepenthone, Norbuprenorphine, Thevinone, Thienorphine, Ethoheptazine, Meptazinol, Metheptazine, Metethoheptazine, Proheptazine, Bezitramide, Piritramide, Clonitazene, Etonitazene, Nitazene, 18-Methoxycoronaridine, 7-Acetoxymitragynine, 7-Hydroxymitragynine, Akuammidine, Akuammine, Eseroline, Hodgkinsine, Mitragynine, Pericine, Pseudoakuammigine, BW373U86, DPI-221, DPI-287, DPI-3290, SNC-80, β-neo-endorphin, dynorphin, Big dynorphin, Dynorphin A, Dynorphin B, Endorphin, Beta-endorphin, Alpha-endorphin, Gamma-endorphin, α-neo-endorphin, β-neo-endorphin, Enkephalin, DADLE • DAMGO • Dermenkephalin, Met-enkephalin, Leu-enkephalin, Adrenorphin, Amidorphin, Casomorphin, DALDA (Tyr-D-Arg-Phe-Lys-NH2), Deltorphin, Dermorphin, DPDPE, Endomorphin, Gliadorphin, Morphiceptin, Nociceptin, Octreotide, Opiorphin, Rubiscolin, TRIMU 5, 3-(3-Methoxyphenyl)-3-ethoxycarbonyltropane, AD-1211, AH-7921, Azaprocin, BDPC, Bisnortilidine, BRL-52537, Bromadoline, C-8813, Ciramadol, Doxpicomine, Enadoline, Faxeladol, GR-89696, Herkinorin, ICI-199,441, ICI-204,448, J-113,397, JTC-801, Ketamine, KNT-42, LPK-26, Methopholine, MT-45, Desmethylclozapine, NNC 63-0532, Nortilidine, O-Desmethyltramadol, Phenadone, Phencyclidine, Prodilidine, Profadol, Ro64-6198, Salvinorin A, SB-612,111, SC-17599, RWJ-394,674, TAN-67, Tapentadol, Tecodine (Oxycodone), Tifluadom, Tilidine, Tramadol, Trimebutine, U-50,488, U-69,593, Viminol, 1-(4-Nitrophenylethyl)piperidylidene-2-(4-chlorophenyl)sulfonamide (W-18), 5'-Guanidinonaltrindole, β-Funaltrexamine, 6β-Naltrexol, Alvimopan, Binaltorphimine, Chlornaltrexamine, Clocinnamox, Cyclazocine, Cyprodime, Diacetylnalorphine, Difenamizole, Diprenorphine (M5050), Fedotozine, JDTic, Levallorphan, Methocinnamox, Methylnaltrexone, Nalfurafine, Nalmefene, Nalmexone, Naloxazone, Naloxonazine, Naloxone, Naloxone benzoylhydrazone, Nalorphine, Naltrexone, Naltriben, Naltrindole, Norbinaltorphimine, Oxilorphan, S-allyl-3-hydroxy-17-thioniamorphinan (SAHTM), Alimadol, Anilopam+HCl, Asimadoline, FE 200665, Fedotozine, MCOPPB, Nalfurafine, Nalorphine, Nalorphine dinicotinate, or SoRI-9409 In some cases, the opioid can be alfentanil, allylprodine, alphaprodine, anileridine, benzylmorphine, bezitramide, buprenorphine, butorphanol, carbiphene, cipramadol, clonitazene, codeine, dextromoramide, dextropropoxyphene, diamorphine, dihydrocodeine, diphenoxylate, dipipanone, fentanyl, hydromorphone, L-alpha acetyl methadol, lofentanil, levorphanol, meperidine, methadone, meptazinol, metopon, morphine, nalbuphine, nalorphine, oxycodone, papaveretum, pethidine, pentazocine, phenazocine, oxymophone, remifentanil, sufentanil, or tramadol.

An analgesic can be merperidine, hydromorphone, fentanyl, codeine, methadone, morphine, oxycodone, oxycodone and ASA, oxycodone and acetaminophen, pentazocine, acetaminophen/caffeine/codeine, acetaminophen/codeine, acetaminophen, acetylsalicylic acid, ibuprofen, naproxen sodium, naproxen, indomethacin, diclofenac, mefenamic acid, ketorolac, celecoxib, erotamin, sumatriptan, butorphanol, zolmitriptan, naratriptan, rizatriptan, almotriptan, apazone, benzpiperylon, benzydramine, caffeine, clonixin, ethoheptazine, flupirtine, nefopam, orphenadrine, propacetamol, or propoxyphene.

An opthalmic preparation can be an anti-angiogenic ophthalmic agent (e.g., aflibercept, ranibizumab, pegaptanib); cysteamine, ocriplasmin, mitomycin, dapiprazole; a mydriatic (e.g., cyclpentolate, phenylephrine, atropine, cyclopentolate/phenylephrine, homatropine, scopolamine, phenylephrine/scopolamine, tropicamide, hydroxyamphetamine/tropicamide, tropicamide); an ophthalmic anesthetic (e.g, lidocaine, proparacaine, tetracaine); ophthalmic anti-infectives (e.g., levofloxacin, natamycin, bactiracin/neomycin/polymyxin b, bactiracin/polymyxin b, tobramycin, moxifloxacin, ciprofloxacin, gatifloxacin, azithromycin, idoxuridine, besifloxacin, norfloxacin, chloramphenicol, bacitracin/polymyxin b, sulfacetamide sodium, chloramphenicaol, boric acid, erythromycin, sulfisoxazole, gentamin, gramicidin/neomycin/polymyxin b, bacitracin, ofloxacin, polymyxin b/trimethoprim, levofloxacin, sulfacetamide sodium, oxytetracycline/polymyxin b, tobramycin, vidarabine, trifluridine, ganciclovir, gatifloxacin); an ophthalmic anti-inflammtory agent (e.g., bromfenac, nepafenac, ketorolac, cyclosporine, fluriprofen, suprofen, diclofenac, bromfenac); ophthalmic antihistamine and decongestant (e.g., ketotifen, nedocromil, azelastine, epinastine, olopatadine, naphazoline/pheniramine, olopatadine, alcaftadine, cromolyn, bepotastine, pemirolast, tetrhydrozolien, tetrahydrozoline/zinc sulfate, iodoxamide, naphazoline, phenylephrine, tetrhydrozoline, naphazoline/zinc sulfate, emedastine, naphazoline/pheniramine, levocabastine); an ophthalmic glaucoma agent (e.g., travoprost, dorzolamide/timolol, bimatoprost, latanoprost, brimonidine, brimonidine/timolol, timolol, levobunolol, brinzolamide, levobetaxolol, carbachol, dorzolamide/timolo, epinephrine/pilocarpine, epinephrine, demecarium bromide, apraclonidine, pilocarpine, acetylcholine, metipranolol, echothiophate iodide, dipivefrin, unoprostone, dorzolamide, tafluprost); an ophthalmic steroid (e.g., dexamethasone, fluocinolone, loteprednol, difluprednate, fluorometholone, loteprednol, prednisolone, medrysone, triamcinolone, rimexolone); an opthlamic steroid with an anti-infective (e.g., fluorometholone/sulfacetamide sodium, dexamethasone/meomycin, dexamethasone/tobramycin, dexamethasone/neomycin/polymyxin b), or orbetaxolol.

An osteoporosis preparation can be alendronate, ibandronate, calcium carbonate, calcium/vitamin D, estradiol, teriparatide, hydrochlorothiazide, calcitonin, conjugated estrogens, conjugated estrogens/medroxyprogesterone, denosumab, zoledronic acid, ibandronate, calcium glubionate, dihydrotachysterol, etidronate, esterified estrogens, raloxifene, alendronate/cholecalciferol, calcium phosphate tribasic, conjugated estrogens/medroxyprogesterone, calcium lactate, estropitate, risedronate or raloxifene.

A pain medication can be ibuprofen, hydroxyzine, celecoxib, meperidien, hydromorphone, amitriptyline, acetaminophen/hydrocodone, acetaminophen/codeine, tapentadol, acetaminophen/diphenhydramine, oxymorphone, oxycodone, acetaminophen, ketorolac, tramadol, diclonfenac, diphenhydramine/ibuprofen, naproxen, acetaminophen/phenyltoloxamine, aspirin/hydrocodone, acetaminophen/pheyltoloxamine, aspirin/caffeine, lidocaine, flurbiprofen, fentanyl, ketoprofen, aluminum hydroxide/aspirin/calcium carbonate/magnesium, trolamine salicylate, morphine, nortriptyline, capsaicin, aspirin/hydrocodone, magnesium salicylate, aspirin/caffeine/salicylamide, benzocain, camphor/menthol, valdecoxib, buprenorphine, aspirin/butalbital/caffeine, acetaminophen/caffeine/phenyltoloxamine/salicylamide, acetaminophen/codeine, clonidine, celecoxib, benzocaine/dextromethorphan, benzocaine, cholline salicylcate/magnesium salicylate, acetaminophen/dextromethorphan/doxylamine, sulindac, methol, ibuprofen/oxycodone, acetaminophen/gauifenesin, acetaminophen/diphenhydramine, pramoxine, aspirin/hydrocodone, acetaminophen/propoxyphene, propoxylphene, aspirin/caffeine/propoxyphene, oxycodone, meperidine, morphine liposomal, diphenhydramine, hydromorphone, diphenhydramine/magnesium salicylate, diflunisal, methadone, capsaicin, acetaminophen/phenyltoloxamine/salicylamide, acetaminophen/caffeine/magnesium, morphine/naltrexone, aspirin/codeine, acetaminophen/oxycodone, aspirin/meprobamate, acetaminophen/aspirin, acetaminophen/caffeine, bupivacaine liposome, aspirin/butalbital/caffeine, piroxicam, pentafluoropropane/tetrafluoroethane, indomethacin, acetaminophen/aspirin/caffeine/salicylamide, levorphanol, etodolac, meclofenamate, meperidine/promethazine, fenoprofen, nalbuphine, tapentadol, oxymorphone, acetaminophen/caffeine/dihydrocodeine, aspirin/caffeine/propxoyphene, menthol, mefenamic acid, propoxyphene, pramoxine, ziconotide, butorphanol, acetaminophen/pentazocine, pentazocine, naloxone/pentazocine, imipramine, tolmetin, acetaminophen/tramadol, acetaminophen/dextromethorphan, choline salicylate/magnesium salicylate, hydrocodone/ibuprofen, rofecoxib, or diclofenac. A pain medication can be a nonsteroidal anti-inflammatory drug (NSAID), a corticosteroid, an opoid, a muscle relaxant, an anti-anxiety drug, an antidepressant, or an anticonvulsant.

An anti-anxiety or panic disorder medication can be alprazolam, clomipramin, lorazepma, nortriptyline, buspirone, venlafaxine, clonazepam, lorazepam, maprotiline, paroxetine, fluoxetine, nefazodone, imipramine, sertraline; a serotonin and norepinephrine reuptake inhibitor (e.g., venlafaxine hydrochloride); a benzodiazepine (e.g., alprazolam, clonazepam, or lorazepam); or a selective serotonin reuptake inhibitor (SSRI; e.g., fluoxetine, paroxetine, or sertraline). A panic disorder medication can be a tricyclic antidepressant (TCA; imipramine hydrochloride, desipramine, clomipramine) or a monoamine oxidase inhibitor (MAOI; e.g., isocaroxazid, phenelzine, tranylcypromine). An anti-anxiety or panic medication can be lemon balm (*Melissa officinalis*), ibergoast (caraway, chamomile, licorice, milk thistle, and peppermint), hops (*Humulus lupulus*), lemon juice, ground giner, honey, catnip, chamomile (*Matricaria recutita*), fennel, L-theanine, Kava Kava, Motherwort, Passionflower, Skullcap (*Scutellaria lateriflora*), omega-3, Valerian (*Valeriana officinalis*), lavender (*Lavandula hybrida*), St. John's Wort, magnesium vitamin B12, vitamin B1, *Aconitum napellus, Argentum nitricum, Arsesnicum album, Gelsemium sempervirens, Natrum muriaticum, Calcarea carbonica, Ignatia amara, Kali arsenicosum, Kali phosphoricum, Lycopodium clavatum, Natrum muriaticum,* phosphorus, *Pulsatilla, Silicea* (Silica), Aconite (*Aconitum napellus*), *Ignatia amara,* Mercurius solubilis, phosphorus, sulphur, borax, Bryonia, Casticum, Anacardium, or Valerian Root.

A prostaglandin can be epoprostanol, dinoprostone, misoprostol, or alprostadil.

A respiratory agent can be an antiasthmatic combination (e.g., dyphylline/guaifensin, guaifenesin/theophylline), an antihistamine (e.g., fexofenadine, loratadine, phenindamine, dexchlorpheniramine, terfenadine, triprolidine, promethazine, brompheniramine, chlorpheniramine, cetirizine, diphenhydramine, carbinoxamine, diphenhydramine, chlorpheniramine, cyproheptadine, levocetirizine, desloratadine, clemastine, astemizole, tripelennamine, carboxamine, pheniramine/phenyltoloxamine/pyrilamine); an antitussive (e.g., carbetapentane, benzonatate, dextromethorphan); a bronchodilator (e.g., an adrenergic bronchodilator (e.g., epinephrine, isoproterenol, salmeterol, levalbuterol, arformoterol, metaproterenol, terbutaline, pirbuterol, albuterol, formoterol, indacaterol, racepinephrine, isoetharine, isoproterenol, bitolterol); an anticholinergic bronchodilator (e.g., ipratropium, aclidinium, tiotropium, ipratropium); a bronchodilator combination (e.g., fluticasone/salmeterol, albuterol/ipratropium, budesonide/formoterol, formoterol/mometasone, isoproterenol/phenylephrine); a methylxanthine (e.g., theophylline, oxtriphylline, dyphylline, aminophylline)); a decongestant (e.g., pseudoephedrine, phenylephrine, phenylpropanolamine, pseudophedrine); an expectorant (e.g., guaifenesin, potassium iodide, carbocysteine, or potassium guaiacolsulfonate); a leukotriene modifier (e.g., zafirlukast, monteukast, zileuton); a lung surfactant (e.g., poractant, calfactant, lucinactant, beractant); alpha 1-proteinase inhibitor, dornase alpha, sodium chloride, nitric oxide); an inhaled anti-infective (e.g., tobramycin, ribavirin, zanamivir, pentamidine); an inhaled corticosteroid (e.g., flunisolide, budesonide, fluticasone, beclomethasone, mometasone, ciclesonide); a mast cell stabilizer (e.g., cromolyn, nedocromil); a mucolytic (e.g., acetylcysteine); a selective phosphodiesterase-4 inhibitor (e.g., roflumilast); loratadine/pseudoephedrine, acetaminophen/chlorpheniramine/pseudoephedrine, chlorpheniramine/phenylephrine, acetaminophen/diphenhydramine/phenylephrine, brompheniramine/pseudoephedrine, codeine/guaifenesin, chlorpheniramine/dextromethorphan/phenylephrine, dextromethorphan/phenylephrine/pyrilamine, acetaminophen/chlorphenirmide/pheylephrine, guaifenesin/pseudoesphedrine, chlorpheniramine/phenylpropanolamine, carbetapentane/pseudoephedrine/pyrilamine, acetaminophen/chlorpheniramine/codeine, chlorpheniramine/dextromethorphan/pseudoephedrine, chlorcyclizine/phenylephrine, chlorpheniramine/pseudoephedrine, or chlorpheniramine/phenylpropanolamine. In some cases, the respiratory agent is albuterol, ephedrine, epinephrine, fomoterol, metaproterenol, terbutaline, budesonide, ciclesonide, dexamethasone, flunisolide, fluticasone propionate, triamcinolone acetonide, ipratropium bromide, pseudoephedrine, theophylline, montelukast, zafirlukast, ambrisentan, bosentan, enrasentan, sitaxsentan, tezosentan, iloprost, treprostinil, or pirfenidone.

A sedative or hypnotic can be a barbiturate (e.g., amobarbital, pentobarbital, secobarbital, phenobarbitol); a benzodiazepine (e.g., clonazepam, diazepam, estrazolam, flunitrazepam, lorazepam, midazolam, nitrazepam, oxazepma, trazolam, temazepma, chlordiazepoxide, alprazolam,); an herbal sedative (e.g., ashwagandha, Duboisia hopwoodii, Prosanthera striatiflora, catnip, kava, mandrake, valerian, marijuana); a non-benzodiazpein "z-drug" sedative (e.g., eszopiclone, zaleplon, zolpidem, zopiclone); an antihistamine (e.g., diphenhydramine, dimenhydrinate, doxylamine, pheneragn, promethazine), chloral hydrate, or alcohol. In some cases, the sedative or hypnotic is butalbital, chlordiazepoxide, diazepam, estazolam, flunitrazepam, flurazepam, lorazepam, midazolam, temazepam, triazolam, zaleplon, zolpidem, zolpidem tartrate, butisol sodim, pentobarbital or zopiclone.

A skin or mucous membrane agent can be an antibiotic (e.g., bacitracin, bacitracin zinc/polymyxin B sulfate; clindamycin phosphate, erythromycin/tretinoin, fusidate sodium, fusidic acid; gramicidin/polymyxin B sulfate; mupirocin; polymyxin B sulfate/bacitracin); an antiviral (e.g., acyclovir, idoxuridine); an antifungal (e.g., clotrimazole, ketoconazole, miconazole nitrate, nystatin, terbinafine HCl, terconazole, tolnaftate); a scabicide or pediculicide (e.g., crotamiton, isopropyl myristate, lindane, permethrin; piperonyl butoxide/pyrethrins); benzoyl peroxide, chlorheidine acetate, chorhexidine gluconate, hydrogen peroxide, metronidazole; metronidazole/avobenzone/octinoxate, metronidazole/nystatin, povidone-iodine, selenium sulfide, silver sulfadiazine, triclosan; an anti-inflammatory agent (e.g., amcinonide, beclomethasone dipropionate, betamethaseon dipropionate in propylene glycol, betamethasone dipropionate/clotrimazole, betamethasone dipropionate/salicyclic acid, betamethasone valerate, budesonide, clobetasol propionate, clobetasone butyrate, desonide, desoximetasone, diflucortolone valerate, diflucortolone valerate/salicyclic acid, fluocinolone acetonide, fluocinonide, fluticasone propionate, halobetasol propionate, hydrocortisone, hydrocortisone acetate, hydrocortisone acetate/zince sulfate, hydrocortisone acetate/zinc sulfate/prmoxine HCl, hydrocortisone valerate, hydrocortisone/dibucaine HCl/esculin/framycetin sulfate; hydrocortisone/urea, mometasone furoate, triamcinolone acetonide); an anipruritic or local anesthetic (e.g., lidocaine HCl, lidocaine/prilocaine); a cell stimulate or proliferant (e.g., tretinoin); a basic ointment or protectant (e.g., dimethicone, petrolatum, zinc oxide); a keratolytic agent (e.g., adapalene, canthadridin/podphyllin/salicyclic acid, dithranol, formaldehyde/lactic acid/salicyclic acid, latic acid/salicyclic acid, podofilox, podophyllin, salicyclic acid); a keratoplastic agent (e.g., coal tar, coal tar/juniper tar/pine tar; coal tar/juniper tar/pine tar/zinc pyrithione, coal tar/salicylic acid, coal tar/salicyclic acid/sulfur); a pigmenting agent (e.g., methoxsalen); acitretin, azelaic acid, calcipotriol, capsaicin, collagenase, fluorouracil, iostretinoin, pimecrolimus, tacrolimus, tazarotene, or vitamin E. In some cases, a skin or mucous membrane agent is isotretinoin, bergapten or methoxsalen.

A smoking cessation aid can be topiramate, fluphenazine, varenicline, nortriptyline, bupropion, clonidine, nicotine, or tryptophan.

A Tourette's syndrome agent can be pimozide, topiramate, olanzapine, clonidine, guanfacine, haloperidol, botulinum toxin type A, methylphenidate, dextroamphetamine, or pergolide.

A urinary tract agent can be lactobacillus acidophilus, amoxicillin, cefazolin, amoxicillin/clavulante, sulfamethoxazole/trimethoprim, cefuroxime, ciprofloxacin, ertapenem, levofloxacin, nitrofurantoin, ceftriaxone, cefixime, ampicillin/sulbactam, doxycycline, piperacillin/tazobactam, hyoscyamine/methenamine/methylene blue/phenyl salicylate, doripenem, cefadroxil, acetohydroxamic acid, nitrofurantoin, methenamine, lomefloxacin, cefepime, cefoxitin, tolteridine, darifenicin, propantheline bromide, or oxybutynin.

A vertigo agent can be promethazine, diphenidol, betahistine or meclizine.

An insomnia medication can be 5-hydroxytryptophan, diphenhydramine/ibuprofen, zolpidem, lorazepam, flurazepam, amitriptyline, triazolam, eszopiclone, estazolam, temezepam, ramelteon, doxepin, doxylamine, zaleplon, acetaminophen/diphenhydramine, diphenhydramine, 5-hydroxytryptophan, tryptophan, chloral hydrate, diphenhydramine/magnesium salicylate, quazepam, eszopiclone, secobarbital, doxepin, olanzapine, clonazepam, quazepam, lorazepam, alprazolam, oxazepam, prazepam, flunitrazepam, melatonin, valerian root, chamomile tea, lemon balm, or 5-L-5-hydroxytryptophan.

A weight loss drug can be megestrol, phentermine/topirmate, phentermine, phenylpropanolamine, lorcaserin, oxandrolone, megestrol, mazindol, orlistat, sibutramine, rimonabant, metformin, exenatide, pramlintide, conjugated linoleic acid, green tea extract, khat, lipoic acid, ECA stack, or raspberry ketone.

An herb, supplement, or vitamin can be aloe, arginine, beta-carotene, black cohosh, chocolate, chondriotin sulfate, coca, coenzyme Q10, cranberry, creatine, DHEA (dehydroepiandrosterone), dong quai, Echinacea, ephedra, evening primrose oil, flaxseed, flaxseed oil, folate, ginkgo, glucosamine, honey, *Lactobacillus acidophilus,* lycopene, marijuana, melatonin, milk thistle, niacin, omega-3 fatty acid, fish oil, alpha-linolenic acid, red yeast rice, SAMe (adenosylmethionine), saw palmetto, soy, St. John's wort, tea tree oil, thiamin, vitamin A, vitamin B12, vitamin B6, vitamin C, vitamin D, vitamin E, whey protein, or zinc. An herb can be a medicinal herb. A medicinal herb can be absinthe wormwood (*Artemisia absinthium*), agrimony (*Agrimonia eupatoria*), aloe vera (*Aloe barbadensis Miller*), alpine rose (*Rhododendron ferrugineum*), angelica (*Angelica silvestris*), anise (*Pimpinella anisum*), arnica (*Arnica montana*), ash (*Fraxinus excelsior*), asparagus (*Asparagus officinalis*), barberry (*Berberis vulgaris*), barley (*Hordeum sativum*), basil (*Ocimum basilicum*), bean (*Phaseolus vulgaris*), bearberry (*Arctostaphylos uva ursi*), beet (*Beta vulgaris*), betony (*Betonica officinalis*), bilberry (*Vaccinium myrtillus*), birch (*Betula pendula*), birdweed (*Polygonum aviculare*), bistort (*Polygonum bistorta*), bitter dock (*Rumex obtusifolis*), bitter root (*Gentiana lutea*), bitterwort (*Gentiana lutea*), blackberry (*Rubus fruticosus*), black chokeberry (*Aronia melanocarpa*), black currant (*Ribes nigrum*), black locust (*Robinia pseudoacacia*), blackthorn (*Prunus spinosa*), blue gum tree (*Eucalyptus globulus*), borage (*Borago officinalis*), broadleaf dock (*Rumex obtusifolis*), broad-leaved dock (*Rumex obtusifolis*), broccoli (*Brassica oleracea var. botrytis*), burdock (*Arctium lappa*), burnet saxifrage (*Pimpinella saxifraga*), butcher's broom (*Ruscus aculeatus*), calamus (*Acorus calamus*), calendula (*Calendula officinalis*), cannabis (*Cannabis sativa*), caraway (*Carum carvi*), carline thistle (*Carlina acaulis*), carrot (*Daucus carota*), cat's claw (*Uncaria tomentosa*), celery (*Apium graveolens*), centaury (*Centaurium umbellatum*), chamomile (*Matricaria chamomilla*), chasteberry (*Vitex agnus-castus*), chickory (*Cichorium intybus*), christ's thorn (*Paliurus spina-christi*), church steples (*Agrimonia eupatoria*), cinnamon (*Cinnamomum zeylandicum*), cinquefoil (*Potentilla reptans*), cleavers (*Galiuma aparine*), clove (*Syzygium aromaticum*), clubmoss (*Lycopodium clavatum*), coltsfoot (*Tussilago farfara*), comfrey (*Symphytum officinale*), common ivy (*Hedera helix*), common polypody (*Polypodium vulgare*), coriander (*Coriandrum sativum*), corn (corn silk) (*Zea mays*), couch grass (*Agropyron repens*), cowslip (*Primula veris*), cranberry (*Vaccinium oxycoccos*), cranesbill (*Geranium macrorrhizum*), creeping cinquefoil (*Potentilla reptans*), creeping thyme (*Thymus serpyllum*), cross gentian (*Gentiana cruciata*), daisy (*Bellis perennis*), dandelion (*Taraxacum officinale*), dill (*Anethum graveolens*), dog rose (*Rosa canina*), dogwood (*Cornus mas*), dwarf everlast (*Helichrysum arenarium*), echinacea (*Echinacea angustifolia*), elder (*Sambucus nigra*), elderberry (*Sambucus nigra*), elecampane (*Inula helenium*), european cornel (*Cornus mas*), european wild ginger (*Asarum europaeum*), evening primrose (*Oenothera biennis*), evening star (*Oenothera biennis*), everlasting flower (*Helichrysum arenarium*), eyebright (*Euphrasia officinalis*), fennel (*Foeniculum vulgare*), fenugreek (*Trigonella foenum-graecum*), fig (*Ficus carica*), flax (*Linum usitatissimum*), garden nasturtium (*Tropaeolum majus*), garlic (*Allium sativum*), garland thorn (*Paliurus spina-christi*), ginger (*Zingiber officinalis*), ginkgo (*Ginkgo biloba*), ginseng (*Araliaceae>Panax*), glossy buckthorn (*Rhamnus frangula*), goat willow (*Salix caprea*), goosegrass (*Galiuma aparine*), goldenrod (*Solidago virgaurea*), gotu kola (*Centella asiatica*), grape vine (*Vitis vinifera*), greater celandine (*Chelidonium majus*), great sallow (*Salix caprea*), great yellow gentian (*Gentiana lutea*), green tea (*Camellia sinensis*), green-winged orchid (*Orchis morio*), ground ivy (*Glechoma hederacea*), gypsyweed (*Veronica officinalis*), haselwort (*Asarum europaeum*), hawthorn (*Crataegus laevigata*), heartsease (*Viola tricolor*), hibiscus (*Hibiscus*), hops (*Humulus lupulus*), horehound (*Marrubium vulgare*), horse chestnut (*Aesculus hippocastanum*), horse-heal (*Inula helenium*), horsetail (*Equisetum arvense*), houseleek (*Sempervivum tectorum*), hyssop (*Hyssopus officinalis*), iceland moss (*Cetraria islandica*), indian cress (*Tropaeolum majus*), ivy (*Hedera helix*), johnny jump up (*Viola tricolor*), juniper (*Juniperus communis*), kidney vetch (*Anthyllis vulneraria*), knotgrass (*Polygonum aviculare*), lady's bedstraw (*Galium verum*), lady's mantle (*Alchemilla vulgaris*), larch (*Larix europaea*), large-leaved lime (*Tilia platyphyllos*), large-leaved linden (*Tilia platyphyllos*), lavender (*Lavandula angustifolia*), lemon balm (*Melissa officinalis*), lemon, citron (*Citrus medica*), lily of the walley (*Convallaria majalis*), linseed (*Linum usitatissimum*), liquorice (*Glycyrrhiza glabra*), loosestrife (*Lythrum salicana*), lovage (*Levisticum officinale*), lungwort (*Pulmonaria officinalis*), mallow (*Malva silvestris*), marigold (*Calendula officinalis*), marjoram (*Majorana hortensis*), marshmallow (*Althaea officinalis*), melilot, yellow (*Melilotus officinalis*), milk thistle (*Silybum marianum*), mint (*Mentha piperita*), mistletoe (*Viscum album*), monks cress (*Tropaeolum majus*), mountain germander (*Teucrium montanum*), mouse-ear hawkweed (*Pilosella officinarum*), mulberry, black (*Morus nigra*), mulberry, white (*Morus alba*), mullein (*Verbascum thapsus*), mustard, black (*Brassica nigra*), mustard, white (*Sinapis*

*alba*), oak (*Quercus*), oat (*Avena sativa*), olive (*Olea europaea*), onion (*Allium cepa*), orchid (*Orchis morio*), oregano (*Origanum vulgare*), parsley (*Petroselinum hortense*), peach (*Prunus persica*), peppermint (*Mentha piperita*), pigweed (*Polygonum aviculare*), pink ipê (*Tabebuia impetiginosa*), plantain, greater (*Plantago major*), plantain, ribwort (*Plantago lanceolata*), plum (*Prunus domestica*), polypody (*Polypodium vulgare*), pomergranate (*Punica granatum*), pumpkin (*Cucurbita pepo L*), purple chokeberry (*Aronia prunifolia*), pussy willow (*Salix caprea*), quackgrass (*Agropyron repens*), quince (*Cydonia oblonga*), radish (*Raphanus sativus*), raspberry (*Rubus idaeus*), ramsons (*Allium ursinum*), red chokeberry (*Aronia arbutifolia*), red currant (*Ribes rubrum*), rest harrow (*Ononis spinosa*), rose de mai (*Rosa centifolia*), rosemary (*Rosmarinus officinalis*), rupturewort (*Herniaria glabra*), rustyback (*Ceterach officinarum*), sage (*Salvia officinalis*), salad burnet (*Sanguisorba minor*), saw palmetto (*Serenoa Repens*), scots pine (*Pinus silvestris*), senna (*Cassia angustifolia*), sesame (*Sesamum indicum*), shepard's purse (*Capsella bursa-pastoris*), silver thistle (*Carlina acaulis*), speedwell (*Veronica officinalis*), starflower (*Borago officinalis*), sticklewort (*Agrimonia eupatoria*), stickyweed (*Galiuma aparine*), stickywilly (*Galiuma aparine*), stinging netle (*Urtica dioica*), st john's wort (*Hypericum perforatum*), strawberry (*Fragaria*), stone fern (*Ceterach officinarum*), sunflower (*Helianthus annuus*), sweetclover, yellow (*Melilotus officinalis*), sweet flag (*Acorus calamus*), sweet woodruff (*Asperula odorata*), taheebo tea (*Tabebuia impetiginosa*), tarragon (*Artemisia dracunculus*), thyme (*Thymus vulgaris*), tetterwort (*Chelidonium majus*), toadflax (*Linaria vulgaris*), tormentil (*Potentilla tormentilla*), valerian (*Valeriana officinalis*), vervian (*Verbena officinalis*), violet (*Viola odorata*), wall germander (*Teucrium chamaedrys*), walnut (*Juglans regia*), water dropwort (*Oenanthe aquatica*), waterlily (*Nymphaea alba*), white lotus (*Nymphaea alba*), wild apple (*Malus sylvestris*), wild cherry (*Prunus serotina*), wild ginger (*Asarum europaeum*), wild pansy (*Viola Tricolor*), wild pear (*Pyrus piraster*), wild strawberry (*Fragaria vesca*), wild thyme (*Thymus serpyllum*), willow herb (*Epilobium parviflorum*), winter savory (*Satureja montana*), woodruff (*Asperula odorata*), wormwood (*Artemisia absinthium*), woundwort (*Solidago virgaurea*), yarrow (*Achilea millefolium*), yellow sweetclover (*Melilotus officinalis*), or yucca (*Agavaceae*).

An agent can be one that is, or can be made to be, vaporizable. In some cases, the drug can be a heat stable drug. Exemplary drugs include acebutolol, acetaminophen, alprazolam, amantadine, amitriptyline, apomorphine diacetate, apomorphine hydrochloride, atropine, azatadine, betahistine, brompheniramine, bumetanide, buprenorphine, bupropion hydrochloride, butalbital, butorphanol, carbinoxamine maleate, celecoxib, chlordiazepoxide, chlorpheniramine, chlorzoxazone, ciclesonide, citalopram, clomipramine, clonazepam, clozapine, codeine, cyclobenzaprine, cyproheptadine, dapsone, diazepam, diclofenac ethyl ester, diflunisal, disopyramide, doxepin, estradiol, ephedrine, estazolam, ethacrynic acid, fenfluramine, fenoprofen, flecainide, flunitrazepam, galanthamine, granisetron, haloperidol, hydromorphone, hydroxychloroquine, ibuprofen, imipramine, indomethacin ethyl ester, indomethacin methyl ester, isocarboxazid, ketamine, ketoprofen, ketoprofen ethyl ester, ketoprofen methyl ester, ketorolac ethyl ester, ketorolac methyl ester, ketotifen, lamotrigine, lidocaine, loperamide, loratadine, loxapine, maprotiline, memantine, meperidine, metaproterenol, methoxsalen, metoprolol, mexiletine HCl, midazolam, mirtazapine, morphine, nalbuphine, naloxone, naproxen, naratriptan, nortriptyline, olanzapine, orphenadrine, oxycodone, paroxetine, pergolide, phenyloin, pindolol, piribedil, pramipexole, procainamide, prochloperazine, propafenone, propranolol, pyrilamine, quetiapine, quinidine, rizatriptan, ropinirole, sertraline, selegiline, sildenafil, spironolactone, tacrine, tadalafil, terbutaline, testosterone, thalidomide, theophylline, tocamide, toremifene, trazodone, triazolam, trifluoperazine, valproic acid, venlafaxine, vitamin E, zaleplon, zotepine, amoxapine, atenolol, benztropine, caffeine, doxylamine, estradiol 17-acetate, flurazepam, flurbiprofen, hydroxyzine, ibutilide, indomethacin norcholine ester, ketorolac norcholine ester, melatonin, metoclopramide, nabumetone, perphenazine, protriptyline HCl, quinine, triamterene, trimipramine, zonisamide, bergapten, chlorpromazine, colchicine, diltiazem, donepezil, eletriptan, estradiol-3,17-diacetate, efavirenz, esmolol, fentanyl, flunisolide, fluoxetine, hyoscyamine, indomethacin, isotretinoin, linezolid, meclizine, paracoxib, pioglitazone, rofecoxib, sumatriptan, tolterodine, tramadol, tranylcypromine, trimipramine maleate, valdecoxib, vardenafil, verapamil, zolmitriptan, zolpidem, zopiclone, bromazepam, buspirone, cinnarizine, dipyridamole, naltrexone, sotalol, telmisartan, temazepam, albuterol, apomorphine hydrochloride diacetate, carbinoxamine, clonidine, diphenhydramine, thambutol, fluticasone proprionate, fluconazole, lovastatin, lorazepam N,O-diacetyl, methadone, nefazodone, oxybutynin, promazine, promethazine, sibutramine, tamoxifen, tolfenamic acid, aripiprazole, astemizole, benazepril, clemastine, estradiol 17-heptanoate, fluphenazine, protriptyline, ethambutal, frovatriptan, pyrilamine maleate, scopolamine, and triamcinolone acetonide or pharmaceutically acceptable analogs or equivalents thereof.

In some cases, an agent is a parasympathomimetic alkaloid. In some cases, the parasympathomimetic alkaloid is nicotine, arecoline, muscarine, or pilocarpine.

In some cases, an agent is a nicotinic acetylcholine receptor agonist. In some cases, the nicotinic acetylcholine receptor agonist is nicotine, acetylcholine, choline, epibatidine, lobeline, or varenicline.

In some cases, an agent inhibits chromatin modifying enzymes (e.g., class I and II histone deaceytlases). In some cases, an agent that inhibits chromation modifying enzymes is nicotine.

In some cases, an agent is a nicotine analog or derivative. In some cases, the nicotine analog is EVP-6124. In some cases, a nicotine analog or derivative is described, e.g., in U.S. Patent Application Publication Nos. 20130157995, 20090234129, 20080108822, 20070186940, or 20080227088 or U.S. Patent Nos. 4,243,605, 5,015,741, 6,503,922, 6,995,265, or 7,132,545.

In some cases, a combination of at least or at most 1, 2, 3, 4, 5, 6, 7, 8, 9, 10, 11, 12, 13, 14, 15, 16, 17, 18, 19, or 20 agents is used. In some cases, a combination of between 1-2, 2-4, 4-6, 6-8, 8-10, 10-12, 12-14, 14-16, 16-18, or 18-20 agents is used. In some cases, a combination of between 1-5, 5-10, 10-15, or 15-20 agents is used.

**Formulations**

Any agent as provided herein for use in the methods and devices described herein can be in a formulation comprising one or more additional substances as provided herein. In some cases, the formulation comprising an agent (e.g., nicotine) and one or more additional substances is a liquid formulation. In some cases, the formulation is liquid at room temperature. In some cases, the liquid formulation is contained in a reservoir as provided herein in a device as provided herein and is liquid at an operating temperature of the device. The operating temperature of any of the devices as described herein can be at, below, or above room temperature. In some cases, the liquid formulation comprising a pharmaceutically active agent (e.g., nicotine) as provided herein is delivered as a liquid to a heater element as provided herein in a device as provided herein when a user inhales from the outlet or mouthpiece of the device. In some cases, the liquid formulation is not a viscous liquid. In some cases, the liquid formulation is not gel-like or a gel. In some cases, a liquid formulation comprising a pharmaceutically active agent (e.g., nicotine) as provided herein is not coated as a solid or film of any thickness onto a heater element as provided herein. In some cases, a liquid formulation comprising nicotine for use in the methods and devices described herein is not admixed with thickening agents and thereby has a viscosity that is reduced or is less than a liquid formulation comprising nicotine that has been admixed with a thickening agent. In some cases, a liquid formulation for use in the methods and devices as provided herein is not applied to or coated on a heater element as provided herein prior to use of the device by a user or subject as provided herein. In some cases, the liquid formulation comprising a pharmaceutically active agent is delivered as a liquid to a heater element in a device as provided herein only upon use of the device. Use of the device can be a user as provided herein inhaling or drawings on an outlet or mouthpiece on a device as provided herein. In some cases, inhalation on the outlet or mouthpiece draws carrier gas (e.g., air) ino the device through an inlet on the device as provided herein, wherein the flow of the carrier gas (e.g., air) through the inlet triggers delivery of a liquid formulation comprising a pharmaceutically active agent (e.g., nicotine) by any of the means provided herein to a heater element contained within the device.. The device can comprise one or more inlets as provided herein, wherein inhalation on an outlet draws carrier gas (e.g., air) through the one or more inlets simultaneously.

In some cases, one or more carriers or excipients is added to a liquid formulation to change a property of the formulation. One or more carrriers can be used to change the density, compressibility, specific weight, viscosity, surface tension, or vapor pressure of a liquid formulation.

### III. eHealth tools

#### Overview

Provided herein are eHealth tools which can include mobile devices, web-based devices, computer readable medium, and an eHealth-enabled electronic agent (e.g., nicotine) delivery platform. In some cases, an eHealth-enabled electronic nicotine delivery platform can help a smoker transition to clean nicotine delivery by delivering a pre-determined nicotine dose with a pre-determined nicotine particle size at a pre-determined time for an individual user of a device. The eHealth-enabled electronic nicotine delivery platform can provide nicotine to an individual user on a particular schedule, which may involve varying the number of doses per day, timing of doses within the day, or amount of nicotine per dose over time. In one embodiment, the eHealth-enabled electronic nicotine delivery platform is used to achieve full smoking cessation. In another embodiment, the eHealth-enabled electronic nicotine delivery platform is used to achieve full nicotine or smoking cessation without relapse to smoking. In another embodiment, the eHealth-enabled electronic nicotine delivery platform is used to achieve full nicotine withdrawal without nicotine withdrawal symptoms. In another embodiment, the eHealth-enabled electronic nicotine delivery platform is used in conjunction with other nicotine replacement therapies, nicotinic agonist or partial agonists (e.g., varenicline or any other $\alpha_4\beta_2$ subtype of the nicotinic acetylcholine receptor) to help achieve a particular smoking goal (e.g., complete cessation). In another embodiment, the eHealth tools can help to ensure user safety when administering doses of nicotine from an electronic nicotine delivery device, so as to prevent overdose.

The methods can be applied to a variety of types of classifications of users of combustible tobacco products, including a new smoker, a trough maintainer smoker, an intermittent smoker, a light smoker, a weight-loss smoker, a heavy smoker, or a very heavy smoker. An intermittent smoker can be an individual who does not smoke every day. A light smoker can be an individual who smokes 1 to 9 cigarettes per day. A moderate smoker can be an individual who smokes 10 to 19 cigarettes a day. A heavy smoker can be an individual who smokes 20 to 29 cigarettes per day. A very heavy smoker can be an individual who smokes 30 or more cigarettes per day. Different smokers may require different approaches to facilitate their transition from smoking.

Provided herein is a method for managing treatment of a condition. The method can comprise providing a device for generating a condensation aerosol comprising a pharmaceutically active agent. The pharmaceutically active agent can be an agent as provided herein. In some cases, the condition is smoking or nicotine addiction. In some cases, the pharmaceutically active agent is nicotine. The device for generating the condensation aerosol can be device as provided herein. The device can comprise a heater element. The heater element can be any heater element as provided herein. The heater element can vaporize a composition comprising the pharmaceutically active agent. In some cases, the formulation is a liquid formulation. The heater element can be in fluid communication with a source of the formulation. The source of the formulation can be a reservoir. The heater element can be in fluid communication with a passageway configured for permitting the condensation of the vaporized formulation to produce particles comprising a size effective for deep lung delivery. The size of the particles can have an MMAD of about 1 to about 5 um. The device can further comprise a programmable controller, wherein the programmable controller comprises a non-transitory computer readable medium comprising one or more algorithms, and an interface for communicating with the programmable controller, wherein the interface is capable of receiving information from and/or transmitting information to a source. The source can be a user of the device, a healthcare provider and/or a counselor. The methods provided herein can include inputting, receiving and/or recording data on the device; analyzing the data; and regulating a dosage, frequency of administration and/or delivery schedule of the condensed formulation comprising the pharmaceutically active agent based on the analysis of the data by the one or more algorithms. The method as provided herein can also comprise adjusting the dosage, frequency of administration and/or delivery schedule of the condensed formulation comprising the pharmaceutically active agent based on the information received from the source. The inputting, analysis, regulating, and, optionally, adjusting can be repeated in order to manage treatment of the condition. Prior to a user engaging in a method or using a device as provided herein for a first time, the dosage, frequency of administration and/or delivery schedule of the condensed formulation comprising the pharmaceutically active agent can be pre-set by a source. The analysis of the data can be performed by the one or more algorithms. The regulation the dosage, frequency of administration and/or delivery schedule of agent as provided herein can be based on an analysis of the data by the one or more algorithms.

Provided herein is a method for facilitating smoking cessation. The method can comprise providing any device for generating a condensation aerosol comprising a pharmaceutically active agent as provided herein, wherein the device comprises a programmable controller. The pharmaceutically active agent can be nicotine. In some cases, the subject inhales the condensation aerosol produced by the device a plurality of times, wherein inhaling a plurality of times produces a desired nicotine blood concentration. The desired nicotine plasma concentration can be a plasma concentration. In some cases, the desired plasma concentration can be an arterial plasma concentration. In some cases, the desired plasma concentration can be a venous plasma concentration. The desired nicotine plasma concentration can be about, more than, less than, or at least 1%, 2%, 3%,4%,5%, 6%, 7%, 8%, 9%, 10%, 11%, 12%, 13%, 14%, 15%, 16%, 17%, 18%, 19%, 20%, 30%, 31%, 32%, 33%, 34%, 35%, 36%, 37%, 38%, 39%, 40%, 41%, 42%, 43%, 44%, 45%, 46%, 47%, 48%, 49%, 50%, 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99%, or 100% of the nicotine plasma concentration achieved by smoking a cigarette. The desired nicotine plasma concentration can be between 1%-10%, 10%-20%, 20%-30%, 30%-40%, 40%-50%, 50%-60%, 60%-70%, 70%-80%, 80%-90%, or 90%-100% of the nicotine plasma concentration achieved by smoking a cigarette. The desired nicotine plasma concentration can be about 1% to about 10%, about 10% to about 20%, about 20% to about 30%, about 30% to about 40%, about 40% to about 50%, about 50% to about 60%, about 60% to about 70%, about 70% to about 80%, about 80% to about 90%, or about 90% to about 100% of the nicotine plasma concentration achieved by smoking a cigarette. Smoking a single cigarette can produce peak increments of plasma nicotine concentration of 5-30 ng/ml. The peak increments of plasma nicotine concentration from smoking a cigarette can be achieved within 10 mintues. The methods provided herein further comprise altering the dosage, frequency of administration, and/or delivery schedule of the condensation aerosol in order to alter the arterial nicotine plasma concentration. The alteration of the dosage, frequency of administration, and/or delivery schedule of the condensation aerosol can facilitate smoking cessation. In some cases, the dosage of the pharmaceutically active agent inhaled during each of the plurality of inhalations can be a percentage of a total dosage required for a specific period of time. The period of time can be a day, wherein each inhalation of the plurality of inhalations can be a percentage of the daily dosage. Each inhalation can be about, more than, less than, or at least 1%, 5%, 10%, 15%, 20%, 25%, 30%, 33%, 50%, or 100% of a total dosage.

An eHealth tool can be a healthcare practice supported by electronic processes and/or communication. In some cases, eHealth tools comprise healthcare practice using the Internet. The eHealth tools can be formatted for use by different types of smokers, including a new smoker, a weight loss smoker, a trough maintainer, a light smoker, a heavy smoker, or a very heavy smoker. The eHealth tools can be formatted for use by different types of patients who may be using nicotine to enhance their cognition or otherwise improve other symptoms of their illness (ulcerative colitis). In some cases the eHealth tools can communicate with a device described herein (e.g., through Bluetooth connectivity), or eHealth tools can be incorporated into a device described herein.

The eHealth tools provided herein include mechanisms for tracking use of a device. For example, the frequency of use of a device can be tracked. Also, provided herein are algorithms for analyzing the use of a device. The algorithms can be used to generate goals for a user of the device. In some cases, the algorithms can suggest a recommended dose of an agent (e.g., nicotine) for a user. The algorithms can suggest an agent (e.g., nicotine) delivery schedule for a user. Algorithms provided herein can change over time based on input from a device or feedback from the user over time. An eHealth nicotine delivery platform described herein can track use of a nicotine delivery device, assess the user in terms of their subjective nicotine craving, mood, or other psychological or behavioral parameters, and adjust nicotine delivery to accomplish desired effects. Smoking behavior can be tracked, as can other symptoms of a disease where nicotine is being used either as a treatment or to enhance deficiencies in cognition associated with a specific illness.

A smoking pattern of a user can be monitored, or use of a device described herein can be monitored. For example, tools provided herein can be used to determine if smoking or use of a device provided herein was used to satisfy a morning craving, determine if smoking occurred, or a device was used, while a subject was bored, drinking, under stress. Tools can be used to assess whether a subject smoked or used a device described herein alone or in the presence of others (e.g., friends), or whether the dose of nicotine administered was successful in enhancing cognition or improving another target medical or psychiatric symptom.

One or more algorithms can be used to devise a plan (e.g., nicotine dose, nicotine delivery schedule) for a user. In some cases, web-based tools can be used to transition a smoker to use of an electronic nicotine delivery device described herein along with customized behavioral input.

The eHealth tools provided herein can be used to enable smoking cessation, partial smoking cessation (e.g., use of an electronic nicotine delivery device at work, in public places, or on a plane), or full nicotine cessation. eHealth tools provided herein can be used to engage smokers, including teenagers and a person in their 20s to alter their smoking habits.

In some cases, the eHealth tools are web-based tools. The web-based tools can enable an appropriate dosing of nicotine for a user of a device described herein. In some cases, the web-based tools can track experiences of a user. In some cases, a web-based tool can track success in making a transition from smoking. Web-based tools described herein can track health benefits derived from using devices described herein. Such tracking can enable generation of rewards (e.g., decreased health premiums). Web-based tools can enable development of constantly-improving use algorithms by obtaining use profiles from a multitude of users in the field, and can provide feedback to users. In some cases, web-based tools described herein can leverage social media to produce ideal health outcomes. The social media can be a social networking site (e.g., Facebook, Google +, MySpace, Bebo), blog or microblog (e.g., Twitter), a content community (e.g., YouTube), a virtual social world (e.g., Second Life), a virtual game world (e.g., World of Warcraft), or a collaborative project (e.g., Wikipedia). Social media can include technologies such as a blog, picture-sharing, vlog, wall-posting, email, instant messaging, music-sharing, crowdsourcing, voice over IP, Internet forums, weblog, social blog, microblog, wiki, podcast, and social bookmarking. The customized feedback can also be specific for users suffering from a medical or psychiatric disorder. For example, nicotine has been shown to have beneficial effects on cognition among patients with schizophrenia. The device could be used to deliver nicotine and also provide therapeutic input to patients to help them manage their nicotine intake in such a way as to provide maximum therapeutic advantage to their cognition or psychiatric symptom control. Other disorders where nicotine has been shown to have beneficial effects on cognition include Parkinson's disease, attention deficient disorder, mild cognitive impairment, and Alzheimer's disease.

In some cases, an eHealth tool is a mobile device. In some cases, the mobile device is an electronic nicotine delivery device. The mobile device can ensure dosing occurs at an appropriate time. The mobile device can comprise on-board tracking of dosing, can provide reminders to a subject, and can provide nicotine craving assessments. Also, a mobile device can comprise complementary advertising opportunities.

The devices provided herein can comprise electronics that control for variability in battery condition and ensure consistent heating.

### Identifying individualized user goals

Customized goals can enable the same nicotine delivery platform to be used to achieve multiple beneficial health benefits for different smokers, from full smoking cessation, to partial cessation (e.g., no smoking during the workday), to partial or full nicotine cessation (i.e., stopping nicotine intake entirely), to cognition remediation. In one embodiment, the customized goals enable the same nicotine delivery platform to be used to achieve full smoking cessation. In another embodiment, the customized goals enable the same nicotine delivery platform to be used to achieve full nicotine or smoking cessation without relapse. In another embodiment, the customized goals enable the same nicotine delivery platform to be used to achieve full nicotine withdrawal without nicotine withdrawal symptoms.

eHealth tools can include Web based and mobile tools. For example, for web-based tools, self-report measures can be used to help a smoker or new user of a device provided herein identify a target goal based on their degree of nicotine dependency, health status, health goals, economic goals (i.e., decrease the amount of money spent on cigarettes), target body weight or change in body weight, or other factors. Tests of attentional bias for smoking stimuli, and other cognitive measures of nicotine dependency, can be assessed on the web backend or an electronic nicotine delivery device to assess risk for relapse upon smoking cessation, which can then be used to identify a pattern of use that will minimize the odds of relapse to smoking. In some cases, biomarkers, such as nicotine receptor polymorphisms, information can also be added to a user's profile to help identify the optimal outcome for an individual user.

When a mobile device is used, smoking patterns can be tracked prior to the transition to an electronic nicotine delivery platform, which can enable a real world, ecologically valid assessment of actual behavior to be used as a foundation for a subsequent prescribed pattern of use of an electronic nicotine delivery device.

## Algorithm development

The high rates of smoking relapse upon cessation (often greater than 90%) reflect the tremendous heterogeneity in the ways in which smoking quit attempts fail, even when using NRTs like a nicotine gum or patch. By systematically tracking user characteristics at the outset of a quit attempt, tracking their actual use of the electronic nicotine delivery device over time in terms of patterns of dosing, and real world risk factors associated with a smoking relapse, algorithms can be generated that can be used to suggest an optimal pattern of use, dose, pH, particle size, and other characteristics (e.g., flavoring) of the electronic nicotine delivery device to maintain use and minimize the risk of smoking relapse. These algorithms can be constantly enhanced through additional user experience, adding to the empirical foundation of the algorithms and enabling more robust and finer-grained algorithms to be customized to an individual user's nicotine dependency and health goals.

For a mobile device, data can be captured from individual users in the field and can be sent to a backend web-based central database for algorithm development. The mobile device can also assess the ecological risk factors for relapse and adjust the dose or dose characteristics of nicotine accordingly to help achieve the desired outcome. An initial trial of several different types of dose characteristics may also be helpful in determining the ideal use algorithm.

In a web-based method, data from real world use of the electronic nicotine delivery device can be collected and used to predict outcomes. Users can also pick from one of several established algorithms that they think will best suit their health or other goals. The central database can issue instructions back to the electronic nicotine device, either in the form of explicit compliance reminders to use the device to achieve the optimal nicotine absorption, or implicit dosing instructions to the device to gradually taper the dose (or other characteristics of the nicotine dose, including its concentration, pH, particle size, flavorings, or flow characteristics coming from the device which can affect back of the throat impaction, which in turn can affect subjective sensations associated with the nicotine dose (i.e., tingling or burning in the back of the throat)) over the days or weeks to help achieve various health or nicotine-related goals.

## Matching users to algorithms

A user's goal when transitioning off of combustible tobacco products may change over time (e.g., a user initially wanting to quit smoking only at work may come to choose full cessation over time). By carefully matching users to an initial use and dose algorithm, and then monitoring their progress over time, adjustments can be made to ensure the maximal probability of success in their individual goals.

For a mobile device, feedback from the mobile device, both in terms of use patterns as well as real-time self-reports of cravings, environmental risk factors for smoking relapse, and on-going tests of psychological dependency can be used help identify an initial use algorithm, as well as make changes to the use algorithm or switch to a new algorithm entirely.

For a web-based device, as new data is used to refine use algorithms, a web-based backend database can communicate subtle and/or gross changes in prescribed use algorithms to the device to help enhance the probability that a target goal will be achieved. In this way, each user can become part of a community helping to refine his/her own and others optimal algorithms to achieve a variety of goals.

## Customized dose, pH, particle size, etc.

By systematically varying different dose characteristics (e.g., dose, particle size, pH, amount of nicotine in the gas vs. particulate phase, air speed velocity coming out of a nicotine delivery device, flavorings, etc.), a differentially reinforcing subjective reward from the nicotine can be created. The probability that certain goals will be achieved (e.g., smoking cessation) can be maximized by varying dose characteristics of nicotine.

Relying on use algorithms matched to individual users regarding their stated goals, physical or psychological nicotine dependency characteristics, and/or biomarkers, the electronic nicotine delivery device can modify dose characteristics of nicotine. These modifications can occur gradually over time (e.g., slowly decreasing the dose of nicotine over days or weeks) to eventually help the user stop all nicotine intake or transition to other forms of nicotine replacement. In some cases, the modifications can change in response to environmental triggers (e.g., by altering the mean particle size of the dose to provide an especially reinforcing dose if the subject reports on the electronic nicotine delivery device a strong craving to return to smoking, or if their pattern of use is indicative of someone likely to return to smoking). In some cases, the modifications can change to help the initial transition off of combustible tobacco (e.g., by altering the pH or flavor of the dose to help match previous stimulus characteristics of smoking).

### Administering nicotine challenge doses

Routine administration of nicotine doses does not allow users to assess their level of nicotine dependency, nor to challenge themselves to achieve certain goals (e.g., full or partial nicotine cessation). An electronic nicotine delivery device can be used to administer challenge doses of nicotine to a user in order to assess their readiness to change use algorithms, assess physical and psychological aspects of nicotine dependency, or, for example, to demonstrate to users that they are increasingly able to wean themselves off of nicotine administration entirely.

As part of a behavioral program to achieve certain health or other nicotine-related goals, the electronic nicotine delivery device can administer one or more nicotine challenge doses. These challenge doses may contain no nicotine, less nicotine than previous doses, or doses of nicotine that vary in regards to other important characteristics (e.g., dose, particle size, pH, amount of nicotine in the gas vs. particulate phase, air speed velocity coming out of a nicotine delivery device, flavorings, etc). An electronic nicotine delivery device can then assess self-reported cravings or changes in a pattern of use that suggests increased or decreased nicotine administration. This feedback can then be used as real world data to help maintain or change the use algorithm to increase the probability that the user will achieve certain health or other nicotine-related goals.

### Customized feedback

By systematically tracking nicotine administration using an electronic nicotine delivery device described herein, and communicating these results to a web-based backend database, feedback to the user can be customized to help promote the achievement of certain health goals. This customized feedback can be used in multiple ways. For example, achieving certain use patterns (e.g., sufficient nicotine administration to forestall strong cravings after initially transitioning from smoking, or administering a new minimum amount of nicotine in accordance with a planned taper to nicotine cessation) can result in a virtual credit or actual monetary reward that can reinforce the user's pattern of nicotine administration.

Customized feedback can also be used to leverage psychological principles important in smoking cessation. For example, a user could be given feedback that his/her pattern of use is consistent with his/her goals (e.g., quitting smoking) or aim to enhance his/her self-efficacy by demonstrating that the user is becoming less dependent on nicotine based on a real world nicotine challenge presented by an electronic nicotine delivery device described herein (e.g., by administering a test dose containing no nicotine or less nicotine than the user had been administering and demonstrating that their nicotine craving did not increase, or significantly increase, after that challenge dose; see **FIG. 19**). Feedback could also engage users using game theory, including the earning of virtual points, which, for example, can be used to enable other actions of the device or to enhance one's standing in a virtual online world, or be redeemed for real-world reinforcements.

In some cases, the electronic nicotine delivery device or web backend system can reinforce a user's self-efficacy by giving him/her feedback that his/her pattern of use is indicative of other users who successfully abstain from smoking. In some cases, an electronic nicotine delivery device or web backend system can give the user feedback that his/her mean nicotine dose is decreasing over time, despite it initially remaining constant or nearly constant, so as to enhance self-efficacy and increase his/her resilience in the face of environmental smoking cues that could otherwise trigger a smoking lapse or relapse **(FIG. 20).**

"About" can mean a referenced numeric indication plus or minus 10% of that referenced numeric indication. For example, the term about 4 can include a range of 3.6 to 4.4.

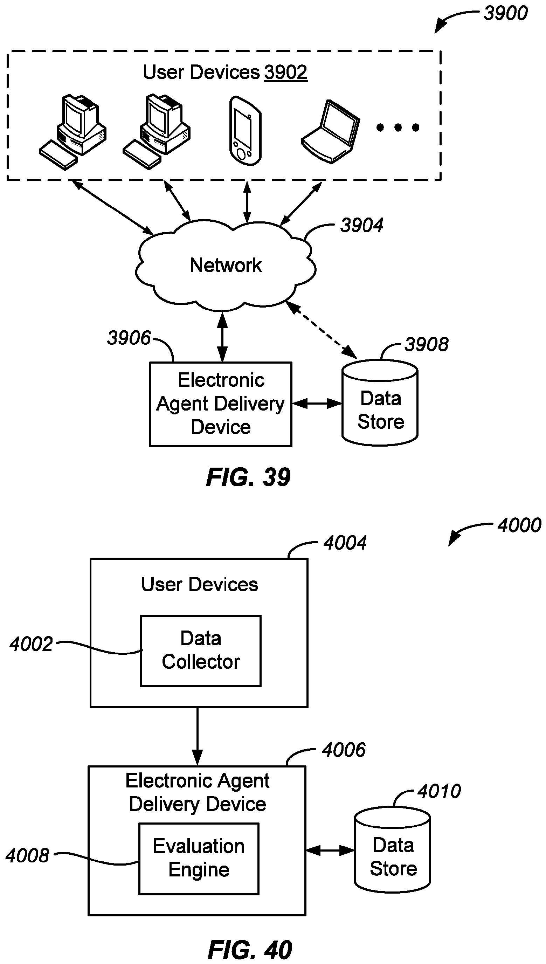

**FIG. 39** illustrates an example environment **3900** for implementing devices and methods described herein in accordance with an embodiment. As illustrated, one or more user devices **3902** connect via a network **3904** to an electronic agent (e.g., nicotine) delivery device **3906** as provided herein which can be configured to produce a condensation aerosol comprising a pharmaceutically active agent (e.g., nicotine) as provided herein. The electronic agent (e.g., nicotine) delivery device **3906** can comprise a controller, which can be programmable, as provided herein and the electronic agent (e.g., nicotine) delivery device **3906** can be connected to the network **3904** through the programmable controller. In some cases, the condensation aerosol comprising the pharmaceutically active agent (e.g., nicotine) is produced from a liquid formulation comprising the pharmaceutically active agent (e.g., nicotine) as provided herein. In various embodiments, the user devices **3902** can include any device capable of communicating with the network **3904,** such as personal computers, workstations, laptops, smartphones, mobile phones, tablet computing devices, smart TVs, game consoles, internet-connected set up boxes, and the like. In some embodiments, the user devices **3902** can include applications such as web browsers and/or applications (e.g., mobile apps) that are capable of communicating with the electronic agent (e.g., nicotine) delivery device **3906** and/or a system that uses the electronic agent (e.g., nicotine) delivery device **3906.** In some cases, the user devices **3902** communicate with the electronic agent (e.g., nicotine) delivery device **3906** via the programmable controller as provided herein. The user can be a patient, and/or a healthcare provider (e.g., physician, physician's assistant, nurse, nurse practioner, pharmacist or other medical professional). In some cases, a first user uses the device, while a second user uses the other user devices **3902.** In some cases, a first user uses the device and the other user devices **3902,** while the second user also uses the user devices **3902.**

In some embodiments, the electronic agent (e.g., nicotine) delivery device **3906** can communicate with a data store **3908** in order perform the functionalities described herein (e.g., track device usage, adjust dose, frequency of administration, delivery schedule, customize feedback, administer challenge doses, etc.). For example, the data store **3908** can be used to store historical (e.g. user use history, dosage history, delivery schedule history, frequency of administration history, etc.), evaluation rules, and the like.

In some embodiments, the data store **3908,** or any other data stores discussed herein, can include one or more data files, databases, (e.g., SQL database), data storage devices (e.g., tape, hard disk, solid-state drive), data storage servers, or the like. The data store **3908** can be connected to the electronic agent (e.g., nicotine) delivery device **3906** locally or remotely via a network. In some embodiments, data store **3908,** or any other data stores discussed herein, can comprise one or more storage services provisioned from a "cloud storage" provider, for example, Amazon Simple Storage Service ("Amazon S3"), provided by Amazon.com, Inc. of Seattle, Washington, Google Cloud Storage, provided by Google, Inc. of Mountain View, California, and the like.

In various embodiments, the network **3904** can include the Internet, a local area network ("LAN"), a wide area network ("WAN"), a cellular network, wireless network or any other public or private data and/or telecommunication network.

**FIG. 40** illustrates example components of an electronic agent (e.g., nicotine) delivery system **4000,** in accordance with an embodiment. In this example, the electronic agent (e.g., nicotine) delivery system **4000** includes a data collector **4002** residing on a user or client device **4004.** The system further comprises an electronic agent (e.g., nicotine) delivery device **4006,** which can be the same as **3906** as depicted in **FIG. 39.** The electronic agent (e.g., nicotine) delivery device **4006** can comprise a programmable controller, wherein the data collector resides on the programmable controller. The data collector can be implemented as a browser script using JavaScript or any other scripting language. The data collector can be configured to communicate with a web-based backend database. For example, the data collector can be configured to collect parameter information about the electronic agent (e.g., nicotine) delivery device **4006** such as discussed herein and transmit such parameter information to the web-based backend database, for example, using an application programming interface (API) provided by the user device **4004.** In some embodiments, the collection and/or communication with the user device **4004** can be triggered by an event on the electronic agent (e.g., nicotine) delivery device **4006.** For example, the event can include a click on a portion (e.g., a button or a link) of a user display on the electronic agent (e.g., nicotine) delivery device **4006,** use of the delivery device by a user or patient, and the like. The user display can be on the programmable controller as provided herein.

In some embodiments, the electronic agent (e.g., nicotine) delivery device **4006** can be configured to receive parameter information (e.g., dosage, frequency of administration, dosing schedule, etc.) provided by the data collector of the user device and to compare and/or analyze the parameter information received from the data collector of the user device to the parameter information from use of the electronic agent (e.g., nicotine) delivery device **4006.** To that end, the electronic agent (e.g., nicotine) delivery device 4006 can utilize an evaluation engine **4008.** The evaluation engine **4008** can be configured to analyze the parameter information in order to customize or adjust output parameters of the electronic agent (e.g., nicotine) delivery device **4006.** In some embodiments, the evaluation engine **4008** can be implemented using one or more server-side library files. In some embodiments, the evaluation engine **4008** can be implemented using one or more algorithms as probided herein for analyzing the respective parameter.

In some embodiments, customized feedback or a treatment regimen (e.g., agent dosage, frequency of administration and/or delivery schedule) can be evaluated based on some or all of the parameters as provided herein. For example, a lookup table (e.g., stored in memory) can be used to determine the weight values associated with some or all of the parameters. The weight values may or may not be further weighted, combined or otherwise processed to derive a final customized feedback or treatment regimen. In some embodiments, the lookup table and the one or more algorithms for deriving the customized feedback or treatment regimen can be included on one or more rules that are pre-determined based on historical data such as past usage and/or user activities. In some embodiments, analysis of parameter information and/or generation of customized feedback or treatment regimen can be performed in real time or nearly real time with respect to the receipt of the parameter information. In other embodiments, any or all of the above operations may be performed in an asynchronous mode, for example, using batch processing.

In some embodiments, the generated feedback and/or treatment regimen can be stored in a data store **4010.** In some embodiments, the data store **4010** can include a memory of a server, one or more data storage device (e.g., SSD, hard disk, taps), or a cloud-based storage service such as discussed in connection with **FIG. 39.** The data store **4010** may or may not be owned and/or operated by the same as the provider of the electronic agent (e.g., nicotine) delivery device **4006.**

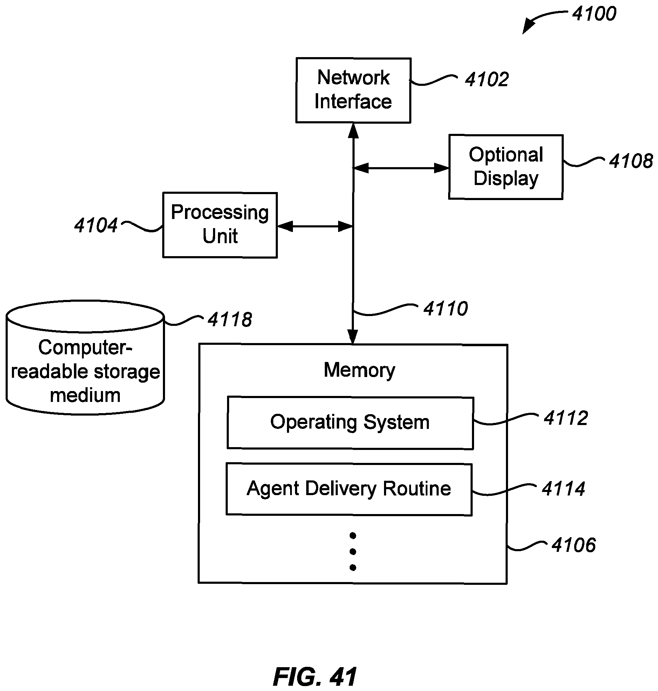

**FIG. 41** illustrates example components of a computer device **4100** for implementing aspects of devices and methods described herein, in accordance with an embodiment. In another embodiment, the computer device **4100** may be configured to implement a user device such as a user device **3902** discussed in connection with

**FIG. 39** and/or components or aspects of the electronic agent (e.g., nicotine) delivery device **3906** such as described in connection with **FIGs. 39** and **40.** In some embodiments, computing device **4100** can include many more components than those shown in **FIG. 4100.** However, it is not necessary that all of these components be shown in order to disclose an illustrative embodiment.

As shown in **FIG. 41,** computing device **4100** includes a network interface **4102** for connecting to a network such as discussed above. In some cases, the computing device **4100** is housed on a programmable controller on an electronic agent (e.g., nicotine) delivery device as provided herein. In various embodiments, the computing device **4100** may include one or more network interfaces **4102** for communicating with one or more types of networks such as the Internet, wireless networks, cellular networks, and any other network.

In an embodiment, computing device **4100** also includes one or more processing units **4104,** a memory **4106,** and an optional display or user interface as provided herein **4108,** all interconnected along with the network interface **4102** via a bus **4110.** The processing unit(s) **4104** can be capable of executing one or more methods or routines stored in the memory **4106.** The display **4108** can be configured to provide a graphical user interface to a user operating the computing device **4100** for receiving user input, displaying output, and/or executing applications. In some cases, such as when the computing device **4100** is a server, the display **4108** may be optional.

The memory **4106** can generally comprise a random access memory ("RAM"), a read only memory ("ROM"), and/or a permanent mass storage device, such as a disk drive. The memory **4106** may store program code for an operating system **4112,** one or more agent (e.g., nicotine) delivery routines **4114,** and other routines. In various embodiments, the program code can be stored on a computer-readable storage medium, for example, in the form of a computer program comprising a plurality of instructions executable by one or more processors. The computer-readable storage medium can be non-transitory. The one or more agent (e.g., nicotine) delivery routines **4114,** when executed, can provide various functionalities associated with the electronic agent (e.g., nicotine) delivery device as described herein.

In some embodiments, the software components discussed above can be loaded into memory **4106** using a drive mechanism associated with a non-transient computer readable storage medium **4118,** such as a floppy disc, tape, DVD/CD-ROM drive, memory card, USB flash drive, solid state drive (SSD) or the like. In other embodiments, the software components can alternatively be loaded via the network interface **4102,** rather than via a non-transient computer readable storage medium **4118.** In an embodiment, the computing device **4100** can also include an optional time keeping device (not shown) for keeping track of the timing of usage of the electronic agent (e.g., nicotine) delivery device.

In some embodiments, the computing device **4100** also communicates via bus **4110** with one or more local or remote databases or data stores such as an online data storage system via the bus **4110** or the network interface **4102.** The bus **4110** can comprise a storage area network ("SAN"), a high-speed serial bus, and/or via other suitable communication technology. In some embodiments, such databases or data stores may be integrated as part of the computing device **4100.**

**EXAMPLES**

**Example 1: Effect of changes in air flow rate, electrical current, duration of heating, and thickness of heater element on particle size of a aerosol generated from a propylene glycol formulation.**

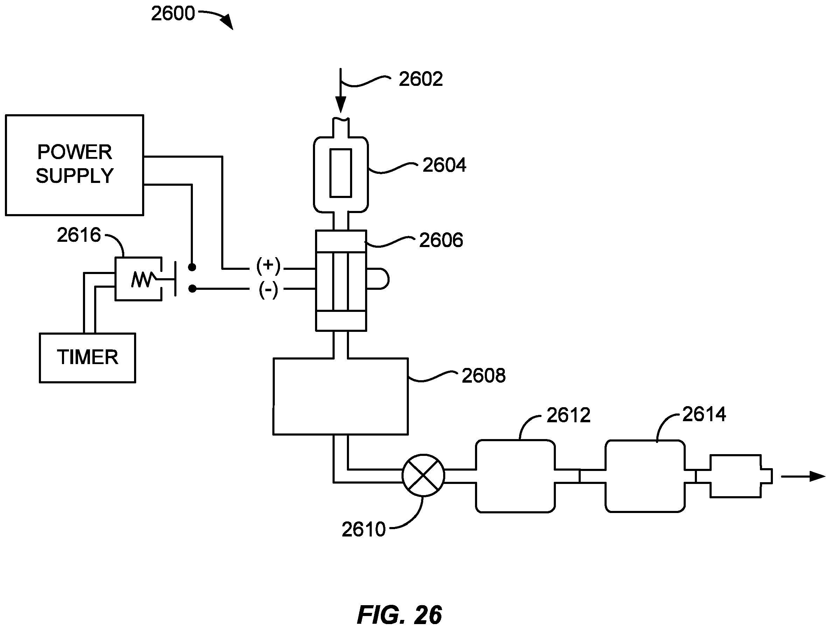

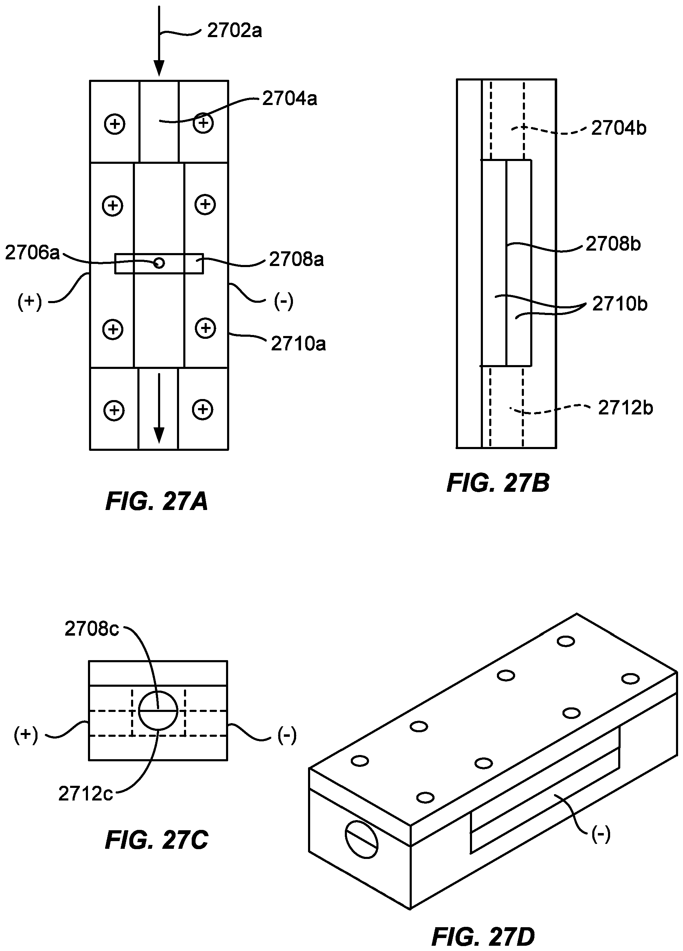

This example describes how changes in specific parameters (i.e. air flow rate, electrical current to a heater element, and thickness of a heater element) affect the size of aerosol particles generated by a test apparatus designed to comprise components and/or parameters of a nicotine delivery device as described herein. **FIG. 26** shows a schematic of the entire test apparatus while **FIG. 27** shows alternates views of the test airway used in the test apparatus. The test bed has an airway created between a block of Delrin (bottom) and a sheet of clear plexiglass (top) with brass sides used to clamp and make electrical contact with a heater element. The heater element is a stainless steel foil of variable thickness (0.0005 inches (about 0.013 mm) or 0.001 inches (about 0.025 mm)), and the formulation used to generate an aerosol is composed of propylene glycol. **FIG. 27A** shows a top view, with airflow **(2702a)** into an an inlet **(2704a).** A hole to deposit drug **(2706a)** is provided and foil is shown **(2708a).** Brass contacts **(2710a)** are provided. The length of the device is 6 inches (about 152.4 mm), and the width is 2.25 inches (about 57.15 mm). **FIG. 27B** shows a side view of the inlet **(2704b),** foil **(2708b),** brass electrical contacts **(2710b),** and outlet **(2712b). FIG. 27C** shows and end view of the foil **(2708c)** and **(2712c)**. **FIG. 27D** shows an isometric view. **Table 2** shows the results of altering heater element thickness, air flow rate, current, and duration of heating on particle size distribution. Based on the results in **Table 2,** as the air flow rate is increased, the particle size diameter (PSD) decreases when the other parameters are held constant.

**Table 2.**

| Sequence | Material | Heater Element Thickness (inches) | Air Flow Rate (Liters/min) | Dose (mg) | Current (Amps) | Duration of Heating (seconds) | Particle Size Diameter (microns) |
|---|---|---|---|---|---|---|---|
| | | | Propylene glycol aerosol data from test airway | | | | |
| 1 | PG | 0.0005 | 1 | 1 | 8 | 0.5 | 2 |
| 2 | PG | 0.0005 | 1 | 1 | 6 | 1 | 2.1-3 |
| 3 | PG | 0.001 | 1 | 1 | 8 | 0.7 | 1 |
| 4 | PG | 0.001 | 3 | 1 | 7 | 1 | 1.8 |
| 5 | PG | 0.001 | 3 | 1 | 7 | 1 | 2 |
| 6 | PG | 0.001 | 3 | 1 | 7 | 1 | 2 |
| 7 | PG | 0.001 | 3 | 1 | 7 | 1 | 1.5-1.8 |
| 8 | PG | 0.001 | 3 | 1 | 7 | 1 | 1.4-1.8 |
| 9 | PG | 0.001 | 3 | 1 | 7 | 1 | 2 |
| 10 | PG | 0.001 | 3 | 1 | 10 | 1 | 1 |
| 11 | PG | 0.001 | 3 | 1 | 10 | 1 | 0.9 |
| 12 | PG | 0.001 | 6 | 1 | 10 | 1 | 0.6 |
| 13 | PG | 0.001 | 6 | 1 | 10 | 1 | 0.6-0.8 |
| 14 | PG | 0.001 | 12 | 1 | 10 | 1 | 0.5 |
| 15 | PG | 0.001 | 12 | 1 | 10 | 1 | 0.5 |

**Example 2: Effect of changes in air flow rate, electrical current, duration of heating, and thickness of heater element on particle size of an aerosol generated from a nicotine/propylene glycol formulation.**

This example describes how changes in specific parameters (i.e. air flow rate, and electrical current to a heater element) affect the size of aerosol particles generated from a 10% nicotine/propylene glycol formulation by a test apparatus as described in Example 1. **Table 3** shows the results of altering heater element thickness, air flow rate, current, and duration of heating on particle size distribution. As shown in **Table 3,** when air flow rate is altered while other parameters are held constant, the higher the air flow rate, the smaller the average particle size diameter (PSD).

**Table 3.**

| Sequence | Material | Heater Element Thickness (inches) | Air Flow Rate (Liters/min) | Dose (mg) | Current (Amps) | Duration of Heating (seconds) | Average Particle Size Diameter (microns) |
|---|---|---|---|---|---|---|---|
| | | | Nicotine/propylene glycol mixture (10%) aerosol data from test airway | | | | |
| 1 | Nic/PG | 0.001 | 4 | 1 | 9 | 1 | 1.35 |
| 2 | Nic/PG | 0.001 | 4 | 1 | 9 | 1 | 1.45 |
| 3 | Nic/PG | 0.001 | 4 | 1 | 9 | 1 | 1.45 |
| 4 | Nic/PG | 0.001 | 2 | 1 | 9 | 1 | 1.85 |
| 5 | Nic/PG | 0.001 | 2 | 1 | 9 | 1 | 2.3 |
| 6 | Nic/PG | 0.001 | 2 | 1 | 9 | 1 | 2.3 |
| 7 | Nic/PG | 0.001 | 4 | 1 | 10 | 1 | 1.55 |

(continued)

| Nicotine/propylene glycol mixture (10%) aerosol data from test airway | | | | | | | |
|---|---|---|---|---|---|---|---|
| Sequence | Material | Heater Element Thickness (inches) | Air Flow Rate (Liters/min) | Dose (mg) | Current (Amps) | Duration of Heating (seconds) | Average Particle Size Diameter (microns) |
| 8 | Nic/PG | 0.001 | 4 | 1 | 10 | 1 | 1.2 |
| 9 | Nic/PG | 0.001 | 4 | 1 | 10 | 1 | 1.325 |

**Example 3: Particle size diameter ranges of aerosols generated from a test apparatus using a heater element comprising a wire coil.**

This example describes the particle size diameters of aerosols generated from either a PG formulation or 10% nicotine/PG formulation using a test apparatus as shown in **FIGs. 26** and **27** and described in Example 1. In this example, the heater element is a stainless steel coil comprising 3.5 coils and a diameter of 0.10 inches (about 2.54 mm). The heater element is heated using a current of 2.5 Amps and the air flow rate is 4 Liters/min (about 6.7 x $10^{-5}$ $m^3$/s). Table 4 shows the results.

**Table 4.**

| Sequence | Material | Air Flow Rate (Liters/min) | Dose (mg) | Current (Amps) | Duration of Heating (seconds) | Particle Size Diameter (microns) |
|---|---|---|---|---|---|---|
| 1 | PG | 4 | 1 | 2.5 | 1 | 1.5-2.2 |
| 2 | PG | 4 | 1 | 2.5 | 1 | 1.5-2.2 |
| 3 | Nic/PG | 4 | 1 | 2.5 | 1 | 1.57-2.2 |
| 4 | Nic/PG | 2 | 1 | 2.5 | 1 | 1.6-2.8 |
| 5 | Nic/PG | 2 | 1 | 2.5 | 1 | 1.52-2.2 |
| 6 | PG | 2 | 1 | 2.5 | 1 | 1.5-2.2 |
| 7 | PG | 4 | 1 | 2.5 | 1 | 1.5-2.3 |
| 8 | PG | 4 | 1 | 2.5 | 1 | 2.4-1.5 |

**Example 4: Particle size diameters of aerosols generated from commercially available e-cigarettes (eCigs).**

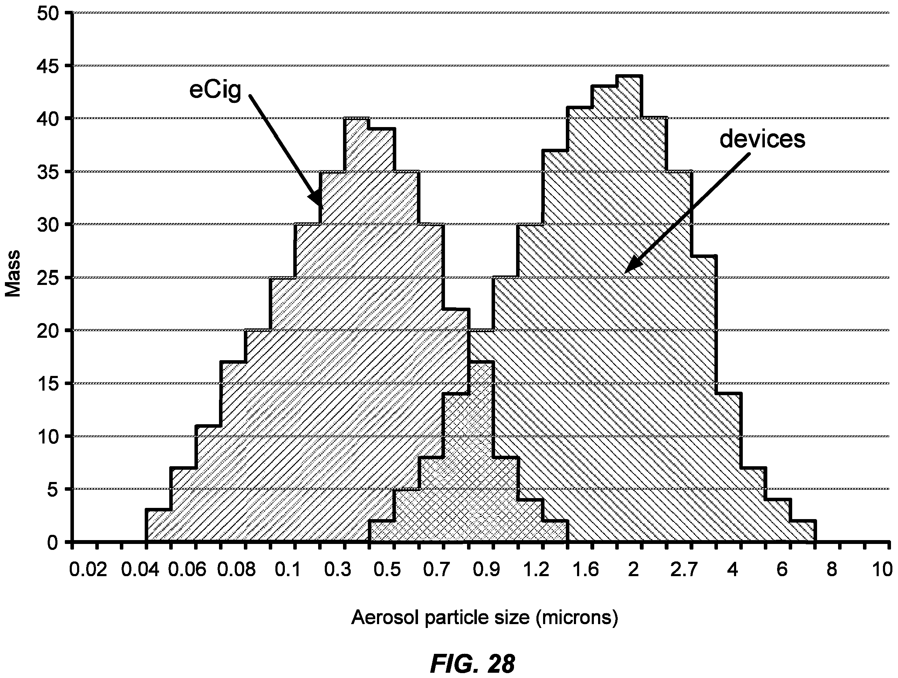

This example describes the particle size diameters of aerosols generated from either one of two brands of eCigs (Finiti and BLU). In this example, a 50 ml volume of an aerosol was pulled from either one of the two brands of eCigs over a period of 3 seconds in order to simulate a human breath. The collected aerosol was then injected into a laser particle size detector set at a flow rate of 14 Liters/min (about 2.33 x $10^{-4}$ $m^3$/s). **Table 5** shows the particle size diameter of the aerosols generated from two brands of eCigs. **FIG. 28** shows a comparison of the particle size distribution for aerosols created by eCigs vs. aerosol created by devices provided herein (devices). As shown in **FIG. 28,** the particle size distribution of aerosols generated by devices provided herein is shifted toward larger particle sizes vs. those generated by eCigs.

**Table 5.**

| Test Number | Brand | Particle Size | | |
|---|---|---|---|---|
| | | Low End | High End | Average |
| 1 | Finiti | 0.5 | 0.5 | 0.5 |
| 2 | Finiti | 0.5 | 0.6 | 0.55 |
| 3 | Finiti | 0.5 | 0.5 | 0.5 |
| 4 | Finiti | 0.5 | 0.5 | 0.5 |

(continued)

| Test Number | Brand | Particle Size | | |
|---|---|---|---|---|
| | | Low End | High End | Average |
| 5 | BLU | 0.5 | 0.5 | 0.5 |
| 6 | BLU | 0.5 | 0.8 | 0.65 |

**Example 5: Effect of changes in valve material, and the diameter of a bypass orifice on particle size of a aerosol generated from a propylene glycol formulation.**

This example describes how changes in specific parameters (i.e. valve material and diameter of a bypass orifice) affect the size of aerosol particles generated by a test apparatus designed to comprise components and/or parameters of a device for generating condensation aerosols as described herein. **FIG. 29A** shows a schematic of the entire test apparatus while **FIG. 29B** shows an internal view of the valve **(2904a)** used in the test apparatus. The valve flap **(2902b)** has a ¾ inch diameter and the diameter of the channel downstream of the valve is 0.375 inches (about 9.53 mm) in length and 0.090 inches (about 2.29 mm) in width. The test bed has a primary airway **(2906a),** and a bypass airway **(2908a),** an aerosol generation chamber **(2912a)** and vacuum source **(2910a).** The aerosol generation chamber comprises a heater element. The inlet to the bypass airway is a slot of varying dimensions (L x W). **Table 6** shows the results using a valve of ¾ inch (about 19.05 mm) diameter and altering valve material and bypass orifice diameter. As shown in **Table 6,** regardless of valve material type and bypass orifice diameter, above inhalation pressures of about 2 inches of H2O (about 498 Pa), the primary flow remains relatively constant, while the bypass flow increases with increasing vacuum pressure. **Table 7** shows the results using a valve of 3/8 inch diameter, a bypass orifice of varying dimensions, and altering the orifice dimensions for the inlet of the primary airway. As shown in **Table 7,** reducing the size of the orifice of the primary airway consistently reduces the flow rate through the primary airway regardless of varying vacuum pressure, dimensions of the bypass orifice, or varying the valve material

**Table 6:** Testing of Flow Control with the device of **FIG. 29.**

| Valve Material | Flow Bypass (LPM) | Flow Primary (LPM) | $\Delta$ P Vac (inches $H_2O$) | Total Flow (LPM) | Bypass $\phi$ (inches) |
|---|---|---|---|---|---|
| .0045" Brown | 15.4 | 4.9 | 2.11 | 20.03 | .149 |
| .0045" Brown | 18.6 | 5.6 | 3 | | .149 |
| .0045" Brown | 21.5 | 6.39 | 4.2 | | .149 |
| .0045" Brown | 24.2 | 6.94 | 5.5 | | .149 |
| .0045" Brown | 28.75 | 7.62 | 8 | | .149 |
| .0045" Brown | 31.7 | 7.9 | 9.6 | | .149 |
| .0045" Brown | 34.6 | 8.2 | 11.3 | | .149 |
| .0045" Brown | 38.2 | 8.5 | 14 | | .149 |
| Green | 9.5 | 1.99 | .3 | | .199 |
| | 17.08 | 3.49 | .93 | | .199 |
| | 24.80 | 4.39 | 2.0 | | .199 |
| | 31.7 | 4.80 | 3.2 | | .199 |
| | 38.2 | 5.0 | 4.7 | | .199 |
| | 44.2 | 5.11 | 6.3 | | .199 |
| | 49.4 | 5.18 | 8.2 | | .199 |
| | 53 | 5.10 | 9.8 | | .199 |

| Valve Slot Size (inches) | Bypass $\phi$ (inches) | Bypass Flow (LPM) | Primary Flow (LPM) | $\Delta$ P Vac (inches $H_2O$) | Valve Material |
|---|---|---|---|---|---|
| .300 | .199 | 6.0 | 2.9 | .1 | Green |
| .300 | .199 | 9.2 | 4.2 | .28 | Green |
| .300 | .199 | 14.1 | 6.2 | .65 | Green |
| .300 | .199 | 17.5 | 7.4 | .99 | Green |
| .300 | .199 | 24.4 | 7.6 | 1.9 | Green |
| .300 | .199 | 28.9 | 7.5 | 2.7 | Green |

(continued)

| Valve Slot Size (inches) | Bypass φ (inches) | Bypass Flow (LPM) | Primary Flow (LPM) | Δ P Vac (inches H$_2$O) | Valve Material |
|---|---|---|---|---|---|
| .300 | .199 | 33.9 | 6.3 | 3.7 | Green |
| .300 | .199 | 38.0 | 5.46 | 4.8 | Green |
| .300 | .199 | 46.7 | 4.76 | 7.5 | Green |
| .300 | .199 | 50.3 | 4.6 | 8.5 | Green |
| .300 | .199 | 54 | 4.6 | 9.8 | Green |
| .300 | 1.99 | 5.9 | 2.6 | .1 | Brown |
| .300 | 1.99 | 7.9 | 3.6 | .2 | Brown |
| .300 | 1.99 | 11.8 | 5.4 | .45 | Brown |
| .300 | 1.99 | 17.7 | 7.9 | 1.0 | Brown |
| .300 | 1.99 | 23.9 | 10.48 | 1.9 | Brown |
| .300 | 1.99 | 28.59 | 11.76 | 2.7 | Brown |
| .300 | 1.99 | 33.2 | 11.9 | 3.7 | Brown |
| .300 | 1.99 | 38.5 | 10.9 | 5.0 | Brown |
| .300 | 1.99 | 42.8 | 10.3 | 6.0 | Brown |
| .300 | 1.99 | 45.5 | 10.2 | 6.8 | Brown |
| .300 | 1.99 | 48.6 | 9.6 | 7.9 | Brown |
| .300 | 1.99 | 49.5 | 9.7 | 8.3 | Brown |

**Table 7:** Re-lay out of valve with 3.8 radius and smaller slot (device of **FIG. 29).**

| Bypass φ (inches) | Primary Slot Size (inches) | Bypass Flow (LPM) | Primary Flow (LPM) | Δ P Vac (inches H$_2$O) | Flap Material (Color) |
|---|---|---|---|---|---|
| .265 | .04 x .150 | 8.75 | .65 | .13 | Brown |
| .265 | .04 x .150 | 12.5 | .95 | .23 | Brown |
| .265 | .04 x .150 | 18.0 | 1.4 | .45 | Brown |
| .265 | .04 x .150 | 40.3 | 3.14 | 2.02 | Brown |
| .265 | .04 x .150 | 25.0 | 1.99 | .84 | Brown |
| .265 | .04 x .150 | 64.0 | 4.5 | | Brown |
| .199Ø Equivalent (EQUI) SLOT | .04 x .150 | 18.7 | 2.82 | 1.38 | Green |
| .199Ø EQIU SLOT | .04 x .150 | 21.8 | 3.19 | 1.8 | Green |
| .199Ø EQIU SLOT | .04 x .150 | 25.5 | 3.68 | 2.54 | Green |
| .199Ø EQIU SLOT | .04 x .150 | 29.5 | 4.07 | 3.26 | Green |
| .199Ø EQIU SLOT | .04 x .150 | 34.1 | 4.45 | 4.19 | Green |
| .199Ø EQIU SLOT | .04 x .150 | 38.7 | 4.75 | 5.21 | Green |
| .199Ø EQIU SLOT | .04 x .150 | 43.3 | 4.88 | 6.2 | Green |
| .199Ø EQIU SLOT | .04 x .150 | 46.2 | 4.97 | 7.0 | Green |
| .199Ø EQIU SLOT | .04 x .150 | 54.1 | 4.79 | 9.12 | Green |
| .199Ø EQIU SLOT | .04 x .150 | 55.0 | 4.69 | 9.9 | Green |
| .199Ø EQIU SLOT | .04 x .150 | 19.8 | 1.05 | 1.5 | .001 KAPTON |
| .199Ø EQIU SLOT | .04 x .150 | 28.6 | 1.37 | 3.17 | .001 KAPTON |
| .199Ø EQIU SLOT | .04 x .150 | 35.7 | 1.10 | 4.56 | .001 KAPTON |
| .199Ø EQIU SLOT | .04 x .150 | 41.7 | .97 | 5.8 | .001 KAPTON |
| .199Ø EQIU SLOT | .04 x .150 | 46.7 | .94 | 7.1 | .001 KAPTON |
| .199Ø EQIU SLOT | .04 x .150 | 60.8 | .94 | 11.5 | .001 KAPTON |

(continued)

| Bypass φ (inches) | Primary Slot Size (inches) | Bypass Flow (LPM) | Primary Flow (LPM) | Δ P Vac (inches H₂O) | Valve Material |
|---|---|---|---|---|---|
| .199 "SLOT" | .040 x .275 | 16.7 | 1.79 | 1.08 | .001 KAPTON |
| .199 "SLOT" | .040 x .275 | 18.1 | 1.87 | 1.3 | .001 KAPTON |
| .199 "SLOT" | .040 x .275 | 25.3 | 2.12 | 3.48 | .001 KAPTON |
| .199 "SLOT" | .040 x .275 | 35.7 | 2.7 | 4.6 | .001 KAPTON |
| .199 "SLOT" | .040 x .275 | 43.5 | 2.8 | 6.4 | .001 KAPTON |
| .199 "SLOT" | .040 x .275 | 50.2 | 2.8 | 8.34 | .001 KAPTON |
| .199 "SLOT" | .040 x .275 | 54.0 | 2.72 | 9.67 | .001 KAPTON |
| .199 "SLOT" | .040 x .275 | 56.3 | 2.64 | 10.4 | .001 KAPTON |
| VALVE REVERSED | | | | | |
| .199 "SLOT" | .040 x .275 | 19.4 | 1.5 | 1.45 | .001 KAPTON |
| .199 "SLOT" | .040 x .275 | 24.8 | 1.89 | 2.3 | .001 KAPTON |
| .199 "SLOT" | .040 x .275 | 36.2 | 2.36 | 4.7 | .001 KAPTON |
| .199 "SLOT" | .040 x .275 | 41.3 | 2.5 | 5.8 | .001 KAPTON |
| .199 "SLOT" | .040 x .275 | 50.4 | 2.6 | 8.3 | .001 KAPTON |
| .199 "SLOT" | .040 x .275 | 55.9 | 2.6 | 9.6 | .001 KAPTON |
| RETEST | | | | | |
| .199 "SLOT" | .040 x .275 | 12.4 | 1.56 | 0.6 | .001 KAPTON |
| .199 "SLOT" | .040 x .275 | 21.1 | 1.65 | 1.71 | .001 KAPTON |
| .199 "SLOT" | .040 x .275 | 30.2 | 2.0 | 3.4 | .001 KAPTON |
| .199 "SLOT" | .040 x .275 | 41.5 | 2.08 | 6.0 | .001 KAPTON |
| .199 "SLOT" | .040 x .275 | 50.1 | 2.03 | 8.4 | .001 KAPTON |
| .199 "SLOT" | .040 x .275 | 57.5 | 1.65 | 11.0 | .001 KAPTON |
| .199 "SLOT" | .040 x .275 | 46.0 | 1.64 | 7.5 | .001 KAPTON |
| .199 "SLOT" | .040 x .275 | 33.7 | 1.55 | 4.32 | .001 KAPTON |
| .199 "SLOT" | .040 x .275 | 19.5 | 1.36 | 1.48 | .001 KAPTON |
| .199 "SLOT" | .040 x .275 | 30.0 | 1.76 | 9.39 | .001 KAPTON |

**Example 6: Particle size diameters of aerosols generated from devices comprising wire coil heater elements and bypass inlets.**

This example describes the particle size diameters (PSD) of aerosols generated from a device comprising a heater element comprising a wire coil. An example of this type of device is shown in **FIGs. 31A-D. FIG. 31A** depicts a device designated ENT-100-A, (two inches (about 50.8 mm) long) comprising a primary carrier gas inlet **(3112a),** positive and negative brass contacts **(3110a),** a heater element **(3106a)** comprising a coil located distally from the inlet to the primary airway **(3112a)** and two bypass inlets **(3104a)** located (disposed) downstream of the heater element but prior to the outlet **(3102a). FIG 31B** depicts a device designated ENT-100-B, which is the same as ENT-100-A except that the heater element has been moved to be proximal to the inlet of the primary airway **(3112b). FIG. 31C** depicts a device designated ENT-100-C, which is similar to the ENT-100-A device except that the wire coil heater element has been moved to an intermediate position relative to the location of the coil in ENT-100-A and ENT-100-B. Any of the devices depicted in **FIG. 31A-C** can comprise the wire coil heater element designated "A Coil" **(3114e)** or "B Coil" **(3116e)** as illustrated in **FIG. 31E.** The coil in both types of heater elements comprise inner diameter of 0.26 inches (about 6.604 mm). The "A Coil" comprises a stretch of coil followed by a straight lead on either end of the coil which connects to the brass contacts. The "B Coil" comprises a stretch of coil, wherein the coil itself connects to the brass contacts. **Tables 8-12** shows the particle size diameter of the aerosols generated from the devices depicted in **FIG. 31A-C. Table 8** shows the PSD of particles generated using an ENT-100-A device with the "B Coil". **Table 9** shows the PSD of particles generated using an ENT-100-B device with the "A Coil". **Table 10** shows the PSD of particles generated using an ENT-100-B device with the "B Coil". **Table 11** shows the PSD of particles generated using an ENT-100-C device with the "A-Coil". **Table 12** shows the PSD of particles generated using an ENT-100-C device with the "B-Coil".

**Table 8:** Testing of ENT-100-A,B prototype

| Total Flow (LPM) | Primary Flow (LPM) | Bypass Flow (LPM) | PSD (microns) | Notes |
|---|---|---|---|---|
| | Dose = 2 mg (propylene glycol formulation), current = 3 amps, duration = 1 sec. |
| 9.7 | N/A | N/A | 1.7 - 1.8 | ENT-100-A Device |
| 9.7 | N/A | N/A | 1.5 - 2.1 | |
| 2.2 | 1.67 | | 0.4 - 0.5 | ENT-100-A Device w/o screen in flow valve |
| 2.2 | 1.67 | | 038 - 0.5 | |
| 2.2 | .7 | | 1.7 - 1.5 | |
| 2.2 | 2.3 | | 0.4 | w/screen |
| 32 | 1.6 | N/A | 0.4 | ENT-100-B (heater coil moved aft) |
| Ø | 0.7 | N/A | 1.7 - 2.0 | |
| Ø | 0.66 | N/A | 1.4 - 1.5 | |
| | 1.7 | Ø | 0.5 - 1.0 | Bypass taped over ENT-100-B |
| | 1.7 | Ø | 0.5 - 1.0 | Bypass taped over ENT-100-B |
| | 1.7 | Ø | 0.5 - 1.0 | Bypass taped over ENT-100-B |
| | 1.7 | Ø | 0.5 - 1.0 | Bypass taped over ENT-100-B |
| | 0.5 | Ø | 3 | Bypass taped over ENT-100-B |
| | 0.51 | Ø | 2.9 | Bypass taped over ENT-100-B |
| | .82 | Ø | 3.3 / 1.8 | Bypass taped over ENT-100-B |
| | .84 | Ø | 3.2 - 3.3 | Bypass taped over ENT-100-B |
| | 1.1 | Ø | 2.7 | Bypass taped over ENT-100-B |
| | 1.11 | Ø | 2.7 - 2.8 | Bypass taped over ENT-100-B |
| | 1.38 | Ø | 2.1 - 2.3 | Bypass taped over ENT-100-B |
| | 1.42 | Ø | 2.2 - 2.4 | Bypass taped over ENT-100-B |
| | 1.72 | Ø | 1.7 | Bypass taped over ENT-100-B |
| | 1.72 | Ø | 1.7 - 1.75 | Bypass taped over ENT-100-B |
| | 2.04 | Ø | .5 - 1.0 | Bypass taped over ENT-100-B |

| Primary Flow (LPM) | Bypass Flow (LPM) | PSD (microns) | Notes | | |
|---|---|---|---|---|---|
| 1.45 | Ø | 2.3 | colspan="3" | ENT-100-B Device Flap removed from flow valve |
| 1.45 | Ø | 2.2 - 2.4 | | | |
| 1.74 | Ø | 1.95 - 2.0 | | | |

(continued)

| Primary Flow (LPM) | Bypass Flow (LPM) | PSD (microns) | Notes |
|---|---|---|---|
| 1.75 | Ø | 1.8 - 1.9 | |
| 2.04 | Ø | 1.7 - 1.8 | |
| 2.04 | Ø | 1.6 - 1.7 | |
| 3.0 | Ø | 0.5 - 1.0 | |
| 3.0 | Ø | 0.5 - 1.0 | |
| 3 | Ø | 0.5 - 1.0 ST | Flow control valve removed/replaced with Black Delyrn W O.196φ hole |
| 3 | Ø | 2.0 - 2.3 | |
| 3 | Ø | 23 - 2.4 | |
| 1.04 | Ø | No trigger | |
| 2.0 | Ø | 3.8 | |
| 2.04 | Ø | 0.5 - 1.0 | With foam (open cell packing foam used to even out air flow, placed upstream from the heater element), no valve |
| 2.04 | Ø | 0.5 - 1.0 ST | |
| 1.05 | Ø | 1.8 - 2.1 | |
| 1.05 | Ø | 2.0 - 2.1 | |
| 1.5 | Ø | .79 - 1.0 | |
| 1.49 | Ø | 1.6 | |
| 1.25 | Ø | 1.6 | |
| 1.24 | Ø | 0.7 - 1.2 | |
| 1.24 | Ø | 0.7 - 1.2 | |
| 2.0 | Ø | 0.5 - 1.0 | |
| 2.0 | Ø | 0.5 - 1.0 | |

**Table 9:** Testing of ENT-100-B device with "A Coil" heater element

| Dose = 2 mg (propylene glycol formulation), 1 sec duration, current 3.1 amps | | |
|---|---|---|
| **Flow (LPM)** | **PSD (Microns)** | **Notes** |
| 1.01 | 3.4 - 3.6 | |
| 1.01 | 3.1 - 3.5 | |
| 1.51 | 2.6 - 2.7 | |
| 1.51 | 2.5 - 2.7 | |
| 2.06 | 2.6 - 2.3 | |
| 2.12 | 2.15 - 2.2 | |
| 2.48 | 1.9 - 2.2 | |
| 2.49 | 1.85 - 1.9 | |
| 3.02 | 1.5 - 1.6 | |
| 3.02 | 1.4 - 1.5 | |
| 3.02 | 1.35 - 1.45 | |
| 3.04 | 1.45 - 1.6 | |
| 3.26 | 1.4 - 1.6 | |
| 3.27 | 1.3 - 1.5 | |
| 4.25 | | |

**Table 10:** Testing of ENT-100-B device with "B Coil" heater element

| Dose = 2 mg (propylene glycol formulation), Duration 1 sec, current 2.0 amps | | | |
|---|---|---|---|
| **Dose (mg)** | **Flow (LPM)** | **PSD (microns)** | **Notes** |
| 2 | 1.5 | 2.9 - 3.1 | With foam |
| 2 | 1.53 | 2.6 - 2.8 | |
| 2 | 1.53 | 2.8 - 2.9 | |
| 2 | 2.49 | 1.8 - 1.9 | |
| 2 | 2.49 | 1.7 - 1.8 | |
| 2 | 3.01 | 1.4 | |
| 2 | 3.01 | 1.4 - 1.5 | |
| 2 | 3.49 | | |
| 2 | 1.55 | 2.5 | With stainless steel (SS) screen to |
| | 1.56 | 2.6 - 2.9 | even flow |
| | 1.56 | 2 - 2.5 | Taped up bypass |
| | 2.52 | 1.5 - 1.6 | |
| | 2.56 | 1.5 | |
| | 2.35 | 1.8 - 2.0 | With foam (taped up bypass) |
| | 2.51 | 1.9 - 2.0 | |
| | 2.48 | 1.9 | |
| | 1.48 | 2.9 - 3.0 | |
| | 1.50 | 2.8 - 3.0 | |
| | 1.5 | 1.8 - 1.9 | Bypass untaped Total flow ~ 8.5 LPM |
| | 1.52 | 1.7 - 1.8 | |
| | 1.48 | 1.2 - 1.1 | With 0.42 φ orifice added to primary inlet (Total flow = 24) |
| | 1.5 | 1.7 - 1.8 | With heater element moved aft |
| | 1.60 | 1.7 - 1.75 | - B configuration (Total flow 12 LPM) |

**Table 11:** Testing of ENT-100-C with "A Coil" heater element, which has 7 coils

| Current set @ 2.0 amps, 1 sec, 2 mg dose (propylene glycol formulation) | | | | |
|---|---|---|---|---|
| **Inlet orifice (inches)** | **Primary Flow (LPM)** | **PSD (microns)** | **$\Delta P$ Vac (inches $H_2O$)** | **Notes** |
| .04 | 1.01 | 4.6 - 5 | 2.48 | No adder |
| .04 | 1.00 | 4.3 - 4.7 | 2.50 | 0.250 straight tube |
| .04 | 3.00 | 1.7 - 1.8 | 17.5 | 2.4 amps |
| .04 | 3.00 | 1.6 - 1.7 | 17.2 | 2.4 amps |
| .04 | 4.85 | ~ 1.0 | LIMIT | |
| .020 + FOAM | 0.98 | 2.2 - 2.4 | .45 | 2.4 amps - No adder |
| .020 + FOAM | 1.00 | 3.5 - 4.0 | .46 | 2.4 amps - No adder |
| .020 + FOAM | 1.00 | 4.2 - 4.7 | .46 | 2.4 amps - No adder |
| .020 + FOAM | 1.00 | 4.0 - 5.7 | .46 | 2.4 amps - No adder |
| .020 + FOAM | 1.00 | 3.0 - 4.3 | .46 | 2.4 amps - No adder |
| .020 + FOAM | 2.09 | 2.2 | 1.52 | 2.4 amps - No adder |
| .020 + FOAM | 2.07 | 2.4 - 2.5 | 1.51 | 2.4 amps - No adder |

(continued)

| Current set @ 2.0 amps, 1 sec, 2 mg dose (propylene glycol formulation) | | | | |
| --- | --- | --- | --- | --- |
| Inlet orifice (inches) | Primary Flow (LPM) | PSD (microns) | $\triangle$ P Vac (inches $H_2O$) | Notes |
| .020 + FOAM | 2.07 | 2.2 - 2.4 | 1.48 | 2.4 amps - No adder |
| .020 + FOAM | 2.08 | 2.4 - 2.5 | 1.53 | 2 amps |
| .020 + FOAM | 2.08 | 2.1 - 2.3 | 1.53 | 2 amps |
| .020 + FOAM | 2.09 | 2.5 - 2.6 | 1.53 | 2 amps |

**Table 12:** Testing of ENT-100-C with "B Coil" heater element, with 0.050 spacer between contacts then spread to .200 in

| Current set @ 2.0 amps, 1 sec, 2 mg dose (propylene glycol formulation) | | | | |
| --- | --- | --- | --- | --- |
| Flow (LPM) | PSD (microns) | $\triangle$ P Vac (inches $H_2O$) | Current (amps) | Notes |
| .94 | 3.0 - 3.2 | .67 | 2.4 | |
| .94 | 2.4 - 2.5 | .67 | 2.8 | |
| .95 | 2.5 - 3.1 | .67 | 2.8 | |
| .95 | 3.3 - 3.4 | .67 | 2.8 | |
| .95 | 2.7 - 3.4 | .67 | 2.8 | |
| 2.11 | 2.3 - 2.4 | 2.58 | 2.8 | |
| 2.11 | 2.3 - 2.7 | 2.58 | 2.8 | |
| 2.11 | 2.6 - 2.7 | 2.58 | 2.8 | |
| New Heater Element .040 ID | | | | |
| 1.91 | 1.7 - 2.0 | .86 | 2.4 | |
| 1.91 | 2.4 - 2.5 | .86 | 2.6 | |
| 1.97 | 2.6 - 2.7 | .86 | 2.6 | |
| 1.91 | 2.4 - 2.5 | .86 | 2.6 | |
| 1.91 | 2.5 - 2.6 | .86 | 2.6 | |
| 1.91 | 2.4 - 2.5 | .86 | 2.8 | |
| 2.04 | 1.8 - 2.0 | .96 | 2.8 | |
| 2.04 | 2.4 - 2.7 | .96 | 2.8 | |
| 2.04 | 2.0 - 1.9 | .96 | 2.8 | |
| New Heater Element .032 ID 0.100 stretch | | | | |
| 2.04 | 2.0 - 2.5 | .93 | 2.6 | |
| 2.04 | 2.0 - 2.2 | .96 | 2.6 | |
| 2.04 | 2.1 - 2.3 | .96 | 2.6 | Spit (nicotine/ propylene glycol was heated under conditions (air flow, heating rate) that lead to the mixture being boiled off of the heater element and "spit" off of the heater element) |
| 2.04 | 2.1 - 2.2 | .89 | 2.6 | spit |

**Example 7: Particle size diameters of aerosols generated from heater element comprising a center exit wire lead.**

This example describes the particle size diameters (PSD) of aerosols generated from a heater element comprising a wire wherein one end of the wire wraps around another segment of the wire, wherein a wire coil is formed with an end of the wire passes thorugh the center of the wire coil. An example of this type of heater element is shown in **FIGs 36-38.** In this example, the heater element is inserted into the device depicted in **FIG. 31D. FIG. 31D** depicts a device designated ENT-100-D with a primary passageway for air to flow through, brass contacts (+/-) embedded within the wall of the primary passageway, and a heater element as described in this example. The wire of the heater element has a diameter of 0.10 inches (about 2.54 mm). The wire coil of the heater element has 9 coils, and the wire coil has an inner diameter of 0.032 inches (about 0.813 mm). In this example, the liquid formulation comprises propylene glycol and it wicks onto the ends of the wire of the heater element and onto the brass contacts. **Table 13** shows the particle size diameter of the aerosols generated from a device comprising the heater element. As shown in **Table 13,** the particle size distribution of aerosols generated by devices with the heater element is unaffected by alterations in current used to heat the wire.

**Table 13:** Propylene glycol (dose: 2 mg) found to wick to ends of heater element and onto brass contacts ENT-100-D

| Heater Element .032 10, 010 0wire, 9 turn, center exit | | | | |
|---|---|---|---|---|
| **Flow (LPM)** | **PSD (microns)** | **$\Delta$ P Vac (inches H$_2$O)** | **Current (amps)** | **Notes** |
| 2.01 | 2 - 2.2 | 1.14 | 2.2 | <u>Foam</u> |
| 2.00 | 2 - 2.2 | 1.14 | 2.2 | |
| 2.00 | 2.0 - 2.2 | 1.14 | 2.0 | |
| 2.0 | 2.1 - 2.2 | 1.14 | 2.0 | |
| 2.0 | 1.8 - 2.1 | 1.14 | 1.8 | |
| 2.0 | 1.9 - 2.1 | 1.14 | 1.8 | |
| 0.99 | 5.0 - 5.3 | .34 | 1.8 | |
| 1.00 | 5.0 - 5.2 | .34 | 1.8 | |
| 1.52 | 2.6 - 2.8 | .71 | 2.0 | |
| 1.52 | 2.6 - 2.7 | .71 | 2.0 | |
| 1.53 | 2.4 - 2.7 | .71 | 1.8 | |
| 1.53 | 2.5 - 2.7 | .71 | 1.8 | |
| 2.02 | 2.1 - 2.2 | | 2.0 | |
| 3.0 | 1.2 - 1.4 | 2.43 | 2.0 | |
| 3.0 | 0.8 - 1.4 | 2.43 | 2.0 | |
| **Flow (LPM)** | **PSD (microns)** | **$\Delta$ P Vac (inches H$_2$O)** | **Current (amps)** | **Notes** |
| 3.0 | .90 - 1.3 | 2.43 | 2.2 | |
| 3.0 | .6 - 1.3 | 2.43 | 2.2 | |

**Example 8: Particle size diameters of aerosols generated from heater element comprising a center exit wire lead when the length of the leads are increased.**

This example describes the particle size diameters (PSD) of aerosols generated from a heater element as described in **FIG. 36.** In this example, the length of the leads connecting the wire coil to the brass contacts are increased as shown in **FIG. 37.** The length of the leads in this example is 0.70 inches (about 17.78 mm). The heater element is inserted into the device depicted in **FIG. 31D. FIG. 31D** depicts a device designated ENT-100-D with a primary passageway for air to flow through, brass contacts (+/-) embedded within the wall of the primary passageway, and a heater element as described in this example. In some cases, the diameter of the inlet is varied from 0.060 inches to either 0.070, 0.071, or 0.041 inches (a range from about 1.524 mm to either 1.78, 1.80, or 1.04 mm. The wire of the heater element has a diameter of 0.10 inches (about 0.254 mm). The wire coil of the heater element has a reduced number of coils, and the wire coil has an inner diameter of 0.032 inches (about 0.813 mm) . In this example, the liquid formulation comprises propylene glycol and it wicks onto the ends of the wire of the heater element and onto the brass contacts. **Table 14** shows the particle size diameter of the aerosols generated from a device comprising the heater element. As shown in **Table 14,** the particle size distribution of aerosols generated by device with the heater element is unaffected by alterations in current used to heat the wire. **Table 14** also shows the effects of altering the airway configuration in the ENT-100-D device. As shown in **Table 14,** altering the configuration of the airway of the ENT-100-D device by adding the airway depicted in **FIG. 32E** (designated the MARK V adders in **Table 14**) downstream of the heater element produces particles with a PSD of about 1 to about 2 $\mu$m.

**Table 14:** Heater element leads lengthened

| Flow (LPM) | PSD (microns) | $\Delta$ P Vac (inches H$_2$O) | Current (amps) | Notes |
|---|---|---|---|---|
| 2.0 | 3.1 - 3.2 | .96 | 2.0 | |
| 2.0 | 3.1 - 3.2 | .96 | 2.0 | |
| 2.01 | 3.1 - 3.2 | .96 | 1.8 | |
| 2.01 | 3.1 - 3.2 | .96 | 1.8 | |
| 2.02 | 3.0 - 3.2 | .96 | 2.2 | Orifice .060 |
| 2.02 | 2.9 - 3.0 | .96 | 2.2 | |
| Test of $\Delta$P affecting PSD | | | | |
| 2.06 | 3.3 - 3.4 | 1.74 | 2.0 | Orifice size = .060 |
| 2.04 | 3.2 - 3.3 | .96 | 2.0 | .071 |
| 2.04 | 3.0 - 3.2 | 7.00 | 2.0 | .041 |
| 2.04 | 3.1 - 3.2 | 7.08 | 2.0 | .041 |
| Flow (LPM) | PSD (microns) | $\Delta$ P Vac (inches H$_2$O) | Current (amps) | Notes |
| Test to see affect of foam | | | | |
| 2.06 | 2.4 - 2.5 | 6.65 | 2.0 | Foam removed |
| 2.06 | 2.4 - 2.5 | 6.65 | 2.0 | |
| 2.0 | 2.7 - 2.9 | 1.63 | 2.0 | Original foam |
| 2.05 | 2.7 - 2.8 | 1.63 | 2.0 | Replaced orifice .070 |
| 2.05 | 2.7 - 2.8 | 1.70 | 2.0 | New foam |
| 2.06 | 2.7 | 1.70 | 2.0 | |
| 2.06 | 2.9 - 3.0 | 1.05 | 2.0 | New foam rotated 90° |
| 2.04 | 2.7 - 2.9 | .98 | 2.0 | |
| | | | | |
| Flow (LPM) | PSD (microns) | $\Delta$ P Vac (inches H$_2$O) | Current (amps) | Notes |
| 2.0 | 2.6 | 1.47 | 2 | Foam rotated |
| 2.0 | 2.6 | 1.47 | 2 | again 90° |
| Foam replaced w/SS screen | | | | |
| 2.05 | 2.6 - 2.8 | .63 | 2 | |
| 2.04 | 2.7 - 3.0 | .63 | 2 | |
| 2.04 | 2.8 - 3.0 | .63 | 2 | |
| 2.06 | 2.8 - 3.0 | .65 | 2 | New screen |
| 2.06 | 3.0 - 3.1 | .65 | 2 | |
| New heater element | | | | |
| 2.03 | 3.0 - 3.2 | .62 | 2 | |
| 2.04 | 2.7 - 2.8 | .62 | 2 | |
| 2.04 | 2.7 - 2.8 | .62 | 2 | |
| 2.04 | 2.9 - 3.0 | .62 | 2 | |
| 2.50 | 2.7 - 2.9 | .9 | 2 | |
| 2.50 | 2.4 - 2.6 | .9 | 2 | |
| 2.54 | 2.6 - 2.8 | .9 | 2 | |
| 2.54 | 2.6 - 2.9 | .9 | 2 | |
| 3.52 | 1.9 | 1.60 | 2 | |
| 3.51 | 2.1 | 1.60 | 2 | |
| 4.53 | 1.8 - 1.9 | 2.54 | 2 | |

(continued)

| Flow (LPM) | PSD (microns) | Δ P Vac (inches H₂O) | Current (amps) | Notes |
|---|---|---|---|---|
| New heater element | | | | |
| 4.51 | 1.8 - 1.9 | 2.54 | 2 | |
| Heater element broke | | | | |
| 2.02 | 2.8 - 3.0 | .61 | 2 | Heater replaced |
| 4.52 | 1.9 | 2.53 | 2 | |
| **Flow (LPM)** | **PSD (microns)** | **Δ P Vac (inches H₂O)** | **Current (amps)** | **Notes** |
| 4.53 | 1.9 | 2.53 | 2 | |
| 6.10 | 1.3 - 1.5 | 4.33 | 2 | |
| 6.10 | 1.4 - 1.5 | 4.35 | 2 | |
| 7.03 | 1.1 - 1.2 | 5.68 | 2 | |

| Flow (LPM) | PSD (microns) | Δ P Vac (inches H₂O) | Notes | |
|---|---|---|---|---|
| 1.48 | 2.8 - 3 | .34 | | |
| 1.48 | 3.2 - 2.4 | .34 | | |
| 1.48 | 2.6 - 2.9 | .34 | | |
| 1.48 | 2.4 - 2.7 | .34 | | |
| 2.04 | 3 - 3.2 | .62 | | |
| 2.04 | 3 - 3.2 | .62 | | |
| .95 | 3.9 - 4.2 | 0.14 | | |
| .95 | 3.9 - 4.2 | 0.14 | | |
| | 1.4 - 1.8 | | Bypass | Adder used (Mark V) |
| 2.08 | | 1.06 | 14.9 | |
| 2.08 | 1.9 - 2.1 | 1.06 | 14.9 | |
| 2.08 | 2.0 - 2.1 | 1.06 | 14.9 | |
| 2.08 | 2.0 - 2.1 | 1.06 | 14.9 | |
| 3.02 | 1.7 - 1.8 | 2.06 | 21.0 | |
| 3.02 | 1.8 | 2.06 | 21.09 | |
| 4.48 | 1.3 - 1.4 | 4.22 | 30.4 | |
| 4.48 | 1.2 - 1.4 | 4.22 | 30.1 | |
| 2.0 | 1.9 - 2 | 1.08 | 0 | Flow meter taped up on bypass |
| 2.0 | 2 | 1.08 | 0 | |
| 2.0 | 2.4 - 2.5 | 1.08 | 0 | |
| 2.01 | 2.2 - 2.3 | 1.08 | 0 | |

**Example 9: Particle size diameters of aerosols generated from heater element comprising a center exit wire lead when the length of the leads are decreased.**

This example describes the particle size diameters (PSD) of aerosols generated from a heater element as described in **FIG. 36.** In this example, the length of the leads connecting the wire coil to the brass contacts is 0.30 inches (about 0.762 mm). The heater element is inserted into the device depicted in **FIG. 31D. FIG. 31D** depicts a device designated ENT-100-D with a primary passageway for air to flow through, brass contacts (+/-) embedded within the wall of the primary passageway, and a heater element as described in this example. The wire of the heater element has a diameter of 0.10 inches (about 2.54 mm). The wire coil of the heater element has an increased number of coils relative to example 8, and the wire coil has an inner diameter of 0.032 inches (about 0.813 mm). In this example, the liquid formulation comprises propylene glycol and it wicks onto the ends of the wire of the heater element and onto the brass contacts. The dose of the formulation is 2 mg. **Table 15** shows the particle size diameter of the aerosols generated from the device described in this example. As shown in **Table 15,** the particle size diameter distribution of aerosols generated by this device is unaffected by alterations in current used to heat the wire.

**Table 15:** Testing using ENT-100-D (side mount) (w/bottom leads) with leads shortened.

| Dose Primary Flow (LPM) | 2 mg, current 2.00 amps (U.N.O.) | | |
| --- | --- | --- | --- |
| | PSD (microns) | $\triangle$ P Vac (inches H$_2$O) | Current (amps) |
| 2.02 | 3.0 - 3.2 | .62 | 2.0 |
| 2.02 | 2.9 - 3.2 | .62 | 2.0 |
| 1.48 | 2.3 - 2.5 | .37 | 2.0 |
| 1.48 | 2.0 - 2.4 | .37 | 2.0 |
| 1.48 | 2.0 - 2.6 | .37 | 1.8 |
| 1.48 | 2.0 - 2.5 | .37 | 1.8 |
| 1.10 | 2.8 - 4.1 | .20 | 1.8 |
| 1.10 | 2.3 - 3.4 | .20 | 1.8 |
| 2.0 | 3.1 - 3.2 | .62 | 2.0 |
| 2.12 | 2.2 | 1.16 | 2.0 |
| 2.12 | 2.2 | 1.16 | 2.0 |
| 1.01 | 2.8 | .30 | 1.8 |
| 1.01 | 2.8 - 3.0 | .30 | 1.8 |
| .49 | 4.7 - 5.4 | .08 | 1.8 |
| .49 | 4.5 - 4.8 | .09 | 1.8 |
| 4.50 | 1.4 - 1.6 | 4.14 | 2.0 |

**Example 10: Particle size diameters of aerosols generated from a device comprising a heater element comprising a center exit wire lead.**

This example describes the particle size diameters (PSD) of aerosols generated from a device comprising a heater element as described in **FIG. 36.** In this example, the heater element is inserted into the device depicted in **FIG. 31D. FIG. 31D** depicts a device designated ENT-100-D with a primary passageway for air to flow through, brass contacts (+/-) embedded within the wall of the primary passageway, and a heater element as described in this example. The wire of the heater element has a diameter of 0.10 inches (about 2.54 mm). The wire coil of the heater element has an inner diameter of 0.032 inches (about 0.813 mm). In this example, the liquid formulation comprises propylene glycol and it wicks onto the ends of the wire of the heater element and onto the brass contacts. The dose of the formulation in this example is 2 mg. **Table 16** shows the particle size diameter of the aerosols generated from a device comprising the heater element described in this example. As shown in **Table 16,** the particle size distribution of aerosols generated by devices with the heater element is unaffected by alterations in current used to heat the wire. Also as shown in **Table 16,** altering the configuration of the airway of the ENT-100-D device by adding the airway depicted in **FIG. 33** (designated the MARK VI adder in **Table 15**) downstream of the heater element produces particles with a PSD of about 1 to about 2 uM, which matches the PSD of the particles generated without the MARK VI adder. The MARK VI adder comprises a primary airway with an internal diameter of 0.25 inches (about 6.35 mm), which narrows to an airway comprising an internal diameter of 0.086 inches (about 2.18 mm)and an external diameter of 0.106 inches (about 2.69 mm).

**Table 16:** Testing of ENT-100-D device

| P Flow (LPM) | B Flow (LPM) | PSD (microns) | $\triangle$ PVac (inches H$_2$O) | Notes |
| --- | --- | --- | --- | --- |
| Dose = 2mg; Current 2 amps; 1 sec duration | | | | |
| 1.97 | Ø | 3.0 - 3.1 | .58 | Straight tube |
| 1.52 | Ø | 2.0 - 2.5 | .37 | |
| 1.52 | Ø | 2.4 | .36 | |
| 1.0 | Ø | 3.2 - 3.7 | .17 | |
| 3.0 | Ø | 2.0 - 2.3 | 1.21 | |
| 3.0 | Ø | 2.3 - 2.4 | 1.22 | |
| 4.53 | Ø | 1.6 - 1.8 | 2.52 | |
| 4.53 | Ø | 1.3 - 1.5 | 2.50 | |
| 6.08 | Ø | 1.2 - 1.3 | 4.23 | |

(continued)

| Dose = 2mg; Current 2 amps; 1 sec duration | | | | |
|---|---|---|---|---|
| P Flow (LPM) | B Flow (LPM) | PSD (microns) | $\Delta$ PVac (inches $H_2O$) | Notes |
| 6.08 | Ø | 0.8 - 1.3 | 4.23 | |
| 6.11 | Ø | 0.7 - 1.2 (ST) | 7.13 | |
| 6.11 | Ø | .6 - 1.2 | 7.13 | w/SS needle in .250 tube |
| 4.48 | Ø | 1.5 - 1.6 | 4.14 | |
| 4.48 | Ø | 1.6 - 1.7 | 4.14 | |
| 3.01 | Ø | 1.7 - 1.9 | 2.05 | |
| 3.01 | Ø | 1.7 - 1.8 | 2.05 | |
| 2.01 | Ø | 2.2 | 1.04 | |
| 2.01 | Ø | 2.2 - 2.7 | 1.04 | |
| 1.47 | Ø | 2.0 - 2.1 | .6 | |
| 1.47 | Ø | 2.1 | .6 | |
| 0.98 | Ø | 2.8 - 3.0 | .29 | |
| 0.98 | Ø | 2.7 - 3.0 | .29 | |
| .48 | Ø | 4.7 - 5.2 | .07 | |
| .48 | Ø | 4.4 - 5.1 | .07 | |
| | | | | |
| P Flow (LPM) | B Flow (LPM) | PSD (microns) | $\Delta$ PVac (inches $H_2O$) | Notes |
| 1.5 | Ø | 2.1 | .6 | Delrin "double cone" |
| 1.5 | Ø | 2.1 - 2.2 | .64 | |
| 2.05 | Ø | 2.3 | 1.04 | |
| 2.05 | Ø | 2.2 | 1.08 | |
| 2.5 | Ø | 2.1 - 2.2 | 1.48 | |
| 3.0 | Ø | 1.9 - 2.0 | 2.04 | |
| 3.0 | Ø | 1.9 - 2.0 | 2.04 | |
| 1.0 | Ø | 2.9 - 3.1 | .29 | |
| 1.24 | Ø | 2.6 - 2.7 | .43 | |
| 1.25 | Ø | 2.5 - 2.7 | .43 | |
| 1.75 | Ø | 2.3 - 2.4 | .76 | |
| 1.75 | Ø | 2.3 | .76 | |
| 1.49 | Ø | 2.1 - 2.2 | .6 | Current changed to 2.2 |
| 1.49 | Ø | 2.1 - 2.2 | 2.41 | Back to 2.0 amps orifice changed .250 w/SS needle 6 slots .100 long x .080 |
| | Adder installed | | | |
| 3.0 | 21.16 | 1.8 | 1.98 | |
| 3.0 | 21.16 | 1.8 - 1.9 | 1.98 | 7x Adder |
| 2.0 | 14.13 | 2.0 - 2.1 | 1.0 | Mark VI |
| 2.0 | 14.13 | 2.0 - 2.1 | 1.0 | |
| .98 | 7.06 | 2.7 - 2.8 | .28 | |
| .98 | 7.00 | 2.8 - 2.9 | .29 | |
| 1.5 | 10.49 | 2.1 - 2.2 | .63 | |
| 1.53 | 10.62 | 2.0 - 2.2 | .63 | |
| .49 | 3.45 | 4.3 - 4.5 | .07 | |
| 4.51 | 31.4 | 1.5 - 1.6 | 4.09 | |
| 4.51 | 31.4 | 1.5 - 1.6 | 4.04 | |
| 6.1 | 4.2 | 1.2 | 7.0 | |
| 1.98 | 3.98 | 2.3 - 2.5 | .98 | |
| 1.98 | 3.98 | 2.3 - 2.4 | .98 | |

(continued)

| P Flow (LPM) | B Flow (LPM) | PSD (microns) | Δ PVac (inches H$_2$O) | Notes |
|---|---|---|---|---|
| 2.02 | 0 | 2.3 - 2.4 | 1.03 | |
| 2 | 28 | 2 | 3.52 | |
| 2 | 28 | 2.0 - 2.1 | 3.52 | |

**Example 11: Particle size diameters of aerosols generated from device comprising a bypass inlet for mixing the condensation aerosol in a larger volume of carrier gas.**

In this example, the particle size diameters (PSD) of a condensation aerosol generated by a device comprising the airway configuration depicted in **FIG. 33** are tested. The device comprises a primary airway with an internal diameter of 0.25 inches (about 6.35 mm), which narrows to an airway comprising an internal diameter of 0.086 inches (about 2.18 mm) and an external diameter of 0.106 inches (about 2.69 mm). The airway confirguation is coupled to a heater element comprising a wire coil, wherein the heater element vaporizes a liquid formulation comprising propylene glycol upstream of where the primary airway narrows. The vaporized formulation then enters the narrowed airway and condenses into particles. The narrowed primary airway is designed to carry the vaporized formulation in a carrier gas (e.g. air) at a flow rate suitable for condensing the vapor into particles of a desired size (e.g. an MMAD of about 1 to about 5 um). In this example, the narrowed primary airway opens up into a wide downstream airway comprising an internal diameter of 0.25 inches (about 6.35 mm) and the condensed particles are mixed with bypass carrier gas (e.g. air) that enters the widened primary airway from inlets located (disposed) in the walls of the primary airway. The carrier gas entering through the inlets is fed from a bypass inlet which is in a wall of a secondary housing that encompasses the primary airway. In this example, the effect of varying the flow rates of the bypass gas (B flow) on the PSD of the condensed is examined. **Table 17** shows the results. As shown in **Table 17,** different rates of B flow have no effect on the PSD. Moreover, the PSD at each B flow rate is between 1 and 3 uM. **Table 18** shows the effect on PSD of limiting the flow of bypass carrier gas through the bypass inlet on the secondary housing. The flow of bypass gas through the bypass inlet is limited by using either a valve or by altering the geometry of the orifice (i.e. forming a slot of different dimensions. As shown in **Table 18,** either the use of a valve or slot to control the flow of bypass gas is effective in producing particles with a PSD of about 1 to about 5 μm.

**Table 17:** Characterization of Primary Flow (P flow), Bypass Flow (B Flow), and particle size diameter of device comprising Mark VI Adder

| P Flow (LPM) | B Flow (LPM) | PSD (microns) | Δ P Vac (inches H$_2$O) | Notes |
|---|---|---|---|---|
| 1.01 | 7 | 2.7 - 2.8 | .29 | |
| 1.02 | 14.2 | 2.5 - 2.8 | 1.99 | |
| 1.0 | 14.03 | 2.5 - 2.7 | 2.11 | |

**Table 18:** Characterization of Primary Flow (P flow), Bypass Flow (B Flow), and particle size diameter of device comprising Mark VI Adder with addition of Flap valve to bypass inlet

| P Flow (LPM) | B Flow (LPM) | Δ P Vac (inches H$_2$O) | Orifice (inches) | Valve |
|---|---|---|---|---|
| 0 | 0 | 0 | .060 | Clear |
| 1.48 | .64 | 1 | .060 | Slot .080 |
| 2.20 | 1.58 | 2 | .060 | x 240 |
| 2.81 | 2.70 | 3.14 | .060 | |
| 3.23 | 3.72 | 4 | .060 | |
| 3.66 | 5.10 | 5 | .060 | |
| 4.42 | 7.3 | 7 | .060 | |
| 5.3 | 10.48 | 10 | .060 | |
| 1.48 | 4.86 | 1 | Tee slot | |
| 1.83 | 6.74 | 1.48 | | |
| 2.25 | 9.02 | 2.08 | | |
| 2.50 | 10.6 | 2.53 | | |

(continued)

| P Flow (LPM) | B Flow (LPM) | ∆ P Vac (inches H$_2$O) | Orifice (inches) | Valve |
|---|---|---|---|---|
| 2.79 | 12.6 | 3.07 | | |
| 3.38 | 17.2 | 4.32 | | |
| 4.14 | 23.7 | 6.24 | | |
| 5.32 | 34.6 | 10.0 | | |
| 1.47 | 5.05 | 1.01 | Internal radius | |
| 1.86 | 6.34 | 1.51 | valve | |
| 2.23 | 7.7 | 2.06 | Blue material | |
| 2.52 | 8.7 | 2.56 | | |
| 1.5 | 5.75 | 1 | Internal radius | |
| 2.2 | 9.2 | 2 | Green | |
| 2.75 | 12.94 | 3 | | |
| 3.27 | 17.5 | 4.06 | | |
| 4.2 | 26.2 | 6.4 | | |
| 5.4 | 38.7 | 10.5 | | |

**Example 12: Effects of gravity on particle size diameters of aerosols generated from an ENT-100-D device.**

In this example, the effects of gravity on the particle size diameters (PSD) of a condensation aerosol generated by an ENT-100-D device as depicted in **FIG. 31D** are tested. The ENT-100-D device is loaded with 2 mg of a liquid propylene glycol formulation and the device is rotated during the use of the device. The device is rotated 90 degrees in all dimensions from a stable baseline position. The particle size diameter is measured at each rotation and found not to change. As a result, the device produces particles of a consistent size regardless of the orientation in space of the device.

**Example 13: Study of the Safety, Tolerability, Pharmacokinetics, and Pharmacodynamics of the eNT-100 Nicotine Inhaler among Healthy Volunteer Cigarette Smokers**

In this example, a study will be conducted to examine the safety, tolerability, pharmacokinetics, and pharmacodynamics of condensation aerosol comprising nicotine produced from a liquid nicotine formulation using the ENT-100 nicotine inhaler. One primary objective is to establish the maximally tolerated dose in the range of 25-100 μg of a condensation aerosol comprising nicotine and propylene glycol (PG) from the eNT-100 nicotine inhaler. A second primary objective is to establish the plasma level-time profiles of nicotine administered as 10 inhalations (single dose) using μg doses from the eNT-100 nicotine inhaler. The study will be conducted in two parts:**Part 1** will be a single-blind, placebo and vehicle-controlled, escalating, single dose design to assess the safety, tolerability, nicotine concentrations, and pharmacodynamics of a condensation aerosol comprising nicotine produced from a liquid nicotine formulation using the eNT-100 nicotine inhaler. Subjects will be abstinent from smoking for at least 12 hours prior to the experimental session. Groups of 12 subjects will be assigned to one of up to seven experimental groups, depending on the maximally tolerated dose within the predetermined range of 25 - 100 μg of nicotine per inhalation (less than a typical cigarette inhalation per puff). Subjects will complete predose assessments of their exhaled CO, smoking urge, nicotine concentrations, spirometry, and pulse oximetry, and a brief training including practice inhalations, and then complete 10 inhalations from the eNT-100 inhaler at approximately 30-second intervals over a 4.5-minute period. Postdose assessments will include nicotine concentrations, safety, tolerability, liking, smoking urge, spirometry, and pulse oximetry assessments. Subjects will receive a follow-up phone call approximately 24 hours after dosing to assess any adverse events (AEs) that have occurred since dosing. Escalation to the next dose group will not take place until adequate safety and tolerability from the previous group has been demonstrated.

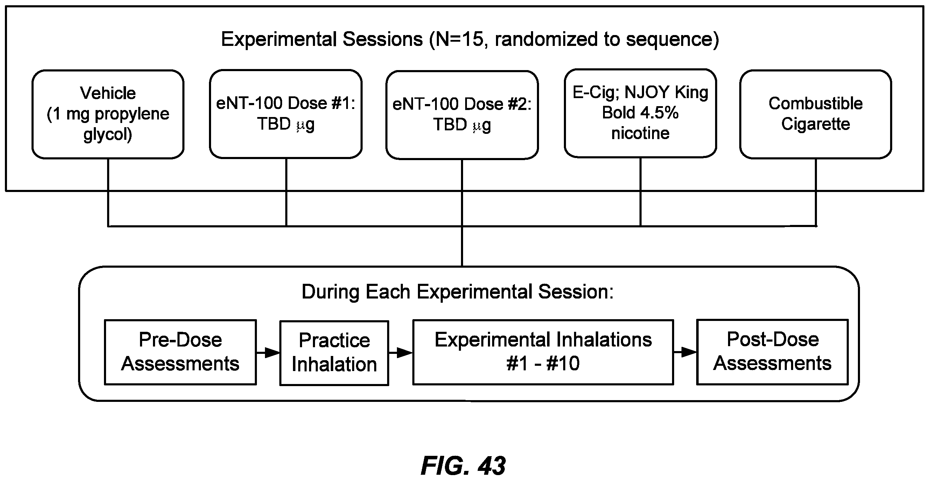

During the escalating-dose sequence portion of the study (see **FIG. 43**), 1.0 mg of total solution (nicotine + PG) will be aerosolized in the vehicle and nicotine dose groups. Then, the final MTD nicotine group (e.g., Group #7), will participate to evaluate the nicotine concentration for the eNT-100 with the nicotine concentration based on the safety and tolerability from the previous groups. Upon reaching the maximally tolerated dose (within the predetermined range of 25 - 100 μg of nicotine per inhalation), a final cohort of subjects will be run at the same nicotine dose, but at twice the nicotine concentration (e.g., if 100 μg of nicotine is well tolerated within 1 mg of PG, [i.e., 10% solution], then an additional group would be run using 100 μg of nicotine within 0.5 mg of PG [i.e., 20% solution]). **Part 2** will be a randomized, single-blind, within-subject, 5-way crossover, vehicle-, e-cig-, and combustible cigarette-controlled design to assess the safety, tolerability, pharmacokinetics and pharmacodynamic effects of the eNT-100 nicotine inhaler. The selected nicotine aerosol concentrations and doses to be administered during Part 2 will be determined upon completion of Part 1 of the study. Subjects will complete predose assessments of exhaled CO, their smoking urge, nicotine PK, spirometry, and pulse oximetry, and a brief training including practice inhalations. Each administration of the study products will include 10 inhalations at approximately 30-second intervals over a 4.5-minute period (or completion of one conventional cigarette). Postdose assessments will include nicotine PK, safety, tolerability, liking, smoking urge, exhaled CO, spirometry, and pulse oximetry assessments. A 36-hour wash-out from nicotine will be required prior to each product administration. See **FIG. 43** for the trial design of this portion of the trial.

### *Study Population and Sample Size*

Parts 1 and 2 will include approximately 48 - 84 (depending on the maximally tolerated aerosol administration) and 15 subjects, respectively.

Subjects will be healthy, adult males and females, 21 - 65 years of age inclusive, who smoke at least 10 cigarettes per day (CPD) for the last 12 months. Ideally, subjects participating in Part 1 will serve as the pool of subjects for Part 2; however, additional subjects will be recruited following completion of Part 1 if necessary to better ensure that 15 subjects are administered study product in Part 2. Potential subjects must fulfill all of the following inclusion criteria to be eligible for participation in the study. Inclusion criteria include: 1. Healthy adult male and female smokers, 21 to 65 years of age, inclusive, at Screening. 2. At least a 12-month smoking history prior to Check-in with a cigarette smoked per day average of 10 or more manufactured cigarettes per day (no restriction on brand). Brief periods (up to 7 consecutive days) of non-smoking (e.g., due to illness, trying to quit, participation in a study where smoking was prohibited) will be permitted at the discretion of the PI. A history of occasional use of e-cigs is allowed, but the subjects should confirm that their primary source of nicotine consumption is smoking conventional cigarettes. 3. Positive urine cotinine at Screening ($\geq$ 500 ng/mL). 4. Exhaled CO > 12 ppm at Screening. 5. Female subjects who are heterosexually active and of childbearing potential (e.g., not surgically sterile [bilateral tubal ligation, hysterectomy, or bilateral oophorectomy at least 6 months prior to Check-in] or at least 2 years naturally postmenopausal) must have been using one of the following forms of contraception and agree to continue using it through completion of the study: hormonal method (e.g., oral, vaginal ring, transdermal patch, implant, or injection) consistently for at least 3 months prior to Check-in; double barrier method (i.e., condom with spermicide or diaphragm with spermicide) consistently for at least 2 weeks prior to Check-in; intrauterine device for at least 3 months prior to Check-in; essure® procedure at least 6 months prior to Check-in; have a partner who has been vasectomized for at least 6 months prior to Check-in. 6. Female subjects of childbearing potential who are not currently engaging in heterosexual intercourse must agree to use one of the above methods of birth control, in the event that they have heterosexual intercourse during the course of the study. 7. Voluntary consent to participate in this study documented on the signed informed consent form (ICF). 8. Willing to comply with the requirements of the study and willing to consider using alternative inhaled forms of nicotine other than conventional cigarettes. 9. Forced Expiratory Flow (FEF) (25 - 75%) at least 70% of the normal values predicted for that individual based on age, gender, and height.

Subjects may be excluded from the study if there is evidence of any of the following criteria at Screening, Check-in, or at anytime during the study as appropriate, in the opinion of the principal investigator (PI): History or presence of clinically significant gastrointestinal, renal, hepatic, neurologic, hematologic, endocrine, oncologic, urologic, pulmonary (especially bronchospastic diseases), immunologic, psychiatric, or cardiovascular disease, or any other condition that, in the opinion of the PI, would jeopardize the safety of the subject or impact the validity of the study results; (**Part 2 only**) clinically significant abnormal findings on the physical examination, ECG, or clinical laboratory results, in the opinion of the PI; (**Part 2 only**) positive test for human immunodeficiency virus (HIV), hepatitis B surface antigen (HbsAg), or hepatitis C virus (HCV); positive urine screen for alcohol or drugs of abuse at Screening or any Check-in; history of drug or alcohol abuse within 24 months of Check-in; an acute illness (e.g., upper respiratory infection, viral infection) requiring treatment within 2 weeks prior to Check-in; fever (> 100.2°F) at Screening or at Check-in; systolic blood pressure > 150 mmHg, diastolic blood pressure > 95 mmHg, or pulse rate > 99 bpm at Screening; body mass index (BMI) < 19 kg/m2 or > 35 kg/m2 at Screening; female subjects who are pregnant, lactating, or intend to become pregnant from Screening through completion of study; consumption of xanthines/caffeine, alcohol, or grapefruit juice within 24 hours of Check-in and during confinement; use of any OTC or prescription smoking cessation treatments, including, but not limited to, nicotine replacement therapies (gum, patches, lozenges, nasal spray, or inhalers), varenicline (Chantix®), or buproprion (Zyban®) within 3 months prior to screening and throughout the study; use of prescription anti-diabetic medication and/or insulin therapy within 12 months of Check-in and throughout the study; concomitant use of inhalers for any reason within 3 months prior to screening and throughout the study; plasma donation within 7 days prior to Check-in, or donation of blood or blood products, had significant blood loss, or received whole blood or a blood product transfusion within 56 days prior to Check-in; participation in a previous clinical study for an investigational drug, device, or biologic within 30 days prior to either Check-in; use of nicotine-containing products other than manufactured cigarettes and occasional e-cig use (e.g., roll-your-own cigarettes, bidis, snuff, nicotine inhaler, pipe, cigar, chewing tobacco, nicotine patch, nicotine spray, nicotine lozenge, or nicotine gum) within four weeks prior to Check-in or during study; or self-reported puffers (i.e., adult smokers who draw smoke from the cigarette into the mouth and throat but do not inhale); FTND score of < 6.

*Study Restrictions:*

Concomitant Medications

Stable doses (i.e., no dosage adjustments within 30 days prior to Check-in) of prescription or over-the-counter medications required to treat a PI-approved disease or condition (e.g., hypertension) are permitted at the discretion of the PI. Hormonal contraceptives (e.g., oral, transdermal patch, implant, injection) and hormonal replacement therapy are permitted. Occasional use of over-the-counter analgesics (e.g., acetaminophen, ibuprofen), antihistamines, and nasal decongestants are permitted. Exceptions may be permitted at the discretion of the PI in consultation with the Sponsor, providing the medication in question would have no impact on the study. Any exceptions will be documented. All concomitant medications (and reasons for their use) taken by subjects during the study will be recorded and coded using the most updated version of the WHO Drug Dictionary available at Celerion (e.g., Sep 2013 or later). During the study, up to 2 g per day of acetaminophen may be administered at the discretion of the PI for intercurrent illness or adverse events. If other drug therapy is required, a joint decision will be made by the PI and Sponsor to continue or discontinue the subject.

Foods and Beverages

Consumption of foods and beverages containing the following substances will be prohibited as indicated: Xanthines/caffeine: 24 hours prior to Check-in and during confinement; alcohol: 24 hours prior to Check-in and during confinement; or grapefruit or grapefruit juice: 24 hours prior to Check-in and during confinement.

Activity

Subjects will not engage in strenuous activity in the 48 hours prior to and at any time during the confinement period.

Subject Numbering

Subjects will be assigned a unique screening number and subject numbers for each part of the study.
In Part 1, once enrolled for study conduct, subjects in the first enrollment cohort will be numbered 101 - 112, subjects in the second enrollment cohort will be numbered 113 - 124, etc.
In Part 2, once enrolled for study conduct, subjects will be assigned a subject number from 201 - 215.
Replacement subjects, if used, will be assigned a number 1000 higher than the subject being replaced (e.g., Subject 1110 would replace Subject 110).
Eligibility for inclusion into Part 1 will be based on a screening visit(s) to assess medical history, concomitant medications, demographics, and smoking history (including the Fagerström Test for Nicotine Dependence [FTND]), an exhaled CO test, urine cotinine and drugs of abuse test, urine pregnancy test, vital signs, and BMI determination.
Subjects participating in Part 2 will complete additional screening events including a physical examination, ECG, clinical laboratory, and serology evaluations. If the pool of subjects from Part 1 is deemed not sufficient to successfully complete Part 2, then additional subjects will be recruited.
However, these subjects must also complete all applicable screening procedures.

**Duration of Study Conduct**

Part 1 will be completed during a Screening visit, a single study visit and a follow-up phone call. Subjects entering into Part 2 will complete an additional 11-day in-clinic confinement. Overall the study is expected to take place over approximately 10 weeks.

**Study Products**

<u>Part 1:</u> Placebo (eNT-100 inhaler delivering air only); Vehicle control (eNT-100 inhaler delivering PG only); and eNT-100 Nicotine Inhaler (potential nicotine concentration range depending on tolerability: 1.25 - 20% nicotine solution in PG vehicle)

**Part 2:** Vehicle control (eNT-100 inhaler delivering PG only); eNT-100 Nicotine Inhaler (potential nicotine concentration range depending on tolerability: 1.25 - 20% nicotine solution in PG vehicle); NJOY King Bold e-cig (4.5% nicotine solution); and subject's usual brand of combustible cigarette

**Product Administration/Experimental Sessions**

**Part 1:** Subjects will participate in one of the experimental groups using the eNT-100 nicotine inhaler involving 10 inhalations resulting in the total nicotine delivery as listed in Table 19 below.

**Table 19: Experimental Groups (Part 1)**

| Group (Total Amount of Nicotine Over 10 Inhalations) | Total Amount of Solution Aerosolized per Inhalation ($\mu$g) | Nicotine per Inhalation ($\mu$g) | Total Nicotine over 10 Inhalations ($\mu$g) | Nicotine Concentration (%) |
|---|---|---|---|---|
| Group #1: Placebo (air only) | 0 | 0 | 0 | 0 |
| Group #2: Vehicle (PG) | 1000 | 0 | 0 | 0 |
| Group #3: 250 $\mu$g | 1000 | 25 | 250 | 2.5 |
| Group #4: 500 $\mu$g | 1000 | 50 | 500 | 5.0 |
| Group #5: 750 $\mu$g | 1000 | 75 | 750 | 7.5 |
| Group #6: 1000 $\mu$g | 1000 | 100 | 1000 | 10.0 |
| Group #{TBD}: MTD | 500 - 2000 | MTD | TBD (range: 250 - 1000) | TBD (range: 1.25 - 20.0) |

If deemed appropriate, in order to identify a dose that is both well-tolerated while minimizing the total amount of chronic exposure to PG that would be expected from eventual chronic use of the eNT inhaler, the total amount of solution aerosolized (starting at 1.0 mg but adjustable to a minimum of 0.5 mg or a maximum of 2.0 mg of total nicotine plus PG solution), as well as the nicotine concentration of the aerosol, can be adjusted following a review of the safety and tolerability from the previous group.

For Groups 2 through 6, **Table 20** below highlights using alternative experimental groups. Initially, subjects will be dosed with 1 mg of total solution being aersolized. If that appears to be poorly tolerated in the vehicle condition, then 0.5 mg of solution will be evaluated. If, on the other hand, the PG appears to be well tolerated but the nicotine concentrations are not well tolerated (e.g., as reflected in AEs at the 25 $\mu$g dose), then 2.0 mg of solution may be evaluated, which would enable evaluation of lower concentrations of nicotine solution. Thus, the total amount of solution to be aerosolized will range from 0.5 to 2 mg, while the nicotine concentration range will be from 1.25 to 20%.

**Table 20: Alternative Experimental Groups (Part 1)**

| Solution | Experimental Groups | Total Amount of eNT Solution (nicotine + PG) Aerosolized per Inhalation (mg) | Nicotine Dose ($\mu$g) (Concentration %) |
|---|---|---|---|
| Placebo (air only) | Group 1 | 0.0 | 0.0 |
| Vehicle (Propylene Glycol only) | Group 2 | 0.5 - 2 | 0.0 |

(continued)

| Solution | Experimental Groups | Total Amount of eNT Solution (nicotine + PG) Aerosolized per Inhalation (mg) | Nicotine Dose (μg) (Concentration %) |
|---|---|---|---|
| | Groups 3-6: Doses to be evaluated if 1.0 mg of PG is not well tolerated | 0.5 | 25 (5.0%) |
| | | 0.5 | 50 (10.0%) |
| | | 0.5 | 75 (15.0%) |
| | | 0.5 | 100 (20.0%) |
| Nicotine & Propylene Glycol (PG) | Groups 3-6: Planned starting doses | 1 | 25 (2.5%) |
| | | 1 | 50 (5.0%) |
| | | 1 | 75 (7.5%) |
| | | 1 | 100 (10.0%) |
| | Groups 3-6: Doses to be evaluated if nicotine | 2 | 25 (1.25%) |
| concentration is not well tolerated | | 2 | 50 (2.5%) |
| | | 2 | 75 (3.75%) |
| | | 2 | 100 (5.0%) |

The final maximally tolerated dose (MTD) nicotine group (e.g., Group #7), will evaluate the nicotine concentration for the eNT-100 with the nicotine concentration based on the safety and tolerability from the previous groups. The maximum nicotine concentration that would be evaluated in any scenario is 20.0%.

Dose-escalation decisions within part 1 will be made based on evaluation of safety data, AEs, and the pharmacodynamic (PD) assessments.

**Part 2:** Subjects will participate in all the experimental sessions according to the randomization schedule outlined in **Table 21.**

**Table 21: Experimental Products (Part 2)**

| Product | Total Amount of Solution Aerosolized per Inhalation (μg) | Nicotine per Inhalation (μg) | Total Nicotine over 10 Inhalations (μg) | Nicotine Concentration (%) |
|---|---|---|---|---|
| Vehicle (propylene glycol only) | 500-2000 | 0 | 0 | 0 |
| eNT-100: TBD μg dose | 500-2000 | TBD | TBD | TBD (range: 1.25 - 20.0) |
| eNT-100: TBD μg dose | 500-2000 | TBD | TBD | TBD (range: 1.25 - 20.0) |
| e-Cig (NJOY King Bold, 4.5% concentration) | Variable | ~113 | ~1130 | 4.5 |
| Combustible cigarette | N/A | 145-199 | ~1450-199 | N/A |

The selected nicotine aerosol concentration and doses to be administered during Part 2 (TBD) will be determined upon completion of Part 1.

Note: % nicotine is % by volume.

**Pharmacokinetic Sample Collection, Parameters, and Analysis:**

During Part 1, serial blood samples will be collected within 15 minutes prior to product administration, and at approximately 5 and 10 minutes after the start of each product administration, and will be used to determine plasma nicotine concentrations. **Table 22** outlines the blood sample collection protocol for Part 1.

**Table 22.** Part 1 Blood Sample Collection Protocol

| Sample Type | Number of Time Points | Approximate Volume per Time Point* (mL) | Approximate Sample Volume Over Course of Study (mL) |
|---|---|---|---|
| PK | 3 | 4 | 12 |
| Total Blood Volume for Study (mL) → | | 12 | |

During Part 2, serial blood samples will be collected within 15 minutes prior to product administration and at approximately 3, 5, 10, 15, 20, 25, 30, and 60 minutes after the start of each product administration and will be used to determine plasma nicotine concentrations. **Table 23** outlines the blood sample collection protocol for Part 2.

**Table 23.** Part 2 Blood Sample Collection Protocol

| Sample Type | Number of Time Points | Approximate Volume per Time Point* (mL) | Approximate Sample Volume Over Course of Study (mL) |
|---|---|---|---|
| Screening laboratory safety tests (including hematology, serum chemistry, serology). | 1 | 12.5 | 12.5 |
| On-study hematology and serum chemistry (including serum pregnancy for women) | 1 | 12.5 | 12.5 |
| PK | 45 | 4 | 180 |
| Total Blood Volume for Study (mL) → | | | 205 |

For Part 2, noncompartmental PK parameters of C5, $C_{max}$, $t_{max}$, and AUCo-t will be calculated from plasma concentrations of nicotine. Additional PK parameters may be calculated if deemed appropriate.

Nicotine concentrations and PK parameters will be summarized by study product using descriptive statistics.

Analyses of variance (ANOVA) or other appropriate statistical tests will be performed on the PK parameters. The ANOVA model will include sequence, study product, and period as fixed effects, and subject nested within sequence as a random effect. Sequence will be tested using subject nested within sequence as the error term. Each ANOVA will include calculation of least-squares means (LSM), differences between product LSM, and the standard error associated with these differences. The above statistical analyses will be performed using the appropriate SAS procedures.

**Pharmacodynamic Assessment and Analysis**

Smoking urge, aversion/tolerability, respiratory tract sensations, and subjective effects will be evaluated via patient-reported outcome (PRO) measures following product administration in both Part 1 and Part 2.

All pharmacodynamic data obtained during both parts of the study will be listed by subject and time point. The data will be summarized by time point using descriptive statistics and an appropriate statistical method (ANOVA or an appropriate non-parametric test as required by the type of data) will be used to characterize the between-group comparisons.

**Safety Assessments and Analysis**

Prior to inclusion into Part 1 of the study, medical history, vital signs, urine drug and alcohol screen, and pregnancy test (females only) will be performed. Part 1 Check-in evaluations will include vital signs, urine drug and alcohol screen, and a pregnancy test (females only).

Additional safety evaluations performed prior to inclusion in Part 2 will include a physical examination, electrocardiogram (ECG), clinical laboratory (clinical chemistry, hematology, urinalysis), and serology. Part 2 Check-in evaluations will include a brief physical examination (symptom-driven), vital signs, clinical laboratory (clinical chemistry, hematology, and urinalysis), urine drug and alcohol screen, and a pregnancy test (females only). End-of-Study (or Early Termination) evaluations will include a brief physical examination (symptom-driven) and vital signs.

In addition, vital signs will be evaluated before and after study product administration.

Adverse events (AEs) spontaneously reported by the subjects or observed by the PI or other study personnel will be monitored and followed up until the symptoms or values return to normal or acceptable levels or until lost to follow-up, as appropriate in the opinion of the PI or his designee.

**Other Non-Safety Assessments and Analysis**

Spirometry, pulse oximetry, and expired CO values for both Part 1 and Part 2 will be listed by subject and time point. Postdose to predose difference in each assessment will be summarized by time point using descriptive statistics and analyzed using an appropriate statistical method.

The dependent measures related to the spirometry device attached to the eNT-100 inhaler used to characterize subjects' inhalations will be listed by subject.

**Description of the eNT-100 Nicotine Inhaler**

The aerosol is created inside the eNT-100 inhaler, which is itself inside a small cylindrical plastic housing that is used to blind the test subject from the test article. The test subject will inhale from a plastic tube that slides over the stainless-steel mouthpiece shown. Inside of the aerosol-generating inhaler is a small heater element that is used to vaporize the nicotine solution under flow conditions that result is a 1.4 to 2.5 micron aerosol particle. The nicotine inhaler further comprises a positive displacement pump to meter out a dose of the nicotine solution onto the heater element.

The eNT-100 is designed to create the aerosol when the inhalation rate reaches 20 lpm (about $3 \times 10^{-4}$ m$^3$/s). At that flow rate the aerosol produced has a particle size of 2.5 micron volume median diameter (VMD) with a GSD of 1.6. The upper end of the inhalation flow rate is determined by the flow rate that can be produced under what is considered an upper limit of vacuum that the human lung can produce by inhalation (13 inches of water is considered that upper limit (about 3235 Pa)). At that vacuum, the inhalation flow rate is 50 lpm (about $8.33 \times 10^{-4}$ m$^3$/s) and the particle size is 1.4 micron VMD with a GSD of 1.2.

The bulk of the aerosol is created within 1 second of the inhaler being breath-activated. Within 1.4 seconds the entire aerosol is created. An estimate of the aerosol produced between the 1 second and the 1.4 second time point is around 5 - 10% of the total amount of the aerosol. As a result, the bulk of the aerosol is delivered to the respiratory tract in the first 1/3 to 1/2 of the volume of the total inhalation volume, thereby allowing the aerosol to be "chased" down into the deep lung by the balance of the inhalation.

The eNT-100 system can generate an emitted dose of +/-20% of the dose (or loaded dose). The dose (or loaded dose) can be the amount of nicotine solution pumped onto the heater element prior to the creation of the aerosol and can be +/-2% of the target dose (the label claimed dose or goal dose). The emitted dose can be 92% to 97% of the dose. For example, the amount actually delivered to the lung if the label claim dose is 100 μg would be between 90% and 99%.

While preferred embodiments have been shown and described herein, it will be obvious to those skilled in the art that such embodiments are provided by way of example only. Numerous variations, changes, and substitutions will now occur to those skilled in the art. It should be understood that various alternatives to the embodiments described herein may be employed. It is intended that the following claims define the scope of the invention and that structures within the scope of these claims be covered thereby.

**Claims**

**1.** A device for generating a condensation aerosol from a liquid formulation comprising:

- a reservoir (2304) comprising the liquid formulation;
- a passageway comprising a heater element (2200) for vaporizing the liquid formulation, an inlet, and an outlet, wherein the heater element (2200) is disposed in the passageway between the inlet and the outlet, wherein the reservoir (2304) is in fluid communication with the heater element (2200), and wherein the passageway is configured to condensate the vaporzied liquid formulation to produce a condensation aerosol in the device, wherein the condensation aerosol has a mass median aerodynamic diameter (MMAD) of from about 1 μm to about 5 μm,
- a tube (2302) disposed within the passageway and connected to the reservoir (2304), wherein the tube (2302) is configured to deliver the liquid formulation to the heater element (2200);

**characterized in that** the device further comprises:

- a positive displacement pump in fluid communication with the reservoir (2304), wherein the displacement pump comprises a peristaltic pump (2306) configured to pump the liquid formulation to the heater element (2200); and
- a baffle (4402) disposed in the passageway between the outlet and the heater element, wherein the baffle (4402) is configured to inhibit flow of particles having an MMAD of greater than 5 $\mu$m.

2. The device of claim 1, wherein the heater element comprises a wire coil.

3. The device of any of the above claims, wherein the passageway has an internal diameter of from 0.20 cm to about 1.3 cm.

4. The device of any one of the previous claims, wherein the tube is a capillary.

5. The device of any one of the previous claims, wherein the tube comprises a second heater element.

6. The device of any of the above claims, wherein at a vacuum of about 249 Pa to about 3738 Pa, a flow rate of gas through the passageway ranges from about 20 liters per minute to about 80 liters per minute.

7. The device of any of the above claims, wherein a flow resistance of the device between the inlet and the outlet ranges from about 0.05 to about 0.15 sqrt (cm-H$_2$O)/LPM.

8. The device of any of the above claims, further comprising a sensor (1710a) to detect inhalation.

**Patentansprüche**

1. Vorrichtung zur Erzeugung eines Kondensationsaerosols aus einer flüssigen Formulierung, umfassend:

   - einen Vorratsbehälter (2304), der die flüssige Formulierung umfasst;
   - einen Durchlassweg, der ein Heizelement (2200) zum Verdampfen der flüssigen Formulierung, einen Einlass und einen Auslass umfasst, wobei das Heizelement (2200) zwischen dem Einlass und dem Auslass in dem Durchlassweg angeordnet ist, wobei der Vorratsbehälter (2304) in Fließverbindung mit dem Heizelement (2200) steht und wobei der Durchlassweg dazu ausgestaltet ist, die verdampfte flüssige Formulierung kondensieren zu lassen, um in der Vorrichtung ein Kondensationsaerosol zu erzeugen, wobei das Kondensationsaerosol einen medianen massenbezogenen aerodynamischen Durchmesser (MMAD) von etwa 1 $\mu$m bis etwa 5 $\mu$m aufweist;
   - ein Röhrchen (2302), das in dem Durchlassweg angeordnet und mit dem Vorratsbehälter (2304) verbunden ist, wobei das Röhrchen (2302) dazu ausgestaltet ist, die flüssige Formulierung dem Heizelement (2200) zuzuführen;

   **dadurch gekennzeichnet, dass** die Vorrichtung ferner umfasst:

   - eine Verdrängerpumpe in Fließverbindung mit dem Vorratsbehälter (2304), wobei die Verdrängerpumpe eine Schlauchpumpe (2306) umfasst, die dazu ausgestaltet ist, die flüssige Formulierung zu dem Heizelement (2200) zu pumpen; und
   - eine Prallplatte (4402), die zwischen dem Auslass und dem Heizelement in dem Durchlassweg angeordnet ist, wobei die Prallplatte (4402) dazu ausgestaltet ist, einen Durchfluss von Partikeln mit einem MMAD von über 5 $\mu$m zu verhindern.

2. Vorrichtung nach Anspruch 1, wobei das Heizelement eine Drahtspule umfasst.

3. Vorrichtung nach einem der obigen Ansprüche, wobei der Durchlassweg einen Innendurchmesser von 0,20 cm bis etwa 1,3 cm aufweist.

4. Vorrichtung nach einem der vorangehenden Ansprüche, wobei das Röhrchen eine Kapillare ist.

5. Vorrichtung nach einem der vorangehenden Ansprüche, wobei das Röhrchen ein zweites Heizelement umfasst.

6. Vorrichtung nach einem der obigen Ansprüche, wobei bei einem Unterdruck von etwa 249 Pa bis etwa 3738 Pa ein Volumenstrom von Gas durch den Durchlassweg etwa 20 Liter pro Minute bis etwa 80 Liter pro Minute beträgt.

7. Vorrichtung nach einem der obigen Ansprüche, wobei ein Strömungswiderstand der Vorrichtung zwischen dem Einlass und dem Auslass etwa 0,05 bis etwa 0,15 Quadratwurzel (cm-$H_2O$)/l/min beträgt.

**8.** Vorrichtung nach einem der obigen Ansprüche, die ferner einen Sensor (1710a) zum Erkennen von Inhalation umfasst.

**Revendications**

**1.** Dispositif de génération d'un aérosol à condensation à partir d'une formulation liquide, comprenant :

- un réservoir (2304) comprenant la formulation liquide ;
- un passage comprenant un élément chauffant (2200) pour vaporiser la formulation liquide, une admission et une sortie, l'élément chauffant (2200) étant disposé dans le passage entre l'admission et la sortie, le réservoir (2304) étant en communication avec l'élément chauffant (2200), et le passage étant configuré pour condenser la formulation liquide vaporisée afin de produire un aérosol à condensation dans le dispositif, l'aérosol à condensation ayant un diamètre aérodynamique moyen en masse (MMAD) d'environ 1 $\mu$m à environ 5 $\mu$m ;
- un tube (2302) disposé dans le passage et raccordé au réservoir (2304), le tube (2302) étant configuré pour fournir la formulation liquide à l'élément chauffant (2200) ;

**caractérisé en ce que** le dispositif comprend en outre :

- une pompe volumétrique en communication fluidique avec le réservoir (2304), la pompe volumétrique comprenant une pompe péristaltique (2306) configurée pour pomper la formulation liquide vers l'élément chauffant (2200) ; et
- un déflecteur (4402) disposé dans le passage entre la sortie et l'élément chauffant, le déflecteur (4402) étant configuré pour empêcher l'écoulement de particules ayant un MMAD supérieur à 5 $\mu$m.

**2.** Dispositif selon la revendication 1, l'élément chauffant comprenant une couronne de fil métallique.

**3.** Dispositif selon l'une quelconque des revendications précédentes, le passage ayant un diamètre interne de 0,20 cm à environ 1,3 cm.

**4.** Dispositif selon l'une quelconque des revendications précédentes, le tube étant un capillaire.

**5.** Dispositif selon l'une quelconque des revendications précédentes, le tube comprenant un deuxième élément chauffant.

**6.** Dispositif selon l'une quelconque des revendications précédentes, un débit de gaz par le passage étant d'environ 20 litres par minute à environ 80 litres par minute, sous un vide d'environ 249 Pa à environ 3 738 Pa.

**7.** Dispositif selon l'une quelconque des revendications précédentes, une résistance à l'écoulement du dispositif entre l'admission et la sortie étant d'environ 0,05 à environ 0,15 sqrt (cm-$H_2$0)/LPM.

**8.** Dispositif selon l'une quelconque des revendications précédentes, comprenant en outre un capteur (1710a) pour détecter l'inhalation.

**FIG. 1**

**FIG. 2A**

**FIG. 2B**

Area liquid is vaporized

Electrical connection

Agent
(e.g., nicotine
ejected)

Thin wall tubing
(0.0005-0.003 inches)

+ | | | | | | -

Battery

**FIG. 3A**

Narrower and higher
resistance section

Area liquid is vaporized

Thick walled
tubing

Agent
(e.g., nicotine
ejected)

+ | | | | | -

Battery

**FIG. 3B**

**FIG. 4**

**FIG. 5**

**FIG. 6**

FIG. 7

FIG. 8

FIG. 9

1004a

1002a

−    +

1006a

**FIG. 10A**

−    +    1004b

1002b

1006a

**FIG. 10B**

1102    1106    1104

1112

$D_J$

1110

L

Air

Flow rate    1108

**FIG. 11**

FIG. 12

FIG. 13

FIG. 14

FIG. 15

FIG. 16A

FIG. 16B

Heater surface rotated to apply mixture

Air Flow

FIG. 17A

Air Flow

FIG. 17B

Air routing upon valve opening

Piston

1806

Air routing after initial part of inhalation

1808

1804

1802

Air Flow

**FIG. 18**

Mean Nicotine Intake

Reduced Challenge Dose

Mean Craving Over Time

Time

**FIG. 19**

Actual Nicotine Intake

Feedback re: Nicotine Intake

Enhanced Self-Efficacy

Time

**FIG. 20**

2108
2104
2110
2114
2112
2102
2106
2116
2118
2100

**FIG. 21**

2204
(+)
(-)
2204
2202
2206
To Reservoir
2208
2200

**FIG. 22**

2302

2306

2304

**FIG. 23**

2402

2404

2406

**FIG. 24**

2504a

2502a

2506a

**FIG. 25A**

2504b

2502b

2506b

**FIG. 25B**

FIG. 26

2702a

2704a

2706a

2708a

(+)

(-)

2710a

**FIG. 27A**

2704b

2708b

2710b

2712b

**FIG. 27B**

2708c

(+)

(-)

2712c

**FIG. 27C**

(-)

**FIG. 27D**

**FIG. 28**

**FIG. 29A**

**FIG. 29B**

**FIG. 30A**

**FIG. 30B**

FIG. 31A

FIG. 31B

3102c

3106c

(-)          (+)

3112c

**FIG. 31C**

3102d

3106d

(+)

(-)

3112d

**FIG. 31D**

3114e

3116e

**FIG. 31E**

**FIG. 32A**

3204a

3206a

3202a

Mixing ratio (7x)

3202b

3204b

3202b

**FIG. 32B**

Mixing ratio (28x)

3202c

3204c

3202c

**FIG. 32C**

**FIG. 32D**

**FIG. 32E**

3302

3306

3302

3304

3308

**FIG. 33**

3702

3704

3706

**FIG. 34**

**FIG. 35**

**FIG. 36**

**FIG. 37A**

**FIG. 37B**

**FIG. 38**

*3900*

User Devices 3902

Network *3904*

*3906*

Electronic Agent Delivery Device

Data Store *3908*

**FIG. 39**

*4000*

*4004*

User Devices

*4002* Data Collector

*4006*

Electronic Agent Delivery Device

*4010* Data Store

*4008* Evaluation Engine

**FIG. 40**

**FIG. 41**

Post-Dose Assessments to Determine MTD

Safety, tolerability, liking, craving reduction

Ascending Dose Sequence

**Group #1:** Placebo — Practice → Inhalations #1 - #10

**Group #2:** Vehicle (1 mg propylene glycol) — Practice → Inhalations #1 - #10

**Group #3:** 250 μg — Practice → 25 μg Inhalations #1 - #10

Safety & Tolerability Review

**Group #4:** 500 μg — Practice → 50 μg Inhalations #1 - #10

Safety & Tolerability Review

**Group #5:** 750 μg — Practice → 75 μg Inhalations #1 - #10

Safety & Tolerability Review

**Group #6:** 1000 μg — Practice → 100 μg Inhalations #1 - #10

Randomization

N=12 per group

*FIG. 42*

Experimental Sessions (N=15, randomized to sequence)

| Vehicle (1 mg propylene glycol) | eNT-100 Dose #1: TBD µg | eNT-100 Dose #2: TBD µg | E-Cig; NJOY King Bold 4.5% nicotine | Combustible Cigarette |

During Each Experimental Session:

Pre-Dose Assessments → Practice Inhalation → Experimental Inhalations #1 - #10 → Post-Dose Assessments

*FIG. 43*

EP 2 925 395 B1

FIG. 44A

FIG. 44B

EP 2 925 395 B1

Flow of Aerosol
4410

Baffle
4402

Outlet
4406

Aerosol Inlet
4404

4408
Carrier Gas

Small Particles
($\leq 5~\mu M$)

Large Particles
($> 5~\mu M$)

FIG. 44C

**EP 2 925 395 B1**

**REFERENCES CITED IN THE DESCRIPTION**

**Patent documents cited in the description**

- WO 9501137 A
- EP 2319334 A1
- EP 1618803 A1
- US 20050235991 A1
- US 20130157995 A
- US 20090234129 A
- US 20080108822 A
- US 20070186940 A
- US 20080227088 A
- US 4243605 A
- US 5015741 A
- US 6503922 B
- US 6995265 B
- US 7132545 B